# EUROPEAN PATENT APPLICATION

(11) **EP 4 818 189 A1**
(43) Date of publication of application: **07.10.2026**
(21) Application number: 24896604.6
(22) Date of filing: 27.11.2024
(51) Int. Cl.: C07K 5/078, A61K 38/05, A61P 25/24

(54) **NITROGEN-CONTAINING HETEROCYCLIC COMPOUND, AND PREPARATION METHOD AND USE THEREFOR**

(30) Priority: 28.11.2023 CN 202311595578; 23.07.2024 CN 202410992479
(71) Applicant: Chengdu Di'ao Pharmaceutical Group Co., Ltd., Chengdu, Sichuan 610041 (CN)
(72) Inventor: CHENG, Xu, Chengdu, Sichuan 610041 (CN); WANG, Yuanqiang, Chengdu, Sichuan 610041 (CN); LIU, Lixin, Chengdu, Sichuan 610041 (CN); JIANG, Fan, Chengdu, Sichuan 610041 (CN); CHEN, Shuang, Chengdu, Sichuan 610041 (CN); YU, Zhou, Chengdu, Sichuan 610041 (CN)
(74) Representative: Kador & Partner Part mbB
(86) International application number: PCT/CN2024/135046
(87) International publication number: WO 2025/113518

(57) **Abstract**

A nitrogen-containing heterocyclic compound or a pharmaceutically acceptable salt, prodrug, deuterated substance, hydrate, solvate, enantiomer, diastereomer or racemate thereof, and a preparation method and use therefor. The compound can be used for preparing quick-acting antidepressant drugs.

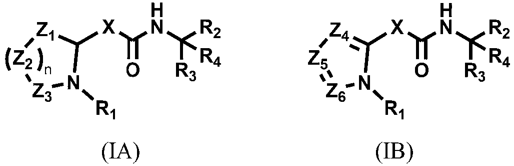

## Description

### TECHNICAL FIELD

The present invention relates to the technical field of medicinal chemistry, and particularly to a compound exerting a rapid antidepressant effect and use therefor.

### BACKGROUND

Depression characterized by a main clinical feature of significant and persistent low mood is a common mental disorder, which is clinically manifested as a depressed mood disproportionate to the circumstances, and the depressed mood ranges from feeling down to extreme sorrow, self-deprecation, and even pessimism, possibly accompanied with suicidal thoughts or behaviors. In severe cases, psychotic symptoms such as hallucinations and delusions may occur. Globally, over 350 million people suffer from depression, with an increase of 18% in the past decade. As of 2017, China alone had more than 54 million patients with depression. Major depressive disorder has a recurrence rate as high as 50%-85% and a suicide rate of 4.0%-10.6%, and has become the most common type of psychiatric disease.

Dysregulation of neurotransmitters in the brain, such as norepinephrine, dopamine and serotonin (5-HT), is considered a major biological factor leading to depression. Main depression treatment methods rely on the use of antidepressants, and based on the efficacy and mechanism of action, the antidepressants are mainly classified into the following four categories: 1) monoamine oxidase inhibitors: such as iproniazid and isocarboxazid, since these drugs contain ingredients with relatively severe side effects, they have been largely discontinued; 2) tricyclic antidepressants: these drugs are effective in alleviating depression but have significant adverse effects on patients with other diseases, and these drugs include clomipramine, imipramine hydrochloride, and the like; 3) selective 5-HT reuptake inhibitors: these drugs can compensate for 5-hydroxytryptamine (5-HT) deficiency in patients with depression, and mainly include fluoxetine, paroxetine, sertraline, citalopram and fluvoxamine; and 4) 5-HT and norepinephrine reuptake inhibitors: these drugs have a dual antidepressant mechanism and relatively high safety, such as venlafaxine and duloxetine. The drugs in the categories 3 and 4 are mainly used, while the drugs in the categories 1 and 2 are largely discontinued.

Since the advent of 5-HT reuptake inhibitor (SSRi), there have been dozens of iterative upgrades. Currently, there are hundreds of research projects that can be searched, mainly focusing on SSRis, selective 5-HT and norepinephrine reuptake inhibitors (SNRIs), norepinephrine and specific 5-HT antidepressants (NaSSAs), 5-HT receptor antagonism and reuptake inhibitors (SARIs), NMDA receptor antagonists, and the like. The antidepressant market is also highly concentrated. Up to now, more than 30 SSRis varieties have been applied globally, with TOP10 being escitalopram, sertraline, venlafaxine, paroxetine, duloxetine, flupentixol + melitracen, mirtazapine, fluoxetine, citalopram and fluvoxamine, collectively accounting for over 90% of the market share.

However, common antidepressants used currently and clinically also have significant limitations, mainly including slow onset and delayed effect. The antidepressants require 3-4 weeks of continuous medication to take effect, and many patients discontinue treatment before achieving efficacy; and patients with suicidal tendencies may not be treated in time to save their lives. A nasal spray formulation of (S)-enantiomer of ketamine (Esketamine) was marketed in the United States in 2019. A single use of the Esketamine can rapidly (within hours) relieve depressive symptoms, and the effect may last for 3-4 days. Although the Esketamine acts quickly, there are severe side effects, including severe somnolence, dissociative hallucinations, potential addiction, and the like. The FDA has also issued a black box warning, stipulating that the Esketamine must be used concomitantly with other oral antidepressants for treatment-resistant depression in adults. These shortcomings limit the clinical application of such drugs. Therefore, there is an urgent clinical need for novel quick-acting, effective and orally administrable antidepressants.

### SUMMARY

An objective of the present invention includes providing a nitrogen-containing heterocyclic compound, or a pharmaceutically acceptable salt, prodrug, deuterated compound, hydrate, solvate, enantiomer, diastereoisomer or racemate thereof;
another objective of the present invention includes providing a pharmaceutical composition including a therapeutically effective amount of any compound above, or the pharmaceutically acceptable salt, prodrug, deuterated compound, hydrate, solvate, enantiomer, diastereoisomer or racemate thereof, and a pharmaceutically acceptable excipient; and
another objective of the present invention includes providing a use of the compound represented by the general formula above, or the pharmaceutically acceptable salt, prodrug, deuterated compound, hydrate, solvate, enantiomer, diastereoisomer or racemate thereof, or the pharmaceutical composition in preparation of a drug for preventing and/or treating depression.

Another objective of the present invention includes providing a preparation method for the compound represented by the general formula above.

In order to achieve the above objectives of the present invention, the technical solution used in the present invention is as follows.

The present invention provides a compound represented by general formula (IA) or (IB), or a pharmaceutically acceptable salt, prodrug, deuterated compound, hydrate, solvate, enantiomer, diastereoisomer or racemate thereof: 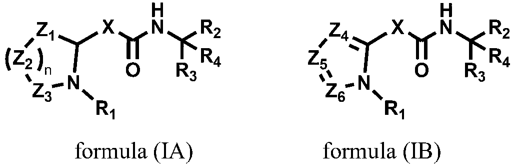 wherein:
X is a bond or CHR₅;
Z₁ and Z₂ are independently selected from CHR₆, NR₇, oxygen or carbonyl, and Z₁ and Z₂ are not both NR₇ or oxygen;
Z₃ is CHR₆ or carbonyl;
Z₄, Z₅ and Z₆ are independently selected from CR₆ or N;
n is an integer from 0 to 4; R₁ is hydrogen, R₆-(CO)-, R₆-(SO)-, R₆-(SO₂)-, R₆-O(CO)-, R₆R₇-N(CO)-, or C₁-C₁₅ linear alkyl, C₁-C₁₅ hetero linear alkyl, C₃-C₁₅ cycloalkyl, C₄-C₁₅ heterocycloalkyl, C₅-C₁₅ aryl or C₅-C₁₅ heteroaryl substituted with 0 to 8 R₆ groups; and R₂ is optionally selected from carboxyl, C₁-C₁₅ linear alkoxycarbonyl, C₁-C₁₅ hetero linear alkoxycarbonyl, C₃-C₁₅ cycloalkoxycarbonyl, C₄-C₁₅ heterocycloalkoxycarbonyl, C₅-C₁₅ aryl or C₅-C₁₅ heteroaryl substituted with 0 to 8 R₆ groups;
R₃ and R₄ are respectively independently selected from hydrogen, or C₁-C₁₅ linear alkyl, C₁-C₁₅ hetero linear alkyl, C₃-C₁₅ cycloalkyl, C₄-C₁₅ heterocycloalkyl, C₅-C₁₅ aryl or C₅-C₁₅ heteroaryl substituted with 0 to 8 R₆ groups;
R₅ is optionally selected from hydrogen, C₁-C₅ alkyl, C₃-C₇ cycloalkyl or C₄-C₇ heterocycloalkyl;
R₆ is optionally selected from hydrogen, halogen, -CN, -NO₂, -OR₇, -NR₇R₈, -SR₇, -COR₇, -SOR₇, -SO₂R₇, -NR₇COR₈, -CONR₇R₈, -OCOR₇, -COOR₇, -OCOOR₇, -OCONR₇R₈, -NR₇CONR₈R₉, -NR₇COOR₈, -NR₇SO₂R₈, -SO₂NR₇R₈, -OSO₂R₇, -SO₃R₇, linear alkyl, hetero linear alkyl, cycloalkyl, heterocycloalkyl, aryl or heteroaryl; wherein each linear alkyl, hetero linear alkyl, cycloalkyl, heterocycloalkyl, aryl or heteroaryl is independently substituted with 0 to 8 R₁₀ groups;
R₇, R₈ and R₉ are respectively independently selected from hydrogen, linear alkyl, hetero linear alkyl, cycloalkyl, heterocycloalkyl, aryl or heteroaryl, wherein each linear alkyl, hetero linear alkyl, cycloalkyl, heterocycloalkyl, aryl or heteroaryl is independently substituted with 0 to 8 halogen, cyano, hydroxyl, sulfhydryl, ether, nitro, alkoxy, amino, carboxyl, sulfo, ester, amide, sulfonate, sulfonamide, alkyl or haloalkyl groups; and
R₁₀ is selected from hydrogen, halogen, cyano, hydroxyl, mercapto, ether, nitro, alkoxy, amino, carboxyl, sulfo, ester, amide, sulfonate, sulfonamide, linear alkyl, hetero linear alkyl, cycloalkyl, heterocycloalkyl, aryl or heteroaryl, wherein each linear alkyl, hetero linear alkyl, cycloalkyl, heterocycloalkyl, aryl or heteroaryl is independently substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups;
wherein, general formula (IA) does not comprise the compound 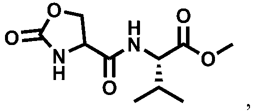 and general formula (IB) does not comprise the compound

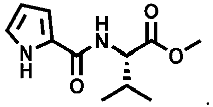

The present invention further provides a compound represented by general formula (Ia) or (Ib), or a pharmaceutically acceptable salt, prodrug, deuterated compound, hydrate, solvate, enantiomer, diastereoisomer or racemate thereof: 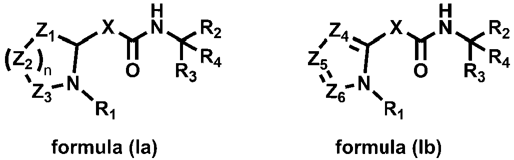 wherein:
X is a bond or CHR₅;
Z₁ and Z₂ are independently selected from CHR₆, NR₇, oxygen or carbonyl, and Z₁ and Z₂ are not both NR₇ or oxygen;
Z₃ is CHR₆ or carbonyl;
Z₄, Z₅ and Z₆ are independently selected from CR₆ or N;
n is an integer from 0 to 4;
R₁ is selected from hydrogen, R₆-(CO)-, R₆-(SO)-, R₆-(SO₂)-, R₆-O(CO)-, R₆R₇N(CO)-, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₆ groups; or R₁ is selected from C₁-C₁₅ linear alkyl containing heteroatoms, wherein the heteroatoms are selected from one or more of O, S and N, and the C₁-C₁₅ linear alkyl containing heteroatoms is optionally substituted with 0 to 8 R₆ groups;
R₂ is selected from -(CO)O-(CH₂)ₘR₆, wherein m is an integer from 0 to 4, or R₂ is selected from C₆-C₁₅ aryl or 5- to 15-membered heteroaryl, wherein C₆-C₁₅ aryl or 5- to 15-membered heteroaryl is optionally substituted with 0 to 8 R₆ groups;
R₃ and R₄ are respectively independently selected from hydrogen, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₆ groups, and R₃ and R₄ are not both hydrogen; or R₃ and R₄ are respectively independently selected from C₁-C₁₅ linear alkyl containing heteroatoms, wherein the heteroatoms are selected from one or more of O, S and N, and the C₁-C₁₅ linear alkyl containing heteroatoms is optionally substituted with 0 to 8 R₆ groups;
R₅ is optionally selected from hydrogen, C₁-C₅ alkyl, C₃-C₇ cycloalkyl or 4- to 7-membered heterocycloalkyl;
R₆ is selected from hydrogen, halogen, -CN, -NO₂, -OR₇, -NR₇R₈, -SR₇, -(CO)-R₇, -(SO)-R₇, -(SO₂)-R₇, -NR₇-(CO)-R₈, -(CO)-NR₇R₈, -O(CO)-R₇, -(CO)OR₇, -O(CO)OR₇, -O(CO)-NR₇R₈, -NR₇-(CO)-NR₈R₉, -NR₇-(CO)OR₈, -NR₇-(SO₂)-R₈, -(SO₂)-NR₇R₈, -O(SO₂)-R₇, -(SO₂)OR₇, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₁₀ groups; or R₆ is selected from C₁-C₁₅ linear alkyl containing heteroatoms, wherein the heteroatoms are selected from one or more of O, S and N, and the C₁-C₁₅ linear alkyl containing heteroatoms is optionally substituted with 0 to 8 R₁₀ groups;
R₇, R₈ and R₉ are respectively independently selected from hydrogen, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups; or R₇, R₈ and R₉ are respectively independently selected from C₁-C₁₅ linear alkyl containing heteroatoms, wherein the heteroatoms are selected from one or more of O, S and N, and the C₁-C₁₅ linear alkyl containing heteroatoms is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups; and
R₁₀ is selected from hydrogen, halogen, cyano, hydroxyl, mercapto, ether, nitro, amino, carboxyl, sulfo, ester, amide, sulfonate, sulfonamide, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups;
wherein, formula (Ia) does not comprise the following compounds: 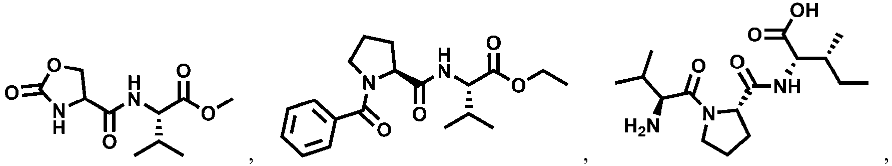 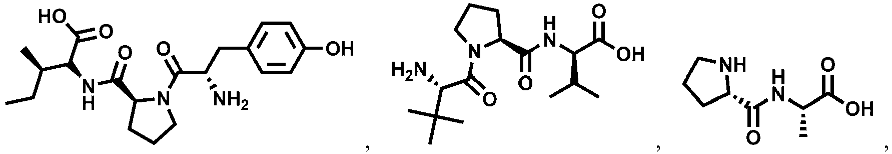 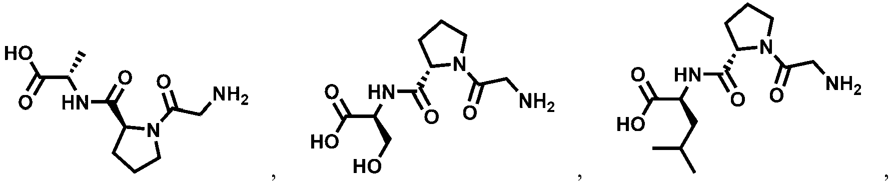 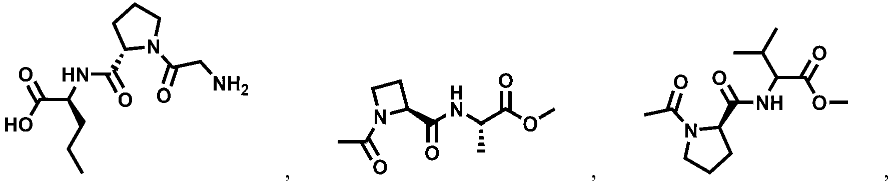 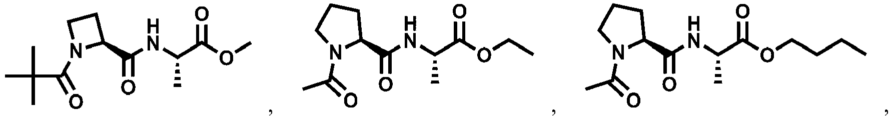 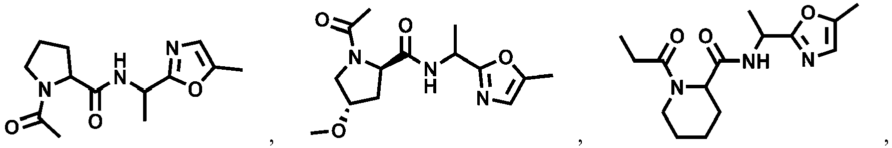 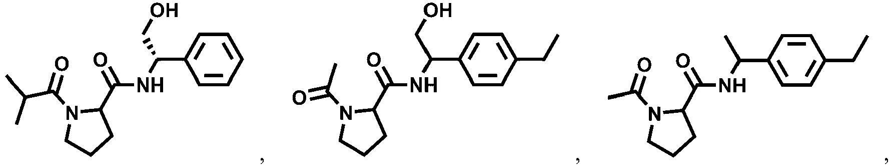 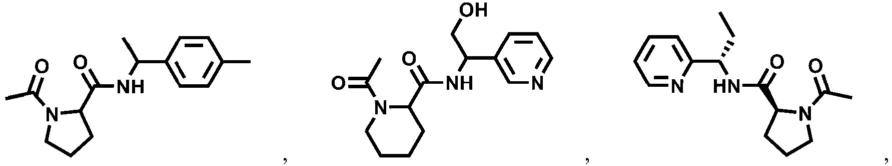 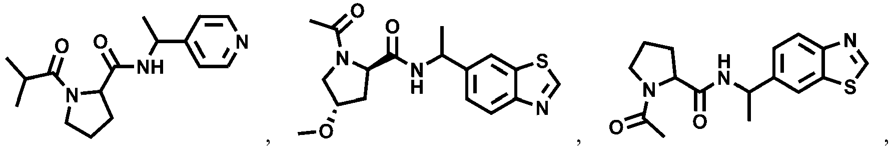 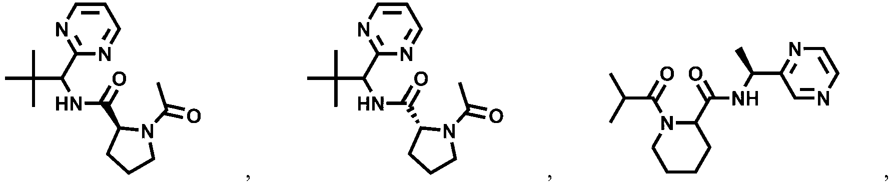 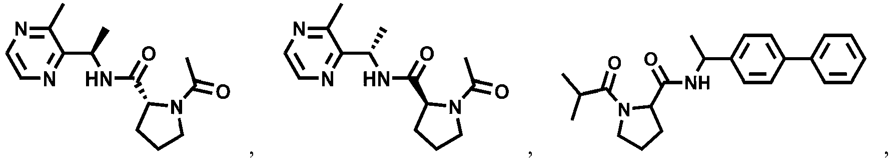 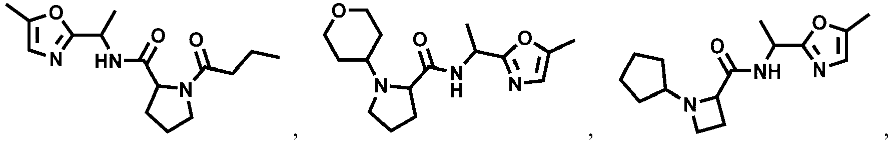 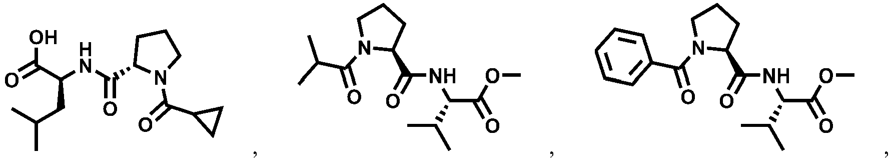 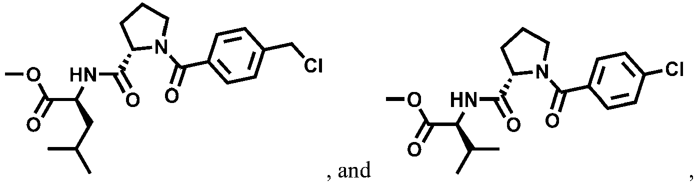
formula (Ib) does not comprise the following compounds:

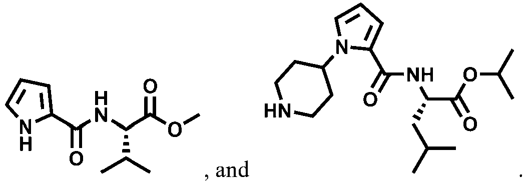

In some embodiments, wherein,
X is a bond;
Z₁ and Z₂ are independently selected from CHR₆, NR₇, oxygen or carbonyl, and Z₁ and Z₂ are not both NR₇ or oxygen;
Z₃ is CHR₆ or carbonyl;
Z₄, Z₅ and Z₆ are independently selected from CR₆ or N;
n is an integer from 0 to 4;
R₁ is selected from hydrogen, R₆-(CO)-, R₆-(SO)-, R₆-(SO₂)-, R₆-O(CO)-, R₆R₇N(CO)-, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₆ groups;
R₂ is selected from -(CO)O-(CH₂)ₘR₆, wherein m is an integer from 0 to 4, or R₂ is selected from C₆-C₁₅ aryl or 5- to 15-membered heteroaryl, wherein C₆-C₁₅ aryl or 5- to 15-membered heteroaryl is optionally substituted with 0 to 8 R₆ groups;
R₃ and R₄ are respectively independently selected from hydrogen or C₁-C₁₅ alkyl, wherein C₁-C₁₅ alkyl is optionally substituted with 0 to 8 R₆ groups; and R₃ and R₄ are not both hydrogen;
R₆ is selected from hydrogen, halogen, -CN, -NO₂, -OR₇, -NR₇R₈, -SR₇, -(CO)-R₇, -(SO)-R₇, -(SO₂)-R₇, -NR₇-(CO)-R₈, -(CO)-NR₇R₈, -O(CO)-R₇, -(CO)OR₇, -O(CO)OR₇, -O(CO)-NR₇R₈, -NR₇-(CO)-NR₈R₉, -NR₇-(CO)OR₈, -NR₇-(SO₂)-R₈, -(SO₂)-NR₇R₈, -O(SO₂)-R₇, -(SO₂)OR₇, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₁₀ groups;
R₇, R₈ and R₉ are respectively independently selected from hydrogen, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups; and
R₁₀ is selected from hydrogen, halogen, cyano, hydroxyl, mercapto, ether, nitro, amino, carboxyl, sulfo, ester, amide, sulfonate, sulfonamide, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups.

In some embodiments, wherein,
the compound represented by formula (Ia) is a compound represented by the following formula (Ia-1), and the compound represented by formula (Ib) is a compound represented by the following formula (Ib-1) 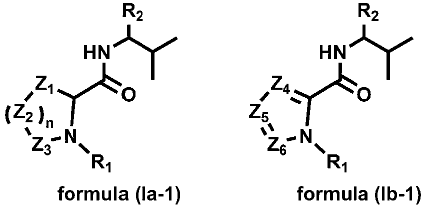
wherein R₁, R₂, Z₁, Z₂, Z₃, Z₄, Z₅, Z₆ and n are as defined above.

In some embodiments, wherein,
the compound represented by formula (Ia) is a compound represented by the following formula (Ia-1'), 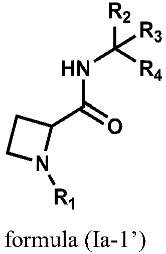
wherein R₁, R₂, R₃ and R₄ are as defined above.

In some embodiments, wherein,
the compound represented by formula (Ia) is a compound represented by the following formula (Ia-2), 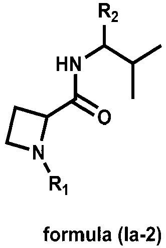
wherein R₁ and R₂ are as defined above.

In some embodiments, wherein,
the compound represented by formula (Ia) is a compound represented by the following formula (Ia-3), 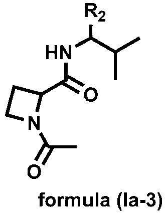
wherein R₂ is as defined above.

In some embodiments, wherein,
R₂ is selected from -(CO)O-(CH₂)ₘR₆, wherein m is an integer from 0 to 4. In some embodiments, wherein,
R₂ is selected from C₆-C₁₅ aryl or 5- to 15-membered heteroaryl, wherein C₆-C₁₅ aryl or 5- to 15-membered heteroaryl is optionally substituted with 0 to 8 R₆ groups.

In some embodiments, wherein,
R₂ is selected from:

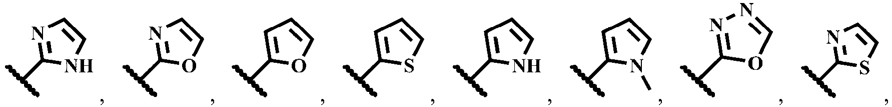

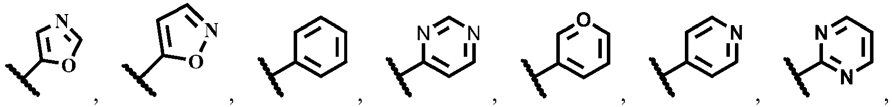

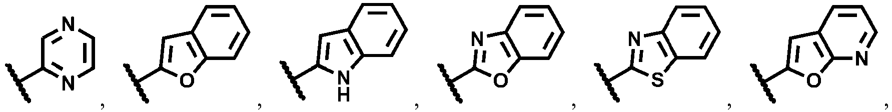

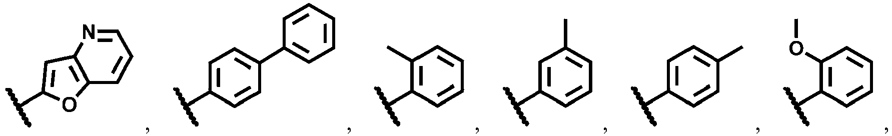

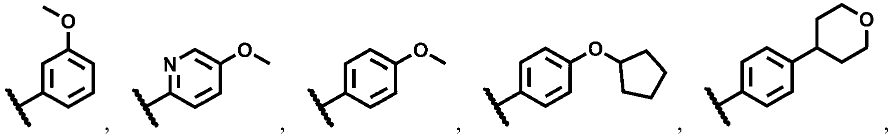

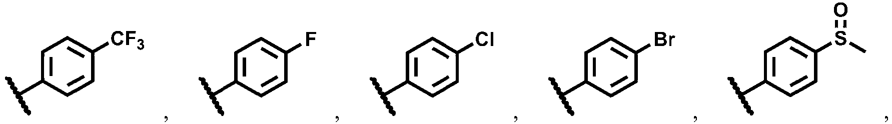

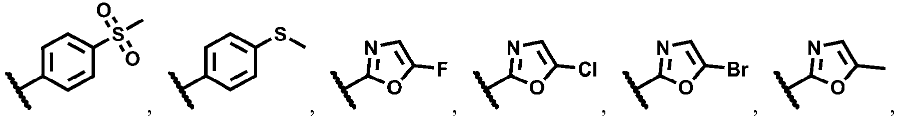

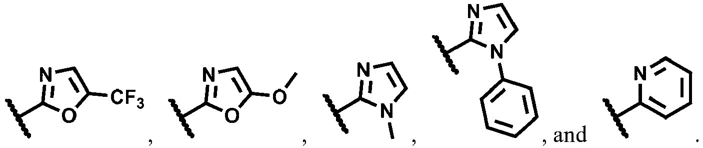

In some embodiments, wherein
X is a bond;
Z₁ and Z₂ are independently selected from CHR₆, NR₇, oxygen or carbonyl, and Z₁ and Z₂ are not both NR₇ or oxygen;
Z₃ is CHR₆ or carbonyl;
Z₄, Z₅ and Z₆ are independently selected from CR₆ or N;
n is an integer from 0 to 4;
R₁ is selected from hydrogen, R₆-(CO)-, R₆-(SO)-, R₆-(SO₂)-, R₆-O(CO)-, R₆R₇N(CO)-, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₆ groups; ,
R₂ is selected from C₆-C₁₅ aryl or 5- to 15-membered heteroaryl, wherein C₆-C₁₅ aryl or 5- to 15-membered heteroaryl is optionally substituted with 0 to 8 R₆ groups;
R₃ and R₄ are respectively independently selected from hydrogen, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₆ groups; and R₃ and R₄ are not both hydrogen;
R₆ is selected from hydrogen, halogen, -CN, -NO₂, -OR₇, -NR₇R₈, -SR₇, -(CO)-R₇, -(SO)-R₇, -(SO₂)-R₇, -NR₇-(CO)-R₈, -(CO)-NR₇R₈, -O(CO)-R₇, -(CO)OR₇, -O(CO)OR₇, -O(CO)-NR₇R₈, -NR₇-(CO)-NR₈R₉, -NR₇-(CO)OR₈, -NR₇-(SO₂)-R₈, -(SO₂)-NR₇R₈, -O(SO₂)-R₇, -(SO₂)OR₇, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₁₀ groups;
R₇, R₈ and R₉ are respectively independently selected from hydrogen, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups; and
R₁₀ is selected from hydrogen, halogen, cyano, hydroxyl, mercapto, ether, nitro, amino, carboxyl, sulfo, ester, amide, sulfonate, sulfonamide, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups.

In some embodiments, wherein,
the compound represented by formula (Ia) is a compound represented by the following formula (Ia-4), and the compound represented by formula (Ib) is a compound represented by the following formula (Ib-2) 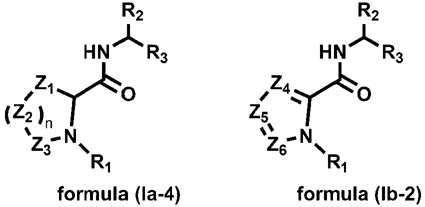
wherein R₁, R₂, R₃, Z₁, Z₂, Z₃, Z₄, Z₅, Z₆ and n are as defined above.

In some embodiments, wherein,
the compound represented by formula (Ia) is a compound represented by the following formula (Ia-5) 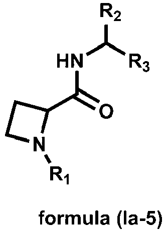
wherein R₁, R₂ and R₃ are as defined above.

In some embodiments, wherein,
the compound represented by formula (Ia) is a compound represented by the following formula (Ia-6) 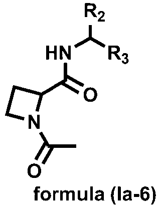
wherein R₂ and R₃ are as defined above.

In some embodiments, wherein,
the compound is selected from any one of the following compounds:

| | | | |
|---|---|---|---|
| A-1-1 | 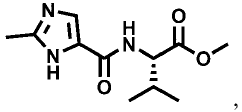 | A-1-2 | 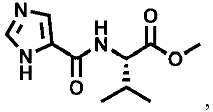 |
| A-1-3 | 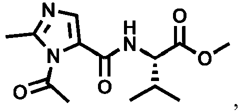 | A-1-4 | 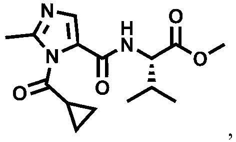 |
| A-1-5 | 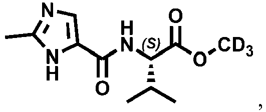 | A-1-6 | 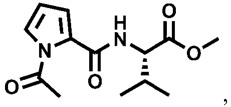 |
| A-2-1 | 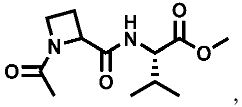 | A-2-2 | 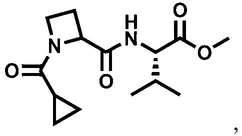 |
| A-2-3 | 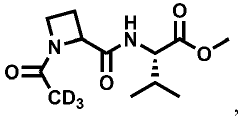 | A-2-4 | 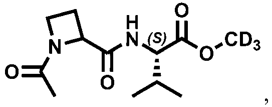 |
| A-2-5 | 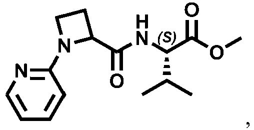 | A-2-6 | 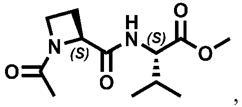 |
| A-2-7 | 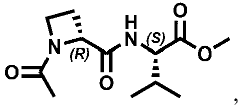 | A-2-8 | 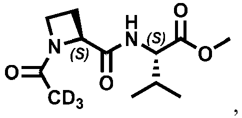 |
| A-2-9 | 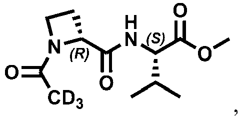 | A-2-10 | 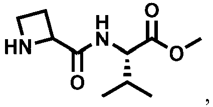 |
| A-2-11 | 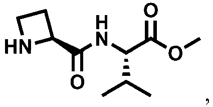 | A-2-12 | 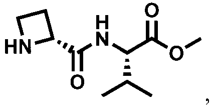 |
| A-2-13 | 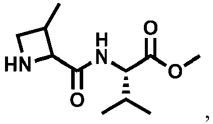 | A-2-14 | 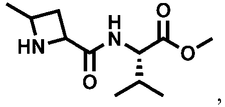 |
| A-2-15 | 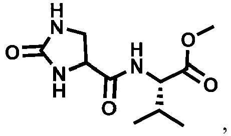 | A-2-16 | 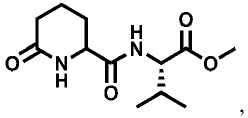 |
| A-2-17 | 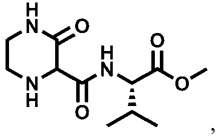 | A-2-18 | 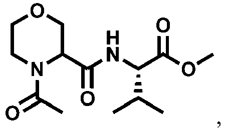 |
| A-2-20 | 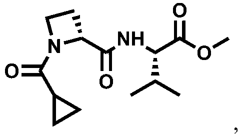 | A-2-21 | 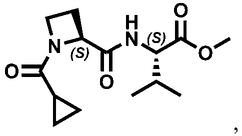 |
| A-2-22 | 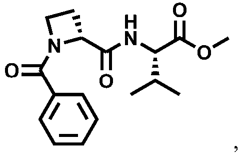 | A-2-23 | 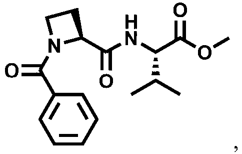 |
| A-2-24 | 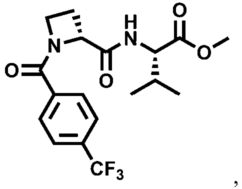 | A-2-25 | 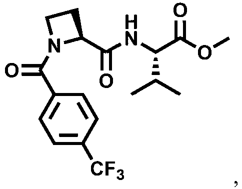 |
| A-2-26 | 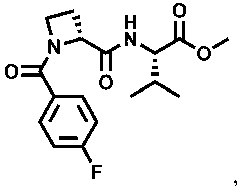 | A-2-27 | 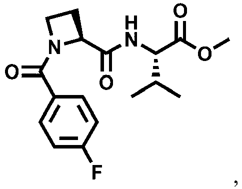 |
| A-2-28 | 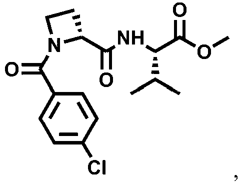 | A-2-29 | 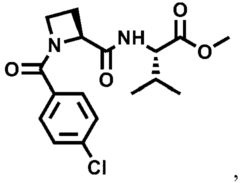 |
| A-2-30 | 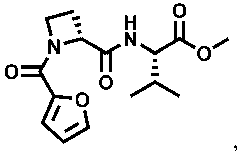 | A-2-31 | 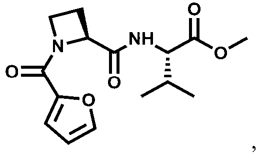 |
| A-2-32 | 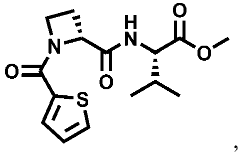 | A-2-33 | 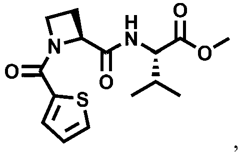 |
| A-2-34 | 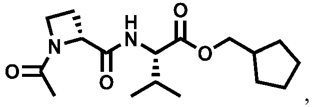 | A-2-35 | 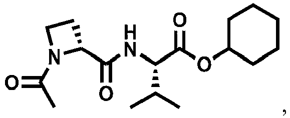 |
| A-2-36 | 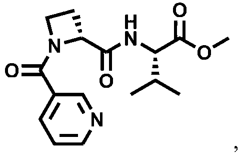 | A-2-37 | 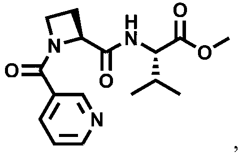 |
| A-2-38 | 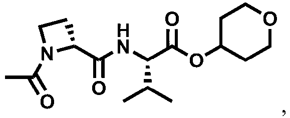 | A-2-39 | 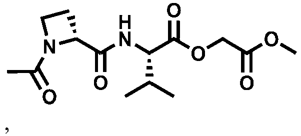 |
| A-2-40 | 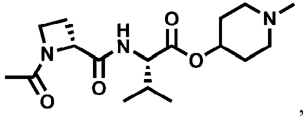 | A-2-41 | 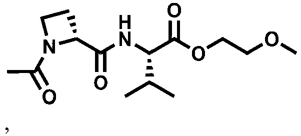 |
| A-2-42 | 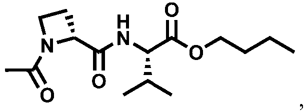 | A-2-43 | 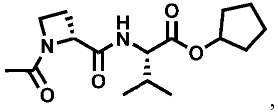 |
| A-2-44 | 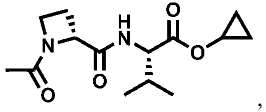 | | |
| B-1-1 | 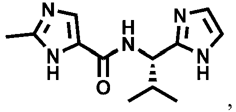 | B-2-1 | 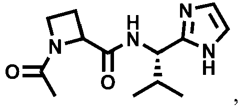 |
| B-2-2 | 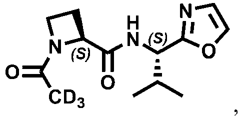 | B-2-3 | 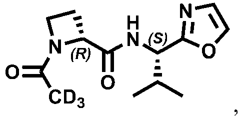 |
| B-2-4 | 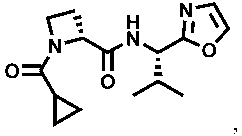 | B-2-5 | 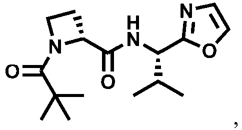 |
| B-2-8 | 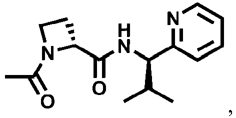 | B-2-9 | 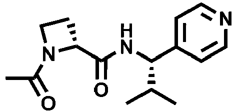 or |
| | | | 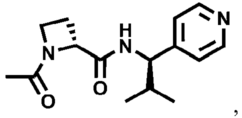 |
| B-2-10 | 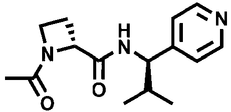 or | B-2-11 | 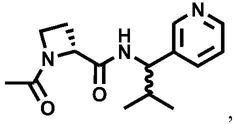 |
| | 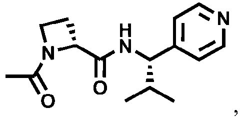 | | |
| B-2-12 | 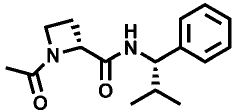 or | B-2-13 | 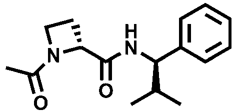 or |
| | 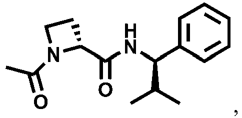 | | 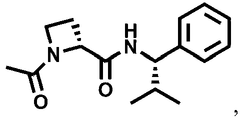 |
| B-2-14 | 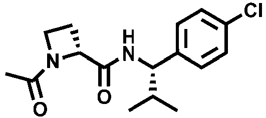 or | B-2-15 | 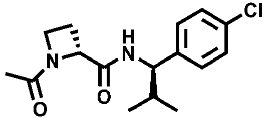 or |
| | 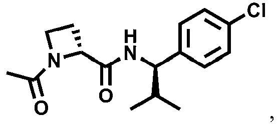 | | 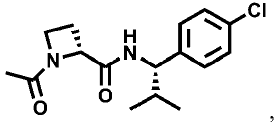 |
| B-2-16 | 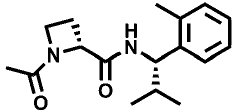 or | B-2-17 | 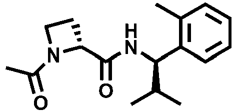 or |
| | 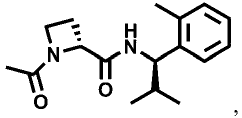 | | 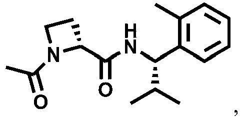 |
| B-2-18 | 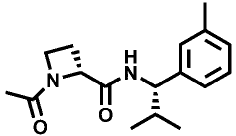 or | B-2-19 | 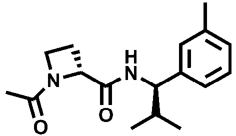 or |
| | 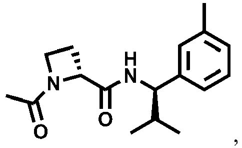 | | 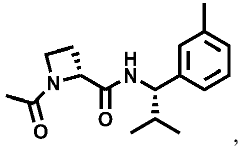 |
| B-2-20 | 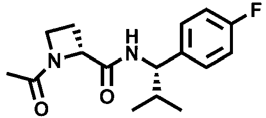 or | B-2-21 | 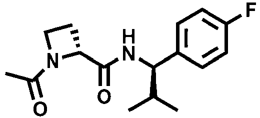 or |
| | 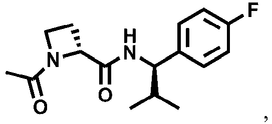 | | 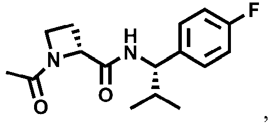 |
| B-2-22 | 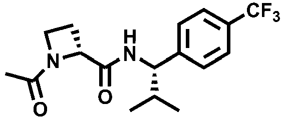 or | B-2-23 | 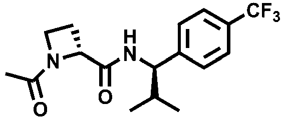 |
| | | | or |
| | 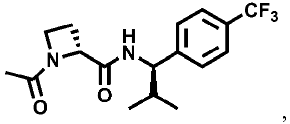 | | 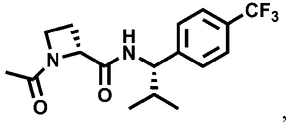 |
| B-2-24 | 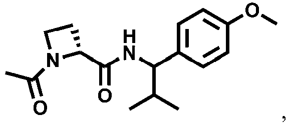 | B-2-25 | 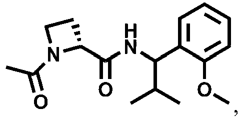 |
| B-2-26 | 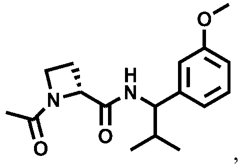 | | 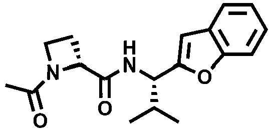 |
| | | B-2-27 | or |
| | | | 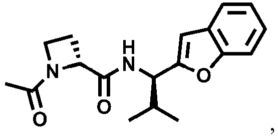 |
| B-2-28 | 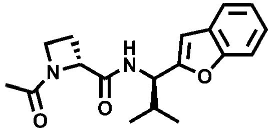 | B-2-29 | 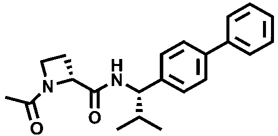 |
| | or | | or |
| | 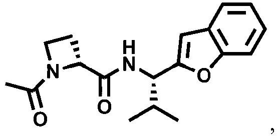 | | 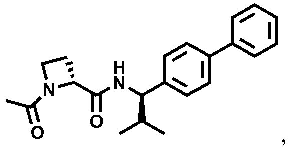 |
| B-2-30 | 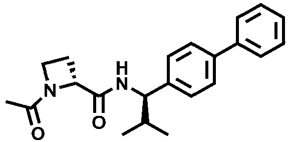 | B-2-31 | 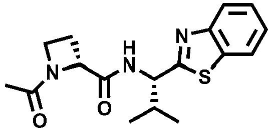 |
| | or | | or |
| | 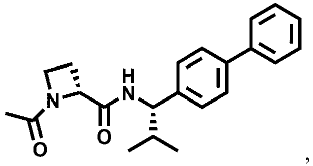 | | 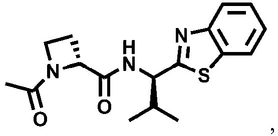 |
| B-2-32 | 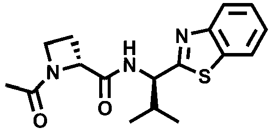 or | B-2-33 | 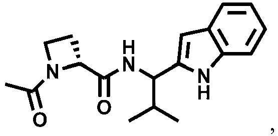 |
| | 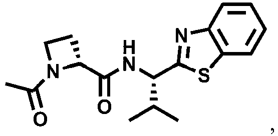 | | |
| B-2-34 | 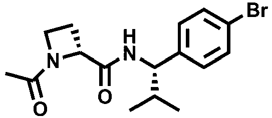 | B-2-35 | 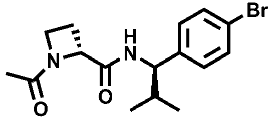 |
| | or | | or |
| | 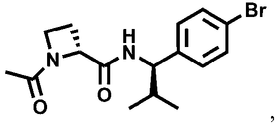 | | 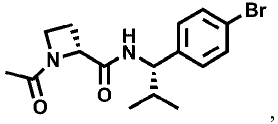 |
| B-2-36 | 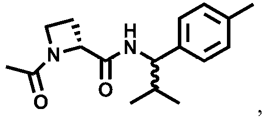 | B-2-37 | 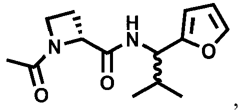 |
| B-2-38 | 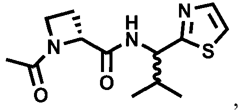 | B-2-39 | 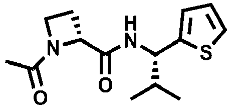 or |
| | | | 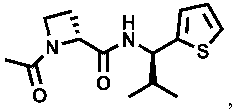 |
| B-2-40 | 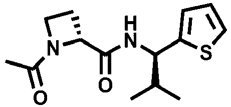 or | B-2-41 | 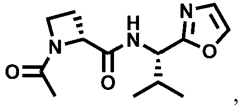 |
| | 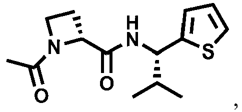 | | |
| B-2-42 | 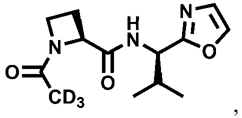 | B-2-43 | 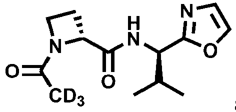 and |
| B-2-44 | 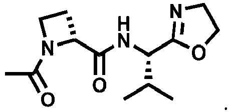 | | |

In some embodiments, wherein,
the compound is selected from any one of the following compounds:

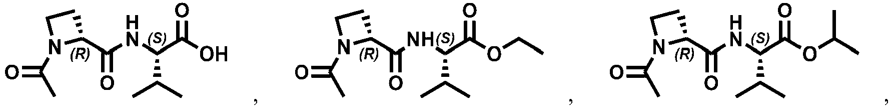

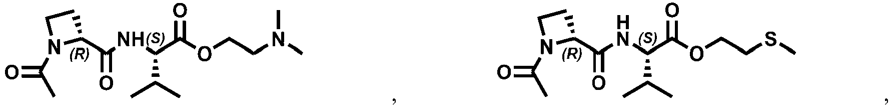

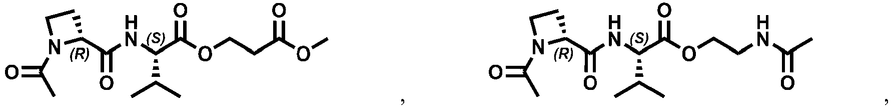

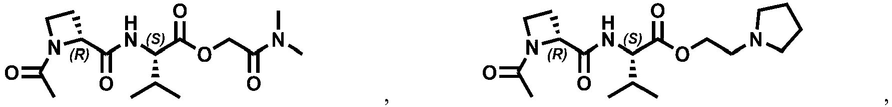

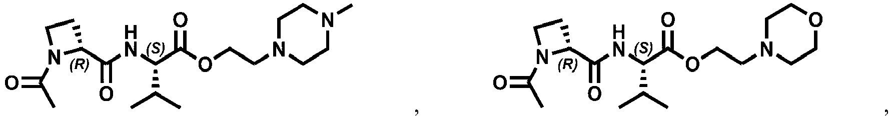

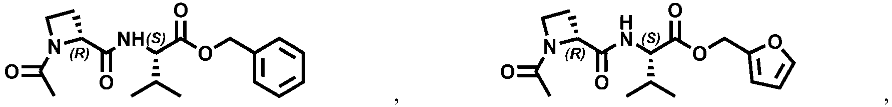

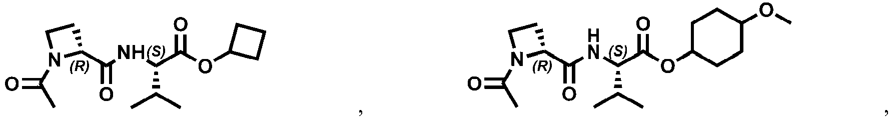

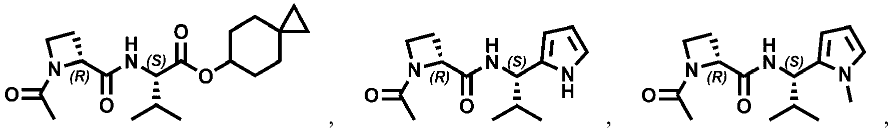

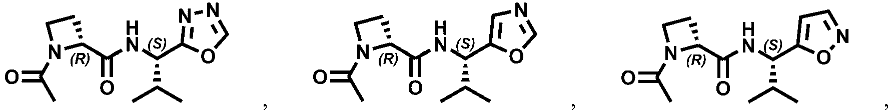

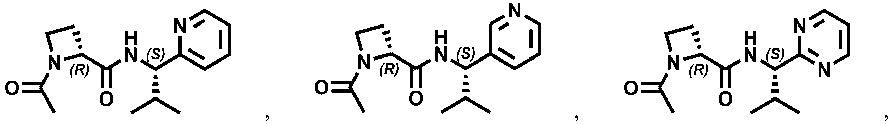

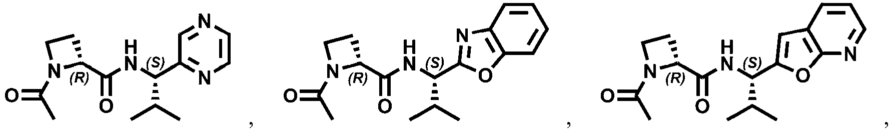

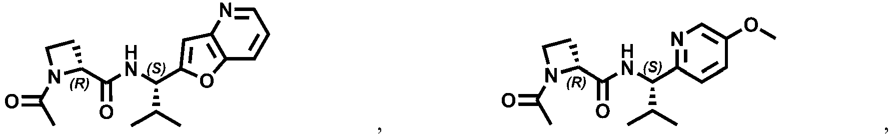

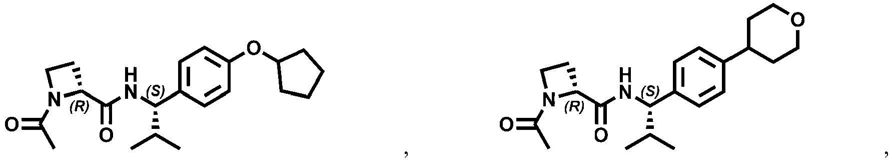

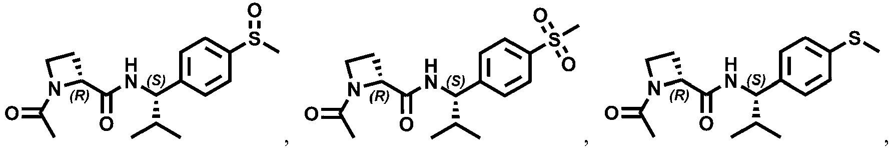

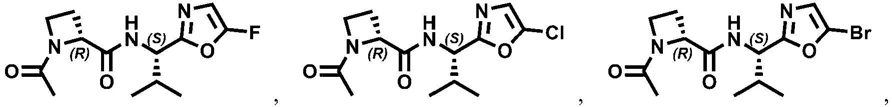

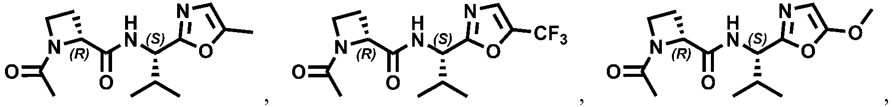

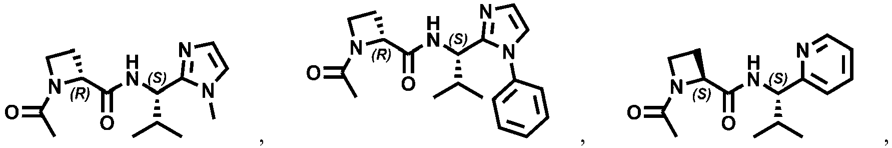

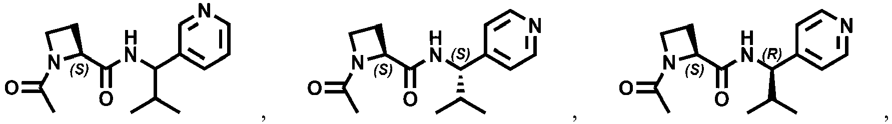

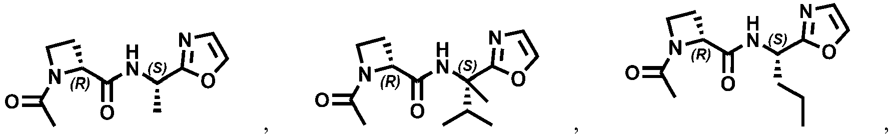

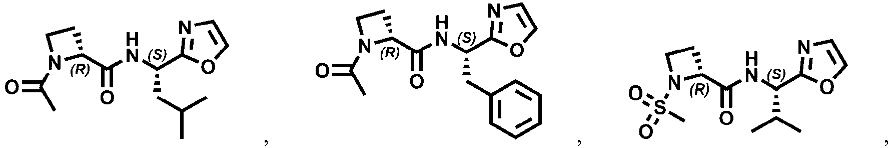

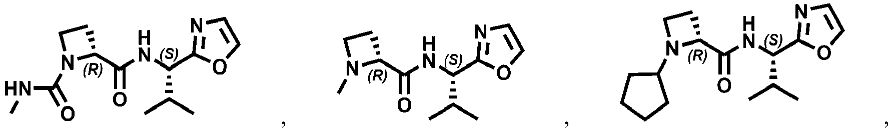

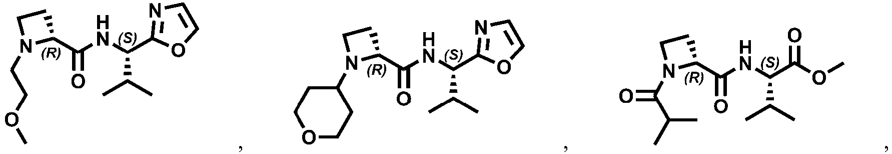

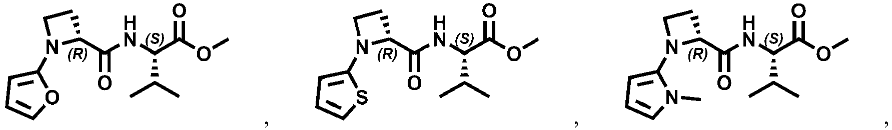

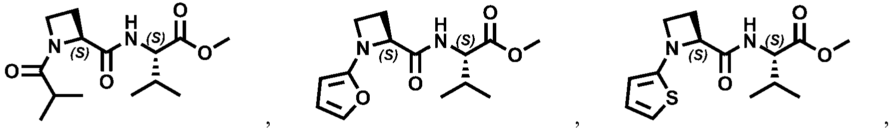

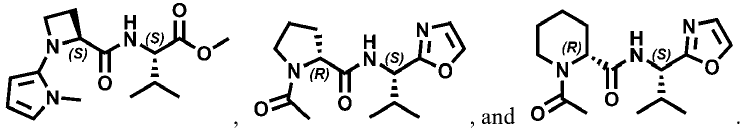

The present invention further provides a pharmaceutical composition including a therapeutically effective amount of the compound herein, or the pharmaceutically acceptable salt, prodrug, deuterated compound, hydrate, solvate, enantiomer, diastereoisomer or racemate thereof, and a pharmaceutically acceptable excipient or an auxiliary ingredient.

The present invention further provides a use of the compound herein, or the pharmaceutically acceptable salt, prodrug, deuterated compound, hydrate, solvate, enantiomer, diastereoisomer or racemate thereof, or the pharmaceutical composition in preparation of a drug for preventing and/or treating depression.

In some embodiments, wherein, the drug in the above use is a quick-acting drug.

The hydrogen herein may be in any isotopic form, including ¹H (protium), ²H (D or deuterium) and ³H (T or tritium).

### Definition

Unless stated to the contrary, the terms used in the specification and the claims have the following meanings.

Carbon, hydrogen, oxygen, sulfur and nitrogen, or F, Cl, Br and I in the groups and compounds of the present invention include their isotopic forms, and carbon, hydrogen, oxygen, sulfur or nitrogen in the groups and compounds of the present invention is optionally further substituted with one or more of their corresponding isotopes, wherein isotopes of carbon include ¹²C, ¹³C and ¹⁴C, isotopes of hydrogen include protium (H), deuterium (D, also known as heavy hydrogen) and tritium (T, also known as super-heavy hydrogen), isotopes of oxygen include ¹⁶O, ¹⁷O and ¹⁸O, isotopes of sulfur include ³²S, ³³S, ³⁴S and ³⁶S, isotopes of nitrogen include ¹⁴N and ¹⁵N, isotopes of fluorine include ¹⁷F and ¹⁹F, isotopes of chlorine include ³⁵Cl and ³⁷Cl, and isotopes of bromine include ⁷⁹Br and ⁸¹Br.

"Alkyl" refers to a straight or branched saturated aliphatic hydrocarbon group having 1 to 20 carbon atoms, preferably alkyl having 1 to 15 carbon atoms, more preferably alkyl having 1 to 8 carbon atoms, and further preferably alkyl having 1, 2, 3, 4, 5 or 6 carbon atoms. Non-limiting examples of alkyl include methyl, ethyl, n-propyl, isopropyl, n-butyl, sec-butyl, neobutyl, tert-butyl, n-pentyl, isopentyl, neopentyl, n-hexyl and various branched isomers thereof. The alkyl may be optionally further substituted with one or more substituents.

"Alkoxy" refers to a group formed by attaching alkyl to an oxygen atom. The alkyl has the same definition as the "alkyl" above. Non-limiting examples of alkoxy include methoxy, ethoxy, n-propoxy, isopropoxy, n-butoxy, sec-butoxy, tert-butoxy, n-pentoxy, n-hexoxy, cyclopropoxy, cyclobutoxy, and the like. The alkoxy may be optionally further substituted with one or more substituents.

"Cycloalkyl" refers to a saturated or partially unsaturated, monocyclic or polycyclic, non-aromatic cyclic substituent, and the cycloalkyl includes 3 to 15 carbon atoms, preferably 3 to 10 carbon atoms, more preferably 3 to 8 carbon atoms (such as 3, 4, 5, 6, 7 or 8 carbon atoms). Non-limiting examples of monocyclic cycloalkyl include cyclopropyl, cyclobutyl, cyclopentyl, cyclopentenyl, cyclopentadienyl, cyclohexyl, cyclohexenyl, cyclohexadienyl, cycloheptyl, cycloheptatrienyl, cyclooctyl, and the like; and the polycyclic cycloalkyl includes spiro, fused and bridged cycloalkyl. The cycloalkyl may be optionally further substituted with one or more substituents.

"Heterocycloalkyl" refers to a substituted or unsubstituted, saturated, non-aromatic ring group, which may be a 3- to 8-membered (such as 3-, 4-, 5-, 6-, 7- or 8-membered) monocyclic ring, 6- to 12-membered (such as 6-, 7-, 8-, 9-, 10-, 11- or 12-membered) bicyclic ring or 10- to 15-membered (such as 10-, 11-, 12-, 13-, 14- or 15-membered) tricyclic ring system, and includes 1, 2 or 3 heteroatoms selected from N, O or S, and is preferably 3- to 8-membered heterocyclyl. 1, 2 or 3 N or S atoms ,which are optionally substituted, in the "heterocycloalkyl" ring may be oxidized to various oxidation states; the "heterocycloalkyl" may be attached to a heteroatom or a carbon atom; and the "heterocycloalkyl" may be a bridged or spiro ring. Non-limiting examples of the "heterocycloalkyl" include oxiranyl, aziridinyl, oxetanyl, azetidinyl, 1,3-dioxolanyl, 1,4-dioxolanyl, 1,3-dioxanyl, azepanyl, piperidinyl, piperidyl, morpholinyl, thiomorpholinyl, 1,3-dithianyl, tetrahydrofuranyl, tetrahydropyrrolyl, tetrahydroimidazolyl, tetrahydrothiazolyl, tetrahydropyranyl, azabicyclo[3.2.1]octyl, azabicyclo[5.2.0]nonyl, oxatricyclo[5.3.1.1]dodecanyl, azaadamantanyl, oxaspiro[3.3]heptanyl, and the like. The heterocycloalkyl may be optionally further substituted with one or more substituents.

"Aryl" refers to a substituted or unsubstituted aromatic ring, which may be a 5- to 8-membered monocyclic ring (such as 5-, 6-, 7- or 8-membered), 6- to 12-membered bicyclic ring (such as 6-, 7-, 8-, 9-, 10-, 11- or 12-membered) or 10- to 15-membered (such as 10-, 11-, 12-, 13-, 14- or 15-membered) tricyclic ring system, and may be a bridged or spiro ring. Non-limiting examples of aryl include phenyl, naphthyl, and the like. The aryl may be optionally further substituted with one or more substituents.

"Heteroaryl" refers to an aromatic ring group having a conjugated planar ring system and containing heteroatoms, which may be a 5- to 8-membered (such as 5-, 6-, 7- or 8-membered) monocyclic ring, 8- to 12-membered (such as 8-, 9-, 10-, 11- or 12-membered) bicyclic ring, or 10- to 15-membered (such as 10-, 11-, 12-, 13-, 14- or 15-membered) tricyclic ring system, and containing 1 to 6 (such as 1, 2, 3, 4, 5 or 6) heteroatoms selected from N, O or S. Non-limiting examples of heteroaryl include oxazolyl, triazolyl, pyridyl, furyl, thienyl, pyranyl, pyrrolyl, pyrimidinyl, pyrazinyl, pyridazinyl, imidazolyl, piperidyl, benzimidazolyl, benzopyridyl, pyrrolopyridyl, and the like. The heteroaryl may be optionally further substituted with one or more substituents.

"Pharmaceutically acceptable salt" refers to a salt formed by reacting a free acid of a compound of the present invention with a non-toxic inorganic or organic base, or a free base of a compound of the present invention with a non-toxic inorganic or organic acid, while retaining the biological effectiveness and properties of the free acid or the free base.

"Pharmaceutical composition" refers to a mixture formed by one or more compounds of the present invention, pharmaceutically acceptable salts or prodrugs thereof, and other chemical constituents, wherein the "other chemical constituents" refer to pharmaceutically acceptable carriers, excipients, and/or one or more other therapeutic agents.

"Optional" or "optionally" means that the subsequently described event or circumstance may, but does not necessarily, occur, and the description includes cases in which the event or circumstance occurs and cases in which the event or circumstance does not occur. For example, "heterocyclyl optionally substituted with alkyl" means that the alkyl may, but does not necessarily, be present, and the description includes cases in which the heterocyclyl is substituted with the alkyl and cases in which the heterocyclyl is not substituted with the alkyl.

As used herein, when " 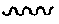 " represents a chemical bond, attachment of a " 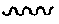 " chemical bond to a certain chiral center of a compound indicates that the chiral center of the compound is of a single configuration, but the absolute configuration is unknown.

### Beneficial technical effects achieved:

(1) The compounds of the present invention have good in-vivo rapid antidepressant activity.
(2) Compared with the compounds disclosed in the prior art, the compounds of the present invention have favorable pharmacokinetic properties, such as good parameters including AUC and bioavailability.

### BRIEF DESCRIPTION OF THE DRAWINGS

FIG. 1 and FIG. 2 show results of a tail suspension test in Experimental Example 5.
FIG. 3 shows results of a forced swimming test in Experimental Example 6.
FIG. 4 shows results of a tail suspension test in Experimental Example 6.

### DETAILED DESCRIPTION

Unless otherwise stated, various raw materials/reagents/instruments used in the examples of the present invention are conventional commercially available products.

The instrument used for ¹HNMR detection in the present invention is a Bruker Advance 400 MHz spectrometer; and the instrument used for MS-ESI detection is an Agilent LCMS 1260-6120.

### Preparation Example 1 Preparation of compound A-1-7

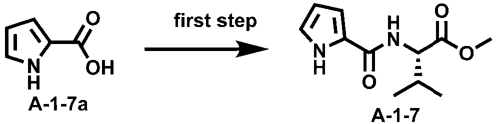

### First step

L-valine methyl ester hydrochloride (779 mg, 4.65 mmol) and 4-dimethylaminopyridine (1.3 g, 10.80 mmol) were dissolved in N,N-dimethylformamide (10 mL), cooled to 0°C, and then added with compound A-1-7a (600 mg, 5.40 mmol) and 1-(3-dimethylaminopropyl)-3-ethylcarbodiimide hydrochloride (1.1 g, 5.94 mmol). The reaction was stirred at room temperature for 16 hours, and completion of the reaction was indicated by TLC. The reaction solution was diluted with water (50 mL) and extracted with ethyl acetate 3 times (50 mL × 3). The organic phases were combined, washed with saturated brine (100 mL), dried with anhydrous sodium sulfate, and concentrated to obtain a crude product, and the crude product was purified by preparative HPLC to obtain compound A-1-7 (580 mg, 2.59 mmol, yield 47.89%). MS (ESI) calculated for [M+H]⁺ was 225.12; found: m/z 224.9.

### Preparation Example 2 Preparation of compound A-1-1

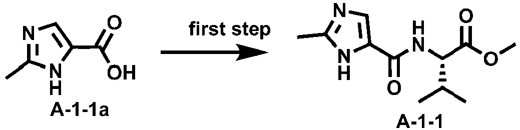

### First step

L-valine methyl ester hydrochloride (686 mg, 4.09 mmol) and 4-dimethylaminopyridine (1.2 g, 9.52 mmol) were dissolved in N,N-dimethylformamide (10 mL), cooled to 0°C, added with compound A-1-1a (600 mg, 4.76 mmol) and 1-(3-dimethylaminopropyl)-3-ethylcarbodiimide hydrochloride (1.0 g, 5.32 mmol). The reaction was stirred at room temperature for 16 hours, and completion of the reaction was indicated by TLC. The reaction solution was diluted with water (50 mL) and extracted with ethyl acetate 3 times (50 mL × 3). The organic phases were combined, washed with saturated brine (100 mL), dried with anhydrous sodium sulfate, and concentrated to obtain a crude product, and the crude product was purified by preparative HPLC to obtain compound A-1-1 (750 mg, 3.13 mmol, yield 65.8%). MS (ESI) calculated for [M+H]⁺ was 240.1; found: m/z 240.0.

### Preparation Example 3 Preparation of compound A-2-1

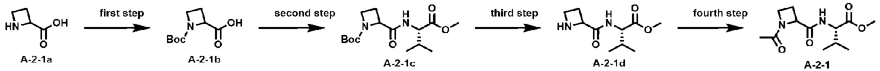

### First step

A-2-1a (5.00 g, 49.45 mmol), di-tert-butyl dicarbonate (12.95 g, 59.34 mmol) and ethanol (60 mL) were added to a 200 mL dry three-necked flask, and fully stirred, and then sodium hydroxide (1.98 g, 49.45 mmol) was dissolved in water (25 mL) and added to the reaction system. The reaction was stirred at room temperature for 16 hours. After completion of the reaction (monitored by LCMS), the reaction solution was diluted with water (60 mL), added with 1 M dilute hydrochloric acid to adjust pH to 3, and extracted with ethyl acetate (150 mL × 3). The organic phases were combined, successively washed with saturated brine, dried with anhydrous sodium sulfate, and concentrated to obtain intermediate A-2-1b (8.60 g, 42.74 mmol, yield 86%).

### Second step

*L*-valine methyl ester hydrochloride (7.52 g, 44.86 mmol), 4-dimethylaminopyridine (10.45 g, 85.45 mmol) and N,N-dimethylformamide (80 mL) were added to a 200 mL dry three-necked flask, cooled to 0°C, and then added with compound A-2-1b (8.60 g, 42.74 mmol) and 1-3-(dimethylaminopropyl)-3-ethylcarbodiimide hydrochloride (9.83 g, 51.29 mmol). The reaction was stirred at room temperature for 16 hours. After completion of the reaction (monitored by LCMS), the reaction solution was diluted with water (100 mL) and extracted with ethyl acetate (200 mL × 3). The organic phases were combined, successively washed with water (500 mL) and saturated brine (500 mL), dried with anhydrous sodium sulfate, and concentrated to obtain intermediate A-2-1c (13.80 g, 43.90 mmol).

### Third step

Compound intermediate A-2-1c (13.80 g, 43.90 mmol) was dissolved in a solution of hydrogen chloride in ethyl acetate (4.0 M, 100 mL). The reaction was stirred at room temperature for 2 hours. After completion of the reaction (monitored by LCMS), the reaction solution was concentrated and purified by medium-pressure preparative chromatography to obtain intermediate A-2-1d (7.56 g, 35.28 mmol, yield 80%).

### Fourth step

Intermediate A-2-1d (1.0 g, 4.67 mmol) was dissolved in dichloromethane (10 mL), and added with acetic anhydride (6.7 g, 65.41 mmol). The reaction was stirred at room temperature for 16 hours, and completion of the reaction was indicated by TLC. The reaction solution was cooled to 0°C, added with 1 M hydrochloric acid to adjust pH to 2, and extracted with ethyl acetate 3 times (50 mL × 3). The organic phases were combined, washed with saturated brine (100 mL), dried with anhydrous sodium sulfate, and concentrated to obtain a crude product, and the crude product was purified by reverse-phase preparative chromatography to obtain compound A-2-1 (500 mg, 1.95 mmol, yield 41.80%). MS (ESI) calculated for [M+H]⁺ was 257.2; found: m/z 257.0.

### Preparation Example 4 Preparation of compound A-2-2

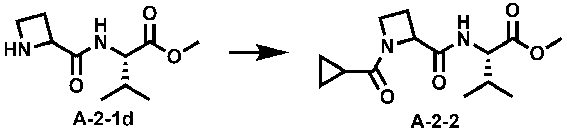

Intermediate A-2-1d (1.00 g, 4.67 mmol) was dissolved in dry dichloromethane (20 mL), added with cyclopropanecarbonyl chloride (0.54 g, 5.14 mmol), and then dropwise added with triethylamine (1.18 g, 11.68 mmol). After completion of the dropwise addition, the reaction was stirred at room temperature for 1 hour. After completion of the reaction (monitored by LCMS), the reaction solution was diluted with water (50 mL) and extracted with ethyl acetate (100 mL × 3). The organic phases were combined, successively washed with saturated brine, dried with anhydrous sodium sulfate, concentrated, and purified by medium-pressure preparative chromatography to obtain compound A-2-2 (0.49 g, 1.74 mmol, yield 37%). MS (ESI) calculated for [M+H]⁺ was 283.2; found: m/z 283.3. ¹H NMR (400 MHz, DMSO-d6) δ 8.58 - 8.21 (m, 1H), 5.13 - 4.66 (m, 1H), 4.32 - 4.10 (m, 2H), 3.86 - 3.71 (m, 1H), 3.64 (dd, *J =* 6.7, 4.2 Hz, 3H), 2.48 - 2.30 (m, 1H), 2.25 - 1.93 (m, 2H), 1.61 - 1.16 (m, 1H), 0.92 - 0.80 (m, 6H), 0. 80 - 0.51 (m, 4H).

### Preparation Example 5 Preparation of compound A-2-4

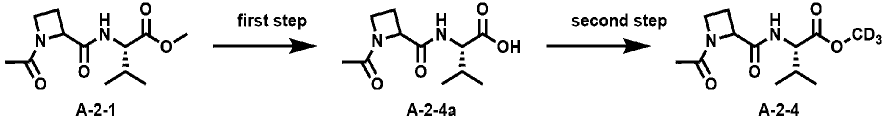

### First step

Compound A-2-1 (0.92 g, 3.59 mmol) and THF (10 mL) were added to a 50 mL dry three-necked flask, and then slowly dropwise added with aqueous sodium hydroxide solution (2 M, 3 mL). After completion of the addition, the reaction was stirred at room temperature for 2 hours. After completion of the reaction (monitored by LCMS), the reaction solution was adjusted to be acidic (pH = 5) with dilute hydrochloric acid, and then extracted with dichloromethane (30 mL × 3). The organic phases were combined, then washed with saturated brine, dried with anhydrous sodium sulfate, and concentrated to obtain a crude product - intermediate A-2-4a (0.80 g, 3.31 mmol, yield 92%).

### Second step

The crude product - intermediate A-2-4a (0.80 g, 3.31 mmol) and deuterated methanol (5 mL) were added to a 50 mL dry three-necked flask, and then slowly dropwise added with concentrated sulfuric acid (0.36 g, 3.64 mmol). After completion of the addition, the reaction was stirred at room temperature for 2 hours. After completion of the reaction (monitored by LCMS), the reaction solution was adjusted to be neutral (pH = 7) with sodium hydroxide solution (2.0 M), and then extracted with dichloromethane (30 mL × 3). The organic phases were combined, washed with saturated brine (50 mL), dried with anhydrous sodium sulfate, concentrated, and purified by medium-pressure preparative chromatography to obtain the product A-2-4 (0.25 g, 0.98 mmol, yield 30%). MS (ESI) calculated for [M+H]⁺ was 260.2; found: m/z 260.2. ¹H NMR (400 MHz, DMSO) δ 8.42 (ddd, *J =* 28.1, 13.8, 6.7 Hz, 1H), 4.97 - 4.64 (m, 1H), 4.32 - 4.13 (m, 1H), 4.08 - 3.93 (m, 1H), 3.83 - 3.62 (m, 1H), 2.41 - 2.29 (m, 1H), 2.16 - 1.91 (m, 2H), 1.83 - 1.56 (m, 3H), 0.96 - 0.77 (m, 6H).

### Preparation Example 6 Preparation of compound A-2-5

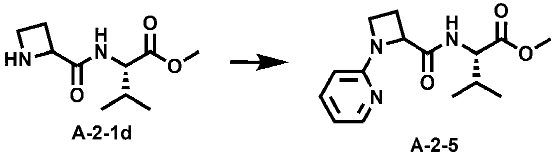

Intermediate A-2-1d (1.00 g, 4.67 mmol), 2-iodopyridine (1.91 g, 9.34 mmol) and N,N-dimethylformamide (30 mL) were added to a 100 mL dry three-necked flask. Subsequently, the mixture was sequentially added with Pd₂(dba)₃ (0.85 g, 0.93 mmol), Xantphos (0.54 g,0.93 mmol) and cesium carbonate (4.55 g, 14.01 mmol). After nitrogen replacement was carried out three times, the mixture was heated to 110°C, and the reaction was stirred for 6 hours. After completion of the reaction (monitored by LCMS), the reaction solution was diluted with water (20 mL) and extracted with dichloromethane (50 mL × 3). The organic phases were combined, successively washed with water (50 mL) and saturated brine (50 mL), dried with anhydrous sodium sulfate, concentrated, and purified by medium-pressure preparative chromatography to obtain the product A-2-5 (0.27 g, 0.93 mmol, yield 20%). MS (ESI) calculated for [M+H]⁺ was 292.2; found: m/z 292.2. ¹H NMR (400 MHz, DMSO-d6) δ 8.71 (dd, *J =* 87.4, 8.3 Hz, 1H), 8.07 (d, *J =* 4.0 Hz, 1H), 7.63 - 7.52 (m, 1H), 6.72 (ddd, *J =* 23.9, 12.0, 5.5 Hz, 1H), 6.43 (dd, *J =* 8.3, 5.8 Hz, 1H), 4.69 (dd, *J =* 16.5, 8.5 Hz, 1H), 4.29 - 4.20 (m, 1H), 3.95 - 3.72 (m, 2H), 3.64 (d, *J =* 9.0 Hz, 3H), 2.46 - 2.35 (m, 2H), 2.16 - 2.00 (m, 1H), 0.91 - 0.79 (m, 6H).

### Preparation Example 7 Preparation of compound A-2-6

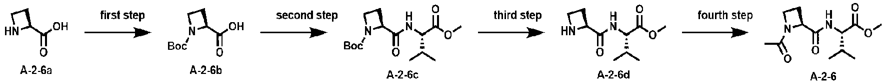

### First step

A-2-6a (5.00 g, 49.45 mmol), di-tert-butyl dicarbonate (12.95 g, 59.34 mmol) and ethanol (60 mL) were added to a 200 mL dry three-necked flask, and fully stirred, and then sodium hydroxide (1.98 g, 49.45 mmol) was dissolved in water (25 mL) and added to the reaction system. The reaction was stirred at room temperature for 16 hours. After completion of the reaction (monitored by LCMS), the reaction solution was diluted with water (60 mL), added with 1.0 M dilute hydrochloric acid to adjust pH to 3, and then extracted with ethyl acetate (150 mL × 3). The organic phases were combined, successively washed with saturated brine, dried with anhydrous sodium sulfate, and concentrated to obtain intermediate A-2-6b (8.60 g, 42.74 mmol, yield 86%).

### Second step

L-valine methyl ester hydrochloride (7.52 g, 44.86 mmol), 4-dimethylaminopyridine (10.45 g, 85.45 mmol) and N,N-dimethylformamide (80 mL) were added to a 200 mL dry three-necked flask, cooled to 0°C, and then added with intermediate A-2-6b (8.60 g, 42.74 mmol) and 1-(3-dimethylaminopropyl)-3-ethylcarbodiimide hydrochloride (9.83 g, 51.29 mmol). The reaction was stirred at room temperature for 16 hours. After completion of the reaction (monitored by LCMS), the reaction solution was diluted with water (100 mL) and extracted with ethyl acetate (200 mL × 3). The organic phases were combined, successively washed with water (500 mL) and saturated brine (500 mL), dried with anhydrous sodium sulfate, and concentrated to obtain a crude product - intermediate A-2-6c (13.80 g, 43.90 mmol).

### Third step

The crude product - intermediate A-2-6c (13.80 g, 43.90 mmol) was dissolved in a solution of hydrogen chloride in ethyl acetate (4.0 M, 100 mL). The reaction was stirred at room temperature for 2 hours. After completion of the reaction (monitored by LCMS), the reaction solution was concentrated and purified by medium-pressure preparative chromatography to obtain intermediate A-2-6d (7.56 g, 35.28 mmol, yield 80%).

### Fourth step

Intermediate A-2-6d (1.00 g, 4.67 mmol) and dichloromethane (15 mL) were added to a 50 mL dry three-necked flask, and then slowly added with acetic anhydride (4.76 g, 46.70 mmol). After completion of the addition, the reaction was stirred at room temperature overnight. After completion of the reaction (monitored by LCMS), the reaction solution was added with saturated aqueous sodium bicarbonate solution (50 mL), stirred for 0.5 hour, and then extracted with dichloromethane (50 mL × 3). The organic phases were combined, then washed with saturated brine, dried with anhydrous sodium sulfate, concentrated, and purified by medium-pressure preparative chromatography to obtain the product A-2-6 (0.92 g, 3.59 mmol, yield 76%). MS (ESI) calculated for [M+H]⁺ was 257.1; found: m/z 257.2. ¹H NMR (400 MHz, CDCl3) δ 8.31 (d, *J* = 7.4 Hz, 1H), 4.93 (dd, *J* = 9.3, 6.5 Hz, 1H), 4.41 (dd, *J* = 8.1, 5.0 Hz, 1H), 4.08 (t, *J =* 7.7 Hz, 2H), 3.74 (d, *J =* 5.0 Hz, 3H), 2.81 - 2.61 (m, 1H), 2.47 - 2.34 (m, 1H), 2.25 - 2.13 (m, 1H), 1.93 (s, 3H), 0.93 (dd, *J =* 8.5, 6.9 Hz, 6H).

### Preparation Example 8 Preparation of compound A-2-7

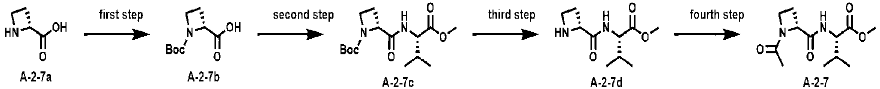

### First step

Compound A-2-7a (5.00 g, 49.45 mmol), di-tert-butyl dicarbonate (12.95 g, 59.34 mmol) and ethanol (60 mL) were added to a 200 mL dry three-necked flask, and fully stirred, and then sodium hydroxide (1.98 g, 49.45 mmol) was dissolved in water (25 mL) and added to the reaction system. The reaction was stirred at room temperature for 16 hours. After completion of the reaction (monitored by LCMS), the reaction solution was diluted with water (60 mL), added with 1.0 M dilute hydrochloric acid to adjust pH to 3, and extracted with ethyl acetate (150 mL × 3). The organic phases were combined, successively washed with saturated brine, dried with anhydrous sodium sulfate, and concentrated to obtain intermediate A-2-7b (8.60 g, 42.74 mmol, yield 86%).

### Second step

*L*-valine methyl ester hydrochloride (7.52 g, 44.86 mmol), 4-dimethylaminopyridine (10.45 g, 85.45 mmol) and *N*,*N*-dimethylformamide (80 mL) were added to a 200 mL dry three-necked flask, cooled to 0°C, and then added with intermediate A-2-7b (8.60 g, 42.74 mmol) and 1-3-(dimethylaminopropyl)-3-ethylcarbodiimide hydrochloride (9.83 g, 51.29 mmol). The reaction was stirred at room temperature for 16 hours. After completion of the reaction (monitored by LCMS), the reaction solution was diluted with water (100 mL) and extracted with ethyl acetate (200 mL × 3). The organic phases were combined, successively washed with water (500 mL) and saturated brine (500 mL), dried with anhydrous sodium sulfate, and concentrated to obtain a crude product - intermediate A-2-7c (13.80 g, 43.90 mmol).

### Third step

The crude product - intermediate A-2-7c (13.80 g, 43.90 mmol) was dissolved in a solution of hydrogen chloride in ethyl acetate (4.0 M, 100 mL). The reaction was stirred at room temperature for 2 hours. After completion of the reaction (monitored by LCMS), the reaction solution was concentrated and purified by medium-pressure preparative chromatography to obtain intermediate A-2-7d (7.56 g, 35.28 mmol, yield 80%).

### Fourth step

Intermediate A-2-7d (1.00 g, 4.67 mmol) and dichloromethane (15 mL) were added to a 50 mL dry three-necked flask, and then slowly added with acetic anhydride (4.76 g, 46.70 mmol). After completion of the addition, the reaction was stirred at room temperature overnight. After completion of the reaction (monitored by LCMS), the reaction solution was added with saturated aqueous sodium bicarbonate solution (50 mL), stirred for 0.5 hour, and then extracted with dichloromethane (50 mL × 3). The organic phases were combined, successively washed with water (500 mL) and saturated brine (500 mL), dried with anhydrous sodium sulfate, concentrated, and purified by medium-pressure preparative chromatography to obtain the product A-2-7 (0.92 g, 3.59 mmol, yield 76%). MS (ESI) calculated for [M+H]⁺ was 257.1; found: m/z 257.2. ¹H NMR (400 MHz, CDCl3) δ 8.28 (d, *J =* 7.9 Hz, 1H), 4.91 (dd, *J =* 9.5, 6.6 Hz, 1H), 4.48 (dd, *J =* 8.4, 4.9 Hz, 1H), 4.13 - 4.00 (m, 2H), 3.73 (s, 3H), 2.73 - 2.58 (m, 1H), 2.52 - 2.35 (m, 1H), 2.30 - 2.15 (m, 1H), 1.95 (s, 3H), 0.96 (dd, *J=* 16.1, 6.9 Hz, 6H).

### Preparation Example 9 Preparation of compound A-2-8

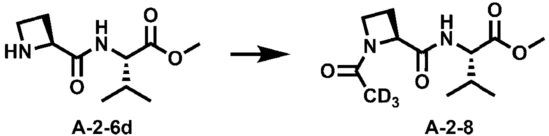

Intermediate A-2-6d (1.4 g, 6.5 mmol) was dissolved in dichloromethane (10 mL), and added with N,N-diisopropylethylamine (3.38 g, 26.2 mmol). After nitrogen replacement was carried out three times, the reaction mixture was cooled to 0°C, and then dropwise added with deuterated acetyl chloride (0.58 g, 7.19 mmol). The reaction solution was stirred at 20°C for 1 hour, then added with saturated sodium bicarbonate solution (20 mL), and extracted with dichloromethane 3 times (50 mL × 3). The organic phases were combined, washed with saturated brine (100 mL), dried with anhydrous sodium sulfate, filtered, concentrated, and purified by silica gel column chromatography (dichloromethane/ethyl acetate = 1/1) to obtain the product A-2-8 (1.31 g, 5.05 mmol, yield 77.3%). MS (ESI) calculated for [M+H]⁺ was 260.2; found: m/z 260. ¹H NMR (400 MHz, CDCl3) δ 8.31 (d, *J = 7.2* Hz, 1H), 4.93 (dd, *J =* 9.2, 6.5 Hz, 1H), 4.41 (dd, *J* = 8.0, 5.0 Hz, 1H), 4.08 (t, *J* = 7.7 Hz, 2H), 3.73 (s, 3H), 2.71(ddd, *J =* 19.2, 13.4, 7.7 Hz, 1H), 2.41 (ddd, *J =* 16.3, 12.0, 7.8 Hz, 1H), 2.20 (td, *J =* 13.6, 6.8 Hz, 1H), 0.93 (dd, *J =* 8.3, 7.1 Hz, 6H).

### Preparation Example 10 Preparation of compound A-2-9

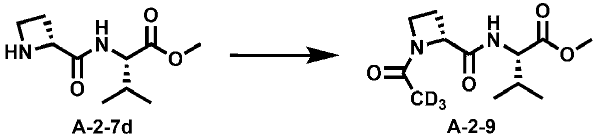

Intermediate A-2-7d (1.5 g, 7.0 mmol) was dissolved in dichloromethane (20 mL), and added with *N*,*N*-diisopropylethylamine (4.52 g, 35 mmol). After gas replacement was carried out under nitrogen protection three times, the reaction mixture was cooled to 0°C, and then dropwise added with deuterated acetyl chloride (630 mg, 7.7 mmol). The reaction solution was stirred at room temperature for 1 hour, then added with saturated aqueous sodium bicarbonate solution (20 mL), and extracted with dichloromethane (50 mL × 3). The organic phases were combined, dried with anhydrous sodium sulfate, filtered, concentrated, and purified by silica gel column chromatography (petroleum ether/ethyl acetate = 2: 1) to obtain the product A-2-9 (1.1 g, 4.24 mmol, yield 60.6%). MS (ESI) calculated for [M+H]⁺ was 260.2; found: m/z 260.1. ¹H NMR (400 MHz, CDCl3) δ 8.28 (d, *J =* 7.4 Hz, 1H), 4.91 (dd, *J =* 9.4, 6.6 Hz, 1H), 4.47 (dd, *J* = 8.4, 4.9 Hz, 1H), 4.12 - 4.03 (m, 2H), 3.72 (s, 3H), 2.70-2.60(m, 1H), 2.47 - 2.38 (m, 1H), 2.28 - 2.15 (m, 1H), 0.95 (dd, *J =* 16.2, 6.9 Hz, 6H).

### Preparation Example 11 Preparation of compound A-2-10

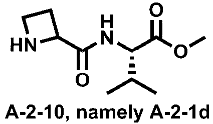

Preparation of compound A-2-10, which was intermediate A-2-1d, referred to Preparation Example 3.

### Preparation Example 12 Preparation of compound A-2-11

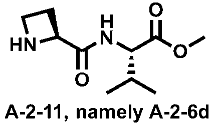

Preparation of compound A-2-11, which was intermediate A-2-6d, referred to Preparation Example 7.

### Preparation Example 13 Preparation of compound A-2-12

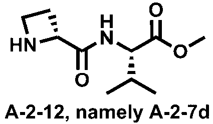

Preparation of compound A-2-12, which was intermediate A-2-7d, referred to Preparation Example 8.

### Preparation Example 14 Preparation of compound A-2-15

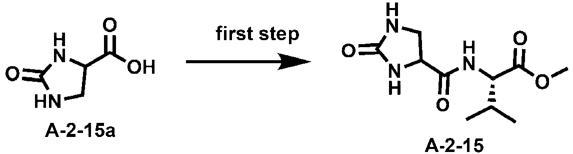

### First step

Compound A-2-15a (480 mg, 3.69 mmol) and *L*-valine methyl ester hydrochloride (680 mg, 4.06 mmol, 1.1 eq) were dissolved in anhydrous *N*,*N*-dimethylformamide (10 mL), sequentially added with 4-dimethylaminopyridine (900 mg, 7.38 mmol) and 1-(3-dimethylaminopropyl)-3-ethylcarbodiimide hydrochloride (780 mg, 4.06 mmol) at 0°C, and stirred at 0°C for 1 hour, and then the mixture was heated to room temperature. The reaction was stirred overnight. The reaction solution was quenched with a small amount of water, concentrated to remove N,N-dimethylformamide, then dissolved in water and acetonitrile, and purified by medium-pressure preparative chromatography to obtain compound A-2-15 (347 mg, 1.43 mmol, yield 38.6%). MS-ESI calculated value [M+H]⁺: 244.1; found: m/z 244.1.

### Preparation Example 15 Preparation of compound A-2-16

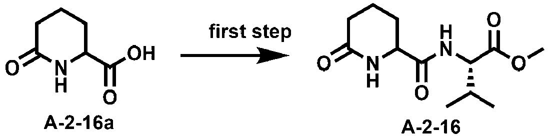

### First step

Compound A-2-16a (440 mg, 3.07 mmol) and *L*-valine methyl ester hydrochloride (567 mg, 3.38 mmol, 1.1 eq) were dissolved in anhydrous *N*,*N*-dimethylformamide (10 mL), sequentially added with 4-dimethylaminopyridine (750 mg, 6.14 mmol) and 1-(3-dimethylaminopropyl)-3-ethylcarbodiimide hydrochloride (650 mg, 3.38 mmol) at 0°C, and stirred at 0°C for 1 hour, and then the mixture was heated to room temperature. The reaction was stirred overnight. The reaction solution was quenched with a small amount of water, concentrated to remove *N*,*N*-dimethylformamide, then dissolved in water and acetonitrile, and purified by medium-pressure preparative chromatography to obtain compound A-2-16 (465 mg, 1.81 mmol, yield 59.0%). MS (ESI) calculated for [M+H]⁺ was: 257.2; found: m/z 257.0.

### Preparation Example 16 Preparation of compound A-2-17

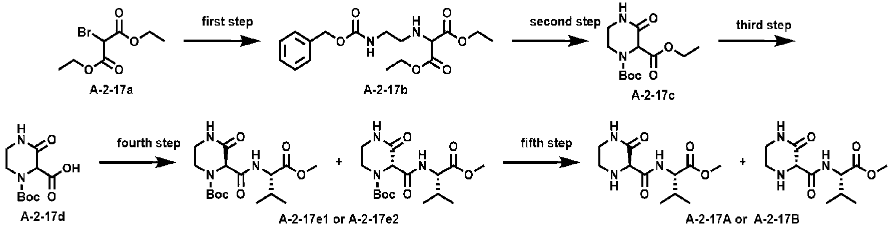

### First step

Compound A-2-17a (10.0 g, 41.9 mmol) was dissolved in *N*,*N*-dimethylformamide (80 mL), added with potassium carbonate (14.3 g, 103.47 mmol), and then added with benzyl(2-aminoethyl)carbamate (12.2 g, 62.9 mmol) at 0°C. The reaction of the reaction mixture was stirred at 100°C for 6 hours. After the reaction was completed, the reaction solution was added with water (50 mL), and then extracted with ethyl acetate (50 mL × 3). The organic phases were combined, washed with brine (100 mL × 3), dried with anhydrous sodium sulfate and concentrated. The resulting residue was separated and purified by silica gel column chromatography (petroleum ether/ethyl acetate = 3/1) to obtain compound A-2-17b (5.8 g, yield 39%). MS (ESI) calculated for [M+H]⁺ was: 353.17; found: m/z 353.16.

### Second step

Compound A-2-17b (5.8 g, 16.5 mmol) was dissolved in methanol (40 mL), and added with palladium on carbon (580 mg). The reaction of the reaction mixture was stirred under hydrogen atmosphere at 25°C for 12 hours. After the reaction was completed, the reaction solution was filtered to remove palladium on carbon, and the filtrate was added with di-tert-butyl dicarbonate (5.4 g, 24.8 mmol). The reaction was stirred at room temperature for 2 hours. After the reaction was completed, the reaction solution was concentrated, and the resulting residue was separated and purified by silica gel column chromatography (dichloromethane/methanol = 20/1) to obtain compound A-2-17c (3.0 g, yield 62%). MS (ESI) calculated for [M+H]⁺ was: 273.14; found: m/z 272.85.

### Third step

Compound A-2-17c (3.0 g, 11 mmol) was dissolved in methanol (30 mL), and added with lithium hydroxide (0.4 g, 16.5 mmol). The reaction mixture was stirred at room temperature for 2 hours. Subsequently, the reaction solution was lyophilized and recrystallized with methanol to obtain compound A-2-17d (2.6 g, yield 96.6%). MS (ESI) calculated for [M+H]⁺ was: 245.11; found: m/z 245.11.

### Fourth step

Compound A-2-17d (2.6 g, 10.6 mmol) was dissolved in N,N-dimethylformamide (30 mL), and added with *L*-valine methyl ester hydrochloride (3.54 g, 21.2 mmol), 1-hydroxybenzotriazole (2.86 g, 21.2 mmol), 1-(3-dimethylaminopropyl)-3-ethylcarbodiimide hydrochloride (4.05 g, 21.2 mmol) and *N*,*N*-diisopropylethylamine (4.12 g, 31.8 mmol). The reaction of the reaction mixture was stirred at room temperature for 4 hours. After the reaction was completed, the reaction solution was added with water (50 mL), and then extracted with ethyl acetate (50 mL × 3). The organic phases were combined, washed with brine (100 mL × 3), dried with anhydrous sodium sulfate, and concentrated to obtain a crude product. The crude product was separated and purified by silica gel column chromatography (dichloromethane/methanol = 15/1), and further purified by preparative HPLC to obtain two isomers of a target product, which were compound A-2-17e1 (300 mg) and compound A-2-17e2 (200 mg). MS (ESI) calculated for [M+H]⁺ was: 358.20; found: m/z 358.02.

### Fifth step

Compound A-2-17e1 (300 mg, 0.84 mmol) was dissolved in dichloromethane (5 mL), and added with trifluoroacetic acid (2 mL). The reaction mixture was stirred at room temperature for 2 hours. After the reaction was completed, the reaction solution was subjected to rotary evaporation to remove the solvent, and then lyophilized to obtain compound A-2-17A (155 mg). MS (ESI) calculated for [M+H]⁺ was: 258.14; found: m/z 258.05. ¹H NMR (400 MHz, DMSO-d6) δ 9.34 (s, 2H), 8.82-8.80 (m, 1H), 8.66-8.61 (m, 1H), 4.81-4.75 (m, 1H), 4.30-4.27(m, 1H), 3.68 - 3.66 (m, 3H), 3.42-3.32 (m, 4H), 2.13-2.07 (m, 1H), 0.91-0.89 (m, 6H).

Compound A-2-17 e2 (200 mg, 0.56 mmol) was dissolved in dichloromethane (5 mL), and added with trifluoroacetic acid (2 mL). The reaction mixture was stirred at room temperature for 2 hours. After the reaction was completed, the reaction solution was subjected to rotary evaporation to remove the solvent, and then purified by preparative HPLC to obtain compound A-2-17B (73 mg). MS (ESI) calculated for [M+H]⁺ was: 258.14; found: m/z 258.00. ¹H NMR (400 MHz, DMSO-d6) δ 9.34 (s, 1H), 8.81 (d, *J =* 8.4 Hz, 1H), 8.63 (d, *J =* 18.0 Hz, 1H), 4.781 (d, *J =* 26.4 Hz, 1H), 4.33-4.27 (m, 1H), 3.68-3.67 (m, 3H), 3.42-3.32 (m, 4H), 2.13-2.07 (m, 1H), 0.91-0.89 (m, 6H).

### Preparation Example 17 Preparation of compound A-2-20

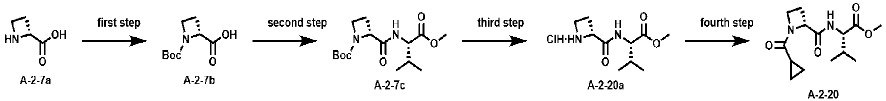

### First step

Compound A-2-7a (10.00 g, 98.91 mmol) was dissolved in ethanol (100 mL), then added with di-tert-butyl dicarbonate (43.17 g, 197.82 mmol), and stirred at room temperature. The mixture was slowly added with 4-dimethylaminopyridine (1.74 g, 14.27 mmol) in portions. Sodium hydroxide (3.96 g, 98.91 mmol) was dissolved in water (100 mL) and added to the above reaction system, and the mixture was stirred at room temperature. After 4 hours, completion of the reaction was monitored by TLC. The above reaction system was diluted with water (100 mL), and added with 1 M hydrochloric acid to adjust pH to 3. The reaction solution was extracted with ethyl acetate (200 mL × 3). The organic phases were combined, washed with saturated brine (200 mL), dried with anhydrous sodium sulfate, and concentrated to obtain a crude product - intermediate A-2-7b (22.10 g). MS (ESI) calculated for [M-H]⁻ was 200.1; found: m/z 200.1.

### Second step

At room temperature, intermediate A-2-7b (10.00 g, 49.70 mmol) and L-valine methyl ester hydrochloride (10.00 g, 59.64 mmol) were added to a single-necked flask, and dissolved by adding dichloromethane (150 mL). Subsequently, the mixture was sequentially added with N,N,N',N'-Tetramethyl-O-(7-azabenzotriazol-1-yl)uronium hexafluorophosphate (22.68 g, 59.64 mmol) and triethylamine (10.06 g, 99.40 mmol). After the mixture was stirred at room temperature for 16 hours, completion of the reaction was indicated by TLC. The reaction solution was diluted with water (200 mL) and extracted with ethyl acetate (200 mL × 3). The organic phases were combined, successively washed with water (500 mL × 3) and saturated brine (500 mL), dried with anhydrous sodium sulfate, concentrated, and purified by silica gel column chromatography (petroleum ether/ethyl acetate = 20/1 - 3/2) to obtain intermediate A-2-7c (13.84 g, 44.02 mmol, yield 88.6%).

### Third step

Intermediate A-2-7c (3.00 g, 9.54 mmol) was added to a single-necked flask, and then added with a solution of hydrogen chloride in ethyl acetate (3.0 M, 5 mL). The mixture was stirred at room temperature for 4 hours. After completion of the reaction (indicated by TLC), the reaction solution was concentrated to obtain intermediate A-2-20a (2.02 g, 8.07 mmol, yield 84.4%). MS (ESI) calculated for [M+H]⁺ was 215.1; found: m/z 215.3.

### Fourth step

Cyclopropylformic acid (0.12 g, 1.44 mmol), *N*,*N*,*N*,*N*-tetramethylchloroformamidinium hexafluorophosphate (0.67 g, 2.40 mmol), *N*-methylimidazole (0.20 g, 2.40 mmol) and triethylamine (0.20 g, 1.98 mmol) were added to a dry three-necked flask. The mixture was added with dichloromethane (5 mL), stirred to react for 0.5 hour, and then added with intermediate A-2-20a (0.30 g, 1.20 mmol). The reaction was stirred at room temperature for 2 hours. After the reaction was completed, the reaction solution was subjected to rotary evaporation to remove the solvent, then added with water (10 mL), and extracted with ethyl acetate (10 mL×3). The organic phases were combined, washed with saturated brine (10 mL × 2), dried with anhydrous sodium sulfate, filtered, concentrated, and purified by silica gel column chromatography (petroleum ether/ethyl acetate = 1/1) to obtain the product A-2-20 (135.3 mg, 0.48 mmol, yield 40.1%). MS (ESI) calculated for [M+H]⁺ was 283.2; found: m/z 283.3. ¹H NMR (400 MHz, Chloroform-d) δ 8.25 (d, *J =* 8.5 Hz, 1H), 4.91 (dd, *J =* 9.5, 6.5 Hz, 1H), 4.47 (dd, *J =* 8.2, 4.8 Hz, 1H), 4.20 (q, *J =* 6.5, 4.5 Hz, 2H), 3.72 (s, 3H), 2.67 (d, *J =* 9.6 Hz, 1H), 2.59 - 2.37 (m, 1H), 2.30 - 2.08 (m, 1H), 1.44 (tq, *J =* 11.2, 6.9, 5.7 Hz, 1H), 1.03 (dt, *J =* 11.7, 5.2 Hz, 2H), 0.93 (dd, *J =* 20.5, 6.9 Hz, 6H), 0.87 - 0.78 (m, 2H).

### Preparation Example 18 Preparation of compound A-2-21

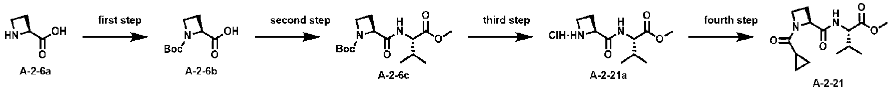

### First step

In a dry three-necked flask, compound A-2-6a (5.20 g, 51.43 mmol) was added to an aqueous sodium hydroxide solution (1 M, 50 mL) at 0°C, dissolved, and then added with ethanol (50 mL). The mixture was dropwise added with di-tert-butyl dicarbonate (13.47 g, 61.72 mmol) at this temperature, and stirred to react at room temperature overnight. After the reaction was completed, the reaction solution was added with a saturated citric acid solution to adjust pH to 4-5, and extracted with dichloromethane (100 mL × 3). The organic phases were combined, washed with a saturated sodium chloride solution (100 mL × 2), dried with anhydrous sodium sulfate, filtered, concentrated, and purified by silica gel column chromatography (petroleum ether/ethyl acetate = 1/1) to obtain intermediate A-2-6b (9.59 g, 47.66 mmol, yield 92.7%). MS (ESI) calculated for [M-H]⁻ was 200.1; found: m/z 200.1. ¹H NMR (400 MHz, Chloroform-d) δ 9.19 (s, 1H), 4.77 (s, 1H), 3.94 (q, *J =* 7.7 Hz, 2H), 2.48 (s, 2H), 1.46 (s, 9H).

### Second step

In a dry three-necked flask, N,N,N',N'-Tetramethyl-O-(7-azabenzotriazol-1-yl)uronium hexafluorophosphate (22.68 g, 59.64 mmol) and triethylamine (10.00 g, 99.40 mmol) were dissolved in dichloromethane (100 mL) at 0°C, and cooled to 0°C. The mixture was added with intermediate A-2-6b (9.59 g, 47.66 mmol), stirred for half an hour, and then added with *L*-valine methyl ester hydrochloride (7.82 g, 59.64 mmol), and the reaction was stirred at room temperature for 16 hours. After the reaction was completed, the reaction solution was added with water (100 mL) and extracted with ethyl acetate (100 mL × 3). The organic phases were combined, successively washed with saturated brine (100 mL × 2), dried with anhydrous sodium sulfate, filtered, concentrated, and purified by silica gel column chromatography (dichloromethane/methanol = 15/1) to obtain intermediate A-2-6c (11.50 g, 36.58 mmol, yield 76.8%). MS (ESI) calculated for [M-H]⁻ was: 313.2; found: m/z 313.0. ¹H NMR (400 MHz, DMSO-d6) δ 8.17 (d, *J =* 8.3 Hz, 1H), 4.72 - 4.50 (m, 1H), 4.22 (dd, *J =* 8.2, 6.1 Hz, 1H), 3.85 - 3.66 (m, 2H), 3.64 (s, 3H), 2.36 (s, 1H), 2.13 - 1.82 (m, 2H), 1.42 - 1.27 (m, 9H), 0.88 (d, *J =* 6.8 Hz, 6H).

### Third step

In a dry three-necked flask, intermediate A-2-6c (2.50 g, 7.95 mmol) was dissolved in 5 mL of dichloromethane solution at 0°C, and dropwise added with a solution of hydrogen chloride in dioxane (4 M, 20 mL). After the addition was completed, the mixture was continuously stirred at room temperature. After the reaction was completed, the reaction solution was concentrated to obtain intermediate A-2-21a (2.00 g, 7.95 mmol, yield 100%). MS (ESI) calculated for [M+H]⁺ was 215.1; found: m/z 215.3.

### Fourth step

Cyclopropylformic acid (0.15 g, 1.20 mmol) and 3-(ethylcarbamoylamino)-*N*,*N*-dimethylpropan-1-amine hydrochloride (0.23 g, 1.20 mmol), 1-hydroxybenzotriazole (0.16 g, 1.20 mmol) and triethylamine (0.20 g, 1.60 mmol) were added to a dry three-necked flask. The mixture was added with dichloromethane (5 mL), stirred to react for 0.5 hour, and then added with intermediate A-2-21a (0.25 g, 1.00 mmol). The reaction was stirred at room temperature for 2 hours. After the reaction was completed, the reaction solution was concentrated, added with water (10 mL), and extracted with ethyl acetate (10 mL×3). The organic phases were combined, washed with saturated brine (10 mL × 2), dried with anhydrous sodium sulfate, filtered, concentrated, and purified by silica gel column chromatography (petroleum ether/ethyl acetate = 1/1) to obtain the product A-2-21 (99.1 mg, 0.35 mmol, yield 35.2%). MS (ESI) calculated for [M+H]⁺ was 283.2; found: m/z 283.3. ¹H NMR (400 MHz, Chloroform-d) δ 8.42 (d, *J =* 8.2 Hz, 1H), 4.93 (dd, *J =* 9.4, 6.4 Hz, 1H), 4.40 (dd, *J =* 8.2, 5.0 Hz, 1H), 4.20 (ddd, *J =* 12.0, 8.9, 4.8 Hz, 2H), 3.63 (s, 3H), 2.74 (ddd, *J =* 12.5, 9.2, 6.2 Hz, 1H), 2.44 (dtd, *J =* 11.9, 9.2, 5.5 Hz, 1H), 2.18 (td, *J =* 7.2, 5.3 Hz, 1H), 1.42 (td, *J =* 8.0, 4.1 Hz, 1H), 1.01 (q, *J =* 3.8 Hz, 2H), 0.90 (dd, *J =* 16.3, 6.9 Hz, 6H), 0.82 (dtd, *J* = 9.3, 5.9, 2.7 Hz, 2H).

### Preparation Example 19 Preparation of compound A-2-22

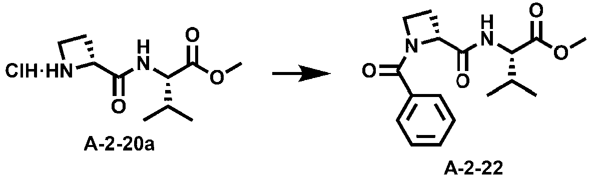

Benzoic acid (146.5 mg, 1.20 mmol), 1-ethyl-(3-dimethylaminopropyl)carbodiimide hydrochloride (299.0 mg, 1.56 mmol) and 1-hydroxybenzotriazole (210.8 mg, 1.56 mmol) were added to a single-necked flask, dissolved by adding dichloromethane (5 mL), and stirred at 0°C for 0.5 hour. Subsequently, the mixture was sequentially added with intermediate A-2-20a (300.0 mg, 1.20 mmol) and diisopropylethylamine (465.3 mg, 3.60 mmol), and stirred at room temperature overnight, and the reaction progress was monitored by TLC. After completion of the reaction (indicated by TLC), the reaction solution was quenched by the addition of water (10 mL), extracted and subjected to liquid separation. Subsequently, the aqueous phase was further extracted with dichloromethane (10 mL × 2), and the organic phases were collected, washed with saturated sodium chloride solution (20 mL), dried with anhydrous sodium sulfate, and concentrated to obtain a crude product. The crude product was purified by silica gel column chromatography (EA: PE = 10%-60%) to obtain the product A-2-22 (227.0 mg, 0.71 mmol, yield 59.59%). MS (ESI) calculated for [M+H]⁺ was 319.2; found: m/z 319.3. ¹H NMR (400 MHz, Chloroform-d) δ 7.61-7.57 (m, 2H), 7.46-7.41 (m, 1H), 7.37 (dd, *J =* 8.1, 6.5 Hz, 2H), 5.09-4.98 (m, 1H), 4.47 (dd, *J =* 8.6, 4.9 Hz, 1H), 4.30 (td, *J =* 8.9, 5.6 Hz, 1H), 4.06 (q, *J =* 8.1 Hz, 1H), 3.64 (s, 3H), 2.69 (s, 1H), 2.43 (s, 1H), 2.23-2.09 (m, 1H), 0.89 (dd, *J =* 21.6, 6.9 Hz, 6H).

### Preparation Example 20 Preparation of compound A-2-23

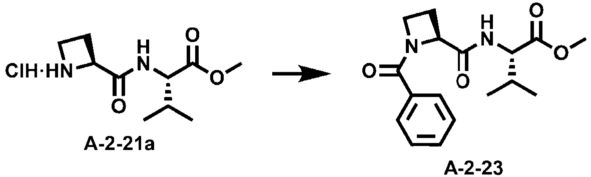

Benzoic acid (0.19 g, 1.54 mmol) and 3-(ethylcarbamoylamino)-*N*,*N*-dimethylpropan-1-amine hydrochloride (0.29 g, 1.54 mmol), 1-hydroxybenzotriazole (0.21 g, 1.54 mmol) and triethylamine (0.26 g, 2.56 mmol) were added to a dry three-necked flask. The mixture was added with dichloromethane (5 mL), stirred to react for 0.5 hour, and then added with intermediate A-2-21a (0.32 g, 1.28 mmol). The reaction was stirred at room temperature for 2 hours. After the reaction was completed, the reaction solution was concentrated, added with water (10 mL), and extracted with ethyl acetate (10 mL×3). The organic phases were combined, washed with saturated brine (10 mL × 2), dried with anhydrous sodium sulfate, filtered, concentrated, and purified by silica gel column chromatography (petroleum ether/ethyl acetate = 1/1) to obtain the product A-2-23 (69.0 mg, 0.22 mmol, yield 17.0%). MS (ESI) calculated for [M+H]⁺ was 319.2; found: m/z 319.3. ¹H NMR (400 MHz, Chloroform-d) δ 8.09-7.85 (m, 1H), 7.65-7.60 (m, 2H), 7.50 (t, *J* = 7.2 Hz, 1H), 7.43 (t, *J* = 7.4 Hz, 2H), 5.12 (t, *J* = 8.1 Hz, 1H), 4.48 (dd, *J =* 8.4, 5.0 Hz, 1H), 4.36 (td, *J =* 8.9, 5.6 Hz, 1H), 4.14 (q, *J =* 8.2 Hz, 1H), 3.74 (s, 3H), 2.84 (s, 1H), 2.44 (s, 1H), 2.29 - 2.09 (m, 1H), 0.92 (dd, *J =* 12.3, 6.9 Hz, 6H).

### Preparation Example 21 Preparation of compound A-2-24

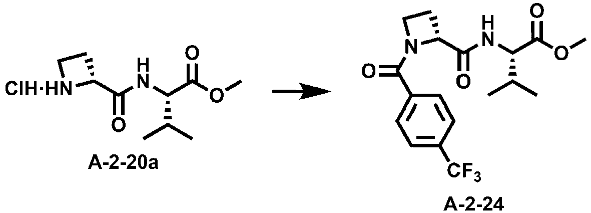

4-(trifluoromethyl)benzoic acid (228.1 mg, 1.20 mmol), 1-ethyl-(3-dimethylaminopropyl)carbodiimide hydrochloride (0.30 g, 1.56 mmol) and 1-hydroxybenzotriazole (0.21 g, 1.56 mmol) were added to a single-necked flask, dissolved by adding dichloromethane (5 mL), and stirred at 0°C for 0.5 hour. Subsequently, the mixture was sequentially added with intermediate A-2-20a (300.0 mg, 1.20 mmol) and diisopropylethylamine (0.47 g, 3.60 mmol), and stirred at room temperature overnight, and the reaction progress was monitored by TLC. After completion of the reaction (indicated by TLC), the reaction solution was quenched by the addition of water (10 mL), extracted and subjected to liquid separation. Subsequently, the aqueous phase was further extracted with dichloromethane (10 mL × 2), and the organic phases were collected, washed with saturated sodium chloride solution (20 mL), dried with anhydrous sodium sulfate, and concentrated to obtain a crude product. The crude product was purified by silica gel column chromatography (EA: PE = 10%-60%) to obtain the product A-2-24 (218.0 mg, 0.56 mmol, yield 47.15%). MS (ESI) calculated for [M+H]⁺ was 387.1; found: m/z 387.4. ¹H NMR (400 MHz, Chloroform-d) δ 7.86 (d, *J =* 7.2 Hz, 1H), 7.77 (d, *J =* 8.2 Hz, 2H), 7.70 (d, *J =* 8.2 Hz, 2H), 5.16 - 5.07 (m, 1H), 4.54 (dd, *J =* 8.6, 4.8 Hz, 1H), 4.36 (td, *J =* 8.9, 5.7 Hz, 1H), 4.12 (q, *J =* 8.2 Hz, 1H), 3.70 (s, 3H), 2.79 (s, 1H), 2.61 - 2.45 (m, 1H), 2.23 (q, *J =* 6.6 Hz, 1H), 0.96 (dd, *J =* 23.5, 6.9 Hz, 6H).

### Preparation Example 22 Preparation of compound A-2-25

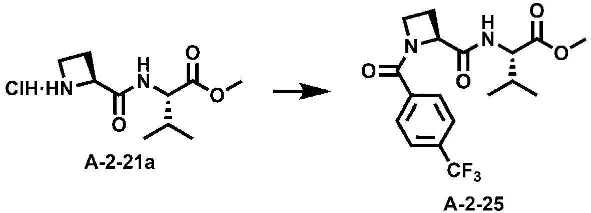

4-(trifluoromethyl)benzoic acid (0.18 g, 0.96 mmol) and 3-(ethylcarbamoylamino)-*N*,*N*-dimethylpropan-1-amine hydrochloride (0.18 g, 0.96 mmol), 1-hydroxybenzotriazole (0.13 g, 0.96 mmol) and triethylamine (0.16 g, 1.60 mmol) were added to a dry three-necked flask. The mixture was added with dichloromethane (5 mL), stirred to react for 0.5 hour, and then added with intermediate A-2-21a (0.20 g, 0.80 mmol). The reaction was stirred at room temperature for 2 hours, and the reaction solution was concentrated, added with water (10 mL), and extracted with ethyl acetate (10 mL×3). The organic phases were combined, washed with saturated brine (10 mL × 2), dried with anhydrous sodium sulfate, filtered, concentrated, and purified by silica gel column chromatography (petroleum ether/ethyl acetate = 1/1) to obtain the product A-2-25 (130.0 mg, 0.34 mmol, yield 42.2%). MS (ESI) calculated for [M+H]⁺ was 387.2; found: m/z 387.4. ¹H NMR(400 MHz, Chloroform-d) δ 7.88 (d, *J =* 8.4 Hz, 1H), 7.73 (q, *J =* 8.3 Hz, 4H), 5.13 (dd, *J =* 9.4, 6.4 Hz, 1H), 4.49 (dd, *J =* 8.4, 4.9 Hz, 1H), 4.37 (td, *J =* 9.0, 5.6 Hz, 1H), 4.22 - 4.03 (m, 1H), 3.75 (s, 3H), 2.85 (d, *J =* 16.4 Hz, 1H), 2.48 (d, *J =* 10.0 Hz, 1H), 2.21 (q, *J =* 6.6 Hz, 1H), 0.92 (dd, *J =* 13.2, 6.9 Hz, 6H).

### Preparation Example 23 Preparation of compound A-2-26

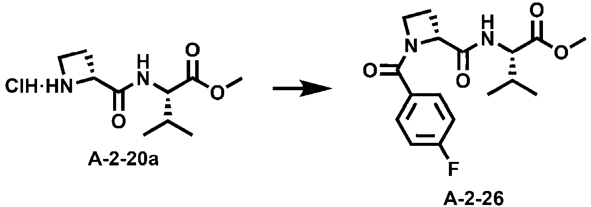

Parafluorobenzoic acid (168.1 mg, 1.20 mmol), 1-ethyl-(3-dimethylaminopropyl)carbodiimide hydrochloride (300 mg, 1.56 mmol) and 1-hydroxybenzotriazole (0.21 g, 1.56 mmol) were added to a single-necked flask, dissolved by adding dichloromethane (5 mL), and stirred at 0°C for 0.5 hour. Subsequently, the mixture was sequentially added with intermediate A-2-20a (300.0 mg, 1.20 mmol) and diisopropylethylamine (0.47 g, 3.60 mmol), and stirred at room temperature overnight, and the reaction progress was monitored by TLC. After completion of the reaction (indicated by TLC), the reaction solution was quenched by the addition of water (10 mL), extracted and subjected to liquid separation. Subsequently, the aqueous phase was further extracted with dichloromethane (10 mL × 2), and the organic phases were collected, washed with saturated sodium chloride solution (20 mL), dried with anhydrous sodium sulfate, and concentrated to obtain a crude product. The crude product was purified by silica gel column chromatography (EA: PE =10%-60%) to obtain the product A-2-26 (270.0 mg, 0.88 mmol, yield 59.34%). MS (ESI) calculated for [M+H]⁺ was 337.2; found: m/z 337.4. ¹H NMR (400 MHz, Chloroform-d) δ 7.85 (s, 1H), 7.62 (dd, *J =* 8.6, 5.3 Hz, 2H), 7.05 (t, *J =* 8.5 Hz, 2H), 5.08 - 4.99 (m, 1H), 4.47 (dd, *J* = 8.6, 4.9 Hz, 1H), 4.30 (td, *J* = 8.8, 5.5 Hz, 1H), 4.14 - 4.02 (m, 1H), 3.64 (s, 3H), 2.70 (s, 1H), 2.44 (s, 1H), 2.15 (dq, *J =* 13.9, 6.7 Hz, 1H), 0.89 (dd, *J =* 21.8, 6.9 Hz, 6H).

### Preparation Example 24 Preparation of compound A-2-27

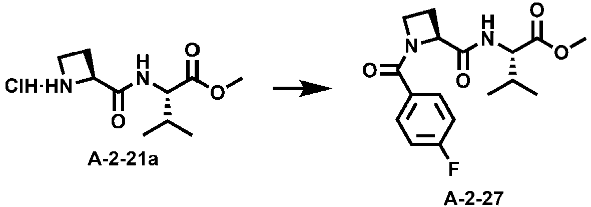

4-fluorobenzoic acid (0.13 g, 0.96 mmol) and 3-(ethylcarbamoylamino)-*N*,*N*-dimethylpropan-1-amine hydrochloride (0.18 g, 0.96 mmol), 1-hydroxybenzotriazole (0.13 g, 0.96 mmol) and triethylamine (0.16 g, 1.60 mmol) were added to a dry three-necked flask. The mixture was added with dichloromethane (5 mL), stirred to react for 0.5 hour, and then added with intermediate A-2-21a (0.20 g, 0.80 mmol). The reaction was stirred at room temperature for 2 hours, and the reaction solution was concentrated, added with water (10 mL), and extracted with ethyl acetate (10 mL×3). The organic phases were combined, washed with saturated brine (10 mL × 2), dried with anhydrous sodium sulfate, filtered, concentrated, and purified by silica gel column chromatography (petroleum ether/ethyl acetate = 1/1) to obtain the product A-2-27 (99.4 mg, 0.28 mmol, yield 35.3%). MS (ESI) calculated for [M+H]⁺ was 337.2; found: m/z 337.4. ¹H NMR (400 MHz, Chloroform-d) δ 7.93 (s, 1H), 7.75 - 7.62 (m, 2H), 7.12 (t, *J =* 8.6 Hz, 2H), 5.11 (s, 1H), 4.47 (dd, *J =* 8.4, 5.0 Hz, 1H), 4.42 - 4.29 (m, 1H), 4.15 (d, *J =* 7.9 Hz, 1H),3.74 (s, 3H), 2.84 (s, 1H), 2.44 (s, 1H), 2.20 (pd, *J =* 7.0, 5.1 Hz, 1H), 0.91 (dd, *J =* 11.7, 6.9 Hz, 6H).

### Preparation Example 25 Preparation of compound A-2-28

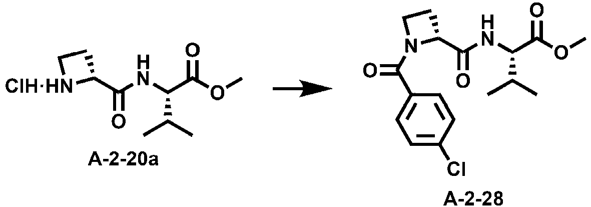

Parachlorobenzoic acid (187.9 mg, 1.20 mmol), 1-ethyl-(3-dimethylaminopropyl)carbodiimide hydrochloride (0.30 g, 1.56 mmol) and 1-hydroxybenzotriazole (0.21 g, 1.56 mmol) were added to a single-necked flask, dissolved by adding dichloromethane (5 mL), and stirred at 0°C for 0.5 hour. Subsequently, the mixture was sequentially added with intermediate A-2-20a (300.0 mg, 1.20 mmol) and diisopropylethylamine (0.47 g, 3.60 mmol), and stirred at room temperature overnight, and the reaction progress was monitored by TLC. After completion of the reaction (indicated by TLC), the reaction solution was quenched by the addition of water (10 mL), extracted and subjected to liquid separation. Subsequently, the aqueous phase was further extracted with dichloromethane (10 mL × 2), and the organic phases were collected, washed with saturated sodium chloride solution (20 mL), dried with anhydrous sodium sulfate, and concentrated to obtain a crude product. The crude product was purified by silica gel column chromatography (EA: PE = 10%-60%) to obtain the product A-2-28 (218.0 mg, 0.56 mmol, yield 47.15%). MS (ESI) calculated for [M+H]⁺ was 353.1; found: m/z 353.4. ¹H NMR (400 MHz, Chloroform-d) δ 7.83 (s, 1H), 7.58 (d, *J =* 8.3 Hz, 2H), 7.39 (d, *J =* 8.2 Hz, 2H), 5.11 - 5.02 (m, 1H), 4.50 (dd, *J =* 8.6, 4.9 Hz, 1H), 4.33 (td, *J =* 8.9, 5.6 Hz, 1H), 4.11 (q, *J =* 7.5, 6.5 Hz, 1H), 3.68 (s, 3H), 2.74 (s, 1H), 2.48 (s, 1H), 2.19 (h, *J =* 6.8 Hz, 1H), 0.93 (dd, *J =* 22.1, 6.9 Hz, 6H).

### Preparation Example 26 Preparation of compound A-2-29

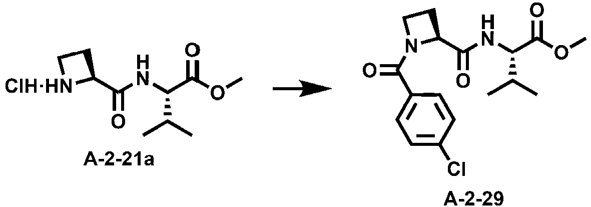

4-chlorobenzoic acid (0.30 g, 1.92 mmol) and 3-(ethylcarbamoylamino)-*N*,*N*-dimethylpropan-1-amine hydrochloride (0.37 g, 1.92 mmol), 1-hydroxybenzotriazole (0.26 g, 1.92 mmol) and triethylamine (0.32 g, 3.20 mmol) were added to a dry three-necked flask. The mixture was added with dichloromethane (5 mL), stirred to react for 0.5 hour, and then added with intermediate A-2-21a (0.40 g, 1.60 mmol). The reaction was stirred at room temperature for 2 hours, and the reaction solution was concentrated, added with water (10 mL), and extracted with ethyl acetate (10 mL× 3). The organic phases were combined, washed with saturated brine (10 mL × 2), dried with anhydrous sodium sulfate, filtered, concentrated, and purified by silica gel column chromatography (petroleum ether/ethyl acetate = 1/1) to obtain the product A-2-29 (64.6 mg, 0.18 mmol, yield 11.5%). MS (ESI) calculated for [M+H]⁺ was 353.1; found: m/z 353.3. ¹H NMR (400 MHz, Chloroform-d) δ 7.91 (d, *J =* 8.3 Hz, 1H), 7.77 - 7.51 (m, 2H), 7.41 (d, *J =* 8.3 Hz, 2H), 5.10 (t, *J =* 8.0 Hz, 1H), 4.47 (dd, *J =* 8.3, 5.0 Hz, 1H), 4.35 (q, *J =* 8.9, 8.4 Hz, 1H), 4.15 (t, *J =* 8.1 Hz, 1H),3.74 (s, 3H), 2.84 (s, 1H), 2.44 (s, 1H), 2.29 - 2.05 (m, 1H), 0.91 (dd, *J =* 12.0, 6.8 Hz, 6H).

### Preparation Example 27 Preparation of compound A-2-30

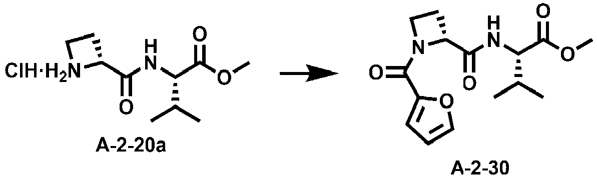

Furan-2-carboxylic acid (165.9 mg, 1.48 mmol), 1-ethyl-(3-dimethylaminopropyl)carbodiimide hydrochloride (0.37 g, 1.92 mmol) and 1-hydroxybenzotriazole (0.26 g, 1.92 mmol) were added to a single-necked flask, dissolved by adding dichloromethane (5 mL), and stirred at 0°C for 0.5 hour. Subsequently, the mixture was sequentially added with compound - intermediate A-2-20a (370.0 mg, 1.48 mmol) and diisopropylethylamine (0.57 g, 4.44 mmol), and stirred at room temperature overnight, and the reaction progress was monitored by TLC. After completion of the reaction (indicated by TLC), the reaction solution was quenched by the addition of water (10 mL), extracted and subjected to liquid separation. Subsequently, the aqueous phase was further extracted with dichloromethane (10 mL × 2), and the organic phases were collected, washed with saturated sodium chloride solution (20 mL), dried with anhydrous sodium sulfate, and concentrated to obtain a crude product. The crude product was purified by silica gel column chromatography (EA: PE = 10%-60%) to obtain the compound A-2-30 (270.0 mg, 0.88 mmol, yield 59.34%). MS (ESI) calculated for [M+H]⁺ was 309.1; found: m/z 309.3. ¹H NMR (400 MHz, Chloroform-d) δ 8.09 (s, 1H), 7.46 (s, 1H), 7.08 (d, *J =* 3.5 Hz, 1H), 6.45 (dd, *J =* 3.5*,* 1.7 Hz, 1H), 5.03 (t, *J =* 8.4 Hz, 1H), 4.44 (dd, *J =* 14.4, 7.2 Hz, 3H), 3.64 (s, 3H), 2.70 (s, 1H), 2.49 (s, 1H), 2.24 - 2.10 (m, 1H), 1.09 - 0.84 (m, 6H).

### Preparation Example 28 Preparation of compound A-2-31

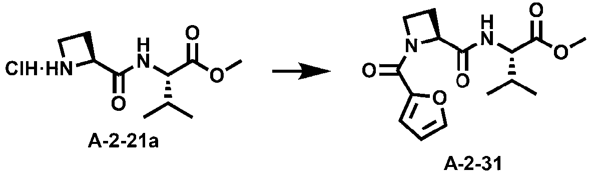

Furan-2-carboxylic acid (0.13 g, 1.20 mmol) and 3-(ethylcarbamoylamino)-*N*,*N*-dimethylpropan-1-amine hydrochloride (0.23 g, 1.20 mmol), 1-hydroxybenzotriazole (0.16 g, 1.20 mmol) and triethylamine (0.20 g, 1.60 mmol) were added to a dry three-necked flask. The mixture was added with dichloromethane (5 mL), stirred to react for 0.5 hour, and then added with intermediate A-2-21a (0.25 g, 1.00 mmol). The reaction was stirred at room temperature for 2 hours, and then the reaction solution was concentrated, added with water (10 mL), and extracted with ethyl acetate (10 mL×3). The organic phases were combined, washed with saturated brine (10 mL × 2), dried with anhydrous sodium sulfate, filtered, concentrated, and purified by silica gel column chromatography (petroleum ether/ethyl acetate = 1/1) to obtain the product A-2-31 (182.2 mg, 0.57 mmol, yield 57.2%). MS (ESI) calculated for [M+H]⁺ was 309.1; found: m/z 309.3. ¹H NMR (400 MHz, Chloroform-d) δ 8.25 (s, 1H), 7.53 (s, 1H), 7.14 (d, *J =* 3.5 Hz, 1H), 6.51 (dd, *J =* 3.6, 1.8 Hz, 1H), 5.21 - 4.93 (m, 1H), 4.47 (d, *J =* 13.1 Hz, 3H), 3.73 (s, 3H), 2.84 (s, 1H), 2.50 (s, 1H), 2.19 (td, *J =* 6.9, 5.1 Hz, 1H), 0.92 (dd, *J =* 8.1, 6.8 Hz, 6H).

### Preparation Example 29 Preparation of compound A-2-32

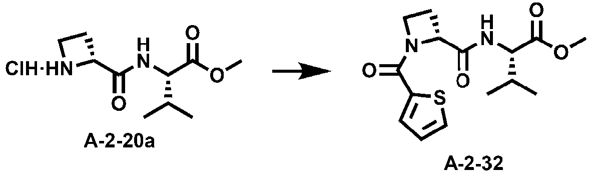

Thiophene-2-carboxylic acid (306.0 mg, 2.39 mmol) was added to a single-necked flask, and dissolved by adding dichloromethane (10 mL). Subsequently, the mixture was sequentially added with compound - intermediate A-2-20a (500.0 mg, 1.99 mmol), 2-(7-azobenzotriazole)-tetramethyluronium hexafluorophosphate (500.0 mg, 2.59 mmol) and triethylamine (402 mg, 3.98 mmol), and stirred at room temperature overnight, and the reaction progress was monitored by TLC. After completion of the reaction (indicated by TLC), the reaction solution was quenched by the addition of water (10 mL), extracted and subjected to liquid separation. Subsequently, the aqueous phase was further extracted with dichloromethane (10 mL × 2), and the organic phases were collected, washed with saturated sodium chloride solution (20 mL), dried with anhydrous sodium sulfate, and concentrated to obtain a crude product. The crude product was purified by silica gel column chromatography (EA: PE = 10%-60%) to obtain the product A-2-32 (302.2 mg, 0.95 mmol, yield 46.7%). MS (ESI) calculated for [M+H]⁺ was 325.1; found: m/z 325.3. ¹H NMR (400 MHz, Chloroform-d) δ 8.09 (s, 1H), 7.52 (dd, *J =* 7.5, 4.2 Hz, 2H), 7.16 - 6.98 (m, 1H), 5.08 (dd, *J =* 9.4, 6.5 Hz, 1H), 4.54 - 4.24 (m, 3H), 3.66 (s, 3H), 2.78 (s, 1H), 2.57 (d, *J =* 27.1 Hz, 1H), 2.17 (dp, *J =* 13.5, 6.8 Hz, 1H), 0.92 (dd, *J =* 17.5, 6.6 Hz, 6H).

### Preparation Example 30 Synthesis of compound A-2-33

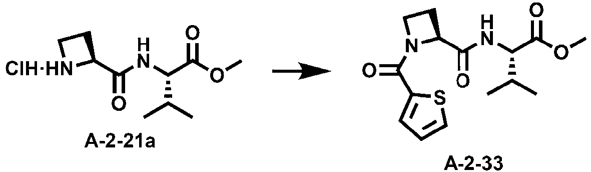

2-thiophenecarboxylic acid (0.15 g, 1.20 mmol)) and 3-(ethylcarbamoylamino)-*N*,*N*-dimethylpropan-1-amine hydrochloride (0.23 g, 1.20 mmol), 1-hydroxybenzotriazole (0.16 g, 1.20 mmol) and triethylamine (0.20 g, 1.60 mmol) were added to a dry three-necked flask. The mixture was added with dichloromethane (5 mL), stirred to react for 0.5 hour, and then added with intermediate A-2-21a (0.25 g, 1.00 mmol). The reaction was stirred at room temperature for 2 hours, and then the reaction solution was concentrated, added with water (10 mL), and extracted with ethyl acetate (10 mL×3). The organic phases were combined, washed with saturated brine (10 mL × 2), dried with anhydrous sodium sulfate, filtered, concentrated, and purified by silica gel column chromatography (petroleum ether/ethyl acetate = 1/1) to obtain the product A-2-33 (77.2 mg, 0.24 mmol, yield 23.9%). MS (ESI) calculated for [M+H]⁺ was 325.1; found: m/z 325.2. ¹H NMR (400 MHz, Chloroform-d) δ 8.16 (s, 1H), 7.75 - 7.37 (m, 2H), 7.12 (dd, *J =* 5.0, 3.8 Hz, 1H), 5.13 (dd, *J* = 9.4, 6.3 Hz, 1H), 4.41 (dtd, *J* = 17.3, 8.4, 5.9 Hz, 3H), 3.73 (s, 3H), 2.86 (s, 1H), 2.51 (s, 1H), 2.20 (pd, *J* = 6.9, 5.1 Hz, 1H), 0.92 (t, *J =* 6.7 Hz, 6H).

### Preparation Example 31 Preparation of compound A-2-34

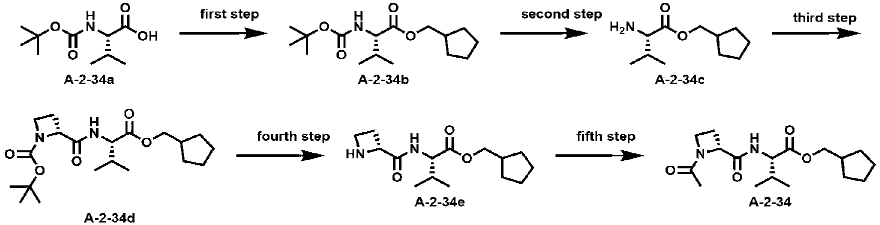

### First step

A compound A-2-34a (3 g, 13.81 mmol) and dichloromethane (50 mL) were added to a dry single-necked flask, and stirred under ice bath for 10 minutes. Subsequently, the mixture was sequentially added with cyclopentylmethanol (2.07 g, 20.71 mmol), 4-dimethylaminopyridine (1.69 g, 13.81 mmol) and N,N'-dicyclohexylcarbodiimide (4.27 g, 20.71 mmol). The reaction was stirred at room temperature for 12 hours, and completion of the reaction was indicated by TLC. The reaction solution was added with water (50 ml) and extracted with dichloromethane (50 ml× 3). The organic phases were combined, washed with saturated brine (20 ml), dried with anhydrous sodium sulfate, concentrated, and purified by silica gel column chromatography to obtain intermediate A-2-34b (3.2 g, 10.68 mmol, yield 77.4%). ¹H NMR (400 MHz, CDCl3) δ 5.07 (d, *J =* 8.8 Hz, 1H), 4.25 (dd, *J =* 9.1, 4.6 Hz, 1H), 4.04 (qd, *J =* 10.6, 7.2 Hz, 2H), 2.25 (dd, *J =* 15.1, 7.5 Hz, 1H), 2.20 - 2.09 (m, 1H), 1.78 (dt, *J =* 7.7, 2.9 Hz, 2H), 1.70 - 1.53 (m, 4H), 1.47 (s, 9H), 1.33 - 1.20 (m, 2H), 1.02 - 0.97 (m, 3H), 0.92 (d, *J =* 6.9 Hz, 3H).

### Second step

Intermediate A-2-34b (3.2 g, 10.68 mmol) and dichloromethane (20 mL) were added to a dry single-necked flask. Subsequently, the mixture was added with 4 M solution of hydrogen chloride in dioxane (20 mL), and stirred at room temperature for 2 hours. After completion of the reaction (indicated by TLC), the reaction solution was directly concentrated to obtain a crude product - intermediate A-2-34c, which was directly used in the next step.

### Third step

The crude product - intermediate A-2-34c and intermediate A-2-7b (1.98 g, 9.94 mmol) were dissolved in dichloromethane (40 mL). Under ice bath, the mixture was added with N,N-diisopropylethylamine (3.85 g, 29.82 mmol), *N*-hydroxy-7-azabenzotriazole (1.35 *g,* 9.94 mmol) and 1-(3-dimethylaminopropyl)-3-ethylcarbodiimide hydrochloride (1.91 g, 9.94 mmol). The mixture was continuously stirred under ice bath for 1 hour, and then placed at room temperature overnight. After completion of the reaction (monitored by TLC), the reaction solution was added with water (50 ml) and extracted with dichloromethane (50 ml× 3). The organic phases were combined, washed with saturated sodium chloride solution (100 mL), dried with anhydrous sodium sulfate, concentrated, and purified by silica gel column chromatography to obtain intermediate A-2-34d (2.4 g, 6.27 mmol, total yield of two steps 58.7%). ¹H NMR (400 MHz, CDCl3) δ 4.79 - 4.64 (m, 1H), 4.59 (dd, *J =* 8.9, 4.6 Hz, 1H), 4.17 - 4.02 (m, 2H), 4.01 - 3.92 (m, 1H), 3.85 (dd, *J =* 14.0, 8.5 Hz, 1H), 2.51 (d, *J =* 6.2 Hz, 1H), 2.40 (s, 1H), 2.31 - 2.19 (m, 2H), 1.79 (d, *J =* 10.4 Hz, 2H), 1.61 (ddd, *J =* 26.9, 13.8, 6.0 Hz, 4H), 1.49 (s, 9H), 1.36 - 1.25 (m, 2H), 1.00 (d, *J =* 6.8 Hz, 3H), 0.95 (d, *J =* 6.9 Hz, 3H).

### Fourth step

Intermediate A-2-34d (1.2 g, 3.13 mmol), dichloromethane (5 mL) and 4 M solution of hydrogen chloride in dioxane (10 mL) were added to a dry single-necked flask, and the reaction was stirred at room temperature for 2 hours. After completion of the reaction (indicated by TLC), the reaction solution was concentrated to obtain a crude product - intermediate A-2-34e.

### Fifth step

The crude product - intermediate A-2-34e was dissolved in tetrahydrofuran (20 mL), and added with N,N-diisopropylethylamine (1.22 g, 9.42 mmol). Under ice bath, the mixture was dropwise added with acetic anhydride (0.64 g, 6.28 mmol), and the reaction was continuously stirred for 30 minutes, and then the reaction mixture was placed at room temperature to continuously react for 2 hours. After completion of the reaction (monitored by TLC), the reaction solution was added with ethanol (5 mL), concentrated, and purified by silica gel column chromatography to obtain the product A-2-34 (0.7 g, 2.16 mmol, yield 68.8%). MS (ESI) calculated for [M+H]⁺ was 325.2; found: m/z 325.2. ¹H NMR (400 MHz, CDCl3) δ 8.20 (d, *J* = 8.4 Hz, 1H), 4.85 (dd, *J =* 9.5, 6.6 Hz, 1H), 4.42 (dd, *J =* 8.6, 4.7 Hz, 1H), 4.00 (t, *J* = 6.4 Hz, 2H), 3.98 - 3.87 (m, 2H), 2.67 - 2.51 (m, 1H), 2.44 - 2.28 (m, 1H), 2.20 (d, *J =* 4.8 Hz, 2H), 1.87 (s, 3H), 1.68 (dd, *J =* 12.1, 5.0 Hz, 2H), 1.60 - 1.44 (m, 4H), 1.23 - 1.14 (m, 2H), 0.92 (d, *J =* 6.9 Hz, 3H), 0.87 (d, *J =* 6.9 Hz, 3H).

### Preparation Example 32 Preparation of compound A-2-35

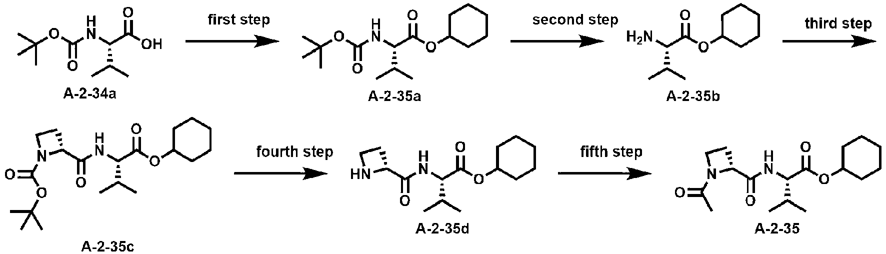

### First step

Compound A-2-34a (3 g, 13.81 mmol) and dichloromethane (50 mL) were added to a dry single-necked flask, and stirred under ice bath for 10 minutes. Subsequently, the mixture was sequentially added with cyclohexanol (2.07 g, 20.71 mmol), 4-dimethylaminopyridine (1.69 g, 13.81 mmol) and N,N'-dicyclohexylcarbodiimide (4.27 g, 20.71 mmol). The reaction was continuously stirred at room temperature for 12 hours. After completion of the reaction (indicated by TLC), the reaction solution was added with water (50 ml) and extracted with dichloromethane (50 ml× 3). The organic phases were combined, washed with saturated sodium chloride solution (100 mL), dried with anhydrous sodium sulfate, and purified by silica gel column chromatography to obtain intermediate A-2-35a (3.5 g, 11.68 mmol, yield 84.66%). ¹H NMR (400 MHz, CDCl3) δ 5.08 (d, *J =* 8.8 Hz, 1H), 4.93 - 4.79 (m, 1H), 4.23 (dd, *J =* 9.0, 4.4 Hz, 1H), 2.18 (dd, *J =* 11.6, 6.7 Hz, 1H), 1.93 - 1.83 (m, 2H), 1.76 (dd, *J =* 7.4, 4.3 Hz, 2H), 1.57 (dd, *J =* 8.4, 3.6 Hz, 2H), 1.48 (s, 9H), 1.46 - 1.28 (m, 4H), 1.00 (d, *J =* 6.9 Hz, 3H), 0.92 (d, *J =* 6.9 Hz, 3H).

### Second step

Intermediate A-2-35a (3.5 g, 11.68 mmol), dichloromethane (20 mL) and 4 M solution of hydrogen chloride in dioxane (20 mL) were added to a dry single-necked flask. The reaction was stirred at room temperature for 2 hours. After completion of the reaction (indicated by TLC), the reaction solution was concentrated to obtain a crude product - intermediate A-2-35b.

### Third step

The crude product - intermediate A-2-35b and intermediate A-2-7b (1.98 g, 9.94 mmol) were dissolved in dichloromethane (40 mL). Under ice bath, the mixture was added with *N,N*-diisopropylethylamine (3.85 g, 29.82 mmol), *N*-hydroxy-7-azabenzotriazole (1.35 *g,* 9.94 mmol) and 1-(3-dimethylaminopropyl)-3-ethylcarbodiimide hydrochloride (1.91 g, 9.94 mmol). The reaction was stirred for 1 hour, and then the reaction mixture was placed at room temperature overnight. After completion of the reaction (monitored by TLC), the reaction solution was added with water (50 ml) and extracted with dichloromethane (50 ml× 3). The organic phases were combined, washed with saturated sodium chloride solution (100 mL), dried with anhydrous sodium sulfate, concentrated, and purified by silica gel column chromatography to obtain intermediate A-2-35c (2.4 g, 6.27 mmol, total yield of two steps 53.7%). ¹H NMR (400 MHz, CDCl3) δ 4.93 - 4.82 (m, 1H), 4.74 - 4.63 (m, 1H), 4.56 (dd, *J* = 9.0, 4.6 Hz, 1H), 3.97 (dd, *J =* 16.3, 8.2 Hz, 1H), 3.90 - 3.79 (m, 1H), 2.45 (d, *J* = 45.1 Hz, 2H), 2.30 - 2.18 (m, 1H), 1.93 - 1.82 (m, 2H), 1.79 - 1.68 (m, 2H), 1.61 - 1.51 (m, 2H), 1.49 (s, 9H), 1.46 - 1.38 (m, 2H), 1.37 - 1.26 (m, 2H), 1.00 (d, *J =* 6.9 Hz, 3H), 0.95 (d, *J =* 6.9 Hz, 3H).

### Fourth step

Intermediate A-2-35c (1.2 g, 3.13 mmol), dichloromethane (5 mL) and 4 M solution of hydrogen chloride in dioxane (10 mL) were added to a dry single-necked flask. The reaction was stirred at room temperature for 2 hours. After completion of the reaction (indicated by TLC), the reaction solution was concentrated to obtain a crude product - intermediate A-2-35d.

### Fifth step

The crude product - intermediate A-2-35d was dissolved in tetrahydrofuran (20 mL). Under ice bath, the mixture was added with N,N-diisopropylethylamine (1.22 g, 9.42 mmol), and slowly dropwise added with acetic anhydride (0.64 g, 6.28 mmol). The reaction was continuously stirred for 30 minutes, and then the reaction was stirred at room temperature for 2 hours. After completion of the reaction (monitored by TLC), the reaction solution was added with ethanol (5 mL), concentrated, and purified by silica gel column chromatography to obtain the product A-2-35 (0.7 g, 2.16 mmol, yield 68.8%). MS (ESI) calculated for [M+H]⁺ was 325.2; found: m/z 325.2. ¹H NMR (400 MHz, CDCl3) δ 8.19 (d, *J =* 8.4 Hz, 1H), 4.85 (dd, *J =* 9.5, 6.5 Hz, 1H), 4.80 - 4.69 (m, 1H), 4.41 (dd, *J =* 8.7, 4.6 Hz, 1H), 4.00 (t, *J =* 7.7 Hz, 2H), 2.67 - 2.54 (m, 1H), 2.37 (ddt, *J* = 9.4, 8.4, 7.0 Hz, 1H), 2.25 - 2.11 (m, 1H), 1.87 (s, 3H), 1.81 - 1.71 (m, 2H), 1.69 - 1.57 (m, 2H), 1.49 - 1.23 (m, 6H), 0.92 (d, *J =* 6.9 Hz, 3H), 0.87 (d, *J =* 6.9 Hz, 3H).

### Preparation Example 33 Preparation of compound A-2-37

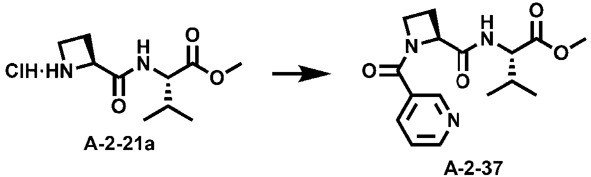

Nicotinic acid (0.12 g, 0.96 mmol), 3-(ethylcarbamoylamino)-*N*,*N*-dimethylpropan-1-amine hydrochloride (0.18 g, 0.96 mmol), 1-hydroxybenzotriazole (0.13 g, 0.96 mmol) and triethylamine (0.16 g, 1.60 mmol) were added to a dry three-necked flask. The mixture was added with dichloromethane (5 mL), stirred to react for 0.5 hour, and then added with intermediate A-2-21a (0.20 g, 0.80 mmol). The reaction was stirred at room temperature for 2 hours, and then the reaction solution was concentrated, added with water (10 mL), and extracted with ethyl acetate (10 mL×3). The organic phases were combined, washed with saturated brine (10 mL × 2), dried with anhydrous sodium sulfate, filtered, concentrated, and purified by silica gel column chromatography (petroleum ether/ethyl acetate = 1/1) to obtain the product A-2-37 (53.5 mg, 0.17 mmol, yield 21.0%). MS (ESI) calculated for [M+H]⁺ was 320.2; found: m/z 320.3. ¹H NMR (400 MHz, Chloroform-d) δ 8.88 (s, 1H), 8.74 (d, *J* = 4.6 Hz, 1H), 8.04 (d, *J =* 7.8 Hz, 1H), 7.84 (d, *J =* 8.2 Hz, 1H), 7.44 (dd, *J =* 7.9, 4.7 Hz, 1H), 5.14 (t, *J =* 8.0 Hz, 1H), 4.69 - 4.37 (m, 2H), 4.21 (q, *J =* 8.3 Hz, 1H), 3.75 (s, 3H), 2.88 (s, 1H), 2.49 (s, 1H), 2.20 (d, *J* = 14.9 Hz, 1H), 0.92 (dd, *J =* 12.7, 6.8 Hz, 6H).

### Preparation Example 34 Preparation of compound B-2-1

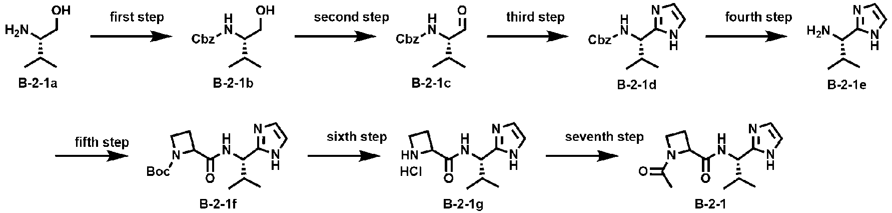

### First step

A starting material B-2-1a (5.00 g, 48.54 mmol) was dissolved in 1,4-dioxane/water (50 mL/10 mL), and slowly added with a solution of benzyl succinimidyl carbonate (12.10 g, 48.54 mmol) in 1,4-dioxane/water (50 mL/10 mL). The reaction of the reaction mixture was stirred at 25°C for 16 hours. After the reaction was completed, the reaction solution was diluted by adding ethyl acetate (200 mL), and then washed with 5% sodium bicarbonate solution and 5% citric acid solution respectively. The organic phases were dried with anhydrous sodium sulfate and concentrated to obtain a crude product - intermediate B-2-1b (10.40 g, 43.83 mmol, yield 90.3%). MS (ESI) calculated for [M+H]⁺ was 238.2; found: m/z 238.1.

### Second step

Intermediate B-2-1b (10.40 g, 43.83 mmol) was dissolved in ethyl acetate (300 mL), and added with Dess-Martin periodinane (22.30 g, 52.60 mmol). The reaction mixture was stirred at room temperature for 24 hours. After the reaction was completed, the reaction solution was filtered through Celite, and the filtrate was concentrated and purified by silica gel column chromatography to obtain intermediate B-2-1c (8.76 g, 37.25 mmol, yield 85%). MS (ESI) calculated for [M+H]⁺ was 236.1; found value 236.1.

### Third step

Intermediate B-2-1c (7.0 g, 29.79 mmol) and a glyoxal solution (15.4 mL, 119.16 mmol) were dissolved in methanol (70 mL), and slowly added with ammonia water (18.9 mL, 148.95 mmol), and the temperature was maintained below 10 °C during the addition. The reaction mixture was stirred at 25°C for 16 hours. After the reaction was completed, the reaction solution was poured into ice water and filtered to collect a precipitated solid, and the resulting filter cake was washed with water and dried to obtain intermediate B-2-1d (6.3 g, 23.05 mmol, yield 77%). MS (ESI) calculated for [M+H]⁺ was 274.1; found: m/z 274.1.

### Fourth step

Intermediate B-2-1d (3.00 g, 10.98 mmol) was dissolved in methanol (75 mL), and then added with palladium on carbon (0.3 g, 10 wt. %). The mixture was subjected to hydrogen replacement three times, and the reaction of the reaction mixture was stirred at 25°C under hydrogen atmosphere for 16 hours. After the reaction was completed, the reaction solution was filtered through Celite, and the filtrate was concentrated to obtain a crude product - intermediate B-2-1e (1.2 g, 8.62 mmol, yield 78%). MS (ESI) calculated for [M+H]⁺ was 140.0; found: m/z 140.1.

### Fifth step

Intermediate B-2-1e (1.2 g, 8.62 mmol) and intermediate A-2-1b (1.82 g, 9.05 mmol) were dissolved in N,N-dimethylformamide (12 mL), and added with 1-hydroxybenzotriazole (1.40 g, 10.34 mmol) and 1-(3-dimethylaminopropyl)-3-ethylcarbodiimide hydrochloride (1.97 g, 10.34 mmol) at -20°C. The reaction mixture was stirred at 0°C for 3 hours. After the reaction was completed, the reaction solution was diluted with water and extracted with ethyl acetate three times. The organic phases were combined, washed with water and brine, dried with anhydrous sodium sulfate and concentrated, and the resulting residue was separated and purified by silica gel column chromatography (dichloromethane/methanol = 15/1), and further purified by reverse-phase preparative chromatography to obtain intermediate B-2-1f (0.90 g, 2.80 mmol, yield 32%). MS (ESI) calculated for [M+H]⁺ was 323.2; found: m/z 323.1.

### Sixth step

Intermediate B-2-1f (0.90 g, 2.80 mmol) was dissolved in ethyl acetate (20 mL), and slowly dropwise added with a solution of hydrogen chloride in dioxane (4.0 M, 3 mL) under stirring at room temperature. After the addition was completed, the mixture was continuously stirred at room temperature for 12 hours. After completion of the reaction was monitored, the reaction solution was concentrated to dryness to obtain a crude product - intermediate B-2-1g (0.60 g, 2.33 mmol, yield 86%). MS (ESI) calculated for [M+H]⁺ was 223.1; found: m/z 223.2.

### Seventh step

Intermediate B-2-1g (0.60 g, 2.33 mmol) was dissolved in toluene (10 mL), added with acetic anhydride (2.38 g, 23.30 mmol), and heated to 110°C to react for 8 hours. After completion of the reaction was monitored, the reaction solution was concentrated to dryness, and the resulting residue was separated and purified by silica gel column chromatography (dichloromethane/methanol = 15/1), and further purified by reverse-phase preparative chromatography to obtain the product B-2-1 (0.20 g, 0.76 mmol, yield 33%). MS (ESI) calculated for [M+H]⁺ was 265.2; found: m/z 265.2. ¹H NMR (400 MHz, DMSO) δ 11.84 (d, *J = 42.0* Hz, 1H), 8.58 - 8.11 (m, 1H), 7.02 - 6.71 (m, 2H), 4.95 - 4.62 (m, 2H), 4.03 (t, *J* = 7.7 Hz, 1H), 3.81 - 3.68 (m, 1H), 2.44 - 2.26 (m, 1H), 2.20 - 1.90 (m, 2H), 1.83 - 1.52 (m, 3H), 0.84 (ddd, *J =* 10.3, 8.3, 5.0 Hz, 3H), 0.75 (t, *J =* 6.7 Hz, 3H).

### Preparation Example 35 Preparation of compound B-2-2

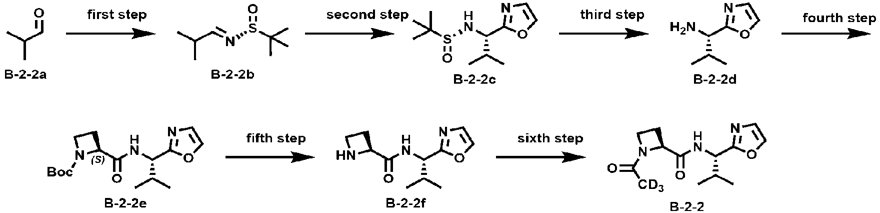

### First step

Compound B-2-2a (15 g, 0.21 mol) was dissolved in dichloromethane (250 mL), and added with (R)-tert-butylsulfinamide (27.5 g, 0.23 mol) and anhydrous copper sulfate (8.6 g, 0.05 mol). The resulting reaction system was stirred at 25°C for 24 hours, then filtered, concentrated, and purified by silica gel column chromatography (petroleum ether/ethyl acetate = 10/1) to obtain intermediate B-2-2b (7 g, 39.3 mmol, yield 17.6%). MS (ESI) calculated for [M+H]⁺ was 176.1; found: m/z 176.1.

### Second step

Compound 1,3-oxazole (1 g, 14.5 mmol) was dissolved in tetrahydrofuran (30 mL). After nitrogen replacement was carried out, the mixture was added with a borane tetrahydrofuran solution (22 mL). The reaction mixture was cooled to -78°C, then added with a solution of n-butyllithium in tetrahydrofuran (2.4 M, 10 mL, 24 mmol), stirred to react for 30 minutes, and then slowly added with intermediate B-2-2b (2.5 g, 14.5 mmol). The reaction mixture was stirred at -78°C for 3 hours. The reaction solution was quenched with saturated aqueous ammonium chloride solution, and extracted with ethyl acetate three times (50 mL × 3). The organic phases were combined, and successively washed with saturated sodium bicarbonate solution (100 mL × 2), saturated ammonium chloride solution (100 mL × 2) and saturated brine (100 mL × 2). The resulting organic phases were dried with anhydrous sodium sulfate, filtered, concentrated, and then purified by silica gel column chromatography (dichloromethane/methanol = 10/1) to obtain intermediate B-2-2c (1 g, 4.1 mmol, yield 28.7%). MS (ESI) calculated for [M+H]⁺ was 245.1; found: m/z 245.1.

### Third step

Intermediate B-2-2c (1 g, 4.1 mmol) was added with HCl/1,4-dioxane (2 M, 10 mL) at 0°C. The reaction mixture was stirred at 0°C for 1 hour, and concentrated to obtain intermediate B-2-2d (400 mg, 2.85 mmol, yield 69.7%). MS (ESI) calculated for [M+H]⁺ was 141.1; found: m/z 141.1.

### Fourth step

A solution of intermediate A-2-6b (2.5 g, 12.4 mmol) in dichloromethane (30 mL) was added with 1-(3-dimethylaminopropyl)-3-ethylcarbodiimide hydrochloride (3.57 g, 18.6 mmol), 1-hydroxybenzotriazole (2.51 g, 18.6 mmol) and N-methylmorpholine (11.29 g, 111.6 mmol), stirred at -15°C for 10 minutes, and then added with intermediate B-2-2d (1.91 g, 13.64 mmol). The reaction mixture was stirred at -15°C for 4 hours. After the reaction was completed, the reaction solution was successively washed with saturated citric acid solution (50 mL × 2) and saturated sodium carbonate solution (50 mL × 2), dried with anhydrous sodium sulfate, filtered, and concentrated. The resulting crude product was purified by silica gel column chromatography (petroleum ether/ethyl acetate = 2/1) to obtain intermediate B-2-2e (2.5 g, 7.7 mmol, yield 56.7 %). MS (ESI) calculated for [M+H]⁺ was 324.2; found: m/z 324.2.

### Fifth step

Intermediate B-2-2e (2.5 g, 6.4 mmol) was dissolved in a solution of trifluoroacetic acid in dichloromethane (V/V = 1: 1, 40 mL). The reaction mixture was stirred at room temperature for 30 minutes. After the reaction was completed, the reaction solution was concentrated to obtain a crude product - intermediate B-2-2f (5.58 g). MS (ESI) calculated for [M+H]⁺ was 224.1; found: m/z 224.1.

### Sixth step

A solution of crude product - intermediate B-2-2f (5.58 g) in dichloromethane (40 mL) was added with N,N-diisopropylethylamine (7.96 g, 61.6 mmol). The mixture was subjected to gas replacement under nitrogen protection three times, stirred at 0°C for 5 minutes, and then added with deuterated acetyl chloride (0.69 g, 8.4 mmol). The reaction mixture was stirred at room temperature for 2 hours. The reaction solution was washed with saturated brine (50 mL × 2), dried with anhydrous sodium sulfate, filtered, concentrated, and purified by silica gel column chromatography (petroleum ether/ethyl acetate = 1/1) to obtain the product B-2-2 (1.57 g, 5.85 mmol, yield 75.6 %). MS (ESI) calculated for [M+H]⁺ was 269.2; found: m/z 269.0. ¹H NMR(400MHz, d6-DMSO) δ 8.64 (dd, *J* = 64.7, 8.7Hz, 1H), 8.02 (dd, *J =* 7.4, 0.6Hz, 1H), 7.13 (d, *J* = 7.8Hz, 1H), 4.92 - 4.61 (m, 2H), 4.00 (t, *J* = 7.6Hz,1H), 3.71 (dt, *J* = 8.8, 6.3Hz, 1H), 2.44 - 2.23 (m, 1H), 2.23 - 1.85 (m, 2H), 0.86 (dd, *J =* 11.2, 6.8Hz, 3H), 0.77 (dd, *J* = 11.0, 6.8Hz, 3H).

### Preparation Example 36 Preparation of compound B-2-3

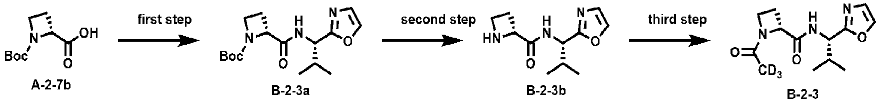

### First step

A solution of compound A-2-7b (2 g, 9.96 mmol) in dichloromethane (50 mL) was added with 1-(3-dimethylaminopropyl)-3-ethylcarbodiimide hydrochloride (2.39 g, 12.45 mmol), 1-hydroxybenzotriazole (1.68 g, 12.45 mmol) and *N,N*-diisopropylethylamine (5.36 g, 41.50 mmol) at -10°C, continuously stirred for 10 minutes, and then added with intermediate B-2-2d (1.47 g, 8.30 mmol). The mixture was continuously stirred at -10°C to react for 2 hours. After the reaction was completed, the reaction solution was successively washed with saturated aqueous citric acid solution (50 mL × 2) and saturated sodium carbonate solution (50 mL × 2). The resulting organic phases were dried with anhydrous sodium sulfate, filtered, concentrated, and then purified by silica gel column chromatography (dichloromethane/methanol = 50/1) to obtain intermediate B-2-3a (2 g, 6.2 mmol, 62.2 %). MS (ESI) calculated for [M+H]⁺ was 324.2; found: m/z 324.0.

### Second step

Intermediate B-2-3a (2 g, 6.2 mmol) was dissolved in a solution of trifluoroacetic acid in dichloromethane (V/V = 1: 1, 20 mL). The reaction mixture was stirred at room temperature for 2 hours. After the reaction was completed, the reaction solution was concentrated to obtain a crude product - intermediate B-2-3b (3.6 g). MS (ESI) calculated for [M+H]⁺ was 224.1; found: m/z 224.0.

### Third step

A solution of crude product - intermediate B-2-3b (3.6 g) in dichloromethane (60 mL) was sequentially added with N,N-diisopropylethylamine (6.4 g, 49.6 mmol) and deuterated acetyl chloride (606 mg, 7.4 mmol) at 0°C. After the addition was completed, the reaction was continuously stirred at 0°C for 1 hour. After the reaction was completed, the reaction solution was concentrated and purified by silica gel column chromatography (dichloromethane/methanol = 30/1) to obtain the product B-2-3 (1.22 g, 4.55 mmol, total yield of two steps 73.5%). MS (ESI) calculated for [M+H]⁺ was 269.2; found: m/z 269.0. ¹H NMR (400 MHz, Chloroform-d) δ 8.50 (d, *J* = 8.5 Hz, 1H), 7.60 (s, 1H), 7.08 (s, 1H), 5.08 (dd, *J* = 8.5, 6.0 Hz, 1H), 4.95 (dd, *J =* 9.6, 6.6 Hz, 1H), 4.06 (t, *J =* 8.0 Hz, 2H), 2.73 - 2.59 (m, 1H), 2.51 - 2.35 (m, 1H), 2.28 (dq, *J =* 13.4, 6.7 Hz, 1H), 0.96 (dd, *J =* 13.9, 6.8 Hz, 6H).

### Preparation Example 37 Preparation of compound B-2-4

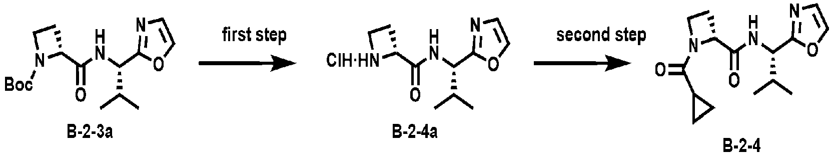

### First step

Intermediate B-2-3a (6 g, 18.55 mmol) was dissolved in dichloromethane (50 mL), and added with 4 M solution of hydrogen chloride in dioxane (15 mL). The reaction was stirred at room temperature for 2 hours, and completion of the reaction was indicated by TLC. The reaction solution was concentrated to obtain a crude product - intermediate B-2-4a (4.83 g, 18.6 mmol, yield 100%). MS (ESI) calculated for [M+H]⁺ was 224.1; found: m/z 224.3.

### Second step

Intermediate B-2-4a (500.0 mg, 1.93 mmol) was added to a dry single-necked flask, dissolved with ultra-dry dichloromethane (5 mL), and subjected to nitrogen replacement. Subsequently, the mixture was added with diisopropylethylamine (0.75 g, 5.79 mmol) at 0°C, and stirred for 10 minutes. Finally, the above reaction system was slowly added with cyclopropanecarbonyl chloride (262.3 mg, 2.51 mmol). The reaction was continuously stirred at 0°C for 2 hours. After completion of the reaction (indicated by TLC), the reaction solution was added with water (10 mL), extracted and subjected to liquid separation. Subsequently, the aqueous phase was further extracted with dichloromethane (10 mL × 2). The organic phases were combined, washed with saturated sodium chloride solution (20 mL), dried with anhydrous sodium sulfate, concentrated, and purified by silica gel column chromatography (EA: PE=10%-50%) to obtain the product B-2-4 (351.0 mg, 1.20 mmol, yield 62.40%). MS (ESI) calculated for [M+H]⁺ was 292.2; found: m/z 292.1. ¹H NMR (400 MHz, Chloroform-d) δ 8.37 (d, *J =* 8.2 Hz, 1H), 7.52 (s, 1H), 6.99 (s, 1H), 4.99 (dd, *J =* 8.5, 6.3 Hz, 1H), 4.87 (dd, *J* = 9.4, 6.7 Hz, 1H), 4.27 - 3.93 (m, 2H), 2.72 - 2.53 (m, 1H), 2.48 - 2.36 (m, 1H), 2.22 - 2.13 (m, 1H), 1.41 - 1.33 (m, 1H), 1.01 - 0.90 (m, 2H), 0.86 (dd, *J =* 13.0, 6.8 Hz, 6H), 0.81 - 0.73 (m, 2H).

### Preparation Example 38 Preparation of compound B-2-5

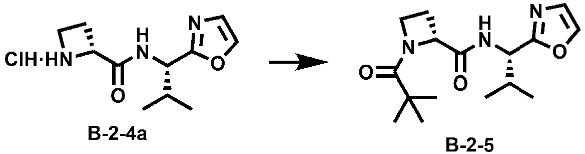

Intermediate B-2-4a (500.0 mg, 1.93 mmol) was added to a dry single-necked flask, dissolved with ultra-dry dichloromethane (5 mL), and subjected to nitrogen replacement. Subsequently, the mixture was added with diisopropylethylamine (0.75 g, 5.79 mmol) at 0°C, and stirred for 10 minutes. Finally, the above reaction system was slowly added with trimethylacetyl chloride (302.5 mg, 2.51 mmol). The reaction was continuously stirred at 0°C for 2 hours. After completion of the reaction (indicated by TLC), the reaction solution was added with water (10 mL), extracted and subjected to liquid separation. Subsequently, the aqueous phase was further extracted with dichloromethane (10 mL × 2). The organic phases were combined, washed with saturated sodium chloride solution (20 mL), dried with anhydrous sodium sulfate, concentrated, and purified by silica gel column chromatography (EA: PE=10%-50%) to obtain the product B-2-5 (170.0 mg, 0.55 mmol, yield 28.7%). MS (ESI) calculated for [M+H]⁺ was 308.2; found: m/z 308.2. ¹H NMR (400 MHz, Chloroform-d) δ 8.17 (d, *J* = 8.4 Hz, 1H), 7.50 (s, 1H), 6.98 (s, 1H), 5.00 (dd, *J* = 8.9, 6.1 Hz, 1H), 4.90 (dd, *J =* 9.6, 5.9 Hz, 1H), 4.24 (t, *J =* 7.7 Hz, 2H), 2.59 (dq, *J =* 14.1, 7.2 Hz, 1H), 2.44 - 232 (m, 1H), 2.27 - 2.09 (m, 1H), 1.14 (s, 9H), 0.87 (dd, *J* = 11.6, 6.8 Hz, 6H).

### Preparation Example 39 Preparation of compound B-2-8

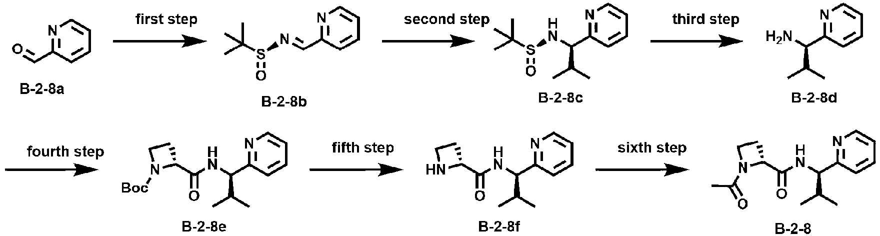

### First step

Intermediate B-2-8a (5 g, 46.68 mmol) was dissolved in ultra-dry dichloromethane (100 mL), and sequentially added with cesium carbonate (18.25 g, 56.02 mmol) and (S)-tert-butylsulfinamide (5.66 g, 46.68 mmol). The mixture was stirred at room temperature for 2 hours. After completion of the reaction (indicated by TLC), the reaction solution was added with water (100 mL), and extracted with dichloromethane (50 mL × 2). The organic phases were combined, washed with saturated sodium chloride solution (200 mL), dried with anhydrous sodium sulfate, and purified by silica gel column chromatography (petroleum ether/ethyl acetate = 1/1) to obtain intermediate B-2-8b (9.56 g, 45.52 mmol, yield 97.4%).

### Second step

Intermediate B-2-8b (9.56 g, 45.46 mmol) was dissolved in ultra-dry tetrahydrofuran (100 mL), subjected to nitrogen protection and cooled to -78°C, and slowly dropwise added with 1 M isopropylmagnesium bromide (50 mL). After the addition was completed, the reaction was continuously stirred for 2 hours. After completion of the reaction (indicated by TLC), the reaction solution was added with water (100 ml) and extracted with dichloromethane (50 ml× 2). The organic phases were combined, washed with saturated sodium chloride solution (200 mL), dried with anhydrous sodium sulfate, and purified by silica gel column chromatography to obtain intermediate B-2-8c (6.15 g, 24.21 mmol, yield 53.2%). MS (ESI) calculated for [M+H]⁺ was 255.2; found: m/z 255.3.

### Third step

Intermediate B-2-8c (6.15 g, 24.21 mmol) was dissolved in dichloromethane (100 mL), and added with 4 M solution of hydrogen chloride in dioxane (30 mL). The reaction was stirred at room temperature for 2 hours. After completion of the reaction (indicated by TLC), the reaction solution was concentrated to obtain a crude product - intermediate B-2-8d.

### Fourth step

The crude product - intermediate B-2-8d (0.5 g, 2.48 mmol) was dissolved in dichloromethane (50 mL), and sequentially added with intermediate A-2-7b (0.56 g, 3.72 mmol), N,N-diisopropylethylamine (0.96 g, 7.44 mmol) and 1-hydroxybenzotriazole (0.5 g, 3.72 mmol) under ice bath. The mixture was added with 1-(3-dimethylaminopropyl)-3-ethylcarbodiimide hydrochloride (0.71 g, 3.72 mmol), and the reaction was continuously stirred under ice bath for 30 minutes. The reaction solution was placed at room temperature. After the reaction was stirred for 3 hours, completion of the reaction of raw materials was indicated by TLC. The reaction solution was added with water (50 mL), extracted with dichloromethane (50 mL × 3), washed with saturated brine (100 mL), dried with anhydrous sodium sulfate, and purified by silica gel column chromatography to obtain intermediate B-2-8e (0.60 g, 1.80 mmol, total yield of two steps: 72.4%). ¹H NMR (400 MHz, CDCl3) δ 8.56 (t, *J =* 5.3 Hz, 1H), 7.85 (s, 1H), 7.63 (td, *J* = 7.7, 1.6 Hz, 1H), 7.19 (t, *J* = 8.5 Hz, 2H), 4.91 (dd, *J* = 8.9, 7.0 Hz, 1H), 4.77 - 4.59 (m, 1H), 3.92 (dd, *J* = 16.3, 8.3 Hz, 1H), 3.79 (td, *J* = 8.6, 5.3 Hz, 1H), 2.46 (s, 1H), 2.22 (dd, *J* = 12.7, 7.1 Hz, 2H), 1.50 (s, 9H), 0.94 (d, *J =* 6.8 Hz, 3H), 0.84 (d, *J =* 6.7 Hz, 3H).

### Fifth step

Intermediate B-2-8e (0.60 g, 1.8 mmol) was dissolved in dichloromethane (5 mL), and added with 4 M solution of hydrogen chloride in dioxane (8 mL). The reaction was stirred at room temperature for 2 hours. After completion of the reaction (indicated by TLC), the reaction solution was concentrated to obtain intermediate B-2-8f (0.42 g, 1.8 mmol, yield: 100%). ¹H NMR (400 MHz, MeOD) δ 8.81 (d, *J* = 5.4 Hz, 1H), 8.65 (t, *J =* 7.5 Hz, 1H), 8.13 (d, *J* = 8.2 Hz, 1H), 8.08 - 7.93 (m, 1H), 5.26 - 5.14 (m, 1H), 4.95 (d, *J =* 7.8 Hz, 1H), 4.19 - 4.05 (m, 1H), 3.95 (td, *J* = 10.0, 6.3 Hz, 1H), 2.94 (dtd, *J* = 15.8, 9.5, 6.3 Hz, 1H), 2.58 (dq, *J* = 12.2, 7.8 Hz, 1H), 2.33 (dd, *J =* 13.9, 6.8 Hz, 1H), 1.11 (dd, *J =* 11.6, 4.3 Hz, 3H), 0.90 (dd, *J =* 12.5, 6.8 Hz, 3H).

### Sixth step

Intermediate B-2-8f (0.42 g, 1.8 mmol) was dissolved in tetrahydrofuran (20 mL), and added with *N*,*N*-diisopropylethylamine (0.70 g, 5.40 mmol). Under ice bath, the mixture was slowly dropwise added with acetic anhydride (0.37 g, 3.60 mmol). The reaction was continuously stirred under ice bath for 30 minutes, and the reaction solution was placed at room temperature. The reaction was stirred for 2 hours, and completion of the reaction was indicated by TLC. The reaction solution was added with methanol (5 mL), concentrated, and separated by silica gel column chromatography to obtain the product B-2-8 (0.25 g, 0.91 mmol, yield 50.4%). MS (ESI) calculated for [M+H]⁺ was 276.2; found: m/z 276.3. ¹H NMR (400 MHz, CDCl3) δ 8.49 (d, *J =* 4.5 Hz, 1H), 8.27 (d, *J =* 8.1 Hz, 1H), 7.68 - 7.51 (m, 1H), 7.19 - 7.05 (m, 2H), 4.86 (dd, *J =* 8.7, 6.0 Hz, 1H), 4.83 - 4.74 (m, 1H), 4.06 - 3.91 (m, 2H), 2.58 - 2.44 (m, 1H), 2.37 (ddd, *J* = 19.4, 11.8, 7.9 Hz, 1H), 2.17 (dd, *J* = 13.5, 6.8 Hz, 1H), 1.91 - 1.83 (m, 3H), 0.85 (t, *J =* 6.9 Hz, 3H), 0.81 - 0.73 (m, 3H).

### Preparation Example 40 Preparation of compounds B-2-9 and B-2-10

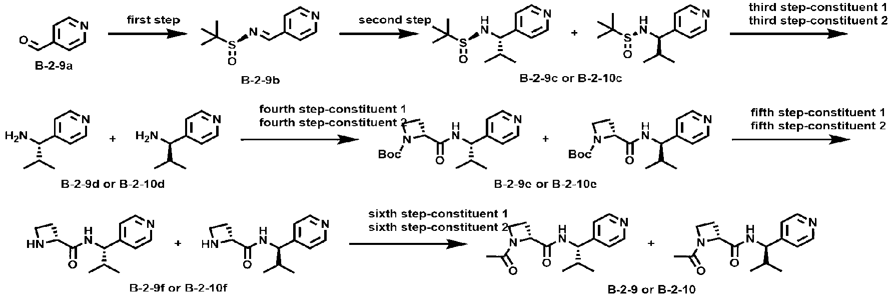

### First step

Intermediate B-2-9a (2 g, 18.67 mmol) was dissolved in ultra-dry dichloromethane (100 mL), sequentially added with cesium carbonate (7.30 g, 22.40 mmol) and (*S*)-tert-butylsulfinamide (2.26 g, 18.67 mmol), and stirred at room temperature for 2 hours. After completion of the reaction (indicated by TLC), the reaction solution was added with water (100 ml) and extracted with dichloromethane (50ml× 2). The organic phases were combined, washed with saturated sodium chloride solution (200 mL), dried with anhydrous sodium sulfate, and purified by silica gel column chromatography to obtain intermediate B-2-9b (3.82 g, 18.02 mmol, yield 97.38%).

### Second step

Intermediate B-2-9b (3.82 g, 18.02 mmol) was dissolved in ultra-dry tetrahydrofuran (100 mL), subjected to nitrogen replacement and cooled to -78°C, and slowly dropwise added with 1 M solution of isopropylmagnesium bromide in tetrahydrofuran (20 mL). After the addition was completed, the reaction was continuously stirred at -78°C for 2 hours. After completion of the reaction (indicated by TLC), the reaction solution was added with water (100 ml) and extracted with dichloromethane (50 ml× 2). The organic phases were combined, washed with saturated sodium chloride solution (200 mL), dried with anhydrous sodium sulfate, and purified by silica gel column chromatography to obtain two constituents.

Constituent 1 with a short retention time was intermediate B-2-9c (1.88 g, 7.43 mmol, yield 41.26%). MS (ESI) calculated for [M+H]⁺ was 255.2, found: m/z 255.3.

Constituent 2 with a long retention time was intermediate B-2-10c (1.83 g, 7.22 mmol, yield 40.10%). MS (ESI) calculated for [M+H]⁺ was 255.2, found: m/z 255.3.

### Third step - constituent 1

Intermediate B-2-9c (1 g, 3.93 mmol) was dissolved in dichloromethane (10 mL), and added with 4 M solution of hydrogen chloride in dioxane (5 mL). The reaction was stirred at room temperature for 3 hours. After completion of the reaction (monitored by TLC), the reaction solution was concentrated to obtain a crude product - intermediate B-2-9d.

### Fourth step - constituent 1

The crude product - intermediate B-2-9d was dissolved by adding dichloromethane (30 mL), and sequentially added with N,N-diisopropylethylamine (1.02 g, 7.86 mmol), intermediate A-2-7b (0.87 g, 4.32 mmol), 1-hydroxybenzotriazole (0.53 g, 3.93 mmol) and 1-(3-dimethylaminopropyl)-3-ethylcarbodiimide hydrochloride (0.75 g, 3.93 mmol). The reaction was stirred at room temperature overnight, and the reaction solution was added with water (50 ml) and extracted with dichloromethane (50 ml× 3). The organic phases were combined, washed with saturated brine (100 mL), dried with anhydrous sodium sulfate, and separated by silica gel column chromatography to obtain intermediate B-2-9e (0.8 g, 2.40 mmol, total yield of two steps 61.1%). ¹H NMR (400 MHz, CDCl3) δ 8.48 (d, *J =* 5.9 Hz, 2H), 7.14 (d, *J =* 5.9 Hz, 2H), 4.71 (dd, *J =* 8.4, 6.3 Hz, 1H), 4.62 (d, *J =* 7.2 Hz, 1H), 3.88 (dd, *J =* 16.3, 8.1 Hz, 1H), 3.76 (tt, *J =* 16.3, 8.2 Hz, 1H), 2.39 (d, *J* = 51.6 Hz, 2H), 2.02 (td, *J* = 13.2, 6.6 Hz, 1H), 1.40 (s, 9H), 0.90-0.67 (m, 6H).

### Fifth step - constituent 1

Intermediate B-2-9e (0.8 g, 2.4 mmol) was dissolved in dichloromethane (5 mL), and added with 4 M solution of hydrogen chloride in dioxane (8 mL). The reaction was stirred at room temperature for 2 hours. After the reaction was completed, the reaction solution was concentrated to obtain a crude product - intermediate B-2-9f.

### Sixth step - constituent 1

The crude product - intermediate B-2-9f was dissolved in tetrahydrofuran (20 mL), and added with *N,N*-diisopropylethylamine (1.02 g, 7.86 mmol). Under ice bath, the mixture was slowly dropwise added with acetic anhydride (0.6 g, 5.9 mmol). The reaction was continuously stirred for 30 minutes, and the reaction solution was placed at room temperature. After the reaction was stirred for 2 hours, completion of the reaction was indicated by TLC. The reaction solution was added with methanol (2 mL), concentrated, and separated by silica gel column chromatography to obtain the product B-2-9 (0.30 g, 1.09 mmol, total yield of two steps 45.4%). ¹H NMR (400 MHz, CDCl3) δ 8.61 (d, *J =* 8.2 Hz, 1H), 8.48 (d, *J =* 5.8 Hz, 2H), 7.15 (d, *J* = 6.0 Hz, 2H), 4.83 (dd, *J* = 9.4, 6.6 Hz, 1H), 4.66 (dd, *J* = 8.2, 6.4 Hz, 1H), 4.03 (t, *J =* 7.7 Hz, 2H), 2.74 - 2.58 (m, 1H), 2.33 (ddt, *J* = 12.1, 9.5, 7.4 Hz, 1H), 1.96 (dd, *J* = 13.4, 6.7 Hz, 1H), 1.91 (s, 3H), 0.81 (d, *J =* 2.6 Hz, 3H), 0.79 (d, *J* = 2.7 Hz, 3H).

### Third step - constituent 2

Intermediate B-2-10c (1 g, 3.93 mmol) was dissolved in dichloromethane (10 mL), and added with 4 M solution of hydrogen chloride in dioxane (5 mL). The reaction was stirred at room temperature for 3 hours. After completion of the reaction (monitored by TLC), the reaction solution was concentrated to obtain a crude product - intermediate B-2-1 0d.

### Fourth step - constituent 2

The crude product - intermediate B-2-10d was dissolved by adding dichloromethane (30 mL), and sequentially added with N,N-diisopropylethylamine (1.02 g, 7.86 mmol), intermediate A-2-7b (0.87 g, 4.32 mmol), 1-hydroxybenzotriazole (0.53 g, 3.93 mmol) and 1-(3-dimethylaminopropyl)-3-ethylcarbodiimide hydrochloride (0.75 g, 3.93 mmol). The reaction was stirred at room temperature overnight, and the reaction solution was added with water (50 ml) and extracted with dichloromethane (50 ml× 3). The organic phases were combined, washed with saturated brine (100 mL), dried with anhydrous sodium sulfate, and separated by silica gel column chromatography to obtain intermediate B-2-10e (0.8 g, 2.40 mmol, total yield of two steps 61.1%). ¹H NMR (400 MHz, CDCl3) δ 8.47 (d, *J =* 5.9 Hz, 2H), 7.09 (d, *J =* 6.0 Hz,2H), 4.75 (dd, *J =* 8.9, 6.4 Hz, 1H), 4.69 (t, *J =* 7.5 Hz, 1H), 3.85 (dd, *J =* 16.4, 8.3 Hz, 1H), 3.72 (dd, *J* = 14.1, 8.4 Hz, 1H), 2.51-2.23 (m, 2H), 2.15 (s, 1H), 1.43 (s, 9H), 0.84 (dd, *J =* 6.7, 4.1 Hz, 6H).

### Fifth step - constituent 2

Intermediate B-2-10e (0.8 g, 2.4 mmol) was dissolved in dichloromethane (5 mL), and added with 4 M solution of hydrogen chloride in dioxane (8 mL). The reaction was stirred at room temperature for 2 hours. After the reaction was completed, the reaction solution was concentrated to obtain a crude product - intermediate B-2-10f.

### Sixth step - constituent 2

The crude product - intermediate B-2-1 0f was dissolved in tetrahydrofuran (20 mL), and added with *N*,*N*-diisopropylethylamine (1.02 g, 7.86 mmol). Under ice bath, the mixture was slowly dropwise added with acetic anhydride (0.6 g, 5.9 mmol). The reaction was continuously stirred for 30 minutes, and the reaction solution was placed at room temperature. After the reaction was stirred for 2 hours, completion of the reaction was indicated by TLC. The reaction solution was added with methanol (2 mL), concentrated, and separated by silica gel column chromatography to obtain the product B-2-10 (0.33 g, 1.2 mmol, total yield of two steps 50.0%). ¹H NMR (400 MHz, CDCl3) δ 8.67 (d, *J =* 8.4 Hz, 1H), 8.47 (d, *J =* 5.8 Hz, 2H), 7.08 (d, *J =* 6.0 Hz, 2H), 4.91 (dd, *J =* 9.3, 6.7 Hz, 1H), 4.72 (dd, *J =* 8.6, 6.4 Hz, 1H), 4.08 - 3.90 (m, 2H), 2.61 (ddt, *J* = 12.1, 9.4, 6.8 Hz, 1H), 2.33 (dtd, *J* = 12.1, 9.2, 5.5 Hz, 1H), 1.97 (dd, *J* = 13.4, 6.7 Hz, 1H), 1.90 (s, 3H), 0.84 (t, *J* = 6.4 Hz, 6H).

### Preparation Example 41 Compound B-2-11

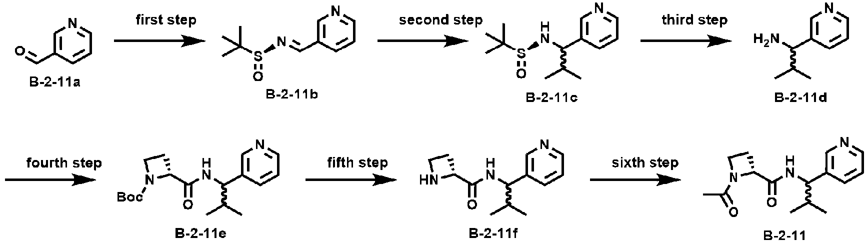

### First step

Intermediate B-2-11a (1 g, 9.33 mmol) was dissolved in ultra-dry dichloromethane (100 mL), and sequentially added with cesium carbonate (3.65 g, 11.20 mmol) and (*S*)-tert-butylsulfinamide (1.13 g, 9.33 mmol). The reaction was stirred at room temperature for 2 hours, and completion of the reaction was indicated by TLC. The reaction solution was added with water (100 ml) and extracted with dichloromethane (50 ml× 2). The organic phases were combined, washed with saturated brine (100 ml), dried with anhydrous sodium sulfate, and purified by silica gel column chromatography to obtain intermediate B-2-11b (1.90 g, 9.01 mmol, yield 97.3%).

### Second step

Intermediate B-2-11b (1.90 g, 9.01 mmol) was dissolved in ultra-dry tetrahydrofuran (100 mL), subjected to nitrogen replacement and cooled to -78°C, and then slowly dropwise added with 1 M isopropylmagnesium bromide (10 mL). The reaction was continuously stirred at this temperature for 2 hours, and then completion of the reaction was indicated by TLC. The reaction solution was added with water (100 ml) and extracted with dichloromethane (50 ml× 2). The organic phases were combined, washed with saturated sodium chloride solution (200 mL), dried with anhydrous sodium sulfate, and purified by silica gel column chromatography to obtain two constituents with single configuration.

A first constituent with a short retention time was intermediate B-2-11c (0.94 g, 3.54 mmol, yield 41.26%). MS (ESI) calculated for [M+H]⁺ was 255.2; found: m/z 255.3.

A second constituent with a long retention time was intermediate B-2-11c constituent 2 (0.47 g, 1.85 mmol, yield 20.10%). MS (ESI) calculated for [M+H]⁺ was 255.2; found: m/z 255.3.

### Third step

Intermediate B-2-11c (0.94 g, 3.54 mmol) was dissolved in dichloromethane (10 mL), and added with 4 M solution of hydrogen chloride in dioxane (5 mL). The reaction was stirred at room temperature for 3 hours. After completion of the reaction (indicated by TLC), the reaction solution was concentrated to obtain a crude product - intermediate B-2-11d.

### Fourth step

The crude product - intermediate B-2-11d was dissolved in dichloromethane (30 mL), and sequentially added with *N*,*N*-diisopropylethylamine (1.02 g, 7.86 mmol), intermediate A-2-7b (0.87 g, 4.32 mmol), 1-hydroxybenzotriazole (0.53 g, 3.93 mmol) and 1-(3-dimethylaminopropyl)-3-ethylcarbodiimide hydrochloride (0.75g, 3.93 mmol). The reaction was stirred at room temperature overnight, and then the reaction solution was added with water (50 ml) and extracted with dichloromethane (50 ml× 3). The organic phases were combined, washed with saturated brine (100 mL), dried with anhydrous sodium sulfate, and purified by silica gel column chromatography to obtain intermediate B-2-11e (0.30 g, 0.9 mmol, total yield of two steps 25.4%). ¹H NMR (400 MHz, CDCl3) δ 8.60 - 8.47 (m, 2H), 7.59 (dt, *J* = 7.8*,* 1.7 Hz, 1H), 7.32 - 7.24 (m, 1H), 4.86 (dd, *J =* 8.7, 7.0 Hz, 1H), 4.77 (t, *J* = 7.5 Hz, 1H), 3.93 (q, *J* = 8.2 Hz, 1H), 3.84 - 3.72 (m, 1H), 2.41 (s, 2H), 2.14 - 2.02 (m, 1H), 1.52 (s, 9H), 0.98 (d, *J =* 6.8 Hz, 3H), 0.93 (d, *J =* 6.7 Hz, 3H).

### Fifth step

Intermediate B-2-11e (0.30 g, 0.9 mmol) was dissolved in dichloromethane (5 mL), and added with 4 M solution of hydrogen chloride in dioxane (8 mL). The reaction was stirred at room temperature for 2 hours. After completion of the reaction (indicated by TLC), the reaction solution was concentrated to obtain a crude product - intermediate B-2-11f.

### Sixth step

The crude product - intermediate B-2-11f was dissolved in tetrahydrofuran (20 mL). Under ice bath, the mixture was added with *N*,*N*-diisopropylethylamine (0.21 g, 1.62 mmol), and slowly dropwise added with acetic anhydride (0.12 g, 1.22 mmol). After the addition was completed, the reaction was continuously stirred under ice bath for 30 minutes, and the reaction solution was placed at room temperature. After the reaction was stirred for 2 hours, completion of the reaction was indicated by TLC. The reaction solution was added with methanol (5 mL), concentrated, and purified by silica gel column chromatography to obtain the product B-2-11 (80 mg, 0.29 mmol, total yield of two steps 32.2%). ¹H NMR (400 MHz, CDCl3) δ 8.73 (d, *J =* 8.1 Hz, 1H), 8.54 - 8.39 (m, 2H), 7.55 (dt, *J* = 7.9, 1.8 Hz, 1H), 7.29 - 7.26 (m, 1H), 4.96 (dd, *J* = 9.4, 6.7 Hz, 1H), 4.79 (dd, *J* = 8.4, 6.9 Hz, 1H), 4.15 - 3.90 (m, 2H), 2.69 - 2.60 (m, 1H), 2.43 - 2.35 (m, 1H), 2.06 - 2.00 (m, 1H), 1.94 (s, 3H), 0.94 (d, *J =* 6.8 Hz, 3H), 0.88 (d, *J* = 6.8 Hz, 3H).

### Preparation Example 42 Preparation of compound ZZL-7

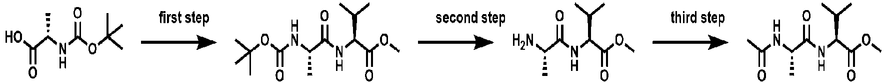

### First step

(Tert-butoxycarbonyl)-*L*-alanine (4.5 g, 23.78 mmol) was added to a dry single-necked flask, dissolved in dichloromethane (50 mL), and stirred under ice bath for 10 minutes. Subsequently, *L*-valine methyl ester (3.12 g, 23.78 mmol), *N*,*N*-diisopropylethylamine (6.15 g, 47.56 mmol), 1-hydroxybenzotriazole (3.53 g, 26.16 mmol) and 1-(3-dimethylaminopropyl)-3-ethylcarbodiimide hydrochloride (5.01 g, 26.16 mmol) were sequentially added to the flask, and stirred at room temperature for 2 hours. After completion of the reaction (indicated by TLC), the reaction solution was added with water (50 mL) for quenching, adjusted to be acidic with 2 M hydrochloric acid, and extracted with dichloromethane (50 mL × 2). The organic phases were combined, adjusted to be alkaline with saturated sodium bicarbonate solution, extracted with dichloromethane (50 mL × 2), washed with saturated sodium chloride solution (20 mL), dried with anhydrous sodium sulfate, and purified by silica gel column chromatography (PE: EA=2: 1) to obtain intermediate (tert-butoxycarbonyl)-*L*-alanyl-*L*-valine methyl ester (6.5 g, 21.50 mmol, yield 90.39%).
LCMS(M+H⁺)=303.1

### Second step

The (tert-butoxycarbonyl)-*L*-alanyl-*L*-valine methyl ester (6.5 g, 21.50 mmol) was added to a dry single-necked flask, and dissolved in dichloromethane (30 mL), and then HCl/1,4-dioxane (10 mL, 4 M) was added to the flask, and stirred at room temperature for 2 hours. After completion of the reaction wa sindicated by TLC, the organic phases were concentrated and purified by silica gel column chromatography (DCM: MeOH=10: 1) to obtain intermediate *L*-alanyl-*L*-valine methyl ester (3.7 g, 18.32 mmol, yield 87.22%).
LCMS(M+H⁺)=203.3
¹HNMR(400 MHz, CDCl3) δ 8.13 (s, 3H), 4.56 (s, 1H), 4.31 (s, 1H), 3.65 (s, 3H), 2.15 (d, *J =* 5.3 Hz, 1H), 1.57 (s, 3H), 0.92 (t, *J* = 6.8 Hz, 6H).

### Third step

The compound *L*-alanyl-*L*-valine methyl ester (3.7 g, 18.32 mmol) and *N,N*-diisopropylethylamine (4.72 g, 36.64 mmol) were dissolved in tetrahydrofuran (50 mL), subjected to full nitrogen replacement and then stirred at 0°C for 10 minutes, and then slowly dropwise added with acetic anhydride (2.8 g, 27.48 mmol). The mixture was stirred at this temperature for 10 minutes, and then stirred at room temperature for 2 hours. After completion of the reaction (indicated by TLC), the reaction solution was added with water (50 mL) for quenching, adjusted to be acidic with 2 M hydrochloric acid, and extracted with dichloromethane (50 mL × 4). The organic phases were combined, washed with saturated sodium chloride solution (10 mL), dried with anhydrous sodium sulfate, and purified by silica gel column chromatography (PE: EA=5: 1) to obtain a compound acetyl-*L*-alanyl-*L*-valine methyl ester (2.6 g, 10.67 mmol, yield 58.23%), which was ZZL-7.
LCMS(M+H⁺)=245.3
¹HNMR(400 MHz, CDCl3) δ 6.68 (d, *J* = 8.5 Hz, 1H), 6.21 (d, *J* = 7.1 Hz, 1H), 4.57 - 4.47 (m, 1H), 4.44 (dd, *J* = 8.7, 4.9 Hz, 1H), 3.68 (s, 3H), 2.18 - 2.04 (m, 1H), 1.94 (s, 3H), 1.31 (d, *J* = 7.0 Hz, 3H), 0.85 (dd, *J =* 10.1, 6.9 Hz, 6H).

### Preparation Example 43 Preparation of compound A-2-38

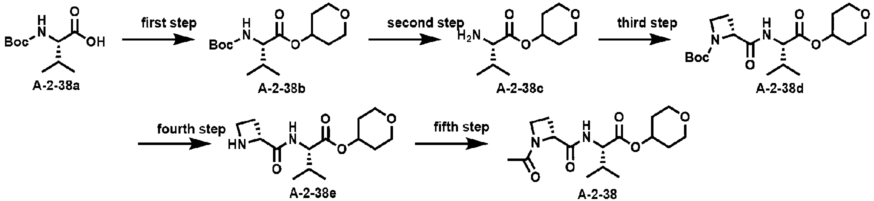

### First step

Intermediate A-2-38a (3.00 g, 13.82 mmol) was dissolved in dichloromethane (50 mL) and stirred for 10 minutes. The mixture was added with tetrahydro-2*H*-pyran-4-ol (2.11 g, 20.71 mmol), 4-dimethylaminopyridine (2.52 g, 20.71 mmol) and *N*,*N*'-dicyclohexylcarbodiimide (4.27 g, 20.71 mmol) in one portion. The mixture was stirred at room temperature for 12 hours, and then completion of the reaction was indicated by TLC. The reaction solution was added with water (50 ml) for quenching, and extracted with dichloromethane (50 ml× 3). The organic phases were combined, successively washed with saturated brine (200 mL), dried with anhydrous sodium sulfate, and purified by silica gel column chromatography to obtain intermediate A-2-38b (2.50 g, 8.30 mmol, yield 60.0%).

### Second step

Intermediate A-2-38b (2.50 g, 8.30 mmol) was dissolved in dichloromethane (20 mL), and added with 4 M solution of hydrogen chloride in dioxane (20 mL). The mixture was stirred at room temperature for 2 hours, and then completion of the reaction was indicated by TLC. The reaction solution was concentrated to obtain a crude product - intermediate A-2-38c, which was used directly in the next reaction step without purification.

### Third step

The crude product - intermediate A-2-38c and intermediate A-2-7b (1.66 g, 8.30 mmol) were dissolved in dichloromethane (40 mL), and added with *N,N-*diisopropylethylamine (3.22 g, 24.90 mmol), *N*-hydroxy-7-azabenzotriazole (1.13 g, 8.30 mmol) and 1-(3-dimethylaminopropyl)-3-ethylcarbodiimide hydrochloride (1.59 g, 8.30 mmol). After the addition was completed, the reaction was stirred under ice bath for 1 hour, and then the reaction mixture was placed at room temperature. After the reaction was stirred overnight, completion of the reaction was indicated by TLC, and the reaction solution was added with water (50 ml) and extracted with dichloromethane (50 ml× 3). The organic phases were combined, successively washed with saturated brine (100 ml), dried with anhydrous sodium sulfate, and purified by silica gel column chromatography to obtain intermediate A-2-38d (2.00 g, 5.20 mmol, total yield of two steps 62.7%).

¹H NMR (400 MHz, CDCl3) δ 5.05 (dt, J = 12.4, 4.1 Hz, 1H), 4.70 (t, J = 8.0 Hz, 1H), 4.57 (dd, J = 8.7, 4.6 Hz, 1H), 4.03 - 3.80 (m, 4H), 3.64 - 3.52 (m, 2H), 2.46 (d, J = 29.1 Hz, 2H), 2.27 (qd, J = 11.7, 6.9 Hz, 1H), 2.03 - 1.90 (m, 2H), 1.73 (ddd, J = 13.1, 8.6, 4.0 Hz, 2H), 1.49 (s,9H), 0.99 (dd, J = 24.2, 6.9 Hz, 6H).

### Fourth step

Intermediate A-2-38d (1.10 g, 2.86 mmol) was dissolved in dichloromethane (5 mL), and added with 4 M solution of hydrogen chloride in dioxane (10 mL). The mixture was stirred at room temperature for 2 hours, and then completion of the reaction was indicated by TLC. The reaction solution was concentrated to obtain a crude product - intermediate A-2-38e, which was used directly in the next reaction step without purification.

### Fifth step

The crude product - intermediate A-2-38e was dissolved in tetrahydrofuran (20 mL), and added with *N*,*N*-diisopropylethylamine (1.11 g, 8.58 mmol). The mixture was stirred under ice bath for 10 minutes, and then slowly dropwise added with acetic anhydride (0.58 g, 5.72 mmol). After the reaction was stirred for 30 minutes, the reaction mixture was placed at room temperature. After the reaction was continuously stirred for 2 hours, completion of the reaction was indicated by TLC. The reaction solution was added with ethanol (10 mL), concentrated, and purified by silica gel column chromatography to obtain the product A-2-38 (0.60 g, 1.84 mmol, total yield of two steps 64.3%).

¹H NMR (400 MHz, CDCl3) δ 8.28 (d, J = 8.3 Hz, 1H), 4.95 (tt, J = 8.1, 4.0 Hz, 1H), 4.86 (dd, J = 9.5, 6.5 Hz, 1H), 4.41 (dd, J = 8.5, 4.7 Hz, 1H), 4.00 (t, J = 7.7 Hz, 2H), 3.88 - 3.74 (m, 2H), 3.49 (ddd, J = 11.6, 8.3, 3.2 Hz, 2H), 2.61 (ddt, J = 12.0, 8.0, 7.2 Hz, 1H), 2.37 (ddt, J = 12.0, 9.5, 7.2 Hz, 1H), 2.17 (dtd, J = 13.8, 6.9, 4.9 Hz, 1H), 1.88 (s, 3H), 1.87 - 1.81 (m, 2H), 1.69 - 1.53 (m, 2H), 0.90 (dd, J = 19.1, 6.9 Hz, 6H).

### Preparation Example 44 Preparation of compound A-2-39

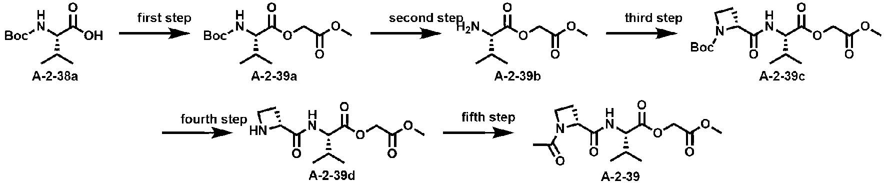

### First step

Intermediate A-2-38a (2.00 g, 9.21 mmol) was dissolved in dichloromethane (50 mL). Under ice bath, the mixture was sequentially added with methyl 2-hydroxyacetate (1.24 g, 13.81 mmol), 4-dimethylaminopyridine (1.69 g, 13.81 mmol) and N,N'-dicyclohexylcarbodiimide (4.27 g, 20.71 mmol). After the addition was completed, the mixture was stirred at room temperature for 12 hours, and completion of the reaction was indicated by TLC. The reaction solution was added with water (50 ml) and extracted with dichloromethane (50 ml× 3). The organic phases were combined, successively washed with saturated brine (200 ml), dried with anhydrous sodium sulfate, and purified by silica gel column chromatography to obtain intermediate A-2-39a (1.50 g, 5.18 mmol, yield 56.2%).

### Second step

Intermediate A-2-39a (1.50 g, 5.18 mmol) was dissolved in dichloromethane (20 mL), and added with 4 M solution of hydrogen chloride in dioxane (20 mL). The mixture was stirred at room temperature for 2 hours, and then completion of the reaction was indicated by TLC. The reaction solution was concentrated to obtain a crude product - intermediate A-2-39b, which was used directly in the next reaction step without purification.

### Third step

The crude product - intermediate A-2-39b and intermediate A-2-7b (1.06 g, 5.29 mmol) were dissolved in dichloromethane (40 mL). Under ice bath, the mixture was sequentially added with *N*,*N*-diisopropylethylamine (1.92 g, 14.91 mmol), *N*-hydroxy-7-azabenzotriazole (0.71 g, 5.29 mmol) and 1-(3-dimethylaminopropyl)-3-ethylcarbodiimide hydrochloride (1.01 g, 5.29 mmol). After the addition was completed, the reaction was continuously stirred under ice bath for 1 hour, and then the reaction solution was placed at room temperature. After the reaction was stirred overnight, completion of the reaction was indicated by TLC, and the reaction solution was added with water (50 ml) and extracted with dichloromethane (50 ml× 3). The organic phases were combined, successively washed with saturated brine (100 ml), dried with anhydrous sodium sulfate, and purified by silica gel column chromatography to obtain intermediate A-2-39c (1.10 g, 2.95 mmol, total yield of two steps 55.8%).

¹H NMR (400 MHz, CDCl3) δ 4.73 (d, J = 15.9 Hz, 1H), 4.60 (t, J = 7.7 Hz, 2H), 4.51 (d, J = 15.9 Hz, 1H), 3.87 (d, J = 8.2 Hz, 1H), 3.77 (dd, J = 14.1, 8.4 Hz, 1H), 3.70 (s, 3H), 2.38 (d, J = 15.2 Hz, 1H), 2.35 - 2.19 (m, 2H), 1.39 (s, 9H), 0.97 (d, J = 6.9 Hz, 3H), 0.93 (d, J = 6.9 Hz, 3H).

### Fourth step

Intermediate A-2-39c (1.10 g, 2.95 mmol) was dissolved in dichloromethane (5 mL), and added with 4 M solution of hydrogen chloride in dioxane (10 mL). The mixture was stirred at room temperature for 2 hours, and then completion of the reaction was indicated by TLC. The reaction solution was concentrated to obtain a crude product - intermediate A-2-39d, which was used directly in the next reaction step without purification.

### Fifth step

The crude product - intermediate A-2-39d was dissolved in tetrahydrofuran (20 mL), and added with *N*,*N*-diisopropylethylamine (1.14 g, 8.85 mmol). The mixture was stirred under ice bath for 10 minutes, and then slowly dropwise added with acetic anhydride (0.60 g, 5.90 mmol), and the reaction was continuously stirred for 30 minutes. The reaction solution was placed at room temperature, and after the reaction was continuously stirred for 2 hours, completion of the reaction was indicated by TLC. The reaction solution was added with ethanol (10 mL), concentrated, and purified by silica gel column chromatography to obtain the product A-2-39 (0.24 g, 0.76 mmol, total yield of two steps 25.8%).

¹H NMR (400 MHz, CDCl3) δ 8.30 (d, J = 8.0 Hz, 1H), 4.85 (dd, J = 9.5, 6.6 Hz, 1H), 4.73 (d, J = 15.9 Hz, 1H), 4.55 - 4.50 (m, 1H), 4.50 - 4.44 (m, 1H), 4.08 - 3.93 (m, 2H), 3.69 (s, 3H), 2.60 (ddt, J = 12.0, 9.0, 6.8 Hz, 1H), 2.37 (dtd, J = 11.9, 8.9, 6.0 Hz, 1H), 2.31 - 2.21 (m, 1H), 1.87 (s, 3H), 0.96 (dd, J = 12.9, 6.9 Hz, 6H).

### Preparation Example 45 Preparation of compound A-2-40

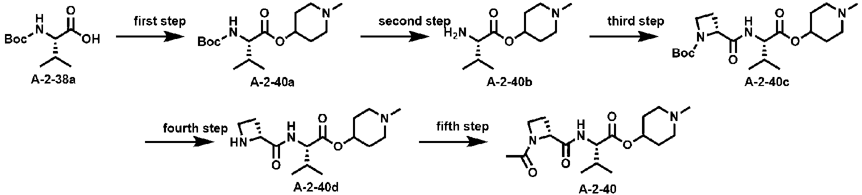

### First step

Intermediate A-2-38a (5.00 g, 23.04 mmol) was dissolved in dichloromethane (50 mL). Under ice bath, the mixture was sequentially added with 1-methylpiperidin-4-ol (3.97 g, 34.56 mmol), 4-dimethylaminopyridine (4.21 g, 34.56 mmol) and *N*,*N*'-dicyclohexylcarbodiimide (7.12 g, 34.56 mmol). After the addition was completed, the mixture was placed at room temperature. After the reaction was continuously stirred for 12 hours, completion of the reaction was indicated by TLC. The reaction solution was added with water (50 ml) and extracted with dichloromethane (50 ml× 3). The organic phases were combined, washed with saturated brine (200 ml), dried with anhydrous sodium sulfate, and purified by silica gel column chromatography to obtain intermediate A-2-40a (4.00 g, 12.72 mmol, yield 55.2%).

### Second step

Intermediate A-2-40a (4.00 g, 12.72 mmol) was dissolved in dichloromethane (20 mL), and added with 4 M solution of hydrogen chloride in dioxane (20 mL). After the reaction was stirred at room temperature for 2 hours, completion of the reaction was indicated by TLC. The reaction solution was concentrated to obtain a crude product - intermediate A-2-40b, which was used directly in the next reaction step without purification.

### Third step

The crude product - intermediate A-2-40b and intermediate A-2-7b (2.67 g, 13.36 mmol) were dissolved in dichloromethane (40 mL). Under ice bath, the mixture was added with *N,N*-diisopropylethylamine (3.16 g, 24.48 mmol), *N*-hydroxy-7-azabenzotriazole (1.8 g, 13.22 mmol) and 1-(3-dimethylaminopropyl)-3-ethylcarbodiimide hydrochloride (2.55 g, 13.36 mmol). After the addition was completed, the reaction was stirred for 1 hour, and then the reaction solution was placed at room temperature. After the reaction was stirred overnight, completion of the reaction was indicated by TLC, and the reaction solution was added with water (50 ml) and extracted with dichloromethane (50 ml× 3). The organic phases were combined, washed with saturated brine (100 ml), dried with anhydrous sodium sulfate, and purified by silica gel column chromatography to obtain intermediate A-2-40c (2.50 g, 6.29 mmol, total yield of two steps 47.1%). ¹H NMR (400 MHz, CDCl3) δ 4.88 - 4.74 (m, 1H), 4.66 - 4.53 (m, 1H), 4.47 (dd, J = 8.8, 4.6 Hz, 1H), 3.87 (q, J = 8.3 Hz, 1H), 3.76 (td, J = 8.5, 5.4 Hz, 1H), 2.54 (s, 2H), 2.47 - 2.27 (m, 2H), 2.22 (s, 3H), 2.17 (ddd, J = 18.4, 9.2, 4.5 Hz, 2H), 2.04 (s, 1H), 1.91 - 1.78 (m, 2H), 1.68 (dqd, J = 16.6, 8.3, 3.7 Hz, 2H), 1.40 (s, 9H), 0.89 (dd, J = 24.2, 6.9 Hz, 6H).

### Fourth step

Intermediate A-2-40c (2.50 g, 6.29 mmol) was dissolved in dichloromethane (5 mL), and added with 4 M solution of hydrogen chloride in dioxane (10 mL). The mixture was stirred at room temperature for 2 hours, and then completion of the reaction was indicated by TLC. The reaction solution was concentrated to obtain a crude product - intermediate A-2-40d, which was used directly in the next reaction step without purification.

### Fifth step

The crude product - intermediate A-2-40d was dissolved in tetrahydrofuran (20 mL), and added with *N*,*N*-diisopropylethylamine (2.44 g, 18.87 mmol). The mixture was stirred under ice bath for 10 minutes, and then slowly dropwise added with acetic anhydride (1.28 g, 12.58 mmol), the reaction was continuously stirred for 30 minutes, and then the reaction solution was placed at room temperature. After the reaction was continuously stirred for 2 hours, completion of the reaction was indicated by TLC. The reaction solution was added with ethanol (10 mL), concentrated, and purified by silica gel column chromatography to obtain the product A-2-40 (1.00 g, 2.95 mmol, total yield of two steps 46.8%). MS (ESI) calculated for [M+H]⁺ was 340.2; found: m/z 340.3. ¹H NMR (400 MHz, CDCl3) δ 8.35 (d, J = 7.4 Hz, 1H), 5.06 (s, 1H), 4.84 (dd, J = 9.3, 6.6 Hz, 1H), 4.29 (dd, J = 7.5, 5.5 Hz, 1H), 4.02 (t, J = 7.9 Hz, 2H), 3.12 (s,4H), 2.73 (s, 3H), 2.64 - 2.54 (m, 1H), 2.45 - 2.31 (m, 2H), 2.11 (dt, J = 13.5, 7.0 Hz, 4H), 1.88 (s, 3H), 0.92 (t, J = 7.0 Hz, 6H).

### Preparation Example 46 Preparation of compound A-2-41

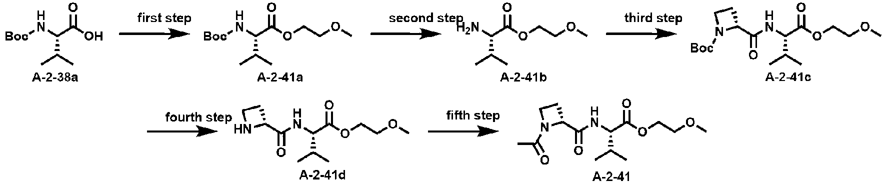

### First step

Intermediate A-2-41a (3.00 g, 13.82 mmol) was dissolved in dichloromethane (50 mL). Under ice bath, the mixture was sequentially added with 2-methoxyethan-1-ol (1.57 g, 20.73 mmol), 4-dimethylaminopyridine (2.52 g, 20.73 mmol) and N,N'-dicyclohexylcarbodiimide (4.27 g, 20.73 mmol). After the addition was completed, the mixture was placed at room temperature. After the reaction was continuously stirred for 12 hours, completion of the reaction was indicated by TLC. The reaction solution was added with water (50 ml) and extracted with dichloromethane (50 ml× 3). The organic phases were combined, washed with saturated brine (200 ml), dried with anhydrous sodium sulfate, and purified by silica gel column chromatography to obtain intermediate A-2-41a (1.50 g, 5.45 mmol, yield 39.4%).

### Second step

Intermediate A-2-41a (1.50 g, 5.45 mmol) was dissolved in dichloromethane (20 mL), and added with 4 M solution of hydrogen chloride in dioxane (20 mL). After the reaction was stirred at room temperature for 2 hours, completion of the reaction was indicated by TLC. The reaction solution was concentrated to obtain a crude product - intermediate A-2-41b, which was used directly in the next reaction step without purification.

### Third step

The crude product - intermediate A-2-41b and intermediate A-2-7b (1.21 g, 6.91 mmol) were dissolved in dichloromethane (40 mL). Under ice bath, the mixture was added with *N,N*-diisopropylethylamine (2.11 g, 16.35 mmol), N-hydroxy-7-azabenzotriazole (1.1 g, 8.18 mmol) and 1-(3-dimethylaminopropyl)-3-ethylcarbodiimide hydrochloride (1.57 g, 8.18 mmol). After the addition was completed, the reaction was stirred for 1 hour, and then the reaction solution was placed at room temperature. After the reaction was stirred overnight, completion of the reaction was indicated by TLC, and the reaction solution was added with water (50 ml) and extracted with dichloromethane (50 ml× 3). The organic phases were combined, washed with saturated brine (100 ml), dried with anhydrous sodium sulfate, and purified by silica gel column chromatography to obtain intermediate A-2-41c (1.00 g, 2.79 mmol, total yield of two steps 51.2%). ¹H NMR (400 MHz, CDCl3) δ 4.72 - 4.64 (m, 1H), 4.60 (dd, J = 8.9, 4.6 Hz, 1H), 4.40 - 4.32 (m, 1H), 4.31 - 4.23 (m, 1H), 3.94 (q, J = 8.2 Hz, 1H), 3.84 (td, J = 8.5, 5.4 Hz, 1H), 3.64 - 3.55 (m, 2H), 3.38 (s, 3H), 2.42 (d, J = 39.7 Hz, 2H), 2.25 (dtd, J = 13.7, 6.9, 4.9 Hz, 1H), 1.47 (s, 9H), 0.99 (d, J = 6.9 Hz, 3H), 0.93 (d, J = 6.9 Hz, 3H).

### Fourth step

Intermediate A-2-41c (1.00 g, 2.79 mmol) was dissolved in dichloromethane (5 mL), and added with 4 M solution of hydrogen chloride in dioxane (10 mL). The mixture was stirred at room temperature for 2 hours, and then completion of the reaction was indicated by TLC. The reaction solution was concentrated to obtain a crude product - intermediate A-2-41d, which was used directly in the next reaction step without purification.

### Fifth step

The crude product - intermediate A-2-41d was dissolved in tetrahydrofuran (20 mL), and added with *N*,*N*-diisopropylethylamine (1.08 g, 8.37 mmol). The mixture was stirred under ice bath for 10 minutes, and then slowly dropwise added with acetic anhydride (0.57 g, 5.58 mmol), the reaction was continuously stirred for 30 minutes, and then the reaction solution was placed at room temperature. After the reaction was continuously stirred for 2 hours, completion of the reaction was indicated by TLC. The reaction solution was added with ethanol (10 mL), concentrated, and purified by silica gel column chromatography to obtain the product A-2-41 (0.30 g, 1.00 mmol, total yield of two steps 35.8%). MS (ESI) calculated for [M+H]⁺ was 301.2; found: m/z 301.4. ¹H NMR (400 MHz, CDCl3) δ 8.29 (d, J = 8.1 Hz, 1H), 4.91 (dd, J = 9.4, 6.6 Hz, 1H), 4.52 (dd, J = 8.4, 4.7 Hz, 1H), 4.40 - 4.29 (m, 1H), 4.27 - 4.18 (m, 1H), 4.11 - 4.02 (m, 2H), 3.65 - 3.55 (m, 2H), 3.37 (s, 3H), 2.74 - 2.59 (m, 1H), 2.44 (dtd, J = 11.9, 8.8, 6.3 Hz, 1H), 2.34 - 2.18 (m, 1H), 1.95 (s, 3H), 0.99 (d, J = 6.9 Hz, 3H), 0.95 (d, J = 6.9 Hz, 3H).

### Preparation Example 47 Preparation of compound A-2-42

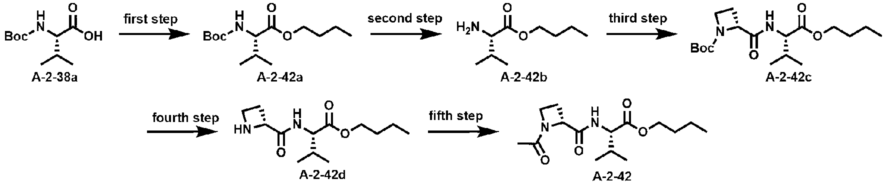

### First step

Intermediate A-2-38a (3.00 g, 13.82 mmol) was dissolved in dichloromethane (50 mL). Under ice bath, the mixture was sequentially added with n-butanol (1.54 g, 20.73 mmol), 4-dimethylaminopyridine (2.52 g, 20.73 mmol) and *N*,*N*'-dicyclohexylcarbodiimide (4.27 g, 20.73 mmol). After the addition was completed, the mixture was placed at room temperature. After the reaction was continuously stirred for 12 hours, completion of the reaction was indicated by TLC. The reaction solution was added with water (50 ml) and extracted with dichloromethane (50 ml× 3). The organic phases were combined, washed with saturated brine (200 ml), dried with anhydrous sodium sulfate, and purified by silica gel column chromatography to obtain intermediate A-2-42a (2.50 g, 9.15 mmol, yield 66.2%).

### Second step

Intermediate A-2-42a (2.50 g, 9.15 mmol) was dissolved in dichloromethane (20 mL), and added with 4 M solution of hydrogen chloride in dioxane (20 mL). After the reaction was stirred at room temperature for 2 hours, completion of the reaction was indicated by TLC. The reaction solution was concentrated to obtain a crude product - intermediate A-2-42b, which was used directly in the next reaction step without purification.

### Third step

The crude product - intermediate A-2-42b and intermediate A-2-7b (2.21 g, 10.98 mmol) were dissolved in dichloromethane (40 mL). Under ice bath, the mixture was added with *N,N*-diisopropylethylamine (3.55 g, 27.45 mmol), *N*-hydroxy-7-azabenzotriazole (1.48 g, 10.98 mmol) and 1-(3-dimethylaminopropyl)-3-ethylcarbodiimide hydrochloride (2.10 g, 10.98 mmol). After the addition was completed, the reaction was stirred for 1 hour, and then the reaction mixture was placed at room temperature. After the reaction was stirred overnight, completion of the reaction was indicated by TLC, and the reaction solution was added with water (50 ml) and extracted with dichloromethane (50 ml× 3). The organic phases were combined, washed with saturated brine (100 ml), dried with anhydrous sodium sulfate, and purified by silica gel column chromatography to obtain intermediate A-2-42c (2.00 g, 5.61 mmol, total yield of two steps 61.3%). ¹H NMR (400 MHz, CDCl3) δ 4.71 - 4.59 (m, 1H), 4.53 (dd, J = 9.0, 4.7 Hz, 1H), 4.18 - 4.08 (m, 2H), 3.92 (dd, J = 16.4, 8.3 Hz, 1H), 3.81 (td, J = 8.5, 5.4 Hz, 1H), 2.40 (d, J = 40.5 Hz, 2H), 2.26 - 2.14 (m, 1H), 1.65 - 1.57 (m, 2H), 1.45 (s, 9H), 1.38 (dd, J = 15.2, 7.4 Hz, 2H), 0.96 (d, J = 6.9 Hz, 3H), 0.94 - 0.89 (m, 6H).

### Fourth step

Intermediate A-2-42c (2.00 g, 5.61 mmol) was dissolved in dichloromethane (5 mL), and added with 4 M solution of hydrogen chloride in dioxane (10 mL). The mixture was stirred at room temperature for 2 hours, and then completion of the reaction was indicated by TLC. The reaction solution was concentrated to obtain a crude product - intermediate A-2-42d, which was used directly in the next reaction step without purification.

### Fifth step

The crude product - intermediate A-2-42d was dissolved in tetrahydrofuran (20 mL), and added with N,N-diisopropylethylamine (2.18 g, 16.83 mmol). The mixture was stirred under ice bath for 10 minutes, and then slowly dropwise added with acetic anhydride (1.15 g, 11.22 mmol), the reaction was continuously stirred for 30 minutes, and then the reaction mixture was placed at room temperature. After the reaction was continuously stirred for 2 hours, completion of the reaction was indicated by TLC. The reaction solution was added with ethanol (10 mL), concentrated, and purified by silica gel column chromatography to obtain the product A-2-42 (0.60 g, 2.01 mmol, total yield of two steps 35.8%). MS (ESI) calculated for [M+H]⁺ was 299.2; found: m/z 299.4. ¹H NMR (400 MHz, CDCl3) δ 8.27 (d, J = 8.3 Hz, 1H), 4.92 (dd, J = 9.5, 6.5 Hz, 1H), 4.48 (dd, J = 8.6, 4.7 Hz, 1H), 4.21 - 4.09 (m, 2H), 4.07 (t, J = 6.0 Hz, 2H), 2.73 - 2.59 (m, 1H), 2.51 - 2.38 (m, 1H), 2.31 - 2.16 (m, 1H), 1.95 (s, 3H), 1.68 - 1.58 (m, 2H), 1.40 (dt, J = 15.1, 7.4 Hz, 2H), 0.99 (d, J = 6.9 Hz, 3H), 0.96 - 0.91 (m, 6H).

### Preparation Example 48 Preparation of compound A-2-43

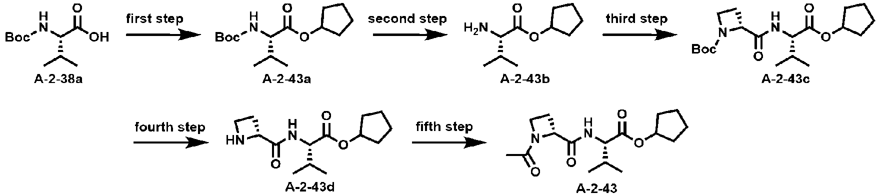

### First step

Intermediate A-2-38a (5.00 g, 23.04 mmol) was dissolved in dichloromethane (50 mL). Under ice bath, the mixture was sequentially added with cyclopentanol (2.97 g, 34.56 mmol), 4-dimethylaminopyridine (4.21 g, 34.56 mmol) and N,N'-dicyclohexylcarbodiimide (7.12 g, 34.56 mmol). After the addition was completed, the mixture was placed at room temperature. After the reaction was continuously stirred for 12 hours, completion of the reaction was indicated by TLC. The reaction solution was added with water (50 ml) and extracted with dichloromethane (50 ml× 3). The organic phases were combined, washed with saturated brine (200 ml), dried with anhydrous sodium sulfate, and purified by silica gel column chromatography to obtain intermediate A-2-43a (3.50 g, 12.28 mmol, yield 53.3%).

### Second step

Intermediate A-2-43a (3.50 g, 12.28 mmol) was dissolved in dichloromethane (20 mL), and added with 4 M solution of hydrogen chloride in dioxane (20 mL). After the reaction was stirred at room temperature for 2 hours, completion of the reaction was indicated by TLC. The reaction solution was concentrated to obtain a crude product - intermediate A-2-43b, which was used directly in the next reaction step without purification.

### Third step

The crude product - intermediate A-2-43b and intermediate A-2-7b (2.45 g, 12.28 mmol) were dissolved in dichloromethane (40 mL). Under ice bath, the mixture was added with *N,N*-diisopropylethylamine (3.16 g, 24.48 mmol), *N*-hydroxy-7-azabenzotriazole (1.65 g, 12.28 mmol) and 1-(3-dimethylaminopropyl)-3-ethylcarbodiimide hydrochloride (2.34 g, 12.28 mmol). After the addition was completed, the reaction was stirred for 1 hour, and then the reaction mixture was placed at room temperature. After the reaction was stirred overnight, completion of the reaction was indicated by TLC, and the reaction solution was added with water (50 ml) and extracted with dichloromethane (50 ml× 3). The organic phases were combined, washed with saturated brine (100 ml), dried with anhydrous sodium sulfate, and purified by silica gel column chromatography to obtain intermediate A-2-43c (2.00 g, 5.43 mmol, total yield of two steps 44.2%). ¹H NMR (400 MHz, CDCl3) δ 5.16 (ddd, J = 8.1, 5.8, 2.2 Hz, 1H), 4.67 - 4.52 (m, 1H), 4.44 (dd, J = 9.0, 4.6 Hz, 1H), 3.87 (dd, J = 16.4, 8.2 Hz, 1H), 3.76 (td, J = 8.5, 5.4 Hz, 1H), 2.35 (d, J = 43.0 Hz, 2H), 2.14 (qd, J = 6.8, 2.0 Hz, 1H), 1.84 - 1.75 (m, 2H), 1.72 - 1.59 (m, 4H), 1.55 (dd, J = 7.8, 3.3 Hz, 2H), 1.40 (s, 9H), 0.90 (d, J = 6.9 Hz, 3H), 0.84 (d, J = 6.9 Hz, 3H).

### Fourth step

Intermediate A-2-43c (2.00 g, 5.43 mmol) was dissolved in dichloromethane (5 mL), and added with 4 M solution of hydrogen chloride in dioxane (10 mL). The mixture was stirred at room temperature for 2 hours, and then completion of the reaction was indicated by TLC. The reaction solution was concentrated to obtain a crude product - intermediate A-2-43d, which was used directly in the next reaction step without purification.

### Fifth step

The crude product - intermediate A-2-43d was dissolved in tetrahydrofuran (20 mL), and added with *N*,*N*-diisopropylethylamine (2.11 g, 16.29 mmol). The mixture was stirred under ice bath for 10 minutes, and then slowly dropwise added with acetic anhydride (1.11 g, 10.86 mmol), the reaction was continuously stirred for 30 minutes, and then the reaction mixture was placed at room temperature. After the reaction was continuously stirred for 2 hours, completion of the reaction was indicated by TLC. The reaction solution was added with ethanol (10 mL), concentrated, and purified by silica gel column chromatography to obtain the product A-2-43 (0.80 g, 2.58 mmol, total yield of two steps 47.5%). ¹H NMR (400 MHz, CDCl3) δ 8.24 (d, J = 8.4 Hz, 1H), 5.21 (td, J = 5.7, 2.9 Hz, 1H), 4.92 (dd, J = 9.5, 6.5 Hz, 1H), 4.45 (dd, J = 8.7, 4.6 Hz, 1H), 4.06 (d, J = 7.2 Hz, 2H), 2.73 - 2.60 (m, 1H), 2.50 - 2.38 (m, 1H), 2.31 - 2.15 (m, 1H), 1.95 (s, 3H), 1.83 (ddd, J = 10.9, 7.3, 5.5 Hz, 2H), 1.78 - 1.65 (m, 4H), 1.61 (dd, J = 7.7, 2.5 Hz, 2H), 0.98 (d, J = 6.9 Hz, 3H), 0.92 (dd, J = 9.9, 4.8 Hz,3H).

### Preparation Example 49 Preparation of compound A-2-44

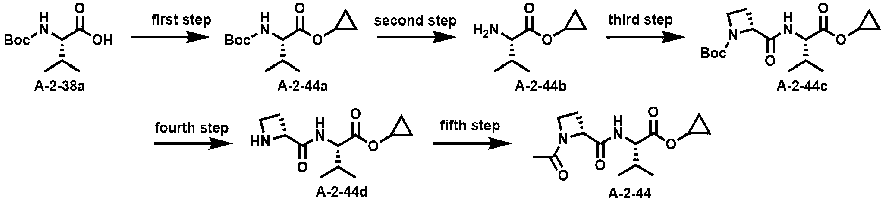

### First step

Intermediate A-2-38a (3.00 g, 13.82 mmol) was dissolved in dichloromethane (50 mL). Under ice bath, the mixture was sequentially added with cyclopropanol (1.20 g, 20.71 mmol), 4-dimethylaminopyridine (2.52 g, 20.71 mmol) and N,N'-dicyclohexylcarbodiimide (4.27 g, 20.71 mmol). After the addition was completed, the mixture was placed at room temperature. After the reaction was continuously stirred for 12 hours, completion of the reaction was indicated by TLC. The reaction solution was added with water (50 ml) and extracted with dichloromethane (50 ml× 3). The organic phases were combined, washed with saturated brine (20 ml), dried with anhydrous sodium sulfate, and purified by silica gel column chromatography to obtain intermediate A-2-44a (2.10 g, 8.17 mmol, yield 59.1%).

### Second step

Intermediate A-2-44a (2.10 g, 8.17 mmol) was dissolved in dichloromethane (20 mL), and added with 4 M solution of hydrogen chloride in dioxane (20 mL). After the reaction was stirred at room temperature for 2 hours, completion of the reaction was indicated by TLC. The reaction solution was concentrated to obtain a crude product - intermediate A-2-44b, which was used directly in the next reaction step without purification.

### Third step

The crude product - intermediate A-2-44b and intermediate A-2-7b (1.63 g, 8.16 mmol) were dissolved in dichloromethane (40 mL). Under ice bath, the mixture was added with *N,N*-diisopropylethylamine (3.16 g, 24.48 mmol), *N*-hydroxy-7-azabenzotriazole (1.10 g, 8.16 mmol) and 1-(3-dimethylaminopropyl)-3-ethylcarbodiimide hydrochloride (1.56 g, 8.16 mmol). After the addition was completed, the reaction was stirred for 1 hour, and then the reaction mixture was placed at room temperature. After the reaction was stirred overnight, completion of the reaction was indicated by TLC, and the reaction solution was added with water (50 ml) and extracted with dichloromethane (50 ml× 3). The organic phases were combined, washed with saturated brine (100 ml), dried with anhydrous sodium sulfate, and purified by silica gel column chromatography to obtain intermediate A-2-44c (1.50 g, 4.41 mmol, total yield of two steps 54.0%).

¹H NMR (400 MHz, CDCl3) δ 4.59 (t, J = 8.0 Hz, 1H), 4.44 (dd, J = 8.9, 4.8 Hz, 1H), 4.16 - 4.08 (m, 1H), 3.87 (dd, J = 16.4, 8.1 Hz, 1H), 3.77 (dd, J = 14.1, 8.4 Hz, 1H), 2.35 (d, J = 34.0 Hz, 2H), 2.16 - 2.05 (m, 1H), 1.40 (s, 9H), 0.90 (d, J = 6.8 Hz, 3H), 0.84 (d, J = 6.9 Hz, 3H), 0.70 - 0.61 (m, 4H).

### Fourth step

Intermediate A-2-44c (1.50 g, 4.41 mmol) was dissolved in dichloromethane (5 mL), and added with 4 M solution of hydrogen chloride in dioxane (10 mL). The mixture was stirred at room temperature for 2 hours, and then completion of the reaction was indicated by TLC. The reaction solution was concentrated to obtain a crude product - intermediate A-2-44d, which was used directly in the next reaction step without purification.

### Fifth step

The crude product - intermediate A-2-44d was dissolved in tetrahydrofuran (20 mL), and added with *N*,*N*-diisopropylethylamine (1.71 g, 13.23 mmol). The mixture was stirred under ice bath for 10 minutes, and then slowly dropwise added with acetic anhydride (0.90 g, 8.82 mmol), the reaction was continuously stirred for 30 minutes, and then the reaction mixture was placed at room temperature. After the reaction was continuously stirred for 2 hours, completion of the reaction was indicated by TLC. The reaction solution was added with ethanol (10 mL), concentrated, and purified by silica gel column chromatography to obtain the product A-2-44 (0.60 g, 2.12 mmol, total yield of two steps 48.2%). MS (ESI) calculated for [M+H]⁺ was 283.2; found: m/z 283.2. ¹H NMR (400 MHz, CDCl3) δ 8.21 (d, J = 8.3 Hz, 1H), 4.84 (dd, J = 9.5, 6.5 Hz, 1H), 4.36 (dd, J = 8.5, 4.8 Hz, 1H), 4.16 - 4.06 (m, 1H), 4.04 - 3.92 (m, 2H), 2.60 (ddt, J = 12.0, 8.8, 6.9 Hz, 1H), 2.37 (tdd, J = 12.0, 8.8, 6.2 Hz, 1H), 2.20 - 2.06 (m, 1H), 1.88 (s, 3H), 0.90 (d, J = 6.9 Hz, 3H), 0.86 (d, J = 6.9 Hz, 3H), 0.73 - 0.58 (m, 4H).

### Preparation Example 50 Preparation of compounds B-2-12 and B-2-13

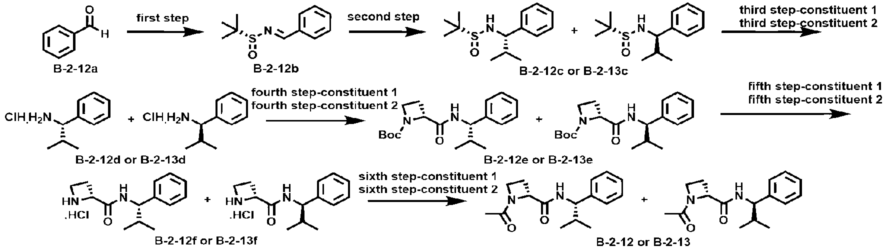

### First step

Intermediate B-2-12a (4.00 g, 37.69 mmol) was dissolved in dichloromethane (50 mL), and sequentially added with (*R*)-tert-butylsulfinamide (4.57 g, 37.69 mmol) and cesium carbonate (12.28 g, 37.69 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain ntermediate B-2-12b (7.00 g, 33.4 mmol, yield 88.7%). MS (ESI) calculated for [M+H]⁺ was 210.1; found: m/z 210.1.

### Second step

Intermediate B-2-12b (4.50 g, 21.50 mmol) was dissolved in tetrahydrofuran (50 mL). The mixture was subjected to nitrogen protection and cooled to -78 °C, and slowly dropwise added with 1 N solution of isopropylmagnesium bromide in tetrahydrofuran (40.00 mL, 40.00 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 3). The organic phases were combined, successively washed with saturated brine (100 mL × 2), dried with anhydrous sodium sulfate, filtered, concentrated, and purified by silica gel column chromatography to obtain two constituents.

Constituent 1 with a short retention time was intermediate B-2-12c (1.00 g, 3.94 mmol, yield 18.4%). MS (ESI) calculated for [M+H]⁺ was 254.2; found: m/z 254.1. ¹H NMR (400 MHz, Chloroform-d) δ 7.29 - 7.23 (m, 2H), 7.22 - 7.14 (m, 3H), 3.38 (d, J = 5.3 Hz, 1H), 2.35 - 1.95 (m, 1H), 1.18 (s, 9H), 0.80 (dd, J = 49.9, 6.8 Hz, 6H).

Constituent 2 with a long retention time was intermediate B-2-13c (0.24 g, 0.95 mmol, yield 4.4%). MS (ESI) calculated for [M+H]⁺ was 254.2; found: m/z 254.1. ¹H NMR (400 MHz, Chloroform-d) δ 7.28 - 7.22 (m, 2H), 7.22 - 7.15 (m, 3H), 4.09 (dd, J = 6.7, 2.2 Hz, 1H), 3.42 (s, 1H), 1.90 (h, J = 6.8 Hz, 1H), 1.12 (s, 9H), 0.83 (dd, J = 72.1, 6.8 Hz, 6H).

### Third step - constituent 1

Intermediate B-2-12c (1.00 g, 3.95 mmol) was dissolved in dichloromethane (10 mL). Under ice bath, the mixture was added with 4 M solution of hydrogen chloride in dioxane (20 mL). The reaction was continuously stirred under ice bath for 1 hour, and then the reaction solution was concentrated to obtain a crude product - intermediate B-2-12d, which was used directly in the next reaction step without purification.

### Fourth step - constituent 1

The crude product B-2-12d was dissolved in dichloromethane (10 mL), and sequentially added with intermediate A-2-7b (1.08 g, 5.38 mmol), *N*-(3-dimethylaminopropyl)-*N*'-ethylcarbodiimide hydrochloride (1.41 g, 7.33 mmol), 1-hydroxybenzotriazole (0.99 g, 7.33 mmol) and ethyldiisopropylamine (1.90 g, 14.67 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-12e (0.55 g, 1.65 mmol, yield 33.8%). MS-ESI theoretical value [M-Boc+2H]⁺: 233.2; found: m/z 233.2. ¹H NMR (400 MHz, Chloroform-d) δ 7.24 (dd, J = 8.1, 6.4 Hz, 2H), 7.17 (td, J = 7.5, 6.7, 2.0 Hz, 3H), 4.74 (dd, J = 9.4, 7.0 Hz, 1H), 4.66 (s, 1H), 4.05 (q, J = 7.1 Hz, 1H), 3.76 (dq, J = 55.3, 8.2 Hz, 2H), 2.32 (s, 2H), 1.97 - 1.92 (m, 1H), 1.42 (s, 9H), 0.83 (dd, J = 27.0, 6.7 Hz, 6H).

### Fifth step - constituent 1

Intermediate B-2-12e (0.55g, 1.65 mmol) was dissolved in dichloromethane (5 mL). Under ice bath, the mixture was added with 4 M solution of hydrogen chloride in dioxane (10 mL), and the reaction was continuously stirred for 2 hours. The reaction solution was concentrated to obtain a crude product - intermediate B-2-12f, which was used directly in the next reaction step without purification.

### Sixth step - constituent 1

The crude product - intermediate B-2-12f was dissolved in dichloromethane (5 mL), cooled to -10°C, and added with acetyl chloride (0.16 g, 2.08 mmol) and ethyldiisopropylamine (0.73 g, 5.67 mmol). The reaction was stirred at -10°C for 1 hour, added with water (5 mL), and extracted with dichloromethane (5 mL × 3). The organic phases were combined, washed with saturated brine (5 mL × 3), dried with anhydrous sodium sulfate, filtered, concentrated, and purified by silica gel column chromatography to obtain the product B-2-12 (86.2 mg, 0.31 mmol, total yield of two steps 19.0%). MS (ESI) calculated for [M+H]⁺ was 275.2; found: m/z 275.1. ¹H NMR (400 MHz, Chloroform-d) δ 8.51 (d, J = 9.1 Hz, 1H), 7.28 - 7.21 (m, 2H), 7.18 - 7.06 (m, 3H), 4.90 (dd, J = 9.5, 6.5 Hz, 1H), 4.71 (dd, J = 9.1, 6.9 Hz, 1H), 4.02 - 3.81 (m, 2H), 2.59 (ddt, J = 12.0, 9.4, 6.7 Hz, 1H), 2.42 - 2.18 (m, 1H), 1.94 (dt, J = 13.6, 6.7 Hz, 1H), 1.86 (s, 3H), 0.83 (dd, J = 33.1, 6.8 Hz, 6H).

### Third step - constituent 2

Intermediate B-2-13c (0.24 g, 0.95 mmol) was dissolved in dichloromethane (2 mL). Under ice bath, the mixture was added with 4 M solution of hydrogen chloride in dioxane (4 mL). The reaction was continuously stirred under ice bath for 1 hour, and then the reaction solution was concentrated to obtain a crude product - intermediate B-2-13d, which was used directly in the next reaction step without purification.

### Fourth step - constituent 2

The crude product B-2-13d was dissolved in dichloromethane (10 mL), and sequentially added with intermediate A-2-7b (0.27 g, 1.33 mmol), N-(3-dimethylaminopropyl)-N'-ethylcarbodiimide hydrochloride (0.35 g, 1.81 mmol), 1-hydroxybenzotriazole (0.25 g, 1.81 mmol) and ethyldiisopropylamine (0.47g, 3.63 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-13e (0.32 g, 0.96 mmol, total yield of two steps 100%). MS-ESI theoretical value [M-Boc+2H]⁺: 233.2; found: m/z 233.2.

### Fifth step - constituent 2

Intermediate B-2-13e (0.32g, 0.96 mmol) was dissolved in dichloromethane (3 mL). Under ice bath, the mixture was added with 4 M solution of hydrogen chloride in dioxane (6 mL), and the reaction was continuously stirred for 2 hours. The reaction solution was concentrated to obtain a crude product - intermediate B-2-13f, which was used directly in the next reaction step without purification.

### Sixth step - constituent 2

The crude product - intermediate B-2-13f was dissolved in dichloromethane (5 mL), cooled to -10°C, and added with acetyl chloride (97.0 mg, 1.23 mmol) and ethyldiisopropylamine (0.43 g, 3.36 mmol). The reaction was stirred at -10°C for 1 hour, added with water (5 mL), and extracted with dichloromethane (5 mL × 3). The organic phases were combined, washed with saturated brine (5 mL × 3), dried with anhydrous sodium sulfate, filtered, concentrated, and purified by silica gel column chromatography to obtain the product B-2-13 (118.2 mg, 0.43 mmol, total yield of two steps 44.8%). MS (ESI) calculated for [M+H]⁺ was 275.2; found: m/z 275.1. ¹HNMR (400 MHz, Chloroform-d) δ 8.45 (d, J = 8.8 Hz, 1H), 7.23 (d, J = 7.2 Hz, 2H), 7.20 - 7.12 (m, 3H), 4.79 (dd, J = 9.5, 6.5 Hz, 1H), 4.66 (dd, J = 8.8, 7.0 Hz, 1H), 4.01 (ddd, J = 9.0, 6.5, 3.8 Hz, 2H), 2.78 - 2.49 (m, 1H), 2.42 - 2.22 (m, 1H), 1.95 (dd, J = 13.4, 6.6 Hz, 1H), 1.89 (s, 3H), 0.79 (dd, J = 25.4, 6.7 Hz, 6H).

### Preparation Example 51 Preparation of compounds B-2-14 and B-2-15

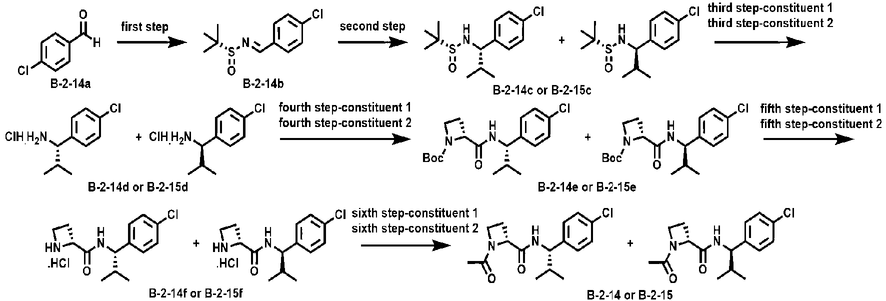

### First step

Intermediate B-2-14a (4.00 g, 28.46 mmol) was dissolved in dichloromethane (50 mL), and sequentially added with (*R*)-tert-butylsulfinamide (3.45 g, 28.46 mmol) and cesium carbonate (13.91 g, 42.69 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain intermediate B-2-14b (6.00 g, 24.6 mmol, yield 86.5%).

### Second step

Intermediate B-2-14b (5.00 g, 20.51 mmol) was dissolved in tetrahydrofuran (50 mL). The mixture was subjected to nitrogen protection and cooled to -78 °C, and slowly dropwise added with 1 N solution of isopropylmagnesium bromide in tetrahydrofuran (41.0 mL, 41.00 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 3). The organic phases were combined, successively washed with saturated brine (100 mL × 2), dried with anhydrous sodium sulfate, filtered, concentrated, and purified by silica gel column chromatography to obtain two constituents.

Constituent 1 with a short retention time was intermediate B-2-14c (0.70 g, 2.43 mmol, yield 11.9%).

Constituent 2 with a long retention time was intermediate B-2-15c (2.00 g, 6.94 mmol, yield 33.9%).

### Third step - constituent 1

Intermediate B-2-14c (0.70 g, 2.43 mmol) was dissolved in dichloromethane (10 mL). Under ice bath, the mixture was added with 4 M solution of hydrogen chloride in dioxane (20 mL). The reaction was continuously stirred under ice bath for 1 hour, and then the reaction solution was concentrated to obtain a crude product - intermediate B-2-14d, which was used directly in the next reaction step without purification.

### Fourth step - constituent 1

The crude product B-2-14d was dissolved in dichloromethane (10 mL), and sequentially added with intermediate A-2-7b (0.65 g, 3.32 mmol), *N*-(3-dimethylaminopropyl)-*N*'-ethylcarbodiimide hydrochloride (0.85 g, 4.41 mmol), 1-hydroxybenzotriazole (0.60 g, 4.41 mmol) and ethyldiisopropylamine (1.14 g, 8.82 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-14e (0.45 g, 1.23 mmol, total yield of two steps 50.5%). MS-ESI theoretical value [M-Boc+2H]⁺: 267.1; found: m/z 267.1.

### Fifth step - constituent 1

Intermediate B-2-14e (0.45 g, 1.23 mmol) was dissolved in dichloromethane (5 mL). Under ice bath, the mixture was added with 4 M solution of hydrogen chloride in dioxane (10 mL), and the reaction was continuously stirred for 2 hours. The reaction solution was concentrated to obtain a crude product - intermediate B-2-14f, which was used directly in the next reaction step without purification.

### Sixth step - constituent 1

The crude product - intermediate B-2-14f was dissolved in dichloromethane (5 mL), cooled to -10°C, and added with acetyl chloride (0.12 g, 1.53 mmol) and ethyldiisopropylamine (0.54 g, 4.17 mmol). The reaction was stirred at **-10°C** for 1 hour, added with water (5 mL), and extracted with dichloromethane (5 mL × 3). The organic phases were combined, washed with saturated brine (5 mL × 3), dried with anhydrous sodium sulfate, filtered, concentrated, and purified by silica gel column chromatography to obtain the product B-2-14 (155.0 mg, 0.50 mmol, total yield of two steps 40.8%). MS (ESI) calculated for [M+H]⁺ was 309.1; found: m/z 309.1. ¹H NMR (400 MHz, Chloroform-d) δ 8.53 (d, J = 8.9 Hz, 1H), 7.21 (d, J = 2.0 Hz, 1H), 7.19 (s, 1H), 7.09 - 7.03 (m, 2H), 4.89 (dd, J = 9.5, 6.5 Hz, 1H), 4.65 (dd, J = 8.8, 6.9 Hz, 1H), 4.08 - 3.82 (m, 2H), 2.45 (ddtd, J = 116.3, 12.0, 9.3, 6.1 Hz, 2H), 1.95 - 1.88 (m, 1H), 1.86 (s, 3H), 0.82 (dd, J = 35.7, 6.7 Hz, 6H).

### Third step - constituent 2

Intermediate B-2-15c (2.00 g, 6.95 mmol) was dissolved in dichloromethane (20 mL). Under ice bath, the mixture was added with 4 M solution of hydrogen chloride in dioxane (40 mL). The reaction was continuously stirred under ice bath for 1 hour, and then the reaction solution was concentrated to obtain a crude product - intermediate B-2-15d, which was used directly in the next reaction step without purification.

### Fourth step - constituent 2

The crude product B-2-15d was dissolved in dichloromethane (20 mL), and sequentially added with intermediate A-2-7b (1.81 g, 8.99 mmol), *N*-(3-dimethylaminopropyl)-*N*'-ethylcarbodiimide hydrochloride (2.35 g, 12.25 mmol), 1-hydroxybenzotriazole (1.66 g, 12.25 mmol) and ethyldiisopropylamine (3.17 g, 24.51 mmol) at -10°C. After the addition was completed, the reaction was continuously stirred at -10°C for 2 hours, and the reaction solution was added with water (10 mL) and extracted with dichloromethane (20 mL × 3). The organic phases were combined, successively washed with saturated brine (20 mL× 3), dried with anhydrous sodium sulfate, filtered, concentrated, and purified by silica gel column chromatography to obtain intermediate B-2-15e (0.70 g, 1.91 mmol, total yield of two steps 27.5%). MS-ESI theoretical value [M-Boc+2H]⁺: 267.1; found: m/z 267.1.

### Fifth step - constituent 2

Intermediate B-2-15e (0.70 g, 1.91 mmol) was dissolved in dichloromethane (10 mL). Under ice bath, the mixture was added with 4 M solution of hydrogen chloride in dioxane (20 mL), and the reaction was continuously stirred for 2 hours. The reaction solution was concentrated to obtain a crude product - intermediate B-2-15f, which was used directly in the next reaction step without purification.

### Sixth step - constituent 2

The crude product - intermediate B-2-15f was dissolved in dichloromethane (5 mL), cooled to -10°C, and added with acetyl chloride (0.16 g, 2.10 mmol) and ethyldiisopropylamine (0.74 g, 5.73 mmol). The reaction was stirred at -10°C for 1 hour, added with water (5 mL), and extracted with dichloromethane (5 mL × 3). The organic phases were combined, washed with saturated brine (5 mL × 3), dried with anhydrous sodium sulfate, filtered, concentrated, and purified by silica gel column chromatography to obtain the product B-2-15 (74.0 mg, 0.24 mmol, total yield of two steps 12.5%). MS (ESI) calculated for [M+H]⁺ was 309.1; found: m/z 309.1. ¹H NMR (400 MHz, Chloroform-d) δ 8.48 (d, J = 8.5 Hz, 1H), 7.33 - 7.17 (m, 2H), 7.16 - 7.00 (m, 2H), 4.79 (dd, J = 9.5, 6.5 Hz, 1H), 4.60 (dd, J = 8.5, 7.0 Hz, 1H), 4.01 (dd, J = 9.0, 6.5 Hz, 2H), 2.66 (dtd, J = 12.0, 8.7, 6.8 Hz, 1H), 2.31 (ddt, J = 12.3, 5.9, 4.7 Hz, 1H), 1.89 (s, 4H), 0.78 (dd, J = 25.4, 6.7 Hz, 6H).

### Preparation Example 52 Preparation of compounds B-2-16 and B-2-17

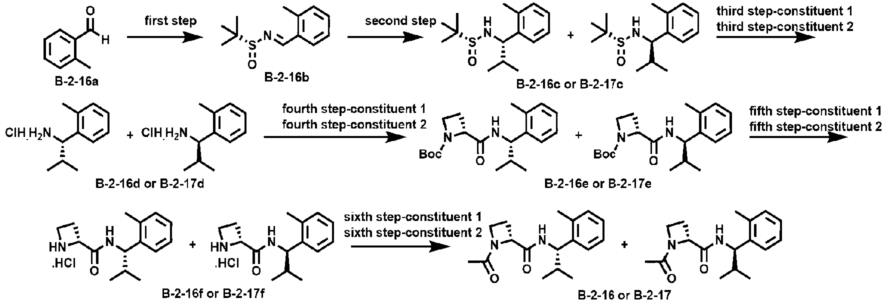

### First step

Intermediate B-2-16a (4.00 g, 33.29 mmol) was dissolved in dichloromethane (50 mL), and sequentially added with (R)-tert-butylsulfinamide (4.03 g, 33.29 mmol) and cesium carbonate (16.27 g, 49.94 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain intermediate B-2-16b (6.50 g, 29.1 mmol, yield 87.4%).

### Second step

Intermediate B-2-16b (5.00 g, 22.39 mmol) was dissolved in tetrahydrofuran (50 mL). The mixture was subjected to nitrogen protection and cooled to -78 °C, and slowly dropwise added with 1 N solution of isopropylmagnesium bromide in tetrahydrofuran (44.5 mL, 40.50 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 3). The organic phases were combined, successively washed with saturated brine (100 mL × 2), dried with anhydrous sodium sulfate, filtered, concentrated, and purified by silica gel column chromatography to obtain two constituents.

Constituent 1 with a short retention time was intermediate B-2-16c (1.86 g, 6.96 mmol, yield 31.1%). MS (ESI) calculated for [M+H]⁺ was 268.2; found: m/z 268.1. ¹H NMR (400 MHz, Chloroform-d) δ 7.19 - 7.03 (m, 4H), 4.24 (dd, J = 7.7, 6.2 Hz, 1H), 3.32 (d, J = 6.2 Hz, 1H), 2.33 (s, 3H), 2.15 - 1.91 (m, 1H), 1.14 (s, 9H), 0.85 (dd, J = 90.1, 6.7 Hz, 6H).

Constituent 2 with a long retention time was intermediate B-2-17c (0.50 g, 1.87 mmol, yield 8.4%). MS (ESI) calculated for [M+H]⁺ was 268.2; found: m/z 268.1. ¹H NMR (400 MHz, Chloroform-d) δ7.19-7.04 (m, 4H), 4.33 (dd, J = 7.6, 2.6 Hz, 1H), 3.62-3.27 (m, 1H), 2.32 (s, 3H), 2.04-1.82 (m, 1H), 1.09 (s, 9H), 0.87 (dd, J = 88.1, 6.7 Hz, 6H).

### Third step - constituent 1

Intermediate B-2-16c (1.86 g, 6.96 mmol) was dissolved in dichloromethane (15 mL). Under ice bath, the mixture was added with 4 M solution of hydrogen chloride in dioxane (30 mL). The reaction was continuously stirred under ice bath for 1 hour, and then the reaction solution was concentrated to obtain a crude product - intermediate B-2-16d, which was used directly in the next reaction step without purification.

### Fourth step - constituent 1

The crude product B-2-16d was dissolved in dichloromethane (20 mL), and sequentially added with intermediate A-2-7b (1.36 g, 6.74 mmol), *N*-(3-dimethylaminopropyl)-*N*'-ethylcarbodiimide hydrochloride (1.76 g, 9.20 mmol), 1-hydroxybenzotriazole (1.24 g, 9.20 mmol) and ethyldiisopropylamine (2.38 g, 18.39 mmol) at -10°C. After the addition was completed, the reaction was continuously stirred at -10°C for 2 hours, and the reaction solution was added with water (10 mL) and extracted with dichloromethane (20 mL × 3). The organic phases were combined, successively washed with saturated brine (20 mL× 3), dried with anhydrous sodium sulfate, filtered, concentrated, and purified by silica gel column chromatography to obtain intermediate B-2-16e (1.30 g, 3.75 mmol, total yield of two steps 53.9%). MS-ESI theoretical value [M-Boc+2H]⁺: 247.2; found: m/z 247.3.

### Fifth step - constituent 1

Intermediate B-2-16e (1.00 g, 2.89 mmol) was dissolved in dichloromethane (10 mL). Under ice bath, the mixture was added with 4 M solution of hydrogen chloride in dioxane (20 mL), and the reaction was continuously stirred for 2 hours. The reaction solution was concentrated to obtain a crude product - intermediate B-2-16f, which was used directly in the next reaction step without purification.

### Sixth step - constituent 1

The crude product - intermediate B-2-16f was dissolved in dichloromethane (10 mL), cooled to -10°C, and added with acetyl chloride (0.25 g, 3.12 mmol) and ethyldiisopropylamine (1.10 g, 8.52 mmol). The reaction was stirred at -10°C for 1 hour, added with water (10 mL), and extracted with dichloromethane (5 mL × 3). The organic phases were combined, washed with saturated brine (10 mL × 3), dried with anhydrous sodium sulfate, filtered, concentrated, and purified by silica gel column chromatography to obtain the product B-2-16 (331.5 mg, 1.15 mmol, total yield of two steps 39.8%). MS (ESI) calculated for [M+H]⁺ was 289.2; found: m/z 289.4. ¹H NMR (400 MHz, Chloroform-d) δ 8.40 (d, J = 8.9 Hz, 1H), 7.13 (t, J = 7.8 Hz, 1H), 7.05 - 6.90 (m, 3H), 4.79 (dd, J = 9.5, 6.4 Hz, 1H), 4.61 (dd, J = 8.9, 7.1 Hz, 1H), 4.10 - 3.91 (m, 2H), 2.78 - 2.60 (m, 1H), 2.30 (dq, J = 9.0, 3.0 Hz, 1H), 2.26 (s, 3H), 1.99 - 1.90 (m, 1H), 1.89 (s, 3H), 0.79 (dd, J = 27.0, 6.8 Hz, 6H).

### Third step - constituent 2

Intermediate B-2-17c (0.50 g, 1.87 mmol) was dissolved in dichloromethane (5 mL). Under ice bath, the mixture was added with 4 M solution of hydrogen chloride in dioxane (10 mL). The reaction was continuously stirred under ice bath for 1 hour, and then the reaction solution was concentrated to obtain a crude product - intermediate B-2-17d, which was used directly in the next reaction step without purification.

### Fourth step - constituent 2

The crude product B-2-17d was dissolved in dichloromethane (20 mL), and sequentially added with intermediate A-2-7b (0.62 g, 3.10 mmol), *N*-(3-dimethylaminopropyl)-*N*'-ethylcarbodiimide hydrochloride (0.81 g, 4.23 mmol), 1-hydroxybenzotriazole (0.57 g, 4.23 mmol) and ethyldiisopropylamine (1.09 g, 8.46 mmol) at -10°C. After the addition was completed, the reaction was continuously stirred at -10°C for 2 hours, and the reaction solution was added with water (10 mL) and extracted with dichloromethane (20 mL × 3). The organic phases were combined, successively washed with saturated brine (20 mL× 3), dried with anhydrous sodium sulfate, filtered, concentrated, and purified by silica gel column chromatography to obtain intermediate B-2-17e (0.50 g, 1.44 mmol, total yield of two steps 77.0%). MS-ESI theoretical value [M-Boc+2H]⁺: 247.2; found: m/z 247.4.

### Fifth step - constituent 2

Intermediate B-2-17e (0.50 g, 1.44 mmol) was dissolved in dichloromethane (5 mL). Under ice bath, the mixture was added with 4 M solution of hydrogen chloride in dioxane (10 mL), and the reaction was continuously stirred for 2 hours. The reaction solution was concentrated to obtain a crude product - intermediate B-2-17f, which was used directly in the next reaction step without purification.

### Sixth step - constituent 2

The crude product - intermediate B-2-17f was dissolved in dichloromethane (5 mL), cooled to -10°C, and added with acetyl chloride (0.13 g, 1.61 mmol) and ethyldiisopropylamine (0.57 g, 4.38 mmol). The reaction was stirred at -10°C for 1 hour, added with water (5 mL), and extracted with dichloromethane (5 mL × 3). The organic phases were combined, washed with saturated brine (5 mL × 3), dried with anhydrous sodium sulfate, filtered, concentrated, and purified by silica gel column chromatography to obtain the product B-2-17 (130.4 mg, 0.45 mmol, total yield of two steps 31.4%). MS (ESI) calculated for [M+H]⁺ was 289.2; found: m/z 289.2. ¹H NMR (400 MHz, Chloroform-d) δ 8.41 (d, J = 8.9 Hz, 1H), 7.18 - 6.97 (m, 4H), 4.88 (t, J = 8.3 Hz, 1H), 4.75 (dd, J = 9.5, 6.4 Hz, 1H), 4.11 - 3.87 (m, 2H), 2.66 (ddt, J = 12.0, 9.2, 6.7 Hz, 1H), 2.36 (s, 3H), 2.35 - 2.19 (m, 1H), 1.95 (d, J = 23.8 Hz, 1H), 1.89 (s, 3H), 0.82 (dd, J = 49.2, 6.7 Hz, 6H).

### Preparation Example 53 Preparation of compounds B-2-18 and B-2-19

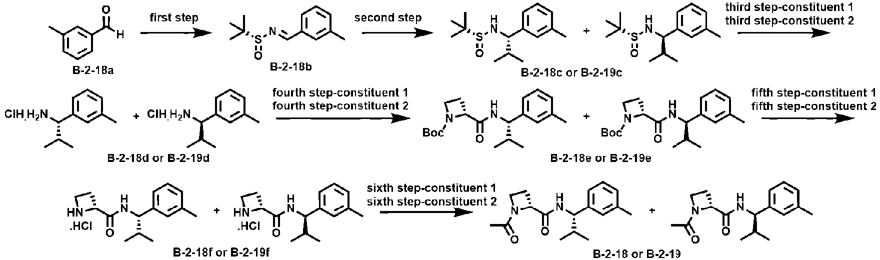

### First step

Intermediate B-2-18a (4.00 g, 33.29 mmol) was dissolved in dichloromethane (50 mL), and sequentially added with (*R*)-tert-butylsulfinamide (4.03 g, 33.29 mmol) and cesium carbonate (16.27 g, 49.94 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain intermediate B-2-18b (7.20 g, 32.24 mmol, yield 96.8%).

### Second step

Intermediate B-2-18b (5.00 g, 22.39 mmol) was dissolved in tetrahydrofuran (50 mL). The mixture was subjected to nitrogen protection and cooled to -78 °C, and slowly dropwise added with 1 N solution of isopropylmagnesium bromide in tetrahydrofuran (44.5 mL, 44.50 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 3). The organic phases were combined, successively washed with saturated brine (100 mL × 2), dried with anhydrous sodium sulfate, filtered, concentrated, and purified by silica gel column chromatography to obtain two constituents.

Constituent 1 with a short retention time was intermediate B-2-18c (0.95 g, 3.55 mmol, yield 15.9%). MS (ESI) calculated for [M+H]⁺ was 268.2; found: m/z 268.2.

Constituent 2 with a long retention time was intermediate B-2-19c (0.50 g, 1.87 mmol, yield 8.4%). MS (ESI) calculated for [M+H]⁺ was 268.2; found: m/z 268.2.

### Third step - constituent 1

Intermediate B-2-18c (0.95 g, 3.55 mmol) was dissolved in dichloromethane (10 mL). Under ice bath, the mixture was added with 4 M solution of hydrogen chloride in dioxane (20 mL). The reaction was continuously stirred under ice bath for 1 hour, and then the reaction solution was concentrated to obtain a crude product - intermediate B-2-18d, which was used directly in the next reaction step without purification.

### Fourth step - constituent 1

The crude product B-2-18d was dissolved in dichloromethane (10 mL), and sequentially added with intermediate A-2-7b (1.22 g, 6.06 mmol), *N*-(3-dimethylaminopropyl)-*N*'-ethylcarbodiimide hydrochloride (1.58 g, 8.27 mmol), 1-hydroxybenzotriazole (1.12 g, 8.27 mmol) and ethyldiisopropylamine (2.14 g, 16.53 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-18e (1.30 g, 3.75 mmol, total yield of two reaction steps 100%). MS (ESI) calculated for [M-Boc+2H]⁺ was 247.2; found: m/z 247.4.

### Fifth step - constituent 1

Intermediate B-2-18e (1.30 g, 3.75 mmol) was dissolved in dichloromethane (10 mL). Under ice bath, the mixture was added with 4 M solution of hydrogen chloride in dioxane (20 mL), and the reaction was continuously stirred for 2 hours. The reaction solution was concentrated to obtain a crude product - intermediate B-2-18f, which was used directly in the next reaction step without purification.

### Sixth step - constituent 1

The crude product - intermediate B-2-18f was dissolved in dichloromethane (10 mL), cooled to -10°C, and added with acetyl chloride (0.35 g, 4.47 mmol) and ethyldiisopropylamine (1.57 g, 12.18 mmol). The reaction was stirred at -10°C for 1 hour, added with water (10 mL), and extracted with dichloromethane (5 mL × 3). The organic phases were combined, washed with saturated brine (10 mL × 3), dried with anhydrous sodium sulfate, filtered, concentrated, and purified by silica gel column chromatography to obtain the product B-2-18 (192.1 mg, 0.67 mmol, total yield of two steps 17.8%). MS (ESI) calculated for [M+H]⁺ was 289.2; found: m/z 289.2. ¹H NMR (400 MHz, Chloroform-d) δ 8.40 (d, J = 8.9 Hz, 1H), 7.13 (t, J = 7.8 Hz, 1H), 7.04 - 6.86 (m, 3H), 4.79 (dd, J = 9.5, 6.4 Hz, 1H), 4.61 (dd, J = 8.9, 7.1 Hz, 1H), 4.14 - 3.83 (m, 2H), 2.68 (ddt, J = 12.0, 9.1, 6.7 Hz, 1H), 2.34 - 2.27 (m, 1H), 2.26 (s, 3H), 1.95 (d, J = 19.6 Hz, 1H), 1.89 (s, 3H), 0.79 (dd, J = 27.0, 6.8 Hz, 6H).

### Third step - constituent 2

Intermediate B-2-19c (0.50 g, 1.87 mmol) was dissolved in dichloromethane (5 mL). Under ice bath, the mixture was added with 4 M solution of hydrogen chloride in dioxane (10 mL). The reaction was continuously stirred under ice bath for 1 hour, and then the reaction solution was concentrated to obtain a crude product - intermediate B-2-19d, which was used directly in the next reaction step without purification.

### Fourth step - constituent 2

The crude product B-2-19d was dissolved in dichloromethane (10 mL), and sequentially added with intermediate A-2-7b (0.64 g, 3.17 mmol), *N*-(3-dimethylaminopropyl)-*N*'-ethylcarbodiimide hydrochloride (0.83 g, 4.32 mmol), 1-hydroxybenzotriazole (0.58 g, 4.32 mmol) and ethyldiisopropylamine (1.12 g, 8.64 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-19e (0.55 g, 1.59 mmol, total yield of two steps 84.9%). MS (ESI) calculated for [M-Boc+2H]⁺ was 247.2; found: m/z 247.3.

### Fifth step - constituent 2

Intermediate B-2-19e (0.55 g, 1.59 mmol) was dissolved in dichloromethane (5 mL). Under ice bath, the mixture was added with 4 M solution of hydrogen chloride in dioxane (10 mL), and the reaction was continuously stirred for 2 hours. The reaction solution was concentrated to obtain a crude product - intermediate B-2-19f, which was used directly in the next reaction step without purification.

### Sixth step - constituent 2

The crude product - intermediate B-2-19f was dissolved in dichloromethane (5 mL), cooled to -10°C, and added with acetyl chloride (0.14 g, 1.78 mmol) and ethyldiisopropylamine (0.63 g, 4.63 mmol). The reaction was stirred at -10°C for 1 hour, added with water (5 mL), and extracted with dichloromethane (5 mL × 3). The organic phases were combined, washed with saturated brine (5 mL × 3), dried with anhydrous sodium sulfate, filtered, concentrated, and purified by silica gel column chromatography to obtain the product B-2-19 (99.9 mg, 0.35 mmol, total yield of two steps 21.8%). MS (ESI) calculated for [M+H]⁺ was 289.2; found: m/z 289.4. ¹H NMR (400 MHz, Chloroform-d) δ 8.47 (d, J = 9.2 Hz, 1H), 7.12 (t, J = 7.5 Hz, 1H), 7.04 - 6.88 (m, 3H), 4.89 (dd, J = 9.6, 6.5 Hz, 1H), 4.68 (dd, J = 9.2, 6.9 Hz, 1H), 4.06 - 3.70 (m, 2H), 2.67 - 2.49 (m, 1H), 2.38 - 2.28 (m, 1H), 2.26 (s, 3H), 2.06 - 1.88 (m, 1H), 1.86 (s, 3H), 0.83 (dd, J = 33.3, 6.8 Hz, 6H).

### Preparation Example 54 Preparation of compounds B-2-20 and B-2-21

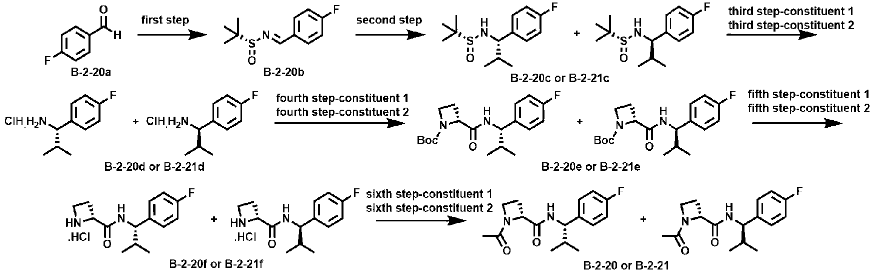

### First step

Intermediate B-2-20a (4.00 g, 33.29 mmol) was dissolved in dichloromethane (50 mL), and sequentially added with (*R*)-tert-butylsulfinamide (4.03 g, 33.29 mmol) and cesium carbonate (16.27 g, 49.94 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain intermediate B-2-20b (7.00 g, 30.79 mmol, yield 92.5%).

### Second step

Intermediate B-2-20b (5.00 g, 22.00 mmol) was dissolved in tetrahydrofuran (50 mL). The mixture was subjected to nitrogen protection and cooled to -78 °C, and slowly dropwise added with 1 N solution of isopropylmagnesium bromide in tetrahydrofuran (40.00 mL, 40.00 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 3). The organic phases were combined, successively washed with saturated brine (100 mL × 2), dried with anhydrous sodium sulfate, filtered, concentrated, and purified by silica gel column chromatography to obtain two constituents.

Constituent 1 with a short retention time was intermediate B-2-20c (1.12 g, 4.12 mmol, yield 18.8%).

Constituent 2 with a long retention time was intermediate B-2-21c (1.12 g, 4.12 mmol, yield 18.8%).

### Third step - constituent 1

Intermediate B-2-20c (1.12 g, 4.12 mmol) was dissolved in dichloromethane (10 mL). Under ice bath, the mixture was added with 4 M solution of hydrogen chloride in dioxane (20 mL). The reaction was continuously stirred under ice bath for 1 hour, and then the reaction solution was concentrated to obtain a crude product - intermediate B-2-20d, which was used directly in the next reaction step without purification.

### Fourth step - constituent 1

The crude product B-2-20d was dissolved in dichloromethane (20 mL), and sequentially added with intermediate A-2-7b (1.39 g, 6.91 mmol), *N*-(3-dimethylaminopropyl)-N'-ethylcarbodiimide hydrochloride (1.81 g, 9.42 mmol), 1-hydroxybenzotriazole (1.27 g, 9.42 mmol) and ethyldiisopropylamine (2.43 g, 18.84 mmol) at -10°C. After the addition was completed, the reaction was continuously stirred at -10°C for 2 hours, and the reaction solution was added with water (10 mL) and extracted with dichloromethane (20 mL × 3). The organic phases were combined, successively washed with saturated brine (20 mL× 3), dried with anhydrous sodium sulfate, filtered, concentrated, and purified by silica gel column chromatography to obtain intermediate B-2-20e (0.55 g, 1.57 mmol, total yield of two reaction steps 38.1%). MS (ESI) calculated for [M-Boc+2H]⁺ was 251.2; found: m/z 251.2.

### Fifth step - constituent 1

Intermediate B-2-20e (0.55 g, 1.57 mmol) was dissolved in dichloromethane (5 mL). Under ice bath, the mixture was added with 4 M solution of hydrogen chloride in dioxane (10 mL), and the reaction was continuously stirred for 2 hours. The reaction solution was concentrated to obtain a crude product - intermediate B-2-20f, which was used directly in the next reaction step without purification.

### Sixth step - constituent 1

The crude product - intermediate B-2-20f was dissolved in dichloromethane (5 mL), cooled to -10°C, and added with acetyl chloride (0.14 g, 1.80 mmol) and ethyldiisopropylamine (0.64 g, 4.92 mmol). The reaction was stirred at -10°C for 1 hour, added with water (5 mL), and extracted with dichloromethane (5 mL × 3). The organic phases were combined, washed with saturated brine (5 mL × 3), dried with anhydrous sodium sulfate, filtered, concentrated, and purified by silica gel column chromatography to obtain the product B-2-20 (76.7 mg, 0.26 mmol, total yield of two steps 16.7%). MS (ESI) calculated for [M+H]⁺ was 293.2; found: m/z 293.2. ¹H NMR (400 MHz, Chloroform-d) δ 8.46 (d, J = 8.6 Hz, 1H), 7.19 - 7.11 (m, 2H), 6.93 (t, J = 8.7 Hz, 2H), 4.79 (dd, J = 9.5, 6.5 Hz, 1H), 4.61 (dd, J = 8.6, 7.1 Hz, 1H), 4.06 - 3.88 (m, 2H), 2.76 - 2.49 (m, 1H), 2.31 (dddd, J = 12.0, 9.5, 8.3, 6.3 Hz, 1H), 1.92 (d, J = 7.0 Hz, 1H), 1.89 (s, 3H), 0.78 (dd, J = 29.5, 6.7 Hz, 6H).

### Third step - constituent 2

Intermediate B-2-21c (1.12 g, 4.19 mmol) was dissolved in dichloromethane (10 mL). Under ice bath, the mixture was added with 4 M solution of hydrogen chloride in dioxane (20 mL). The reaction was continuously stirred under ice bath for 1 hour, and then the reaction solution was concentrated to obtain a crude product - intermediate B-2-21d, which was used directly in the next reaction step without purification.

### Fourth step - constituent 2

The crude product B-2-21d was dissolved in dichloromethane (20 mL), and sequentially added with intermediate A-2-7b (1.39 g, 6.91 mmol), *N*-(3-dimethylaminopropyl)-*N*'-ethylcarbodiimide hydrochloride (1.81 g, 9.42 mmol), 1-hydroxybenzotriazole (1.27 g, 9.42 mmol) and ethyldiisopropylamine (2.43 g, 18.84 mmol) at -10°C. After the addition was completed, the reaction was continuously stirred at -10°C for 2 hours, and the reaction solution was added with water (10 mL) and extracted with dichloromethane (20 mL × 3). The organic phases were combined, successively washed with saturated brine (20 mL× 3), dried with anhydrous sodium sulfate, filtered, concentrated, and purified by silica gel column chromatography to obtain intermediate B-2-21e (1.50 g, 4.28 mmol, total yield of two steps 100%). MS (ESI) calculated for [M-Boc+2H]⁺ was 251.1; found: m/z 251.3.

### Fifth step - constituent 2

Intermediate B-2-21e (1.00 g, 2.85 mmol) was dissolved in dichloromethane (10 mL). Under ice bath, the mixture was added with 4 M solution of hydrogen chloride in dioxane (20 mL), and the reaction was continuously stirred for 2 hours. The reaction solution was concentrated to obtain a crude product - intermediate B-2-21f, which was used directly in the next reaction step without purification.

### Sixth step - constituent 2

The crude product - intermediate B-2-21f was dissolved in dichloromethane (10 mL), cooled to -10°C, and added with acetyl chloride (0.28 g, 3.52 mmol) and ethyldiisopropylamine (1.24 g, 9.60 mmol). The reaction was stirred at -10°C for 1 hour, added with water (10 mL), and extracted with dichloromethane (10 mL × 3). The organic phases were combined, washed with saturated brine (10 mL × 3), dried with anhydrous sodium sulfate, filtered, concentrated, and purified by silica gel column chromatography to obtain the product B-2-21 (0.49 g, 1.68 mmol, total yield of two steps 58.8%). MS (ESI) calculated for [M+H]⁺ was 293.2; found: m/z 293.2. ¹H NMR (400 MHz, Chloroform-d) δ 8.65 - 8.34 (m, 1H), 7.12 - 7.02 (m, 2H), 7.00 - 6.79 (m, 2H), 4.89 (dd, J = 9.5, 6.5 Hz, 1H), 4.67 (dd, J = 8.9, 7.0 Hz, 1H), 4.13 - 3.79 (m, 2H), 2.60 (ddt, J = 12.0, 9.4, 6.7 Hz, 1H), 2.31 (dtd, J = 12.0, 9.3, 5.5 Hz, 1H), 1.95 - 1.87 (m, 1H), 1.86 (s, 3H), 0.82 (dd, J = 38.1, 6.7 Hz, 6H).

### Preparation Example 55 Preparation of compounds B-2-22 and B-2-23

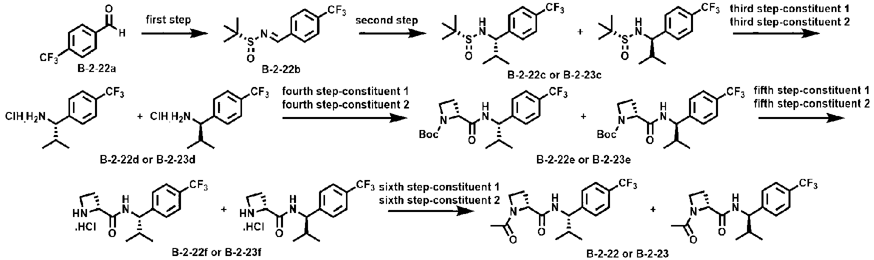

### First step

Intermediate B-2-22a (4.00 g, 22.79 mmol) was dissolved in dichloromethane (50 mL), and sequentially added with (*R*)-tert-butylsulfinamide (2.78 g, 22.97 mmol) and cesium carbonate (11.23 g, 34.45 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain intermediate B-2-22b (6.00 g, 21.6 mmol, yield 94.9%).

### Second step

Intermediate B-2-22b (5.00 g, 18.03 mmol) was dissolved in tetrahydrofuran (50 mL). The mixture was subjected to nitrogen protection and cooled to -78 °C, and slowly dropwise added with 1 N solution of isopropylmagnesium bromide in tetrahydrofuran (36.0 mL, 36.0 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 3). The organic phases were combined, successively washed with saturated brine (100 mL × 2), dried with anhydrous sodium sulfate, filtered, concentrated, and purified by silica gel column chromatography to obtain two constituents.

Constituent 1 with a short retention time was intermediate B-2-22c (1.00 g, 3.11 mmol, yield 17.3%). MS (ESI) calculated for [M+H]⁺ was 322.1; found: m/z 322.4. ¹H NMR (400 MHz, Chloroform-d) δ 7.67 - 7.48 (m, 2H), 7.38 - 7.28 (m, 2H), 4.12 (t, J = 6.3 Hz, 1H), 3.41 (d, J = 6.4 Hz, 1H), 2.42 - 1.95 (m, 1H), 1.18 (s, 9H), 0.81 (dd, J = 52.3, 6.7 Hz, 6H).

Constituent 2 with a long retention time was intermediate B-2-23c (1.00 g, 3.11 mmol, yield 17.3%). MS (ESI) calculated for [M+H]⁺ was 322.1; found: m/z 322.4. ¹H NMR (400 MHz, Chloroform-d) δ 7.51 (d, J = 8.1 Hz, 2H), 7.31 (d, J = 8.0 Hz, 2H), 4.17 (dd, J = 6.6, 2.0 Hz, 1H), 3.46 (d, J = 2.0 Hz, 1H), 1.95 - 1.87 (m, 1H), 1.13 (s, 9H), 0.83 (dd, J = 69.5, 6.8 Hz, 6H).

### Third step - constituent 1

Intermediate B-2-22c (1.00 g, 3.11 mmol) was dissolved in dichloromethane (10 mL). Under ice bath, the mixture was added with 4 M solution of hydrogen chloride in dioxane (20 mL). The reaction was continuously stirred under ice bath for 1 hour, and then the reaction solution was concentrated to obtain a crude product - intermediate B-2-22d, which was used directly in the next reaction step without purification.

### Fourth step - constituent 1

The crude product B-2-22d was dissolved in dichloromethane (20 mL), and sequentially added with intermediate A-2-7b (1.02 g, 5.06 mmol), *N*-(3-dimethylaminopropyl)-*N*'-ethylcarbodiimide hydrochloride (1.32 g, 6.90 mmol), 1-hydroxybenzotriazole (0.93 g, 6.90 mmol) and ethyldiisopropylamine (1.78g, 13.80 mmol) at -10°C. After the addition was completed, the reaction was continuously stirred at -10°C for 2 hours, and the reaction solution was added with water (10 mL) and extracted with dichloromethane (20 mL × 3). The organic phases were combined, successively washed with saturated brine (20 mL× 3), dried with anhydrous sodium sulfate, filtered, concentrated, and purified by silica gel column chromatography to obtain intermediate B-2-22e (1.10 g, 2.75 mmol, total yield of two reaction steps 88.3%).

### Fifth step - constituent 1

Intermediate B-2-22e (1.10 g, 2.75 mmol) was dissolved in dichloromethane (10 mL). Under ice bath, the mixture was added with 4 M solution of hydrogen chloride in dioxane (20 mL), and the reaction was continuously stirred for 2 hours. The reaction solution was concentrated to obtain a crude product - intermediate B-2-22f, which was used directly in the next reaction step without purification.

### Sixth step - constituent 1

The crude product - intermediate B-2-22f was dissolved in dichloromethane (10 mL), cooled to -10°C, and added with acetyl chloride (0.23 g, 2.93 mmol) and ethyldiisopropylamine (1.03 g, 7.98 mmol). The reaction was stirred at -10°C for 1 hour, added with water (10 mL), and extracted with dichloromethane (5 mL × 3). The organic phases were combined, washed with saturated brine (10 mL × 3), dried with anhydrous sodium sulfate, filtered, concentrated, and purified by silica gel column chromatography to obtain the product B-2-22 (152.1 mg, 0.44 mmol, total yield of two steps 16.2%). MS (ESI) calculated for [M+H]⁺ was 343.2; found: m/z 343.2. ¹H NMR (400 MHz, Chloroform-d) δ 8.58 (d, J = 8.3 Hz, 1H), 7.50 (d, J = 8.1 Hz, 2H), 7.32 (d, J = 8.1 Hz, 2H), 4.80 (dd, J = 9.5, 6.5 Hz, 1H), 4.67 (dd, J = 8.4, 6.9 Hz, 1H), 4.06 - 3.95 (m, 2H), 2.73 - 2.58 (m, 1H), 2.31 (ddt, J = 12.1, 9.5, 6.9 Hz, 1H), 2.00 - 1.92 (m, 1H), 1.90 (s, 3H), 0.80 (dd, J = 22.1, 6.8 Hz, 6H).

### Third step - constituent 2

Intermediate B-2-23c (1.00 g, 3.11 mmol) was dissolved in dichloromethane (10 mL). Under ice bath, the mixture was added with 4 M solution of hydrogen chloride in dioxane (20 mL). The reaction was continuously stirred under ice bath for 1 hour, and then the reaction solution was concentrated to obtain a crude product - intermediate B-2-23d, which was used directly in the next reaction step without purification.

### Fourth step - constituent 2

The crude product B-2-23d was dissolved in dichloromethane (20 mL), and sequentially added with intermediate A-2-7b (1.07 g, 5.31 mmol), *N*-(3-dimethylaminopropyl)-*N*'-ethylcarbodiimide hydrochloride (1.39 g, 7.25 mmol), 1-hydroxybenzotriazole (0.98 g, 7.25 mmol) and ethyldiisopropylamine (1.87 g, 14.49 mmol) at -10°C. After the addition was completed, the reaction was continuously stirred at -10°C for 2 hours, and the reaction solution was added with water (10 mL) and extracted with dichloromethane (20 mL × 3). The organic phases were combined, successively washed with saturated brine (20 mL× 3), dried with anhydrous sodium sulfate, filtered, concentrated, and purified by silica gel column chromatography to obtain intermediate B-2-23e (1.00 g, 2.50 mmol, total yield of two steps 80.3%).

### Fifth step - constituent 2

Intermediate B-2-23e (1.00 g, 2.50 mmol) was dissolved in dichloromethane (10 mL). Under ice bath, the mixture was added with 4 M solution of hydrogen chloride in dioxane (20 mL), and the reaction was continuously stirred for 2 hours. The reaction solution was concentrated to obtain a crude product - intermediate B-2-23f, which was used directly in the next reaction step without purification.

### Sixth step - constituent 2

The crude product - intermediate B-2-23f was dissolved in dichloromethane (10 mL), cooled to -10°C, and added with acetyl chloride (0.20g, 2.56 mmol) and ethyldiisopropylamine (0.90 g, 6.99 mmol). The reaction was stirred at -10°C for 1 hour, added with water (10 mL), and extracted with dichloromethane (10 mL × 3). The organic phases were combined, washed with saturated brine (10 mL × 3), dried with anhydrous sodium sulfate, filtered, concentrated, and purified by silica gel column chromatography to obtain the product B-2-23 (284.6 mg, 0.83 mmol, total yield of two steps 33.2%). MS (ESI) calculated for [M+H]⁺ was 343.2; found: m/z 343.2. ¹H NMR (400 MHz, Chloroform-d) δ 8.64 (d, J = 8.8 Hz, 1H), 7.49 (d, J = 8.0 Hz, 2H), 7.24 (d, J = 8.1 Hz, 2H), 4.90 (dd, J = 9.5, 6.5 Hz, 1H), 4.74 (dd, J = 8.8, 6.8 Hz, 1H), 4.08 - 3.78 (m, 2H), 2.68 - 2.52 (m, 1H), 2.31 (dtd, J = 12.0, 9.3, 5.5 Hz, 1H), 2.01 - 1.91 (m, 1H), 1.88 (s, 3H), 0.84 (dd, J = 30.1, 6.8 Hz, 6H).

### Preparation Example 56 Preparation of compound B-2-24

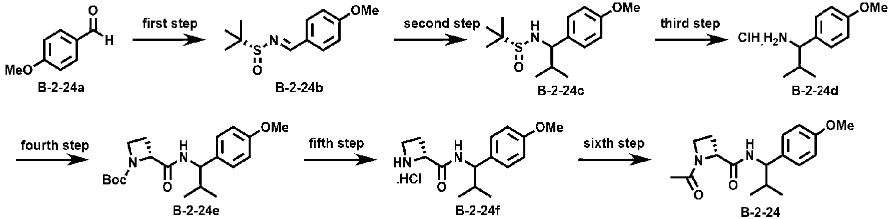

### First step

Intermediate B-2-24a (4.00 g, 29.38 mmol) was dissolved in dichloromethane (50 mL), and sequentially added with (R)-tert-butylsulfinamide (4.57 g, 29.38 mmol) and anhydrous copper sulfate (4.69 g, 29.38 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain intermediate B-2-24b (4.00 g, 16.7 mmol, yield 56.9%). MS (ESI) calculated for [M+H]⁺ was 240.1; found: m/z 240.1.

### Second step

Intermediate B-2-24b (2.00 g, 8.36 mmol) was dissolved in tetrahydrofuran (20 mL). The mixture was subjected to nitrogen protection and cooled to -78 °C, and slowly dropwise added with 1 N solution of isopropylmagnesium bromide in tetrahydrofuran (16.72 mL, 16.72 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (20 mL × 3). The organic phases were combined, successively washed with saturated brine (20 mL× 2), dried with anhydrous sodium sulfate, filtered, concentrated, and purified by silica gel column chromatography to obtain a mixture of two constituents, which are recorded as intermediate B-2-24c (1.00 g, 3.53 mmol, yield 42.2%). MS (ESI) calculated for [M+H]⁺ was 284.2; found: m/z 284.1.

### Third step

Constituent 1 of intermediate B-2-24c (1.00 g, 3.53 mmol) was dissolved in dichloromethane (10 mL). Under ice bath, the mixture was added with 4 M solution of hydrogen chloride in dioxane (20 mL). The reaction was continuously stirred under ice bath for 1 hour, and then the reaction solution was concentrated to obtain a crude product - intermediate B-2-24d, which was used directly in the next reaction step without purification.

### Fourth step

The crude product B-2-24d was dissolved in dichloromethane (10 mL), and sequentially added with intermediate A-2-7b (1.11 g, 5.02 mmol), N-(3-dimethylaminopropyl)-N'-ethylcarbodiimide hydrochloride (1.44 g, 7.53 mmol), 1-hydroxybenzotriazole (1.02 g, 7.53 mmol) and ethyldiisopropylamine (1.95g, 15.06 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-24e (1.20 g, 3.31 mmol, total yield of two reaction steps 93.8%). MS-ESI theoretical value [M-Boc+2H]⁺: 263.2; found: m/z 263.2.

### Fifth step

Intermediate B-2-24e (1.20 g, 3.31 mmol) was dissolved in dichloromethane (10 mL). Under ice bath, the mixture was added with 4 M solution of hydrogen chloride in dioxane (20 mL), and the reaction was continuously stirred for 2 hours. The reaction solution was concentrated to obtain a crude product - intermediate B-2-24f, which was used directly in the next reaction step without purification.

### Sixth step - constituent 1

The crude product - intermediate B-2-24f was dissolved in dichloromethane (10 mL), cooled to -10°C, and added with acetyl chloride (0.28 g, 3.61 mmol) and ethyldiisopropylamine (1.27 g, 9.84 mmol). The reaction was stirred at -10°C for 1 hour, added with water (10 mL), and extracted with dichloromethane (10 mL × 3). The organic phases were combined, washed with saturated brine (10 mL × 3), dried with anhydrous sodium sulfate, filtered, concentrated, and purified by silica gel column chromatography to obtain the product B-2-24 (332.9 mg, 1.09 mmol, total yield of two steps 33.0%). MS (ESI) calculated for [M+H]⁺ was 305.2; found: m/z 305.2. ¹H NMR (400 MHz, Chloroform-d) δ 8.40 (dd, J = 25.0, 8.9 Hz, 1H), 7.14 - 7.00 (m, 2H), 6.81 - 6.72 (m, 2H), 4.83 (ddd, J = 41.8, 9.5, 6.5 Hz, 1H), 4.62 (ddd, J = 21.1, 8.9, 7.1 Hz, 1H), 4.16 - 3.86 (m, 2H), 3.71 (d, J = 2.8 Hz, 3H), 2.79 - 2.47 (m, 1H), 2.43 - 2.17 (m, 1H), 1.99 - 1.90 (m, 1H), 1.87 (d, J = 15.0 Hz, 3H), 0.89 - 0.64 (m, 6H).

### Preparation Example 57 Preparation of compound B-2-25

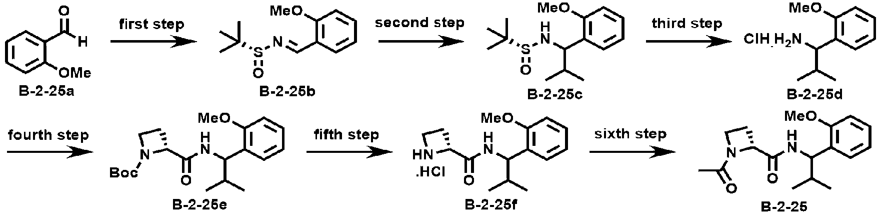

### First step

Intermediate B-2-25a (5.00 g, 36.72 mmol) was dissolved in dichloromethane (50 mL), and sequentially added with (R)-tert-butylsulfinamide (4.90 g, 40.39 mmol) and anhydrous copper sulfate (5.86 g, 36.72 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain intermediate B-2-25b (8.00 g, 27.28 mmol, yield 74.3%). MS (ESI) calculated for [M+H]⁺ was 240.1; found: m/z 240.1.

### Second step

Intermediate B-2-25b (5.00 g, 17.05 mmol) was dissolved in tetrahydrofuran (50 mL). The mixture was subjected to nitrogen protection and cooled to -78°C, and slowly dropwise added with 1 N solution of isopropylmagnesium bromide in tetrahydrofuran (41.78 mL, 41.78 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 3). The organic phases were combined, successively washed with saturated brine (100 mL× 2), dried with anhydrous sodium sulfate, filtered, concentrated, and purified by silica gel column chromatography to obtain a mixture of two constituents, which are recorded as intermediate B-2-25c (2.30 g, 8.12 mmol, yield 47.6%). MS (ESI) calculated for [M+H]⁺ was 284.2; found: m/z 284.2.

### Third step

Constituent 1 of intermediate B-2-25c (1.30 g, 4.59 mmol) was dissolved in dichloromethane (10 mL). Under ice bath, the mixture was added with 4 M solution of hydrogen chloride in dioxane (20 mL). The reaction was continuously stirred under ice bath for 1 hour, and then the reaction solution was concentrated to obtain a crude product - intermediate B-2-25d, which was used directly in the next reaction step without purification.

### Fourth step

The crude product B-2-25d was dissolved in dichloromethane (10 mL), and sequentially added with intermediate A-2-7b (1.45 g, 7.36 mmol), *N*-(3-dimethylaminopropyl)-*N*'-ethylcarbodiimide hydrochloride (1.92g, 10.04 mmol), 1-hydroxybenzotriazole (1.36 g, 10.04 mmol) and ethyldiisopropylamine (2.59 g, 20.07 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-25e (1.50 g, 4.14 mmol, total yield of two reaction steps 90.2%). MS-ESI theoretical value [M-Boc+2H]⁺: 263.2; found: m/z 263.4.

### Fifth step

Intermediate B-2-25e (1.50 g, 4.14 mmol) was dissolved in dichloromethane (10 mL). Under ice bath, the mixture was added with 4 M solution of hydrogen chloride in dioxane (20 mL), and the reaction was continuously stirred for 2 hours. The reaction solution was concentrated to obtain a crude product - intermediate B-2-25f, which was used directly in the next reaction step without purification.

### Sixth step

The crude product - intermediate B-2-25f was dissolved in dichloromethane (10 mL), cooled to -10°C, and added with acetyl chloride (0.33 g, 4.19 mmol) and ethyldiisopropylamine (1.48 g, 11.43 mmol). The reaction was stirred at -10°C for 1 hour, added with water (10 mL), and extracted with dichloromethane (10 mL × 3). The organic phases were combined, washed with saturated brine (10 mL × 3), dried with anhydrous sodium sulfate, filtered, concentrated, and purified by silica gel column chromatography to obtain the product B-2-25 (431.5 g, 1.44 mmol, total yield of two steps 34.9%). MS (ESI) calculated for [M+H]⁺ was 305.2; found: m/z 305.2. ¹H NMR (400 MHz, Chloroform-d) δ 8.36 (t, J = 12.2 Hz, 1H), 7.17 - 6.99 (m, 2H), 6.86 - 6.78 (m, 2H), 4.91 - 4.81 (m, 1H), 4.77 - 4.57 (m, 1H), 4.05 - 3.90 (m, 2H), 3.78 (d, J = 13.5 Hz, 3H), 2.70 - 2.48 (m, 1H), 2.32 (ddtd, J = 15.1, 11.9, 9.3, 5.7 Hz, 1H), 2.13 - 2.01 (m, 1H), 1.86 (d, J = 18.2 Hz, 3H), 0.90 - 0.69 (m, 6H).

### Preparation Example 58 Preparation of compound B-2-26

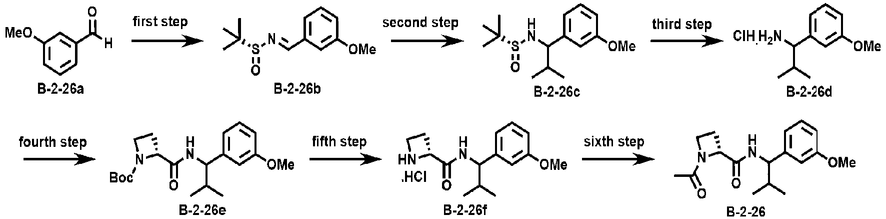

### First step

Intermediate B-2-26a (5.00 g, 36.72 mmol) was dissolved in dichloromethane (50 mL), and sequentially added with (R)-tert-butylsulfinamide (4.90 g, 40.39 mmol) and anhydrous copper sulfate (5.86 g, 36.72 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain intermediate B-2-26b (6.00 g, 25.07 mmol, yield 68.3%). MS (ESI) calculated for [M+H]⁺ was 240.1; found: m/z 240.1.

### Second step

Intermediate B-2-26b (5.00 g, 20.89 mmol) was dissolved in tetrahydrofuran (50 mL). The mixture was subjected to nitrogen protection and cooled to -78°C, and slowly dropwise added with 1 N solution of isopropylmagnesium bromide in tetrahydrofuran (41.78 mL, 41.78 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 3). The organic phases were combined, successively washed with saturated brine (100 mL× 2), dried with anhydrous sodium sulfate, filtered, concentrated, and purified by silica gel column chromatography to obtain a mixture of two constituents, which are recorded as intermediate B-2-26c (2.30 g, 8.12 mmol, yield 38.8%). MS (ESI) calculated for [M+H]⁺ was 284.2; found: m/z 284.1.

### Third step

Intermediate B-2-26c (1.50 g, 5.29 mmol) was dissolved in dichloromethane (10 mL). Under ice bath, the mixture was added with 4 M solution of hydrogen chloride in dioxane (20 mL). The reaction was continuously stirred under ice bath for 1 hour, and then the reaction solution was concentrated to obtain a crude product - intermediate B-2-26d, which was used directly in the next reaction step without purification.

### Fourth step

The crude product B-2-26d was dissolved in dichloromethane (10 mL), and sequentially added with intermediate A-2-7b (1.73 g, 8.59mmol), N-(3-dimethylaminopropyl)-N'-ethylcarbodiimide hydrochloride (2.25g, 11.71 mmol), 1-hydroxybenzotriazole (1.58 g, 11.71 mmol) and ethyldiisopropylamine (3.03 g, 23.43 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-26e (1.70 g, 4.69 mmol, total yield of two reaction steps 88.7%). MS-ESI theoretical value [M-Boc+2H]⁺: 263.2; found: m/z 263.2.

### Fifth step

Intermediate B-2-26e (1.00 g, 2.76 mmol) was dissolved in dichloromethane (10 mL). Under ice bath, the mixture was added with 4 M solution of hydrogen chloride in dioxane (20 mL), and the reaction was continuously stirred for 2 hours. The reaction solution was concentrated to obtain a crude product - intermediate B-2-26f, which was used directly in the next reaction step without purification.

### Sixth step

The crude product - intermediate B-2-26f was dissolved in dichloromethane (10 mL), cooled to -10°C, and added with acetyl chloride (0.24 g, 3.01 mmol) and ethyldiisopropylamine (1.06 g, 8.22 mmol). The reaction was stirred at -10°C for 1 hour, added with water (10 mL), and extracted with dichloromethane (10 mL × 3). The organic phases were combined, washed with saturated brine (10 mL × 3), dried with anhydrous sodium sulfate, filtered, concentrated, and purified by silica gel column chromatography to obtain the product B-2-26 (199.8 mg, 0.66 mmol, total yield of two steps 23.8%). MS (ESI) calculated for [M+H]⁺ was 305.2; found: m/z 305.2. ¹H NMR (400 MHz, Chloroform-d) δ 8.40 (dd, J = 25.0, 8.9 Hz, 1H), 7.14 - 7.00 (m, 2H), 6.81 - 6.72 (m, 2H), 4.83 (ddd, J = 41.8, 9.5, 6.5 Hz, 1H), 4.62 (ddd, J = 21.1, 8.9, 7.1 Hz, 1H), 4.16 - 3.86 (m, 2H), 3.71 (d, J = 2.8 Hz, 3H), 2.79 - 2.47 (m, 1H), 2.43 - 2.17 (m, 1H), 1.99 - 1.90 (m, 1H), 1.87 (d, J = 15.0 Hz, 3H), 0.89 - 0.64 (m, 6H).

### Preparation Example 59 Preparation of compounds B-2-27 and B-2-28

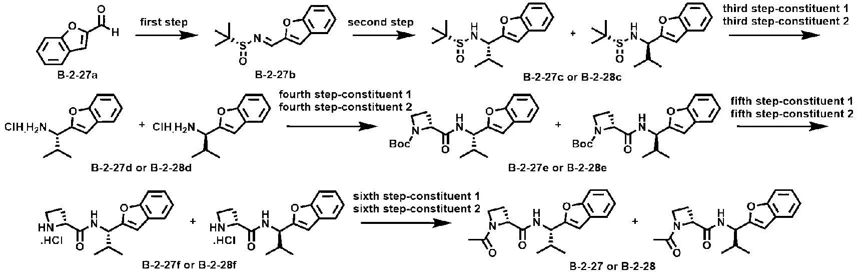

### First step

Intermediate B-2-27a (5.00 g, 34.21 mmol) was dissolved in dichloromethane (50 mL), and sequentially added with (*R*)-tert-butylsulfinamide (4.56 g, 37.63 mmol) and cesium carbonate (22.29 g, 68.42 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain intermediate B-2-27b (4.00 g, 16.04 mmol, yield 46.9%). MS (ESI) calculated for [M+H]⁺ was 250.1; found: m/z 250.1.

### Second step

Intermediate B-2-27b (4.00 g, 16.04 mmol) was dissolved in tetrahydrofuran (50 mL). The mixture was subjected to nitrogen protection and cooled to -78°C, and slowly dropwise added with 1 N solution of isopropylmagnesium bromide in tetrahydrofuran (32.00 mL, 32.00 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 3). The organic phases were combined, successively washed with saturated brine (100 mL × 2), dried with anhydrous sodium sulfate, filtered, concentrated, and purified by silica gel column chromatography to obtain two constituents.

Constituent 1 with a short retention time was intermediate B-2-27c (1.30 g, 4.43 mmol, yield 27.6%). MS (ESI) calculated for [M+H]⁺ was 294.1; found: m/z 294.1. ¹H NMR (400 MHz, Chloroform-d) δ 7.53 - 7.28 (m, 2H), 7.18 - 7.04 (m, 2H), 6.64 (d, J = 0.8 Hz, 1H), 4.18 (ddd, J = 8.8, 6.2, 0.7 Hz, 1H), 3.54 (d, J = 8.7 Hz, 1H), 2.26 (dq, J = 13.4, 6.7 Hz, 1H), 1.19 (d, J = 5.3 Hz, 9H), 0.90 (dd, J = 12.3, 6.8 Hz, 6H).

Constituent 2 with a long retention time was intermediate B-2-28c (1.00 g, 3.41 mmol, yield 21.2%). MS (ESI) calculated for [M+H]⁺ was 294.1; found: m/z 294.1. ¹HNMR (400 MHz, Chloroform-d) δ 7.58 - 7.30 (m, 2H), 7.22 - 7.12 (m, 2H), 6.52 (t, J = 0.7 Hz, 1H), 4.30 (dd, J = 6.4, 4.7 Hz, 1H), 3.46 (d, J = 4.7 Hz, 1H), 2.29 - 2.06 (m, 1H), 1.13 (s, 9H), 0.94 (dd, J = 29.7, 6.8 Hz, 6H).

### Third step - constituent 1

Intermediate B-2-27c (1.00 g, 3.41 mmol) was dissolved in dichloromethane (10 mL). Under ice bath, the mixture was added with 4 M solution of hydrogen chloride in dioxane (10 mL). The reaction was continuously stirred under ice bath for 1 hour, and then the reaction solution was concentrated to obtain a crude product - intermediate B-2-27d, which was used directly in the next reaction step without purification.

### Fourth step - constituent 1

The crude product B-2-27d was dissolved in dichloromethane (10 mL), and sequentially added with intermediate A-2-7b (0.94 g, 4.65 mmol), *N*-(3-dimethylaminopropyl)-*N*'-ethylcarbodiimide hydrochloride (1.22 g, 6.35 mmol), 1-hydroxybenzotriazole (0.86 g, 6.35 mmol) and ethyldiisopropylamine (1.64 g, 12.69 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-27e (0.80 g, 2.15 mmol, total yield of two reaction steps 63.0%). MS (ESI) calculated for [M-Boc+2H]⁺ was 273.2; found: m/z 273.2.

### Fifth step - constituent 1

Intermediate B-2-27e (0.50 g, 1.34 mmol) was dissolved in dichloromethane (5 mL). Under ice bath, the mixture was added with 4 M solution of hydrogen chloride in dioxane (5 mL), and the reaction was continuously stirred for 2 hours. The reaction solution was concentrated to obtain a crude product - intermediate B-2-27f, which was used directly in the next reaction step without purification.

### Sixth step - constituent 1

The crude product - intermediate B-2-27f was dissolved in dichloromethane (5 mL), cooled to -10°C, and added with acetyl chloride (0.12 g, 1.50 mmol) and ethyldiisopropylamine (0.53 g, 4.08 mmol). The reaction was stirred at -10°C for 1 hour, added with water (5 mL), and extracted with dichloromethane (5 mL × 3). The organic phases were combined, washed with saturated brine (5 mL × 3), dried with anhydrous sodium sulfate, filtered, concentrated, and purified by silica gel column chromatography to obtain the product B-2-27 (75.0 mg, 0.24 mmol, total yield of two steps 17.8%). MS (ESI) calculated for [M+H]⁺ was 315.2; found: m/z 315.2. ¹H NMR (400 MHz, Chloroform-d) δ 8.44 (d, J = 9.0 Hz, 1H), 7.53 - 7.41 (m, 2H), 7.20 (dtd, J = 18.6, 7.3, 1.3 Hz, 2H), 6.59 (d, J = 0.9 Hz, 1H), 4.97 (ddd, J = 53.4, 9.2, 6.4 Hz, 2H), 4.27 - 3.94 (m, 2H), 2.76 (ddt, J = 12.0, 9.0, 6.7 Hz, 1H), 2.45 - 2.35 (m, 1H), 2.34 - 2.22 (m, 1H), 1.96 (s, 3H), 0.94 (dd, J = 6.8, 1.2 Hz, 6H).

### Third step - constituent 2

Intermediate B-2-28c (1.00 g, 3.41 mmol) was dissolved in dichloromethane (2 mL). Under ice bath, the mixture was added with 4 M solution of hydrogen chloride in dioxane (4 mL). The reaction was continuously stirred under ice bath for 1 hour, and then the reaction solution was concentrated to obtain a crude product - intermediate B-2-28d, which was used directly in the next reaction step without purification.

### Fourth step - constituent 2

The crude product B-2-28d was dissolved in dichloromethane (10 mL), and sequentially added with intermediate A-2-7b (0.82 g, 4.07 mmol), *N*-(3-dimethylaminopropyl)-*N*'-ethylcarbodiimide hydrochloride (1.06 g, 5.55 mmol), 1-hydroxybenzotriazole (0.75 g, 5.55 mmol) and ethyldiisopropylamine (1.43g, 11.10 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-28e (0.80 g, 2.15 mmol, total yield of two steps 63.0%).

### Fifth step - constituent 2

Intermediate B-2-28e (0.80g, 2.15 mmol) was dissolved in dichloromethane (10 mL). Under ice bath, the mixture was added with 4 M solution of hydrogen chloride in dioxane (10 mL), and the reaction was continuously stirred for 2 hours. The reaction solution was concentrated to obtain a crude product - intermediate B-2-28f, which was used directly in the next reaction step without purification.

### Sixth step - constituent 2

The crude product - intermediate B-2-28f was dissolved in dichloromethane (5 mL), cooled to -10°C, and added with acetyl chloride (0.18 g, 2.34 mmol) and ethyldiisopropylamine (0.83 g, 6.39 mmol). The reaction was stirred at -10°C for 1 hour, added with water (5 mL), and extracted with dichloromethane (5 mL × 3). The organic phases were combined, washed with saturated brine (5 mL × 3), dried with anhydrous sodium sulfate, filtered, concentrated, and purified by silica gel column chromatography to obtain the product B-2-28 (189.0 g, 0.60 mmol, total yield of two steps 28.0%). MS (ESI) calculated for [M+H]⁺ was 315.2; found: m/z 315.4. ¹HNMR (400 MHz, Chloroform-d) δ 8.46 (d, J = 9.1 Hz, 1H), 7.60 - 7.38 (m, 2H), 7.26 - 7.17 (m, 2H), 6.51 (d, J = 0.9 Hz, 1H), 5.24 - 4.87 (m, 2H), 4.12 - 3.82 (m, 2H), 2.68 (ddt, J = 12.0, 9.2, 6.9 Hz, 1H), 2.49 - 2.36 (m, 1H), 2.35 - 2.15 (m, 1H), 1.94 (s, 3H), 0.98 (dd, J = 9.6, 6.8 Hz, 6H).

### Preparation Example 60 Preparation of compounds B-2-29 and B-2-30

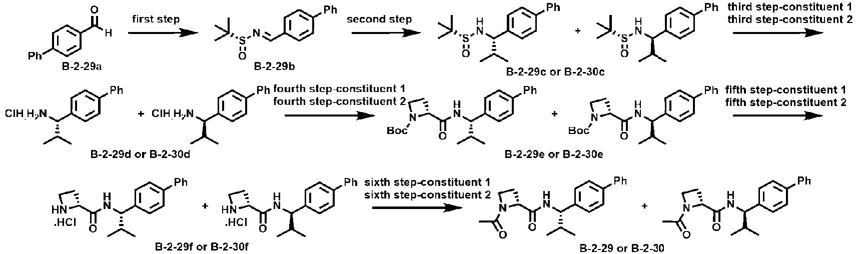

### First step

Intermediate B-2-29a (5.00 g, 27.44 mmol) was dissolved in dichloromethane (50 mL), and sequentially added with (*R*)-tert-butylsulfinamide (3.66 g, 30.18mmol) and cesium carbonate (17.88 g, 54.88 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain intermediate B-2-29b (7.00 g, 24.53 mmol, yield 89.4%). MS (ESI) calculated for [M+H]⁺ was 286.1; found: m/z 286.2.

### Second step

Intermediate B-2-29b (5.00 g, 17.52 mmol) was dissolved in tetrahydrofuran (50 mL). The mixture was subjected to nitrogen protection and cooled to -78°C, and slowly dropwise added with 1 N solution of isopropylmagnesium bromide in tetrahydrofuran (35.00 mL, 35.00 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 3). The organic phases were combined, successively washed with saturated brine (100 mL × 2), dried with anhydrous sodium sulfate, filtered, concentrated, and purified by silica gel column chromatography to obtain two constituents.

Constituent 1 with a short retention time was intermediate B-2-29c (1.20 g, 3.64 mmol, yield 20.8%). MS (ESI) calculated for [M+H]⁺ was 330.2; found: m/z 330.2.

Constituent 2 with a long retention time was intermediate B-2-30c (1.40 g, 4.25 mmol, yield 24.3%). MS (ESI) calculated for [M+H]⁺ was 330.2; found: m/z 330.2.

### Third step - constituent 1

Intermediate B-2-29c (1.20 g, 3.64 mmol) was dissolved in dichloromethane (10 mL). Under ice bath, the mixture was added with 4 M solution of hydrogen chloride in dioxane (10 mL). The reaction was continuously stirred under ice bath for 1 hour, and then the reaction solution was concentrated to obtain a crude product - intermediate B-2-29d, which was used directly in the next reaction step without purification.

### Fourth step - constituent 1

The crude product B-2-29d was dissolved in dichloromethane (10 mL), and sequentially added with intermediate A-2-7b (0.79 g, 3.91 mmol), *N*-(3-dimethylaminopropyl)-*N*'-ethylcarbodiimide hydrochloride (1.02 g, 5.32 mmol), 1-hydroxybenzotriazole (0.72 g, 5.32 mmol) and ethyldiisopropylamine (1.38 g, 10.65 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-29e (0.98 g, 2.40 mmol, total yield of two reaction steps 65.9%). MS (ESI) calculated for [M-Boc+2H]⁺ was 309.2; found: m/z 309.1.

### Fifth step - constituent 1

Intermediate B-2-29e (0.98 g, 2.40 mmol) was dissolved in dichloromethane (5 mL). Under ice bath, the mixture was added with 4 M solution of hydrogen chloride in dioxane (10 mL), and the reaction was continuously stirred for 2 hours. The reaction solution was concentrated to obtain a crude product - intermediate B-2-29f, which was used directly in the next reaction step without purification.

### Sixth step - constituent 1

The crude product - intermediate B-2-29f was dissolved in dichloromethane (10 mL), cooled to -10°C, and added with acetyl chloride (0.21 g, 2.64 mmol) and ethyldiisopropylamine (0.93 g, 7.20 mmol). The reaction was stirred at -10°C for 1 hour, added with water (10 mL), and extracted with dichloromethane (10 mL × 3). The organic phases were combined, washed with saturated brine (10 mL × 3), dried with anhydrous sodium sulfate, filtered, concentrated, and purified by silica gel column chromatography to obtain the product B-2-29 (0.45 g, 1.28 mmol, total yield of two steps 53.5%). MS (ESI) calculated for [M+H]⁺ was 351.2; found: m/z 351.3. ¹H NMR (400 MHz, Chloroform-d) δ 8.57 (d, J = 8.7 Hz, 1H), 7.60 - 7.52 (m, 4H), 7.46 - 7.30 (m, 5H), 4.88 (dd, J = 9.5, 6.4 Hz, 1H), 4.77 (dd, J = 8.8, 6.9 Hz, 1H), 4.19 - 3.95 (m, 2H), 2.75 (ddt, J = 12.0, 9.0, 6.8 Hz, 1H), 2.43 - 2.28 (m, 1H), 2.10 - 2.00 (m, 1H), 1.97 (s, 3H), 0.90 (dd, J = 23.2, 6.7 Hz, 6H).

### Third step - constituent 2

Intermediate B-2-30c (1.40g, 4.25 mmol) was dissolved in dichloromethane (10 mL). Under ice bath, the mixture was added with 4 M solution of hydrogen chloride in dioxane (10 mL). The reaction was continuously stirred under ice bath for 1 hour, and then the reaction solution was concentrated to obtain a crude product - intermediate B-2-30d, which was used directly in the next reaction step without purification.

### Fourth step - constituent 2

The crude product B-2-30d was dissolved in dichloromethane (10 mL), and sequentially added with intermediate A-2-7b (0.88 g, 4.39 mmol), N-(3-dimethylaminopropyl)-N'-ethylcarbodiimide hydrochloride (1.15 g, 5.99 mmol), 1-hydroxybenzotriazole (0.81 g, 5.99 mmol) and ethyldiisopropylamine (1.55 g, 11.97 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-30e (0.80 g, 1.96 mmol, total yield of two steps 46.1%). MS (ESI) calculated for [M-Boc+2H]⁺ was 309.2; found: m/z 308.9.

### Fifth step - constituent 2

Intermediate B-2-30e (0.80g, 1.96 mmol) was dissolved in dichloromethane (3 mL). Under ice bath, the mixture was added with 4 M solution of hydrogen chloride in dioxane (6 mL), and the reaction was continuously stirred for 2 hours. The reaction solution was concentrated to obtain a crude product - intermediate B-2-30f, which was used directly in the next reaction step without purification.

### Sixth step - constituent 2

The crude product - intermediate B-2-30f was dissolved in dichloromethane (5 mL), cooled to -10°C, and added with acetyl chloride (0.17 g, 2.15 mmol) and ethyldiisopropylamine (0.76 g, 5.85 mmol). The reaction was stirred at -10°C for 1 hour, added with water (5 mL), and extracted with dichloromethane (5 mL × 3). The organic phases were combined, washed with saturated brine (5 mL × 3), dried with anhydrous sodium sulfate, filtered, concentrated, and purified by silica gel column chromatography to obtain the product B-2-30 (0.49 g, 1.40 mmol, total yield of two steps 71.3%). MS (ESI) calculated for [M+H]⁺ was 351.2; found: m/z 351.3. ¹H NMR (400 MHz, Chloroform-d) δ 8.63 (d, J = 9.0 Hz, 1H), 7.63 - 7.48 (m, 4H), 7.43 (dd, J = 8.4, 6.9 Hz, 2H), 7.35 - 7.30 (m, 1H), 7.28 (s, 1H), 7.26 (s, 1H), 4.90 (ddd, J = 61.6, 9.3, 6.6 Hz, 2H), 4.10 - 3.92 (m, 2H), 2.68 (ddt, J = 12.0, 9.4, 6.7 Hz, 1H), 2.39 (dtd, J = 12.0, 9.3, 5.5 Hz, 1H), 2.08 - 2.00 (m, 1H), 1.94 (s, 3H), 0.94 (dd, J = 30.7, 6.7 Hz, 6H).

### Preparation Example 61 Preparation of compounds B-2-31 and B-2-32

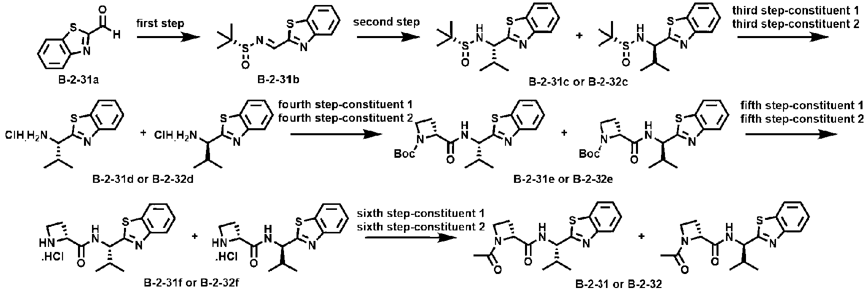

### First step

Intermediate B-2-31a (4.00 g, 24.51 mmol) was dissolved in dichloromethane (50 mL), and sequentially added with (R)-tert-butylsulfinamide (2.97 g, 24.51 mmol) and cesium carbonate (15.97 g, 49.02 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain intermediate B-2-31b (6.53 g, 24.5 mmol, yield 100%). MS (ESI) calculated for [M+H]⁺ was 267.1; found: m/z 267.2.

### Second step

Intermediate B-2-31b (5.00 g, 18.77 mmol) was dissolved in tetrahydrofuran (50 mL). The mixture was subjected to nitrogen protection and cooled to -78°C, and slowly dropwise added with 1 N solution of isopropylmagnesium bromide in tetrahydrofuran (37.5 mL, 37.5 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 3). The organic phases were combined, successively washed with saturated brine (100 mL × 2), dried with anhydrous sodium sulfate, filtered, concentrated, and purified by silica gel column chromatography to obtain two constituents.

Constituent 1 with a short retention time was intermediate B-2-31c (1.12 g, 3.62 mmol, yield 19.2%). MS (ESI) calculated for [M+H]⁺ was 311.1; found: m/z 311.2.

Constituent 2 with a long retention time was intermediate B-2-32c (0.45 g, 1.45 mmol, yield 7.7%). MS (ESI) calculated for [M+H]⁺ was 311.1; found: m/z 311.2.

### Third step - constituent 1

Intermediate B-2-31c (1.12 g, 3.62 mmol) was dissolved in dichloromethane (10 mL). Under ice bath, the mixture was added with 4 M solution of hydrogen chloride in dioxane (10 mL). The reaction was continuously stirred under ice bath for 1 hour, and then the reaction solution was concentrated to obtain a crude product - intermediate B-2-31d, which was used directly in the next reaction step without purification.

### Fourth step - constituent 1

The crude product B-2-31d was dissolved in dichloromethane (10 mL), and sequentially added with intermediate A-2-7b (0.75 g, 3.73 mmol), *N*-(3-dimethylaminopropyl)-*N*'-ethylcarbodiimide hydrochloride (0.97 g, 5.08 mmol), 1-hydroxybenzotriazole (0.69 g, 5.08 mmol) and ethyldiisopropylamine (1.31 g, 10.17 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-31e (0.90 g, 2.31 mmol, total yield of two reaction steps 63.8%).

### Fifth step - constituent 1

Intermediate B-2-31e (0.90g, 2.31 mmol) was dissolved in dichloromethane (10 mL). Under ice bath, the mixture was added with 4 M solution of hydrogen chloride in dioxane (10 mL), and the reaction was continuously stirred for 2 hours. The reaction solution was concentrated to obtain a crude product - intermediate B-2-31f, which was used directly in the next reaction step without purification.

### Sixth step - constituent 1

The crude product - intermediate B-2-31f was dissolved in dichloromethane (10 mL), cooled to -10°C, and added with acetyl chloride (0.20 g, 2.55 mmol) and ethyldiisopropylamine (0.90 g, 6.96 mmol). The reaction was stirred at -10°C for 1 hour, added with water (5 mL), and extracted with dichloromethane (5 mL × 3). The organic phases were combined, washed with saturated brine (5 mL × 3), dried with anhydrous sodium sulfate, filtered, concentrated, and purified by silica gel column chromatography to obtain the product B-2-31 (0.40 g, 1.21 mmol, total yield of two steps 52.3%). MS (ESI) calculated for [M+H]⁺ was 332.1; found: m/z 332.2. ¹HNMR (400 MHz, Chloroform-d) δ 8.73 (d, J = 8.4 Hz, 1H), 8.07 - 7.74 (m, 2H), 7.53 - 7.29 (m, 2H), 5.28 (dd, J = 8.4, 5.3 Hz, 1H), 4.99 (dd, J = 9.4, 6.4 Hz, 1H), 4.21 - 3.95 (m, 2H), 2.87 - 2.75 (m, 1H), 2.60 - 2.34 (m, 2H), 1.97 (s, 3H), 0.99 (dd, J = 22.7, 6.8 Hz, 6H).

### Third step - constituent 2

Intermediate B-2-32c (0.45 g, 1.45 mmol) was dissolved in dichloromethane (5 mL). Under ice bath, the mixture was added with 4 M solution of hydrogen chloride in dioxane (5 mL). The reaction was continuously stirred under ice bath for 1 hour, and then the reaction solution was concentrated to obtain a crude product - intermediate B-2-32d, which was used directly in the next reaction step without purification.

### Fourth step - constituent 2

The crude product B-2-32d was dissolved in dichloromethane (5 mL), and sequentially added with intermediate A-2-7b (0.32 g, 1.59 mmol), *N*-(3-dimethylaminopropyl)-*N*'-ethylcarbodiimide hydrochloride (0.42 g, 2.17 mmol), 1-hydroxybenzotriazole (0.29 g, 2.17 mmol) and ethyldiisopropylamine (0.56 g, 4.35 mmol) at -10°C. After the addition was completed, the reaction was continuously stirred at -10°C for 2 hours, and the reaction solution was added with water (5 mL) and extracted with dichloromethane (10 mL × 3). The organic phases were combined, successively washed with saturated brine (5 mL× 3), dried with anhydrous sodium sulfate, filtered, concentrated, and purified by silica gel column chromatography to obtain intermediate B-2-32e (0.38 g, 0.98 mmol, total yield of two steps 67.6%).

### Fifth step - constituent 2

Intermediate B-2-32e (0.38 g, 0.98 mmol) was dissolved in dichloromethane (5 mL). Under ice bath, the mixture was added with 4 M solution of hydrogen chloride in dioxane (5 mL), and the reaction was continuously stirred for 2 hours. The reaction solution was concentrated to obtain a crude product - intermediate B-2-32f, which was used directly in the next reaction step without purification.

### Sixth step - constituent 2

The crude product - intermediate B-2-32f was dissolved in dichloromethane (5 mL), cooled to -10°C, and added with acetyl chloride (80.0 mg, 1.02 mmol) and ethyldiisopropylamine (0.36 g, 2.79 mmol). The reaction was stirred at -10°C for 1 hour, added with water (5 mL), and extracted with dichloromethane (5 mL × 3). The organic phases were combined, washed with saturated brine (5 mL × 3), dried with anhydrous sodium sulfate, filtered, concentrated, and purified by silica gel column chromatography to obtain the product B-2-32 (71.4 mg, 0.22 mmol, total yield of two steps 22.0%). MS (ESI) calculated for [M+H]⁺ was 332.1; found: m/z 332.2. ¹HNMR (400 MHz, Chloroform-d) δ 8.71 (d, J = 8.7 Hz, 1H), 7.92 (ddt, J = 60.4, 8.0, 0.9 Hz, 2H), 7.46 (ddd, J = 8.3, 7.2, 1.3 Hz, 1H), 7.36 (ddd, J = 8.3, 7.2, 1.2 Hz, 1H), 5.33 (dd, J = 8.7, 5.2 Hz, 1H), 5.00 (dd, J = 9.6, 6.3 Hz, 1H), 4.14 - 4.06 (m, 2H), 2.78 - 2.59 (m, 1H), 2.62 - 2.34 (m, 2H), 2.01 (s, 3H), 1.03 (dd, J = 25.1, 6.8 Hz, 6H).

### Preparation Example 62 Preparation of compound B-2-33

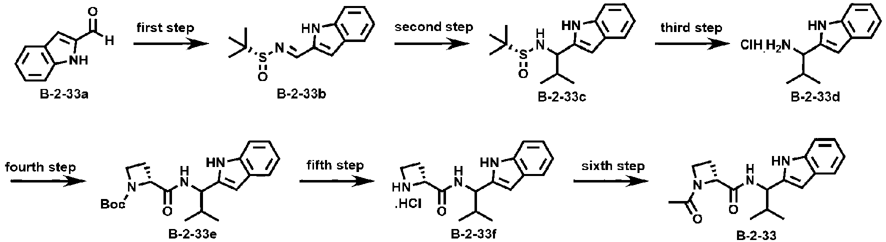

### First step

Intermediate B-2-33a (4.50 g, 31.00 mmol) was dissolved **in** dichloromethane (50 mL), and sequentially added with (R)-tert-butylsulfinamide (4.13 g, 34.10 mmol) and cesium carbonate (20.20 g, 62.00 mmol). After the addition was completed, the reaction was stirred overnight. After completion of the reaction (indicated by TLC), the reaction solution was filtered, and the resulting filtrate was washed with saturated brine (100 mL × 2), dried with anhydrous sodium sulfate, filtered, concentrated, and purified by silica gel column chromatography to obtain intermediate B-2-33b (7.50 g, 30.2 mmol, yield 97.4%). MS (ESI) calculated for [M+H]⁺ was 249.1; found: m/z 249.1.

### Second step

Intermediate B-2-33b (5.00 g, 20.13 mmol) was dissolved in tetrahydrofuran (50 mL). The mixture was subjected to nitrogen protection and cooled to -78°C, and slowly dropwise added with 1 N solution of isopropylmagnesium bromide in tetrahydrofuran (40.26 mL, 40.26 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 3). The organic phases were combined, successively washed with saturated brine (100 mL× 2), dried with anhydrous sodium sulfate, filtered, concentrated, and purified by silica gel column chromatography to obtain a mixture of two constituents, which are recorded as intermediate B-2-33c (3.70 g, 12.7 mmol, yield 62.8%).

### Third step

Intermediate B-2-33c (1.40 g, 4.79 mmol) was dissolved in dichloromethane (10 mL). Under ice bath, the mixture was added with 4 M solution of hydrogen chloride in dioxane (10 mL). The reaction was continuously stirred under ice bath for 1 hour, and then the reaction solution was concentrated to obtain a crude product - intermediate B-2-33d, which was used directly in the next reaction step without purification.

### Fourth step

The crude product B-2-33d was dissolved in dichloromethane (10 mL), and sequentially added with intermediate A-2-7b (1.06 g, 5.26 mmol), *N*-(3-dimethylaminopropyl)-*N*'-ethylcarbodiimide hydrochloride (1.37 g, 7.17 mmol), 1-hydroxybenzotriazole (0.97 g, 7.17 mmol) and ethyldiisopropylamine (1.85 g, 14.37 mmol) at -10°C. After the addition was completed, the reaction was continuously stirred at -10°C for 2 hours, and the reaction solution was added with water (10 mL) and extracted with dichloromethane (10 mL × 3). The organic phases were combined, successively washed with saturated brine (10 mL× 3), dried with anhydrous sodium sulfate, filtered, concentrated, and purified by silica gel column chromatography to obtain intermediate B-2-33e (0.93 g, 2.50 mmol, total yield of two reaction steps 52.3%). MS (ESI) calculated for [M+H]⁺ was 372.2; found: m/z 372.3.

### Fifth step

Intermediate B-2-33e (0.93 g, 2.50 mmol) was dissolved in dichloromethane (10 mL). Under ice bath, the mixture was added with 4 M solution of hydrogen chloride in dioxane (20 mL), and the reaction was continuously stirred for 2 hours. The reaction solution was concentrated to obtain a crude product - intermediate B-2-33f, which was used directly in the next reaction step without purification.

### Sixth step

The crude product - intermediate B-2-33f was dissolved in dichloromethane (10 mL), cooled to -10°C, and added with acetyl chloride (0.22 g, 2.76 mmol) and ethyldiisopropylamine (0.97 g, 7.53 mmol). The reaction was stirred at -10°C for 1 hour, added with water (10 mL), and extracted with dichloromethane (10 mL × 3). The organic phases were combined, washed with saturated brine (10 mL × 3), dried with anhydrous sodium sulfate, filtered, concentrated, and purified by silica gel column chromatography to obtain the product B-2-33 (272.5 mg, 0.87 mmol, total yield of two steps 34.8%). MS (ESI) calculated for [M+H]⁺ was 314.2; found: m/z 314.3. ¹HNMR (400 MHz, Chloroform-d) δ 8.91 (d, J = 21.2 Hz, 1H), 8.33 (dd, J = 53.4, 8.5 Hz, 1H), 7.55 (dt, J = 7.8, 1.0 Hz, 1H), 7.33 (ddq, J = 7.7, 5.8, 1.0 Hz, 1H), 7.19 - 6.99 (m, 2H), 6.34 (ddt, J = 14.5, 1.9, 0.8 Hz, 1H), 5.02 - 4.71 (m, 2H), 4.10 - 3.97 (m, 2H), 2.69 (dddd, J = 11.9, 9.0, 7.4, 6.3 Hz, 1H), 2.47 - 2.25 (m, 2H), 1.92 (d, J = 14.7 Hz, 3H), 1.01 (ddd, J = 23.0, 14.1, 6.8 Hz, 6H).

### Preparation Example 63 Preparation of compounds B-2-34 and B-2-35

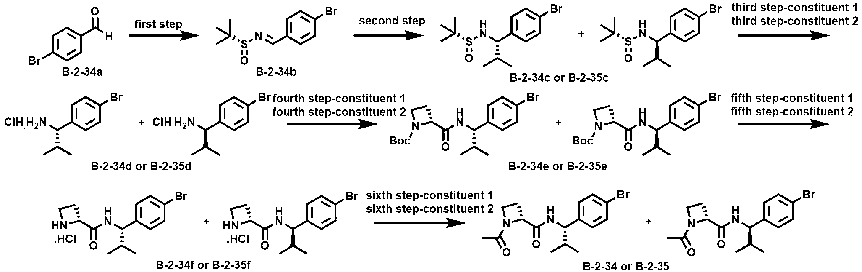

### First step

Intermediate B-2-34a (8.00 g, 43.24 mmol) was dissolved in dichloromethane (100 mL), and sequentially added with (R)-tert-butylsulfinamide (5.24 g, 43.24 mmol) and cesium carbonate (28.18 g, 86.48 mmol). After the addition was completed, the reaction was stirred overnight. After completion of the reaction (indicated by TLC), the reaction solution was added with water (100 mL) and extracted with dichloromethane (100 mL× 2). The organic phases were combined, washed with saturated brine (200 mL), dried with anhydrous sodium sulfate, filtered, concentrated, and purified by silica gel column chromatography to obtain intermediate B-2-34b (10.13 g, 35.15 mmol, yield 81.3%). ¹H NMR (400 MHz, Chloroform-d) δ 8.46 (s, 1H), 7.66 - 7.62 (m, 2H), 7.56 - 7.52 (m, 2H), 1.19 (s, 9H).

### Second step

Intermediate B-2-34b (8.00 g, 27.76 mmol) was dissolved in tetrahydrofuran (100 mL). The mixture was subjected to nitrogen protection and cooled to -78°C, and slowly dropwise added with 1 N solution of isopropylmagnesium bromide in tetrahydrofuran (50.0 mL, 50.0 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 2). The organic phases were combined, successively washed with saturated brine (200 mL), dried with anhydrous sodium sulfate, filtered, concentrated, and purified by silica gel column chromatography to obtain two constituents.

Constituent 1 with a short retention time was intermediate B-2-34c (1.40 g, 4.21 mmol, yield 15.18%). MS (ESI) calculated for [M+H]⁺ was 332.1; found: m/z 332.2. ¹H NMR (400 MHz, Chloroform-d) δ 7.44 - 7.36 (m, 2H), 7.14 - 7.02 (m, 2H), 4.05 - 4.01 (m, 1H), 3.35 (d, J = 5.9 Hz, 1H), 1.17 (s, 9H), 0.85 (d, J = 6.7 Hz, 3H), 0.72 (d, J = 6.7 Hz, 3H).

Constituent 2 with a long retention time was intermediate B-2-35c (1.44 g, 4.34 mmol yield 15.6,%). MS (ESI) calculated for [M+H]⁺ was 332.1; found: m/z 332.2. ¹H NMR (400 MHz, Chloroform-d) δ 7.42 - 7.33 (m, 2H), 7.09 - 7.03 (m, 2H), 4.06 (dt, J = 6.7, 1.7 Hz, 1H), 3.41 (d, J = 1.9 Hz, 1H), 1.12 (s, 9H), 0.90 (d, J = 6.7 Hz, 3H), 0.72 (d, J = 6.8 Hz, 3H).

### Third step - constituent 1

Intermediate B-2-34c (1.40 g, 4.21 mmol) was dissolved in methanol (10 mL). Under ice bath, the mixture was added with 4 M solution of hydrogen chloride in ethyl acetate (5 mL). The mixture was placed at room temperature, the reaction was continuously stirred for 3 hours, and then concentrated to obtain a crude product - intermediate B-2-34d, which was used directly in the next reaction step without purification.

### Fourth step - constituent 1

Intermediate A-2-7b (1.09 g, 5.41 mmol) was dissolved in dichloromethane (20 mL), and sequentially added with *N*,*N*-diisopropylethylamine (1.04 g, 5.41 mmol), 1-hydroxybenzotriazole (730.7 mg, 5.41 mmol) and *N*-(3-dimethylaminopropyl)-*N*'-ethylcarbodiimide hydrochloride (1.61 g, 12.48 mmol) at -10°C. After the reaction was continuously stirred for 30 minutes, the reaction mixture was added with the crude product - intermediate B-2-34d, the reaction was continuously stirred for 2 hours, and completion of the reaction was indicated by TLC. The reaction solution was concentrated, added with water (20 mL), and extracted with ethyl acetate (20 mL×3). The organic phases were combined, successively washed with saturated brine (50 mL), dried with anhydrous sodium sulfate, concentrated, and purified by silica gel column chromatography to obtain intermediate B-2-34e (0.77 g, 1.87 mmol, total yield of two reaction steps 44.4%). MS-ESI theoretical value [M-Boc+2H]⁺: 311.1; found: m/z 311.2.

### Fifth step - constituent 1

Intermediate B-2-34e (0.77 g, 1.87 mmol) was dissolved in methanol (10 mL), cooled to -5°C, and added with 4 M solution of hydrogen chloride in ethyl acetate (10 mL). The mixture was placed at room temperature, the reaction was continuously stirred for 2 hours, and then concentrated to obtain a crude product - intermediate B-2-34f, which was used directly in the next reaction step without purification. Free ammonia MS (ESI) calculated for [M+H]⁺ was 311.1, found: m/z 311.2.

### Sixth step - constituent 1

The crude product - intermediate B-2-34f was dissolved in dichloromethane (20 mL), cooled to -10°C, and added with acetyl chloride (0.17 g, 2.16 mmol) and N,N-diisopropylethylamine (0.56 g, 4.32 mmol). After the reaction was stirred at -10°C for 2 hours, completion of the reaction was indicated by TLC. The reaction solution was added with water (30 mL), and extracted with ethyl acetate (20 mL×3). The organic phases were combined, washed with saturated brine (50 mL), dried with anhydrous sodium sulfate, filtered, concentrated, and purified by silica gel column chromatography to obtain the product B-2-34 (496 mg, 1.40 mmol, total yield of two steps 74.9%). MS (ESI) calculated for [M+H]⁺ was 353.1; found: m/z 353.2. ¹H NMR (400 MHz, Chloroform-d) δ 8.54 (d, J = 8.8 Hz, 1H), 7.35 (d, J = 8.4 Hz, 2H), 7.00 (d, J = 8.5 Hz, 2H), 4.88 (dd, J = 9.5, 6.5 Hz, 1H), 4.63 (dd, J = 8.8, 6.9 Hz, 1H), 4.04 - 3.85 (m, 2H), 2.61 - 2.52 (m, 1H), 2.37 - 2.26 (m, 1H), 1.86 (s, 3H), 0.86 (d, J = 6.7 Hz, 3H), 0.77 (d, J = 6.7 Hz, 3H).

### Third step - constituent 2

Intermediate B-2-35c (1.44 g, 4.33 mmol) was dissolved in methanol (10 mL). Under ice bath, the mixture was added with 4 M solution of hydrogen chloride in ethyl acetate (5 mL). The mixture was placed at room temperature, the reaction was continuously stirred for 3 hours, and completion of the reaction was indicated by TLC. The reaction solution was concentrated to obtain a crude product - intermediate B-2-35d, which was used directly in the next reaction step without purification.

### Fourth step - constituent 2

Intermediate A-2-7b (1.09 g, 5.41 mmol) was dissolved in dichloromethane (20 mL), and sequentially added with *N*,*N*-diisopropylethylamine (1.04 g, 5.41 mmol), 1-hydroxybenzotriazole (730.7 mg, 5.41 mmol) and *N*-(3-dimethylaminopropyl)-*N*'-ethylcarbodiimide hydrochloride (1.61 g, 12.48 mmol) at -10°C. After the reaction was continuously stirred for 30 minutes, the reaction mixture was added with the crude product - intermediate B-2-35d, the reaction was continuously stirred for 2 hours, and completion of the reaction was indicated by TLC. The reaction solution was concentrated, added with water (20 mL), and extracted with ethyl acetate (20 mL×3). The organic phases were combined, successively washed with saturated brine (50 mL), dried with anhydrous sodium sulfate, concentrated, and purified by silica gel column chromatography to obtain intermediate B-2-35e (1.00 g, 2.43 mmol, total yield of two reaction steps 56.4%). MS (ESI) calculated for [M-Boc+2H]⁺ was 311.1; found: m/z 311.2.

### Fifth step - constituent 2

Intermediate B-2-35 (1.00, 2.43 mmol) was dissolved in methanol (10 mL). Under ice bath, the mixture was added with 4 M solution of hydrogen chloride in ethyl acetate (10 mL), and the reaction was continuously stirred for 2 hours. The reaction solution was concentrated to obtain a crude product - intermediate B-2-35f, which was used directly in the next reaction step without purification. MS (ESI) calculated for [M+H]+ was 311.1, found: m/z 311.2.

### Sixth step - constituent 2

The crude product - intermediate B-2-35f was dissolved in dichloromethane (20 mL), cooled to -10°C, and added with acetyl chloride (0.25 g, 3.24 mmol) and N,N-diisopropylethylamine (0.84 g, 6.48 mmol). After the reaction was stirred at -10°C for 2 hours, completion of the reaction was indicated by TLC. The reaction solution was added with water (30 mL), and extracted with ethyl acetate (20 mL×3). The organic phases were combined, washed with saturated brine (50 mL), dried with anhydrous sodium sulfate, filtered, concentrated, and purified by silica gel column chromatography to obtain the product B-2-35 (0.74 g, 2.10 mmol, total yield of two steps 86.4%). MS (ESI) calculated for [M+H]⁺ was 353.1; found: m/z 353.2. ¹H NMR (400 MHz, Chloroform-d) δ 8.48 (d, J = 8.5 Hz, 1H), 7.36 (d, *J* = 8.5 Hz, 2H), 7.07 (d, *J* = 8.4 Hz, 2H), 4.78 (dd, *J* = 9.5, 6.5 Hz, 1H), 4.58 (dd, *J* = 8.5, 6.9 Hz, 1H), 4.00 (t, *J* = 7.8 Hz, 2H), 2.67 - 2.58 (m, 1H), 2.34 - 2.24 (m, 1H), 1.88 (s, 3H), 0.81 (d, *J* = 6.8 Hz, 3H), 0.75 (d, *J =* 6.7 Hz, 3H).

### Preparation Example 64 Preparation of compound B-2-36

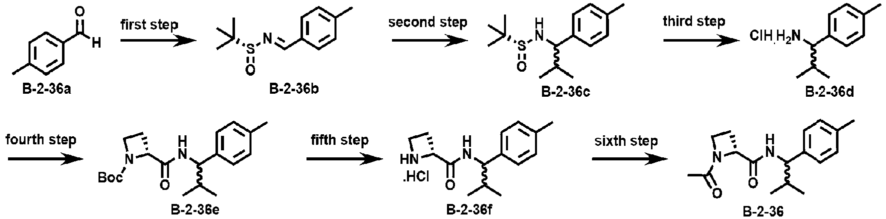

### First step

Intermediate B-2-36a (5.00 g, 41.61 mmol) was dissolved **in** dichloromethane (100 mL), and sequentially added with (*R*)-tert-butylsulfinamide (5.04 g, 41.61 mmol) and cesium carbonate (27.11 g, 83.22 mmol). After the addition was completed, the reaction was stirred for 2 hours. After completion of the reaction (indicated by TLC), the reaction solution was added with water (100 mL) and extracted with dichloromethane (100 mL× 2). The organic phases were combined, washed with saturated brine (200 mL), dried with anhydrous sodium sulfate, filtered, concentrated, and purified by silica gel column chromatography to obtain intermediate B-2-36b (8.79 g, 39.36 mmol, yield 94.6%). ¹H NMR (400 MHz, Chloroform-d) δ 8.47 (s, 1H), 7.70 - 7.63 (m, 2H), 7.19 (d, J = 7.9 Hz, 2H), 2.33 (s, 3H), 1.18 (s, 9H).

### Second step

Intermediate B-2-36b (8.00 g, 35.82 mmol) was dissolved in tetrahydrofuran (50 mL). The mixture was subjected to nitrogen protection and cooled to -78°C, and slowly dropwise added with 1 N solution of isopropylmagnesium bromide in tetrahydrofuran (70.0 mL, 70.0 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 2). The organic phases were combined, successively washed with saturated brine (200 mL), dried with anhydrous sodium sulfate, filtered, concentrated, and purified by silica gel column chromatography to obtain two constituents.

Constituent with a short retention time was intermediate B-2-36c (2.01 g, 7.52 mmol, yield 21.0%). The other constituent was discarded.

### Third step

Intermediate B-2-36c (2.00 g, 7.48 mmol) was dissolved in methanol (10 mL). Under ice bath, the mixture was added with 4 M solution of hydrogen chloride in ethyl acetate (10 mL). The mixture was placed at room temperature, the reaction was continuously stirred for 3 hours, and then concentrated to obtain a crude product - intermediate B-2-36d, which was used directly in the next reaction step without purification.

### Fourth step

Intermediate A-2-7b (2.33 g, 11.56 mmol) was dissolved in dichloromethane (20 mL), and sequentially added with *N*,*N*-diisopropylethylamine (2.99 g, 23.13 mmol), 1-hydroxybenzotriazole (1.35 g, 10.02 mmol) and *N*-(3-dimethylaminopropyl)-*N*'-ethylcarbodiimide hydrochloride (1.92 g, 10.02 mmol) at -10°C. After the reaction was continuously stirred for 30 minutes, the reaction mixture was added with the crude product - intermediate B-2-36d, the reaction was continuously stirred for 2 hours, and completion of the reaction was indicated by TLC. The reaction solution was concentrated, added with water (20 mL), and extracted with ethyl acetate (20 mL×3). The organic phases were combined, successively washed with saturated brine (50 mL), dried with anhydrous sodium sulfate, concentrated, and purified by silica gel column chromatography to obtain intermediate B-2-36e (2.11 g, 6.09 mmol, total yield of two reaction steps 81.6%). MS (ESI) calculated for [M-Boc+2H]⁺ was 247.2; found: m/z 247.3.

### Fifth step

Intermediate B-2-36e (2.11 g, 6.09 mmol) was dissolved in methanol (10 mL), cooled to -5°C, and added with 4 M solution of hydrogen chloride in ethyl acetate (10 mL). The mixture was placed at room temperature, the reaction was continuously stirred for 2 hours, and thenconcentrated to obtain a crude product - intermediate B-2-36f, which was used directly in the next reaction step without purification. Free ammonia MS (ESI) calculated for [M+H]⁺ was 247.2, found: m/z 247.3.

### Sixth step

The crude product - intermediate B-2-36f was dissolved in dichloromethane (20 mL), cooled to -10°C, and added with acetyl chloride (0.62 g, 7.95 mmol) and N,N-diisopropylethylamine (2.05 g, 15.90 mmol). After the reaction was stirred at -10°C for 2 hours, completion of the reaction was indicated by TLC. The reaction solution was added with water (30 mL), and extracted with ethyl acetate (20 mL×3). The organic phases were combined, washed with saturated brine (50 mL), dried with anhydrous sodium sulfate, filtered, concentrated, and purified by silica gel column chromatography to obtain the product B-2-36 (1.43 g, 4.96 mmol, total yield of two steps 81.4%). MS (ESI) calculated for [M+H]⁺ was 289.2; found: m/z 289.2. ¹H NMR (400 MHz, Chloroform-d) δ 8.40 (d, J = 8.8 Hz, 1H), 7.08 (d, J = 8.2 Hz, 2H), 7.04 (d, J = 8.0 Hz, 2H), 4.77 (dd, J = 9.5, 6.4 Hz, 1H), 4.61 (dd, J = 8.8, 7.1 Hz, 1H), 4.03 - 3.96 (m, 2H), 2.66 (ddt, J = 11.9, 9.1, 6.7 Hz, 1H), 2.33 - 2.26 (m, 1H), 2.24 (s, 3H), 1.88 (s, 3H), 0.82 (d, J = 6.8 Hz, 3H), 0.75 (d, J = 6.7 Hz, 3H).

### Preparation Example 65 Preparation of compound B-2-37

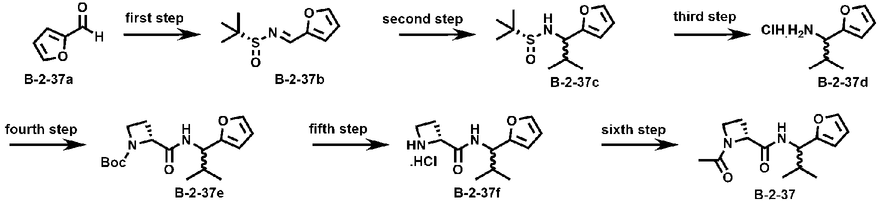

### First step

Intermediate B-2-37a (5.00 g, 52.04 mmol) was dissolved in dichloromethane (100 mL), and sequentially added with (*R*)-tert-butylsulfinamide (6.31 g, 52.04 mmol) and cesium carbonate (33.91 g, 104.08 mmol). After the addition was completed, the reaction was stirred for 2 hours. After completion of the reaction (indicated by TLC), the reaction solution was added with water (100 mL) and extracted with dichloromethane (100 mL× 2). The organic phases were combined, washed with saturated brine (200 mL), dried with anhydrous sodium sulfate, filtered, concentrated, and purified by silica gel column chromatography to obtain intermediate B-2-37b (10.02 g, 50.28 mmol, yield 96.6%). MS (ESI) calculated for [M+H]⁺ was 200.1, found: m/z 200.2.

### Second step

Intermediate B-2-37b (10.00 g, 27.76 mmol) was dissolved in tetrahydrofuran (100 mL). The mixture was subjected to nitrogen protection and cooled to -78°C, and slowly dropwise added with 1 N solution of isopropylmagnesium bromide in tetrahydrofuran (50.0 mL, 50.0 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 2). The organic phases were combined, successively washed with saturated brine (200 mL), dried with anhydrous sodium sulfate, filtered, concentrated, and purified by silica gel column chromatography to obtain two constituents.

Constituent with a short retention time was intermediate B-2-37c (1.37 g, 7.68 mmol, yield 15.3%). MS (ESI) calculated for [M+H]⁺ was 244.1; found: m/z 244.3. The other constituent was discarded.

### Third step

Intermediate B-2-37c (1.87 g, 7.68 mmol) was dissolved in methanol (10 mL). Under ice bath, the mixture was added with 4 M solution of hydrogen chloride in ethyl acetate (5 mL). The mixture was placed at room temperature, the reaction was continuously stirred for 3 hours, and then concentrated to obtain a crude product - intermediate B-2-37d, which was used directly **in** the next reaction step without purification.

### Fourth step

Intermediate A-2-7b (1.00 g, 4.14 mmol) was dissolved in dichloromethane (20 mL), and sequentially added with *N*,*N*-diisopropylethylamine (1.61 g, 12.45 mmol), 1-hydroxybenzotriazole (0.67 g, 4.98 mmol) and *N*-(3-dimethylaminopropyl)-*N*'-ethylcarbodiimide hydrochloride (0.95 g, 4.98 mmol) at -10°C. After the reaction was continuously stirred for 30 minutes, the reaction mixture was added with the crude product - intermediate B-2-37d, and then stirred for 2 hours, and completion of the reaction was indicated by TLC. The reaction solution was concentrated, added with water (20 mL), and extracted with ethyl acetate (20 mL×3). The organic phases were combined, successively washed with saturated brine (50 mL), dried with anhydrous sodium sulfate, concentrated, and purified by silica gel column chromatography to obtain intermediate B-2-37e (0.91 g, 2.82 mmol, total yield of two reaction steps 36.7%). MS (ESI) calculated for [M-Boc+2H]⁺ was 223.1; found: m/z 223.3.

### Fifth step

Intermediate B-2-37e (0.91 g, 2.82 mmol) was dissolved in methanol (10 mL), cooled to -5°C, and added with 4 M solution of hydrogen chloride in ethyl acetate (10 mL). The mixture was placed at room temperature, the reaction was continuously stirred for 2 hours, and then concentrated to obtain a crude product - intermediate B-2-37f, which was used directly in the next reaction step without purification.

### Sixth step

The crude product - intermediate B-2-37f was dissolved in dichloromethane (20 mL), cooled to -10°C, and added with acetyl chloride (0.36 mmol, 4.59 mmol) and *N*,*N*-diisopropylethylamine (1.14 g, 9.18 mmol). After the reaction was stirred at -10°C for 2 hours, completion of the reaction was indicated by TLC. The reaction solution was added with water (30 mL), and extracted with ethyl acetate (20 mL×3). The organic phases were combined, washed with saturated brine (50 mL), dried with anhydrous sodium sulfate, filtered, concentrated, and purified by silica gel column chromatography to obtain the product B-2-37 (0.57 g, 2.16 mmol, total yield of two steps 76.5%). MS (ESI) calculated for [M+H]⁺ was 265.1; found: m/z 265.4. ¹H NMR (400 MHz, Chloroform-d) δ 8.30 (d, J = 9.1 Hz, 1H), 7.33 (dd, J = 1.9, 0.9 Hz, 1H), 6.28 (dd, J = 3.2, 1.8 Hz, 1H), 6.18 (d, J = 3.2 Hz, 1H), 4.88 (td, J = 9.6, 6.4 Hz, 2H), 4.11 - 4.04 (m, 2H), 2.73 (ddt, J = 11.9, 8.9, 6.8 Hz, 1H), 2.45 - 2.33 (m, 1H), 1.92 (s, 3H), 0.91 - 0.85 (m, 6H).

### Preparation Example 66 Preparation of compound B-2-38

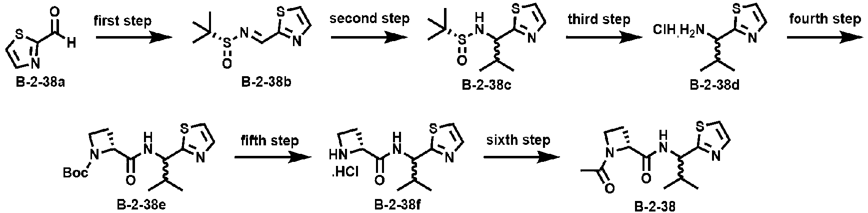

### First step

Intermediate B-2-38a (5.00 g, 44.19 mmol) was dissolved in dichloromethane (100 mL), and sequentially added with (*R*)-tert-butylsulfinamide (5.36 g, 44.19 mmol) and cesium carbonate (28.80 g, 88.38 mmol). After the addition was completed, the reaction was stirred for 2 hours. After completion of the reaction (indicated by TLC), the reaction solution was added with water (100 mL) and extracted with dichloromethane (100 mL× 2). The organic phases were combined, washed with saturated brine (200 mL), dried with anhydrous sodium sulfate, filtered, concentrated, and purified by silica gel column chromatography to obtain intermediate B-2-38b (8.27 g, 38.23 mmol, yield 86.5%).

### Second step

Intermediate B-2-38b (5.00 g, 23.11 mmol) was dissolved in tetrahydrofuran (100 mL). The mixture was subjected to nitrogen protection and cooled to -78°C, and slowly dropwise added with 1 N solution of isopropylmagnesium bromide in tetrahydrofuran (50.0 mL, 50.0 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 2). The organic phases were combined, successively washed with saturated brine (200 mL), dried with anhydrous sodium sulfate, filtered, concentrated, and purified by silica gel column chromatography to obtain two constituents.

Constituent 1 with a short retention time was intermediate B-2-38c (2.51 g, 9.63 mmol, yield 41.7%). MS (ESI) calculated for [M+H]⁺ was 261.1; found: m/z 261.1. ¹H NMR (400 MHz, Chloroform-d) δ 7.66 (d, J = 3.3 Hz, 1H), 7.21 (d, J = 3.2 Hz, 1H), 4.45 - 4.38 (m, 2H), 2.21 - 2.12 (m, 1H), 1.21 (s, 9H), 0.87 (dd, J = 6.8, 3.8 Hz, 6H).

### The other constituent was discarded.

### Third step

Intermediate B-2-38c (2.51 g, 9.63 mmol) was dissolved in methanol (10 mL). Under ice bath, the mixture was added with 4 M solution of hydrogen chloride in ethyl acetate (5 mL). The mixture was placed at room temperature, the reaction was continuously stirred for 3 hours, and then concentrated to obtain a crude product - intermediate B-2-38d, which was used directly in the next reaction step without purification.

### Fourth step

Intermediate A-2-7b (1.31 g, 6.53 mmol) was dissolved in dichloromethane (50 mL), and sequentially added with *N*,*N*-diisopropylethylamine (2.11 g, 16.32 mmol), 1-hydroxybenzotriazole (0.88 g, 6.53 mmol) and *N*-(3-dimethylaminopropyl)-*N*'-ethylcarbodiimide hydrochloride (1.25 g, 6.53 mmol) at -10°C. After the reaction was continuously stirred for 30 minutes, and then added with the crude product - intermediate B-2-38d, the reaction was continuously stirred for 2 hours, and completion of the reaction was indicated by TLC. The reaction solution was concentrated, added with water (20 mL), and extracted with ethyl acetate (20 mL×3). The organic phases were combined, successively washed with saturated brine (50 mL), dried with anhydrous sodium sulfate, concentrated, and purified by silica gel column chromatography to obtain intermediate B-2-38e (1.38 g, 4.07 mmol, total yield of two reaction steps 42.3%).

### Fifth step

Intermediate B-2-38e (1.38 g, 4.07 mmol) was dissolved in methanol (10 mL), cooled to -5°C, and added with 4 M solution of hydrogen chloride in ethyl acetate (10 mL). The mixture was placed at room temperature, the reaction was continuously stirred for 2 hours, and then concentrated to obtain a crude product - intermediate B-2-38f, which was used directly in the next reaction step without purification.

### Sixth step

The crude product - intermediate B-2-38f was dissolved in dichloromethane (20 mL), cooled to -10°C, and added with acetyl chloride (0.48 g, 6.07 mmol) and *N*,*N*-diisopropylethylamine (1.57 g, 4.32 mmol). After the reaction was stirred at -10°C for 2 hours, completion of the reaction was indicated by TLC. The reaction solution was added with water (30 mL), and extracted with ethyl acetate (20 mL×3). The organic phases were combined, washed with saturated brine (50 mL), dried with anhydrous sodium sulfate, filtered, concentrated, and purified by silica gel column chromatography to obtain the product B-2-38 (496.0 mg, 1.40 mmol, total yield of two steps 34.4%). MS (ESI) calculated for [M+H]⁺ was 282.2; found: m/z 282.4. ¹H NMR (400 MHz, Chloroform-d) δ 8.66 (d, J = 8.5 Hz, 1H), 7.75 (d, J = 3.3 Hz, 1H), 7.23 (d, J = 3.3 Hz, 1H), 5.21 (dd, J = 8.6, 5.4 Hz, 1H), 4.95 (dd, J = 9.5, 6.4 Hz, 1H), 4.14 - 4.07 (m, 2H), 2.82 - 2.70 (m, 1H), 2.46 - 2.38 (m, 2H), 1.96 (s, 3H), 0.96 (d, J = 6.8 Hz, 3H), 0.91 (d, J = 6.9 Hz, 3H).

### Preparation Example 67 Preparation of compounds B-2-39 and B-2-40

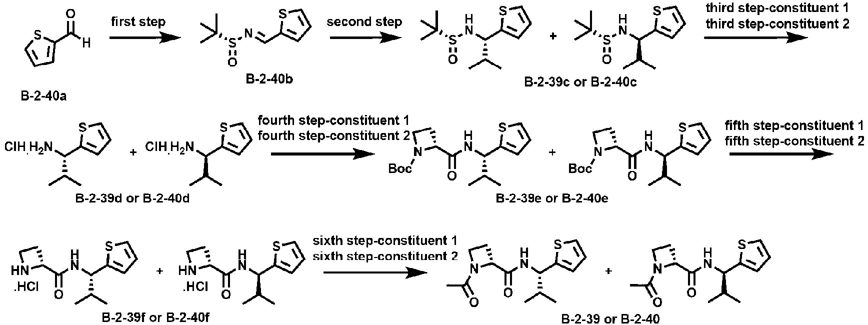

### First step

Intermediate B-2-40a (5.00 g, 44.58 mmol) was dissolved in dichloromethane (100 mL), and sequentially added with (*R*)-tert-butylsulfinamide (5.40 g, 44.58 mmol) and cesium carbonate (29.05 g, 89.16 mmol). After the addition was completed, the reaction was stirred for 2 hours. After completion of the reaction (indicated by TLC), the reaction solution was added with water (100 mL) and extracted with dichloromethane (100 mL× 2). The organic phases were combined, washed with saturated brine (200 mL), dried with anhydrous sodium sulfate, filtered, concentrated, and purified by silica gel column chromatography to obtain intermediate B-2-40b (7.87 g, 36.55 mmol, yield 92.0%).MS (ESI) calculated for [M+H]⁺ was 216.0, found: m/z 216.2.

### Second step

Intermediate B-2-40b (5.00 g, 23.23 mmol) was dissolved in tetrahydrofuran (100 mL). The mixture was subjected to nitrogen protection and cooled to -78°C, and slowly dropwise added with 2 N solution of isopropylmagnesium bromide in tetrahydrofuran (24.0 mL, 48.0 mmol). After the addition was completed, the reaction was continuously stirred at -78°C for 2 hours, and completion of the reaction was indicated by TLC. The reaction solution was added with saturated ammonium chloride solution to adjust pH to 6, and then extracted with ethyl acetate (100 mL × 2). The organic phases were combined, successively washed with saturated brine (200 mL), dried with anhydrous sodium sulfate, filtered, concentrated, and purified by silica gel column chromatography to obtain two constituents.

Constituent 1 with a short retention time was intermediate B-2-40c (2.51 g, 9.68 mmol, yield 41.7%).

Constituent 2 with a long retention time was intermediate B-2-39c (0.76 g, 2.90 mmol, yield 12.6%).

### Third step - constituent 1

Intermediate B-2-40c (1.12 g, 4.30 mmol) was dissolved in methanol (10 mL). Under ice bath, the mixture was added with 4 M solution of hydrogen chloride in ethyl acetate (10 mL). The mixture was placed at room temperature, the reaction was continuously stirred for 3 hours, and then concentrated to obtain a crude product - intermediate B-2-40d, which was used directly in the next reaction step without purification.

### Fourth step - constituent 1

Intermediate A-2-7b (671.1 mg, 3.36 mmol) was dissolved in dichloromethane (20 mL), and sequentially added with *N*,*N*-diisopropylethylamine (1.09 g, 8.41 mmol), 1-hydroxybenzotriazole (454.0 mg, 3.36 mmol) and *N*-(3-dimethylaminopropyl)-*N*'-ethylcarbodiimide hydrochloride (644.1 mg, 3.36 mmol) at -10°C. After the reaction was continuously stirred for 30 minutes, the reaction mixture was added with the crude product - intermediate B-2-40d, the reaction was continuously stirred for 2 hours, and completion of the reaction was indicated by TLC. The reaction solution was concentrated, added with water (20 mL), and extracted with ethyl acetate (20 mL×3). The organic phases were combined, successively washed with saturated brine (50 mL), dried with anhydrous sodium sulfate, concentrated, and purified by silica gel column chromatography to obtain intermediate B-2-40e (742.0 mg, 2.19 mmol, total yield of two reaction steps 51.0%).MS (ESI) calculated for [M-Boc+2H]⁺ was 239.1; found: m/z 239.2.

### Fifth step - constituent 1

Intermediate B-2-40e (742.0 mg, 2.19 mmol) was dissolved in methanol (10 mL), cooled to -5°C, and added with 4 M solution of hydrogen chloride in ethyl acetate (10 mL). The mixture was placed at room temperature, the reaction was continuously stirred for 2 hours, and then concentrated to obtain a crude product - intermediate B-2-40f, which was used directly in the next reaction step without purification.

### Sixth step - constituent 1

The crude product - intermediate B-2-40f was dissolved in dichloromethane (20 mL), cooled to -10°C, and added with acetyl chloride (0.26 g, 3.29 mmol) and *N*,*N*-diisopropylethylamine (0.85 g, 6.57 mmol). After the reaction was stirred at -10°C for 2 hours, completion of the reaction was indicated by TLC. The reaction solution was added with water (30 mL), and extracted with ethyl acetate (20 mL×3). The organic phases were combined, washed with saturated brine (50 mL), dried with anhydrous sodium sulfate, filtered, concentrated, and purified by silica gel column chromatography to obtain the product B-2-40 (468.0 mg, 1.67 mmol, total yield of two steps 76.2%). MS (ESI) calculated for [M+H]⁺ was 281.1; found: m/z 281.2. ¹H NMR (400 MHz, Chloroform-d) δ 8.42 (d, J = 9.0 Hz, 1H), 7.16 (dd, J = 4.6, 1.6 Hz, 1H), 6.96 - 6.91 (m, 2H), 5.07 (dd, J = 8.9, 6.4 Hz, 1H), 4.87 (dd, J = 9.5, 6.4 Hz, 1H), 4.10 - 4.03 (m, 2H), 2.78 - 2.67 (m, 1H), 2.44 - 2.32 (m, 1H), 2.09 (h, J = 6.7 Hz, 1H), 1.94 (s, 3H), 0.93 (d, J = 2.0 Hz, 3H), 0.91 (d, J = 1.9 Hz, 3H).

### Third step - constituent 2

Intermediate B-2-39c (0.76 g, 2.90 mmol) was dissolved in methanol (5 mL). Under ice bath, the mixture was added with 4 M solution of hydrogen chloride in ethyl acetate (5 mL). The mixture was placed at room temperature, the reaction was continuously stirred for 3 hours, and then concentrated to obtain a crude product - intermediate B-2-39d, which was used directly in the next reaction step without purification.

### Fourth step - constituent 2

Intermediate A-2-7b (700 mg, 3.48 mmol) was dissolved in dichloromethane (50 mL), and sequentially added with *N*,*N*-diisopropylethylamine (1.12 g, 8.70 mmol), 1-hydroxybenzotriazole (0.47 g, 3.48 mmol) and *N*-(3-dimethylaminopropyl)-*N*'-ethylcarbodiimide hydrochloride (0.67 g, 3.48 mmol) at -10°C. After the reaction was continuously stirred for 30 minutes, the reaction mixture was added with the crude product - intermediate B-2-39d, the reaction was continuously stirred for 2 hours, and completion of the reaction was indicated by TLC. The reaction solution was concentrated, added with water (20 mL), and extracted with ethyl acetate (20 mL×3). The organic phases were combined, successively washed with saturated brine (50 mL), dried with anhydrous sodium sulfate, concentrated, and purified by silica gel column chromatography to obtain intermediate B-2-39e (0.74 g, 2.19 mmol, total yield of two reaction steps 75.5%).

### Fifth step - constituent 2

Intermediate B-2-39e (0.74 g, 2.19 mmol) was dissolved in methanol (10 mL), cooled to -5°C, and added with 4 M solution of hydrogen chloride in ethyl acetate (10 mL). The mixture was placed at room temperature, the reaction was continuously stirred for 2 hours, and then concentrated to obtain a crude product - intermediate B-2-39f, which was used directly in the next reaction step without purification.

### Sixth step - constituent 2

The crude product - intermediate B-2-39f was dissolved in dichloromethane (20 mL), cooled to -10°C, and added with acetyl chloride (0.26 g, 3.29 mmol) and *N*,*N*-diisopropylethylamine (0.85 g, 6.57 mmol). After the reaction was stirred at -10°C for 2 hours, completion of the reaction was indicated by TLC. The reaction solution was added with water (30 mL), and extracted with ethyl acetate (20 mL×3). The organic phases were combined, washed with saturated brine (50 mL), dried with anhydrous sodium sulfate, filtered, concentrated, and purified by silica gel column chromatography to obtain the product B-2-39 (0.37 g, 1.32 mmol, total yield of two steps 60.3%). MS (ESI) calculated for [M+H]⁺ was 281.1; found: m/z 282.4. ¹H NMR (400 MHz, Chloroform-d) δ 8.40 (d, J = 9.2 Hz, 1H), 7.09 (dd, J = 5.1, 1.2 Hz, 1H), 6.86 (dd, J = 5.1, 3.5 Hz, 1H), 6.80 (dt, J = 3.5, 1.1 Hz, 1H), 5.05 (dd, J = 8.9, 6.2 Hz, 1H), 4.87 (dd, J = 9.6, 6.4 Hz, 1H), 4.00 - 3.94 (m, 2H), 2.66 - 2.56 (m, 1H), 2.38 - 2.31 (m, 1H), 2.03 (h, J = 6.7 Hz, 1H), 1.87 (s, 3H), 0.88 (dd, J = 6.8, 5.5 Hz, 6H).

### Preparation Example 68 Preparation of compound B-2-41

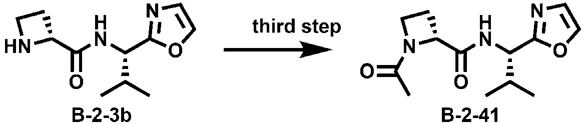

Intermediate B-2-3b (1.10 g, 4.93 mmol) and *N*,*N*-diisopropylethylamine (2.55 g, 19.73 mmol) were dissolved in anhydrous dichloromethane (20 mL), and cooled to -10°C. The mixture was slowly dropwise added with acetyl chloride (0.77 g, 9.86 mmol). The reaction mixture was stirred at 0°C for 2 hours, and completion of the reaction was indicated by TLC. The reaction solution was added with water (50 mL), and extracted with ethyl acetate (100 mL × 3). The organic phases were combined, dried with anhydrous sodium sulfate, filtered, concentrated, and purified by silica gel column chromatography to obtain the product B-2-41 (0.40 g, 1.51 mmol, yield 30.6%). MS (ESI) calculated for [M+H]⁺ was 266.1; found: m/z 266.1. ¹H NMR (400 MHz, DMSO-d6) δ 8.76 (d, J = 8.6 Hz, 0.5H), 8.54 (d, J = 8.9 Hz, 0.5H), 8.10 - 8.03 (m, 1H), 7.18 (s, 1H), 4.94 - 4.67 (m, 2H), 4.03 (t, J = 7.7 Hz, 1H), 3.75 (dt, J = 9.0, 6.1 Hz, 1H), 2.49 - 2.32 (m, 1H), 2.25 - 1.95 (m, 2H), 1.77 (s, 1.5H), 1.57 (s, 1.5H), 0.89 (dd, J = 16.4, 6.7 Hz, 3H), 0.80 (dd, J = 8.6, 6.7 Hz, 3H).

### Preparation Example 69 Preparation of compound B-2-42

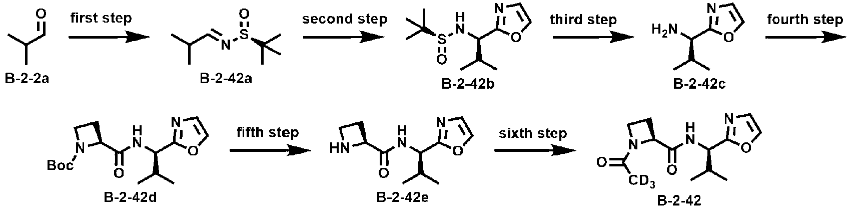

### First step

Intermediate B-2-2a (10.00 g, 138.68 mmol) was dissolved **in** dichloromethane (300 mL), and added with (*S*)-tert-butylsulfinamide (16.81 g, 138.68 mmol) and copper sulfate (5.53 g, 34.67 mmol). The reaction was stirred for 16 hours, and completion of the reaction was indicated by TLC. The reaction solution was filtered, concentrated, dissolved by adding ethyl acetate (150 mL), and then slowly added with petroleum ether (450 mL). The mixture was stirred for 12 hour and filtered, and the filtrate was concentrated to obtain a crude product - intermediate B-2-42a (15.00 g, 85.6 mmol, yield 61.7%).

### Second step

Under nitrogen protection, 1 M solution of borane in tetrahydrofuran (171.14 mL, 171.14 mmol) was added with oxazole (17.73 g, 256.71 mmol), and cooled to -78°C. The mixture was slowly added with 2.5 M solution of n-butyllithium in tetrahydrofuran (68.46 mL, 171.14 mmol), the reaction was continuously stirred for 30 minutes, and then the reaction mixture was slowly added with the crude product - intermediate B-2-42a (15.00 g, 85.6 mmol). After the reaction was continuously stirred for 4 hours, completion of the reaction was indicated by TLC, and the reaction solution was poured into saturated aqueous ammonium chloride solution (200 mL) and extracted with ethyl acetate (100 mL × 3). The organic phases were combined, washed with saturated brine (200 mL), dried with anhydrous sodium sulfate, filtered, concentrated, and purified by silica gel column chromatography to obtain intermediate B-2-42b (10.0 g, 40.9 mmol, yield 47.8%).

### Third step

Intermediate B-2-42b (10.0 g, 40.9 mmol) was dissolved in methanol (200 mL), and the mixture was added with 4 M solution of hydrogen chloride in dioxane (200 mL) at 0°C. The reaction was continuously stirred for 2 hours, and completion of the reaction was indicated by TLC. The reaction solution was added with ammonia water to adjust pH to 7 , and concentrated. The resulting residue was dissolved in methanol (50 mL), then heated to 60°C and stirred for 1 hour, added with acetonitrile (500 mL) and continuously stirred for 1 hour. The mixture was slowly cooled to room temperature to precipitate a solid, then subjected to suction filtration, and dried to obtain intermediate B-2-42c (5.00 g, 35.7 mmol, yield 87.2%). ¹H NMR (400 MHz, MeOD) δ 8.01 (s, 1H), 7.26 (s, 1H), 4.42 (d, J = 6.4 Hz, 1H), 2.45 - 2.22 (m, 1H), 1.09 (d, J = 6.8 Hz, 3H), 0.96 (d, J = 6.8 Hz, 3H).

### Fourth step

Intermediate A-2-6b (1.43 g, 7.11 mmol) was dissolved in dichloromethane (40 mL), cooled to -10°C, and added with 1-ethyl-(3-dimethylaminopropyl)carbodiimide hydrochloride (2.05 g, 10.70 mmol) and 1-hydroxybenzotriazole (1.45 g, 10.70 mmol). After the reaction was continuously stirred for 10 minutes, the reaction mixture was added with intermediate B-2-42c (1.00 g, 7.14 mmol) and N,N-diisopropylethylamine (2.76 g, 21.39 mmol). The reaction was continuously stirred for 3 hours, and completion of the reaction was indicated by TLC. The reaction solution was added with dichloromethane (50 mL), and washed with water (100 mL). The organic phases were collected, dried with anhydrous sodium sulfate, concentrated, and purified by silica gel column chromatography to obtain intermediate B-2-42d (0.60 g, 1.86 mmol, yield 26.1%).

### Fifth step

Intermediate B-2-42d (0.60 g, 1.86 mmol) was dissolved in dichloromethane (25 mL), and added with 4 M solution of hydrogen chloride in dioxane (5 mL). After the reaction was stirred for 1 hour, completion of the reaction was indicated by TLC, and the reaction solution was concentrated to obtain a crude product - intermediate B-2-42e, which was used directly in the next reaction step without purification.

### Sixth step

Deuterated acetic acid (0.57 g, 8.96 mmol) was dissolved in N,N-dimethylformamide (20 mL),
and added with 2-(7-azabenzotriazol-1-yl)-N,N,N',N'-tetramethyluronium hexafluorophosphate (3.41 g, 8.96 mmol). After the reaction was stirred for 30 minutes, the reaction mixture was sequentially added with the crude product - intermediate B-2-42e and N,N-diisopropylethylamine (1.29 g, 10 mmol). The reaction was continuously stirred for 16 hours, and completion of the reaction was indicated by TLC. The reaction solution was added with water (80 mL), and extracted with ethyl acetate (60 mL×3). The organic phases were combined, washed with water (100 mL), dried with anhydrous sodium sulfate, filtered, concentrated, and purified by silica gel column chromatography to obtain the product B-2-42 (45.1 mg, 0.17 mmol, total yield of two reaction steps 9.0%). MS (ESI) calculated for [M+H]⁺ was 269.2; found: m/z 269.4. ¹H NMR (400 MHz, CDCl3) δ 8.37 (d, J = 8.2 Hz, 1H), 7.53 (s, 1H), 7.00 (s, 1H), 4.99 (dt, J = 19.6, 9.8 Hz, 1H), 4.87 (dd, J = 9.4, 6.6 Hz, 1H), 4.09 - 3.93 (m, 1H), 3.72 - 3.54 (m, 1H), 2.63 - 2.50 (m, 1H), 2.45 - 2.30 (m, 1H), 2.19 (td, J = 13.4, 6.8 Hz, 1H), 0.93 - 0.81 (m, 6H).

### Preparation Example 70 Preparation of compound B-2-43

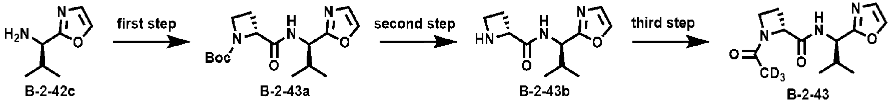

### First step

Intermediate A-2-7b (1.43 g, 7.13 mmol) was dissolved in dichloromethane (40 mL), cooled to -10°C, and added with 1-ethyl-(3-dimethylaminopropyl)carbodiimide hydrochloride (2.05 g, 10.70 mmol) and 1-hydroxybenzotriazole (1.45 g, 10.70 mmol). After the reaction was continuously stirred for 10 minutes, the reaction mixture was added with intermediate B-2-42c (1.00 g, 7.14 mmol) and N,N-diisopropylethylamine (2.76 g, 21.39 mmol). The reaction was continuously stirred for 3 hours, and completion of the reaction was indicated by TLC. The reaction solution was added with dichloromethane (50 mL), and washed with water (100 mL). The organic phases were collected, dried with anhydrous sodium sulfate, concentrated, and purified by silica gel column chromatography to obtain intermediate B-2-43a (0.40 g, 1.24 mmol, yield 17.3%).

### Second step

Intermediate B-2-43a (0.40 g, 1.24 mmol) was dissolved in dichloromethane (25 mL), and added with 4 M solution of hydrogen chloride in dioxane (5 mL). After the reaction was stirred for 1 hour, completion of the reaction was indicated by TLC, and the reaction solution was concentrated to obtain a crude product - intermediate B-2-43b, which was used directly in the next reaction step without purification.

### Third step

Deuterated acetic acid (0.20 g, 3.14 mmol) was dissolved in N,N-dimethylformamide (10 mL), and added with 2-(7-azabenzotriazol-1-yl)-N,N,N',N'-tetramethyluronium hexafluorophosphate (1.19g, 3.14 mmol). After the reaction was stirred for 30 minutes, the reaction solution was sequentially added with the crude product - intermediate B-2-43b and N,N-diisopropylethylamine (0.61 g, 4.71 mmol). The reaction was continuously stirred for 16 hours, and completion of the reaction was indicated by TLC. The reaction solution was added with water (80 mL), and extracted with ethyl acetate (60 mL×3). The organic phases were combined, washed with water (100 mL), dried with anhydrous sodium sulfate, filtered, concentrated, and purified by silica gel column chromatography to obtain the product B-2-43 (78.2 mg, 0.29 mmol, total yield of two reaction steps 23.4%). MS (ESI) calculated for [M+H]⁺ was 269.2; found: m/z 269.4. ¹H NMR (400 MHz, CDCl3) δ 8.50 (d, J = 7.7 Hz, 1H), 7.54 (d, J = 8.6 Hz, 1H), 7.01 (s, 1H), 4.98 (dd, J = 8.4, 5.9 Hz, 1H), 4.87 (dd, J = 9.3, 6.4 Hz, 1H), 4.10 - 3.93 (m, 2H), 2.79 - 2.58 (m, 1H), 2.43 - 2.29 (m, 1H), 2.18 (tt, J = 11.6, 5.8 Hz, 1H), 0.92 - 0.69 (m, 6H).

### Preparation Example 71 Preparation of compound B-2-44

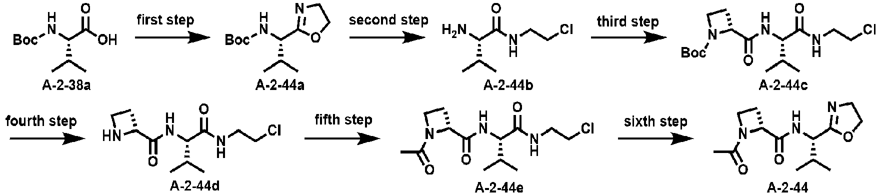

### First step

Intermediate A-2-38a (3.00 g, 13.81 mmol) was dissolved in methanol, and sequentially added with 2-bromoethylamine hydrobromide (2.83 g, 13.81 mmol), 4-(4,6-dimethoxy-1,3,5-triazin-2-yl)-4-methylmorpholinium chloride (3.82 g, 13.81 mmol) and 4-methylmorpholine (1.40 g, 13.81 mmol). After the addition was completed, the mixture was stirred at room temperature to react for 1 hour, added with a solution of sodium hydroxide in methanol (2.71 g, 48.24 mmol, dissolved in 125 mL of methanol), refluxed to react for 2.5 hours, and then concentrated. The residue was added with water (100 mL), and extracted with dichloromethane (100 mL × 3). The organic phases were combined, washed with saturated brine (100 mL × 2), dried with anhydrous sodium sulfate, filtered, concentrated, and purified by silica gel column chromatography to obtain intermediate B-2-44a (1.80 g, 7.43 mmol, yield 53.8%). MS (ESI) calculated for [M+H]⁺ was 243.2; found: m/z 243.2.

### Second step

Intermediate B-2-44a (1.80 g, 7.43 mmol) was dissolved in dichloromethane (10 mL). Under ice bath, the mixture was added with 4 M solution of hydrogen chloride in dioxane (20 mL). After the addition was completed, the reaction was stirred for 2 hours, and then the reaction solution was concentrated to obtain a crude product - intermediate B-2-44b, which was used directly in the next step without purification. MS (ESI) calculated for [M+H]⁺ was 179.1; found: m/z 179.1.

### Third step

Intermediate B-2-44b was dissolved in dichloromethane (10 mL), and added with intermediate A-2-7b (1.13 g, 5.60 mmol). The mixture was cooled to -10°C, and then added with N,N-diisopropylethylamine (2.17 g, 16.80 mmol), N-(3-dimethylaminopropyl)-N'-ethylcarbodiimide hydrochloride (1.40 g, 7.28 mmol) and 1-hydroxybenzotriazole (0.98 g, 7.28 mmol). The reaction was continuously stirred for 3 hours. The reaction solution was concentrated, added with water (20 mL), and extracted with ethyl acetate (20 mL×3). The organic phases were combined, washed with saturated brine (20 mL × 2), dried with anhydrous sodium sulfate, filtered, concentrated, and purified by silica gel column chromatography to obtain intermediate B-2-44c (1.80 g, 5.24 mmol, total yield of two reaction steps 70.5%). MS (ESI) calculated for [M+H]⁺ was 362.2; found: m/z 362.4.

### Fourth step

Under ice bath, intermediate B-2-44c (1.20 g, 3.32 mmol) was dissolved in dichloromethane (10 mL), and added with 4 M solution of hydrogen chloride in dioxane (20 mL). After the reaction was continuously stirred for 2 hours, the reaction solution was concentrated to obtain a crude product - intermediate B-2-44d, which was used directly in the next reaction step without purification. MS (ESI) calculated for [M+H]⁺ was 262.1; found: m/z 262.1.

### Fifth step

The crude product - intermediate B-2-44d was dissolved in dichloromethane (10 mL), cooled to -10°C, and added with N,N-diisopropylethylamine (1.48 g, 11.46 mmol). The mixture was slowly added with acetyl chloride (0.36 g, 4.58 mmol). The reaction was continuously stirred for 4 hours, and the reaction solution was added with water (20 mL), and extracted with ethyl acetate (20 mL×3). The organic phases were combined, washed with saturated brine (20 mL× 2), dried with anhydrous sodium sulfate, filtered, concentrated, and purified by silica gel column chromatography to obtain intermediate B-2-44e (0.80 g, 2.63 mmol, total yield of two reaction steps 79.3%). MS (ESI) calculated for [M+H]⁺ was 304.1; found: m/z 304.1.

### Sixth step

Intermediate B-2-44e (0.46 g, 1.5 mmol) was dissolved in methanol (10 mL), added with potassium hydroxide (0.35 g, 6.30 mmol), and heated to reflux, the reaction was stirred for 2.5 hours. After the reaction was completed, the reaction solution was subjected to rotary evaporation to remove the solvent, then added with water (10 mL), and extracted with ethyl acetate (10 mL×3). The organic phases were combined, washed with saturated brine (10 mL × 2), dried with anhydrous sodium sulfate, filtered, concentrated, and purified by silica gel column chromatography to obtain intermediate B-2-44 (0.20 g, 0.75 mmol, yield 49.8%). MS (ESI) calculated for [M+H]⁺ was 268.2; found: m/z 268.2. ¹H NMR (400 MHz, Chloroform-d) δ 8.13 (dd, J = 47.5, 8.8 Hz, 1H), 4.84 (dt, J = 9.6, 6.6 Hz, 1H), 4.58 - 4.46 (m, 1H), 4.27 - 4.13 (m, 2H), 4.04 - 3.91 (m, 2H), 3.84 - 3.69 (m, 2H), 2.73 - 2.48 (m, 1H), 2.43 - 2.23 (m, 1H), 2.09 (qd, J = 6.9, 5.3 Hz, 1H), 1.86 (d, J = 4.0 Hz, 3H), 0.98 - 0.68 (m, 6H).

### Comparative Example 1 Compound D4-pEL

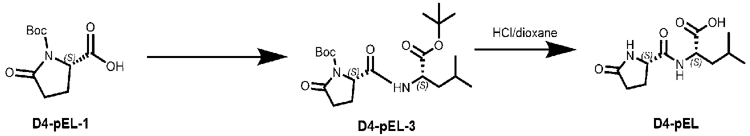

### First step

**D4-pEL-1** (1.00 g, 4.36 mmol), *L*-leucine tert-butyl ester hydrochloride (0.98 g, 5.23 mmol), triethylamine (1.32 g, 13.08 mmol), *N*,*N*-dimethylformamide (20 mL), 1-hydroxybenzotriazole (1.18 g, 8.72 mmol) and 1-(3-dimethylaminopropyl)-3-ethylcarbodiimide hydrochloride (1.25 g, 6.54 mmol) were sequentially added to a 100 mL three-necked flask. After the addition was completed, the reaction was stirred at room temperature for 5 hours. After completion of the reaction (monitored by LCMS and TLC), the reaction solution was diluted by adding saturated brine (100 mL), and extracted with ethyl acetate (100 mL × 3). The organic phases were collected, dried with anhydrous sodium sulfate, filtered, and concentrated under reduced pressure. The residue was purified by normal-phase column chromatography to obtain **D4-pEL-3** (1.3 g, 3.26 mmol, yield 74.8%). ESI-LCMS: m/z 243.2 [M-*^{t}*Bu-Boc+H]⁺.

### Second step

**D4-pEL-3** (1.3 g, 3.26 mmol) and HCl/1,4-dioxane (4.0 M, 20 mL) were added to a 100 mL three-necked flask. The reaction mixture was stirred at room temperature for 2 hours. After completion of the reaction (monitored by LCMS and TLC), the reaction solution was concentrated, and diluted by adding dichloromethane (50 mL). Subsequently, the reaction solution was concentrated under reduced pressure to remove residual HCl/1,4-dioxane. The residue was purified by reverse-phase column chromatography, and **D4-pEL** (200 mg, 0.83 mmol, yield 25.5%)was obtained by freeze-drying. ESI-LCMS: m/z 243.3 [M+H]⁺.

¹H NMR (400 MHz, DMSO-*d₆*) δ 7.90 (s, 1H), 7.69 (d, *J* = 8.0 Hz, 1H), 4.08 - 3.91 (m, 2H), 2.29 - 1.97 (m, 3H), 1.94 - 1.83 (m, 1H), 1.69 - 1.55 (m, 1H), 1.55 - 1.46 (m, 1H), 1.46 - 1.35 (m, 1H), 0.84 (t, *J* = 6.4 Hz, 6H).

### Comparative Example 2 Compound D5-PA

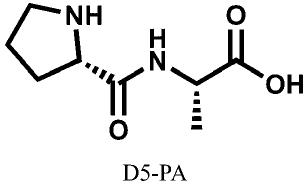

D5-PA was purchased from Shanghai Bide Pharmaceutical Technology Co., Ltd., Cat. No. BD133842, Batch DSY151, purity 97%.

### Experimental Example 1 Plasma stability determination

Mouse plasma (purchased from IPHASE, Batch No. 23B007) was thawed in a 37°C water bath, centrifuged to remove an upper clot, and the pH value was recorded as 7-8. An appropriate volume of stock solution of test compound in acetonitrile (at a concentration of 10 mM) was diluted with acetonitrile to 1 mM, so as to serve as a test drug solution. An appropriate volume of test drug solution was added to a corresponding volume of plasma and mixed uniformly, such that the concentration of the test compound in the incubation system was 5 µM, and the content of the organic solvent acetonitrile was 0.5%. 50 µL of mixed plasma was transferred into a 96-well plate (N=2), incubated at 37°C in a water bath for a designated time, and then added with 300 µL of internal standard-containing methanol solution to terminate the reaction. The reaction mixture was vortexed and centrifuged, and the supernatant was subjected to LC-MS/MS analysis. Propantheline and ZZL-7 were used as positive controls, and processed and tested under the same conditions as the test compound. A curve was plotted with the remaining percentage of the compound against incubation time to obtain a k value, and the half-life of each compound was calculated. The formula is as follows: $in vitro {t}_{\frac{1}{2}} \left(min\right) = - \frac{0.693}{k}$

**Table 1 Plasma stability data of some compounds**

| **Compound** | **T_{1/2} (min)** |
|---|---|
| Propantheline | 25.01 |
| ZZL-7 | 25.29 |
| A-2-20 | 171.2 |
| D5-PA | 52.6 |
| A-2-21 | >289.1 |
| A-2-22 | >289.1 |
| A-2-23 | >289.1 |
| A-2-24 | >289.1 |
| A-2-25 | 194.5 |
| A-2-26 | 229.6 |
| A-2-27 | 284.8 |
| A-2-28 | >289.1 |
| A-2-29 | 226.6 |
| A-2-30 | >289.1 |
| A-2-31 | >289.1 |
| A-2-32 | 139.1 |
| A-2-33 | >289.1 |
| A-2-37 | >289.1 |
| B-2-8 | >289.1 |
| B-2-9 | >289.1 |
| B-2-10 | >289.1 |
| B-2-11 | >289.1 |
| B-2-34 | >289.1 |
| B-2-35 | >289.1 |
| B-2-36 | >289.1 |
| B-2-37 | >289.1 |
| B-2-38 | >289.1 |
| B-2-40 | >289.1 |
| B-2-39 | >289.1 |
| B-2-44 | >289.1 |
| B-2-12 | >289.1 |
| B-2-14 | >289.1 |
| B-2-13 | >289.1 |
| B-2-15 | >289.1 |
| B-2-16 | >289.1 |
| B-2-17 | >289.1 |
| B-2-31 | >289.1 |
| B-2-32 | >289.1 |
| B-2-33 | >289.1 |
| B-2-18 | >289.1 |
| B-2-19 | >289.1 |
| B-2-20 | >289.1 |
| B-2-21 | >289.1 |
| B-2-22 | >289.1 |
| B-2-23 | >289.1 |
| B-2-24 | >289.1 |
| B-2-25 | >289.1 |
| B-2-26 | >289.1 |
| B-2-27 | >289.1 |
| B-2-28 | >289.1 |
| B-2-29 | >289.1 |
| B-2-30 | >289.1 |
| B-2-42 | >289.1 |
| B-2-43 | >289.1 |
| A-2-35 | 276.9 |
| A-2-40 | >289.1 |
| A-2-43 | 198.3 |
| B-2-41 | >289.1 |

| | |
|---|---|
| Note: if the remaining percentage of the compound was still greater than 75% after 120 minutes, the T_{1/2} was recorded as >289.1 minutes. | |

Table 1 showed the plasma stability data of some compounds, and the results indicated that the compounds in the examples of the present invention showed excellent plasma stabilities, which were significantly superior to those of ZZL-7 and D5-PA.

### Experimental example 2 Permeability detection of compounds

MDR1-MDCK II cells (imported from the Netherlands Cancer Institute) were seeded in a 96-well cell plate at a density of 3.33×10⁵ cells/mL, and cultured in a carbon dioxide incubator for 4-7 days to form a cell monolayer. A Hank's balanced salt solution (HBSS, pH 7.40 ± 0.05) containing 10.0 mM 4-(2-hydroxyethyl)-1-piperazineethanesulfonic acid (HEPES) was used as a transport buffer. A DMSO stock solution of compound was diluted to 2 µM (DMSO < 1.0%) with the transport buffer, and added to the basolateral (B) side and apical (A) side respectively (N=2). The bidirectional (A-B and B-A) incubation concentration of Digoxin, a P-gp substrate, was 10 µM, and the unidirectional (A-B) incubateion concentrations of Nadolol, a low-permeability control, and Metoprolol, a high-permeability control, were 2 µM. The cell plate was incubated in a 5% CO₂ cell incubator at 37°C and saturated humidity for 2.5 hours, then final samples from a receiver side and a donor side were collected, added with the transport buffer and a precipitant at an appropriate ratio, and analyzed by LC-MS/MS, and an apparent permeability coefficient, an efflux ratio and a solution recovery were calculated.

After the transport experiment was completed, an integrity of the MDR1-MDCK II cell layer was detected by a lucifer yellow detection experiment. Residual solution in apical and basolateral wells was removed, and 75 µL of transport buffer containing 100 µM lucifer yellow was added to the apical wells and 250 µL of transport buffer was added to the basolateral wells respectively. The cell plate was incubated in a 5% CO₂ cell incubator at 37 °C and saturated humidity for 30 minutes, then 20 µL of sample was taken from the apical side and mixed with 60 µL of transport buffer and 80 µL of sample was taken from the basolateral side. A relative fluorescence intensity was detected by a microplate reader at excitation/emission wavelengths of 425/528 nm, and the permeability of Lucifer Yellow was calculated.

The apparent permeability coefficient (Pₐₚₚ), efflux ratio (ER) and %solution recovery were respectively calculated by following formulas: ${P}_{app} = \frac{{V}_{R}}{Area \times Time} \times \frac{{\left[drug\right]}_{receiver}}{{\left[drug\right]}_{initial , donor}} = \frac{{V}_{R}}{Area \times Time} \times \frac{{C}_{R}}{{C}_{0}}$ $Efflux Ratio = \frac{{P}_{app} \left(B-A\right)}{{P}_{app} \left(A-B\right)}$ $%Solution Recovery = \frac{{C}_{R} \times {V}_{R} + {C}_{D} \times {V}_{D}}{{C}_{0} \times {V}_{D}} \times 100$

*V_{R}* was the volume of solution in the receiver side (0.075 mL for the apical side and 0.25 mL for the basolateral side respectively), *V_{D}* was the volume of solution in the donor side (0.075 mL for the apical side and 0.25 mL for the basolateral side respectively), *C_{R}* was the peak area ratio of compound in the receiver side, *C_{D}* was the peak area ratio of compound in the donor side, *C₀* was the initial peak area ratio of compound in the donor side, Area was the surface area (0.143 cm²) of the cell monolayer, and Time was incubation time of 9000 seconds (150 minutes). Pₐₚₚ(B-A) and Pₐₚₚ(A-B) respectively referred to the apparent permeability coefficient from the basolateral side to the apical side and the apparent permeability coefficient from the apical side to the basolateral side.

The permeability of Lucifer Yellow(%Lucifer Yellow) was calculated by the following formula: $%Lucifer Yellow = \frac{{V}_{Basolateral} \times {RFU}_{Basolateral}}{{V}_{Apical} \times {RFU}_{Apical} + {V}_{Basolateral} \times {RFU}_{Basolateral}} \times 100$

*RFU_{Apical}* and *RFU_{Basolareral}* respectively referred to relative fluorescence intensities of lucifer yellow in the apical side and the basolateral side. *V_{Apical}* and *V_{Basolateral}* respectively referred to loading volumes (0.075 mL and 0.25 mL respectively) in the apical side and the basolateral side.

**Table 2 Permeability data of some compounds**

| **Compound** | **Mean Papp (10⁻⁶ cm/s)** | | **Rank** |
|---|---|---|---|
| | A to B | B to A | Pₐₚₚ |
| B-2-3 | 2.42 | 3.22 | Moderate |
| B-2-41 | 4.07 | 5.61 | Moderate |
| D4-pEL | <0.216 | 0.302 | Low |
| D5-PA | 0.15 | 0.30 | Low |

The compounds of the present patent showed moderate permeabilities, which were superior to those of the control compounds D4-pEL and D5-PA.

### Experimental Example 3 Mouse pharmacokinetic detection

The detection was carried out in 6-8-week-old CD1 mice (purchased from: Speifu (Beijing) Biotechnology Co., Ltd.). A test compound was dissolved in an aqueous solution of 10% hydroxypropyl-β-cyclodextrin containing 10% DMSO, and administered via intravenous injection (10 mg/kg) or oral gavage (10 mg/kg). The concentration of the compound in a plasma sample was analyzed by LC-MS/MS. Pharmacokinetic calculation was carried out by using WinNonlin (Phoenix^{™}, Version 8.3) or other similar software. The following pharmacokinetic parameters were calculated based on plasma concentration and time data: Oral administration: AUCₗₐₛₜ, bioavailability (F). Statistical calculation was carried out on the above parameter data.

**Table 3 Pharmacokinetic data of some compounds administered orally in mice**

| **Compound** | **AUCₗₐₛₜ (h*ng/mL)** | **F (%)** |
|---|---|---|
| ZZL-7 | 138.5 | 7.5 |
| A-1-7 | 154.3 | 27.3 |
| A-1-1 | 628.6 | 35.3 |
| A-2-1 | 3134.9 | 156.4 |
| A-2-2 | 404.8 | 21.5 |
| A-2-4 | 3766.4 | 80.2 |
| A-2-6 | 3343.9 | 148.2 |
| A-2-7 | 3986.9 | 83.9 |
| A-2-8 | 3313.3 | 88.5 |
| A-2-9 | 5254.5 | 84.7 |
| B-2-1 | 1660.8 | 44.9 |
| B-2-2 | 6949.6 | 153.0 |
| B-2-3 | 10916.5 | 159.0 |
| B-2-8 | 2553 | 72.5 |
| B-2-33 | 3361 | 68.3 |
| B-2-41 | 11566 | 90 |
| D4-pEL | 555 | 7.7 |
| D5-PA | 38 | 3.8 |

| | | |
|---|---|---|
| Note: ZZL-7 was administered at a dose of 100 mg/kg via PO. | | |

### Experimental Example 4 Rat pharmacokinetic detection

The detection was carried out in 6-8-week-old male SD rats (purchased from:SPF (Beijing) Biotechnology Co., Ltd.). A test compound was dissolved in a solution containing 10% DMSO, 15% Solutol HS and 75% (aqueous solution of 10% hydroxypropyl-β-cyclodextrin), and administered via intravenous injection (10 mg/kg) or oral gavage (15 mg/kg). A concentration of the compound in a plasma sample was analyzed by LC-MS/MS. Pharmacokinetic calculation was carried out by using WinNonlin (Phoenix^{™}, Version 8.3). The following pharmacokinetic parameters were calculated based on plasma concentration and time data: Oral administration: AUCₗₐₛₜ, bioavailability (F). Statistical calculation was carried out on the above parameter data.

**Table 4 Pharmacokinetic data of some compounds administered orally in rats**

| **Compound** | **AUCₗₐₛₜ (h*ng/mL)** | **F (%)** |
|---|---|---|
| B-2-3 | 24315 | 65.97 |
| D4-pEL | 98.6 | 1.04 |
| D5-PA | 11.5 | 0.71 |

Tables 3 and 4 showed the area under the plasma concentration-time curve (AUC) and the bioavailability (F) data of some compounds administered orally. The results indicated that the compounds in the examples of the present invention had favorable pharmacokinetic properties, which were significantly superior to those of ZZL-7, D4-pEL and D5-PA.

### Experimental Example 5 In-vivo pharmacodynamic detection (1)

6-8-week-old C57/B6 mice (purchased from: Jinan Pengyue Laboratory Animal Breeding Co., Ltd.) were used to establish a 28-day chronic unpredictable mild stress (CUMS) animal model, and an experimental procedure referred to document description^{[1,2]}. After one week of acclimatization, seven experiments, including food and water deprivation, foot shock, wet bedding, cage tilting, restraint stress, shaker stress, and olfactory stimulation, were randomly applied to the animals within one week to achieve unpredictability, and the stimulation was continued for 4 consecutive weeks. The successful establishment of the model was verified by an open field test (OFT)^{[3]}, and then pharmacodynamic evaluation was carried out. The animals were divided into a blank control group (Control), a model group (Model), a vehicle control group (Vehicle), a fluoxetine control group, ZZL-7 experimental group and a compound group in the experiment. The test compound was orally administered once at a dose of 25 mg/kg, a tail suspension test (TST)^{[4,5]} was carried out 2 hours later to evaluate the depression of the mice, and prolonged immobility time in the TST indicated behavioral despair of the mice. After summary statistics of data, SPSS data statistics software was used for analysis, and Graph Pad software was used for graph generation based on SPSS analysis results.

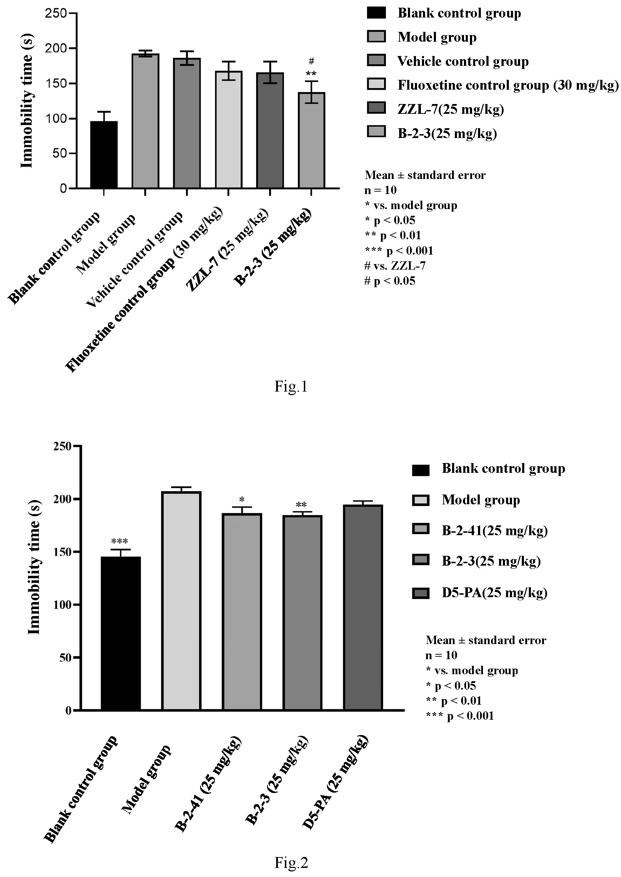

The results of the tail suspension test were shown in FIG. 1 and FIG. 2. The test results indicated that the compounds B-2-3 and B-2-41 could rapidly reverse the prolonged immobility time of the CUMS model mice in the tail suspension test, which proved that the compounds in the examples of the present invention had rapid antidepressant effects.

### Experimental Example 6 In-vivo pharmacodynamic detection (2)

6-8-week-old C57/B6 mice (purchased from: Jinan Pengyue Laboratory Animal Breeding Co., Ltd.) were used to establish a 28-day chronic unpredictable mild stress (CUMS) animal model, and an experimental procedure referred to document description^{[1,2]}. After one week of acclimatization, seven experiments, including food and water deprivation, foot shock, wet bedding, cage tilting, restraint stress, shaker stress, and olfactory stimulation, were randomly applied to the animals within one week to achieve unpredictability, and the stimulation was continued for 4 consecutive weeks. The successful establishment of the model was verified by an open field test (OFT)^{[3]}, and then pharmacodynamic evaluation was carried out. The animals were divided into a blank control group (Control), a model group (Model), a vehicle control group (Vehicle), a fluoxetine control group and a compound group in the experiment. The test compound was intravenously (i.v.) administered once at a dose of 100 mg/kg, a tail suspension test (TST) and a forced swimming test (FST)^{[4,5]} were carried out 2 hours later to evaluate the depression of the mice, and prolonged immobility time in the TST and the FST indicated behavioral despair of the mice. After summary statistics of data, SPSS data statistics software was used for analysis, and Graph Pad software was used for graph generation based on SPSS analysis results.

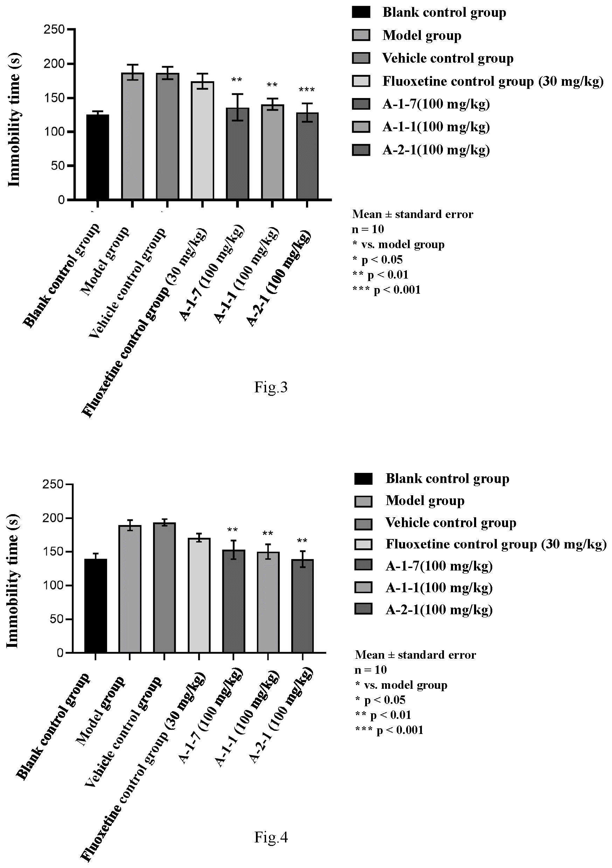

The results of the forced swimming test were shown in FIG. 3, and the results of the tail suspension test were shown in FIG. 4. The test results indicated that the compounds A-1-7, A-1-1 and A-2-1 could rapidly reverse the prolonged immobility time of the CUMS model mice in the forced swimming test and the tail suspension test, which proved that the compounds in the examples of the present invention had rapid antidepressant effects.

### References:

[1] Willner P, Towell A, Sampson D, Sophokleous S, Muscat R. Reduction of sucrose preference by chronic unpredictable mild stress, and its restoration by a tricyclic antidepressant. Psychopharmacology (Berl) 1987; 93: 358-64.
[2] Chen Z, Gu J, Lin S, et al. Saffron essential oil ameliorates CUMS-induced depression-like behavior in mice via the MAPK-CREB1-BDNF signaling pathway[J]. Journal of Ethnopharmacology, 2023, 300: 115719.
[3] Pentkowski, Nathan S., et al. "Anxiety and Alzheimer's disease: Behavioral analysis and neural basis in rodent models of Alzheimer's-related neuropathology." Neuroscience & Biobehavioral Reviews 127 (2021): 647-658.
[4] Can, Adem, et al. "The mouse forced swim test." JoVE (Journal of Visualized Experiments) 59 (2012): e3638.
[5] Can, Adem, et al. "The tail suspension test." JoVE (Journal of Visualized Experiments) 59 (2012): e3769.

## Claims

1. A compound represented by general formula (IA) or (IB), or a pharmaceutically acceptable salt, prodrug, deuterated compound, hydrate, solvate, enantiomer, diastereoisomer or racemate thereof: 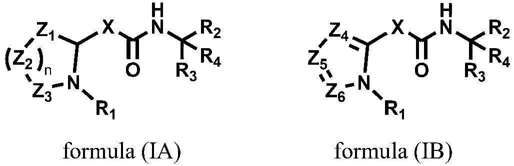 wherein:
X is a bond or CHR₅;
Z₁ and Z₂ are independently selected from CHR₆, NR₇, oxygen or carbonyl, and Z₁ and Z₂ are not both NR₇ or oxygen;
Z₃ is CHR₆ or carbonyl;
Z₄, Z₅ and Z₆ are independently selected from CR₆ or N;
n is an integer from 0 to 4; R₁ is hydrogen, R₆-(CO)-, R₆-(SO)-, R₆-(SO₂)-, R₆-O(CO)-, R₆R₇-N(CO)-, or C₁-C₁₅ linear alkyl, C₁-C₁₅ hetero linear alkyl, C₃-C₁₅ cycloalkyl, C₄-C₁₅ heterocycloalkyl, C₅-C₁₅ aryl or C₅-C₁₅ heteroaryl substituted with 0 to 8 R₆ groups; and R₂ is optionally selected from carboxyl, C₁-C₁₅ linear alkoxycarbonyl, C₁-C₁₅ hetero linear alkoxycarbonyl, C₃-C₁₅ cycloalkoxycarbonyl, C₄-C₁₅ heterocycloalkoxycarbonyl, C₅-C₁₅ aryl or C₅-C₁₅ heteroaryl substituted with 0 to 8 R₆ groups;
R₃ and R₄ are respectively independently selected from hydrogen, or C₁-C₁₅ linear alkyl, C₁-C₁₅ hetero linear alkyl, C₃-C₁₅ cycloalkyl, C₄-C₁₅ heterocycloalkyl, C₅-C₁₅ aryl or C₅-C₁₅ heteroaryl substituted with 0 to 8 R₆ groups;
R₅ is optionally selected from hydrogen, C₁-C₅ alkyl, C₃-C₇ cycloalkyl or C₄-C₇ heterocycloalkyl;
R₆ is optionally selected from hydrogen, halogen, -CN, -NO₂, -OR₇, -NR₇R₈, -SR₇, -COR₇, -SOR₇, -SO₂R₇, -NR₇COR₈, -CONR₇R₈, -OCOR₇, -COOR₇, -OCOOR₇, -OCONR₇R₈, -NR₇CONR₈R₉, -NR₇COOR₈, -NR₇SO₂R₈, -SO₂NR₇R₈, -OSO₂R₇, -SO₃R₇, linear alkyl, hetero linear alkyl, cycloalkyl, heterocycloalkyl, aryl or heteroaryl; wherein each linear alkyl, hetero linear alkyl, cycloalkyl, heterocycloalkyl, aryl or heteroaryl is independently substituted with 0 to 8 R₁₀ groups;
R₇, R₈ and R₉ are respectively independently selected from hydrogen, linear alkyl, hetero linear alkyl, cycloalkyl, heterocycloalkyl, aryl or heteroaryl, wherein each linear alkyl, hetero linear alkyl, cycloalkyl, heterocycloalkyl, aryl or heteroaryl is independently substituted with 0 to 8 halogen, cyano, hydroxyl, sulfhydryl, ether, nitro, alkoxy, amino, carboxyl, sulfo, ester, amide, sulfonate, sulfonamide, alkyl or haloalkyl groups; and
R₁₀ is selected from hydrogen, halogen, cyano, hydroxyl, mercapto, ether, nitro, alkoxy, amino, carboxyl, sulfo, ester, amide, sulfonate, sulfonamide, linear alkyl, hetero linear alkyl, cycloalkyl, heterocycloalkyl, aryl or heteroaryl, wherein each linear alkyl, hetero linear alkyl, cycloalkyl, heterocycloalkyl, aryl or heteroaryl is independently substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups;
wherein, general formula (IA) does not comprise the compound 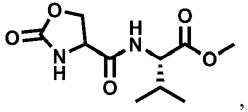 and general formula (IB) does not comprise the compound

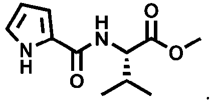

2. A compound represented by general formula (Ia) or (Ib), or a pharmaceutically acceptable salt, prodrug, deuterated compound, hydrate, solvate, enantiomer, diastereoisomer or racemate thereof: 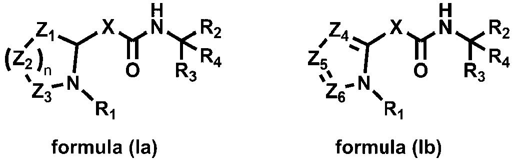 wherein:
X is a bond or CHR₅;
Z₁ and Z₂ are independently selected from CHR₆, NR₇, oxygen or carbonyl, and Z₁ and Z₂ are not both NR₇ or oxygen;
Z₃ is CHR₆ or carbonyl;
Z₄, Z₅ and Z₆ are independently selected from CR₆ or N;
n is an integer from 0 to 4;
R₁ is selected from hydrogen, R₆-(CO)-, R₆-(SO)-, R₆-(SO₂)-, R₆-O(CO)-, R₆R₇N(CO)-, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₆ groups; or R₁ is selected from C₁-C₁₅ linear alkyl containing heteroatoms, wherein the heteroatoms are selected from one or more of O, S and N, and the C₁-C₁₅ linear alkyl containing heteroatoms is optionally substituted with 0 to 8 R₆ groups;
R₂ is selected from -(CO)O-(CH₂)ₘR₆, wherein m is an integer from 0 to 4, or R₂ is selected from C₆-C₁₅ aryl or 5- to 15-membered heteroaryl, wherein C₆-C₁₅ aryl or 5- to 15-membered heteroaryl is optionally substituted with 0 to 8 R₆ groups;
R₃ and R₄ are respectively independently selected from hydrogen, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₆ groups, and R₃ and R₄ are not both hydrogen; or R₃ and R₄ are respectively independently selected from C₁-C₁₅ linear alkyl containing heteroatoms, wherein the heteroatoms are selected from one or more of O, S and N, and the C₁-C₁₅ linear alkyl containing heteroatoms is optionally substituted with 0 to 8 R₆ groups;
R₅ is optionally selected from hydrogen, C₁-C₅ alkyl, C₃-C₇ cycloalkyl or 4- to 7-membered heterocycloalkyl;
R₆ is selected from hydrogen, halogen, -CN, -NO₂, -OR₇, -NR₇R₈, -SR₇, -(CO)-R₇, -(SO)-R₇, -(SO₂)-R₇, -NR₇-(CO)-R₈, -(CO)-NR₇R₈, -O(CO)-R₇, -(CO)OR₇, -O(CO)OR₇, -O(CO)-NR₇R₈, -NR₇-(CO)-NR₈R₉, -NR₇-(CO)OR₈, -NR₇-(SO₂)-R₈, -(SO₂)-NR₇R₈, -O(SO₂)-R₇, -(SO₂)OR₇, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₁₀ groups; or R₆ is selected from C₁-C₁₅ linear alkyl containing heteroatoms, wherein the heteroatoms are selected from one or more of O, S and N, and the C₁-C₁₅ linear alkyl containing heteroatoms is optionally substituted with 0 to 8 R₁₀ groups;
R₇, R₈ and R₉ are respectively independently selected from hydrogen, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups; or R₇, R₈ and R₉ are respectively independently selected from C₁-C₁₅ linear alkyl containing heteroatoms, wherein the heteroatoms are selected from one or more of O, S and N, and the C₁-C₁₅ linear alkyl containing heteroatoms is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups; and
R₁₀ is selected from hydrogen, halogen, cyano, hydroxyl, mercapto, ether, nitro, amino, carboxyl, sulfo, ester, amide, sulfonate, sulfonamide, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3-to 15-membered heterocycloalkyl, C₆-C₁₅ aryl or 5- to 15-membered heteroaryl is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups;
wherein, formula (Ia) does not comprise the following compounds: 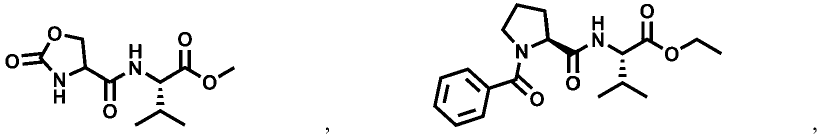 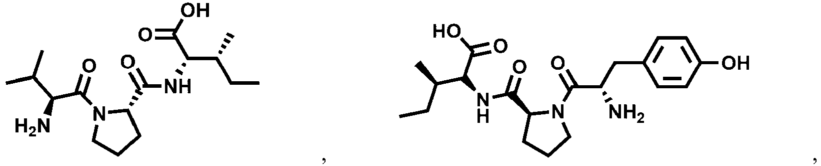 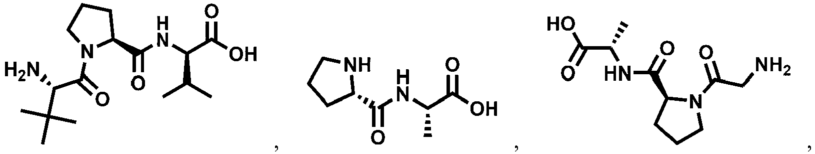 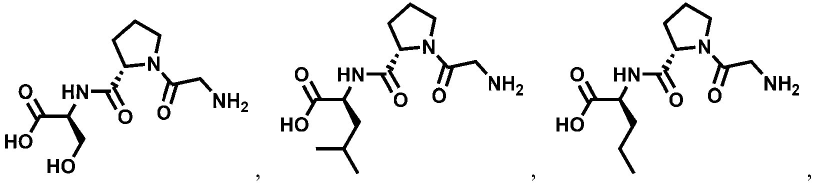 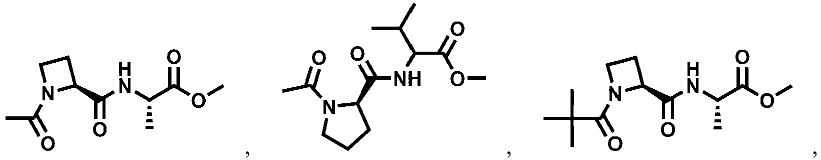 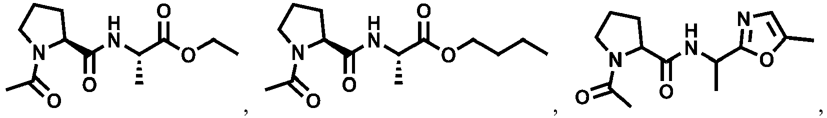 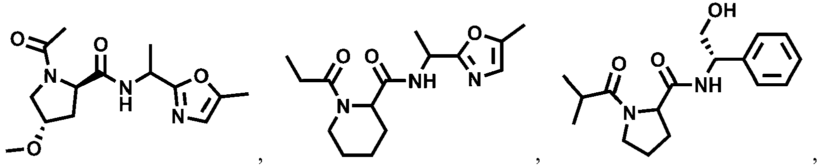 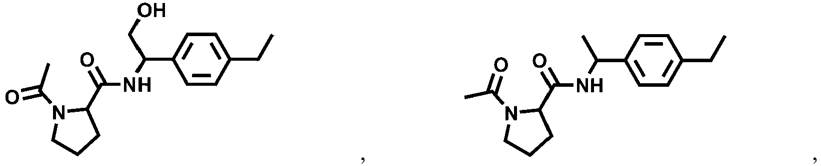 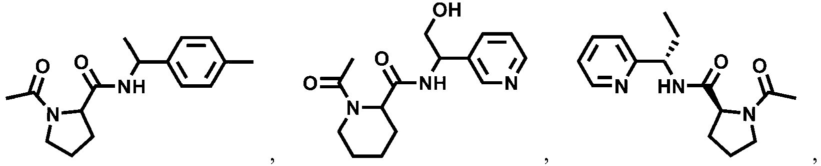 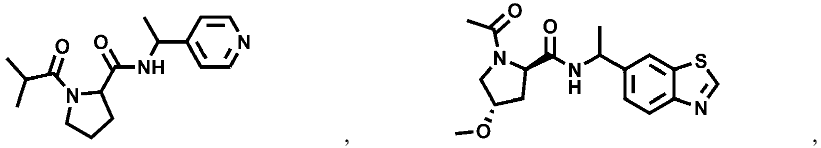 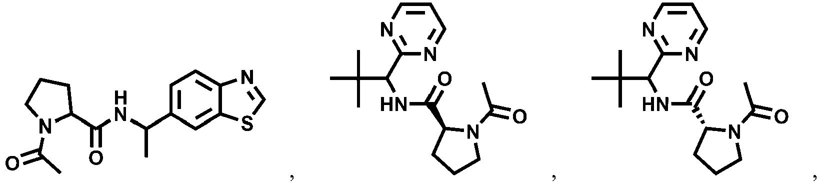 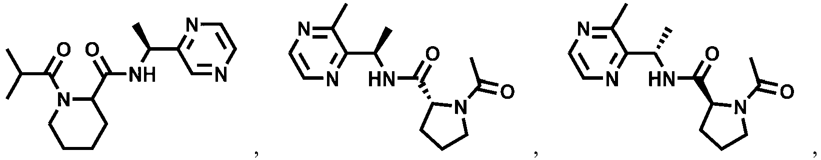 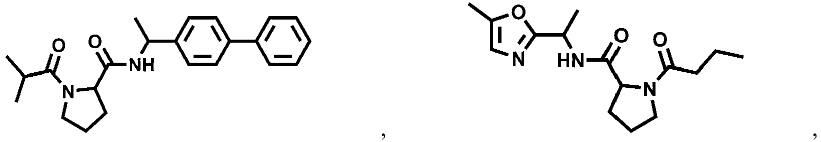 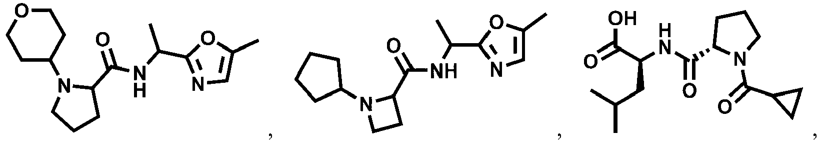 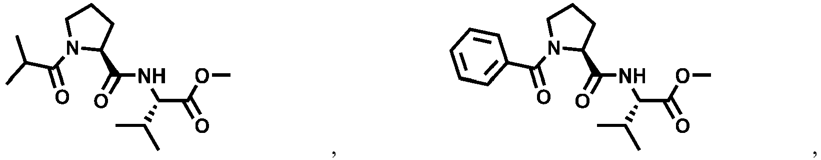 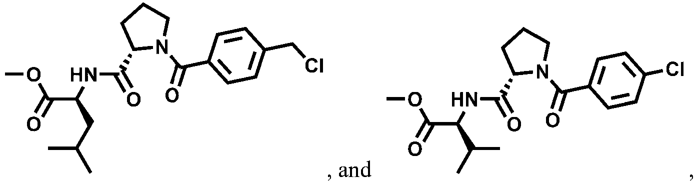
formula (Ib) does not comprise the following compounds:

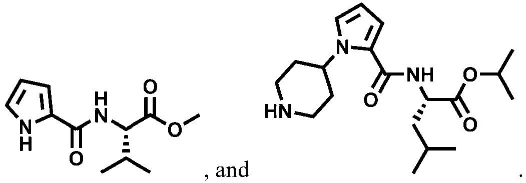

3. The compound represented by formula (Ia) or (Ib), or the pharmaceutically acceptable salt, prodrug, deuterated compound, hydrate, solvate, enantiomer, diastereoisomer or racemate thereof according to claim 2,
wherein:
X is a bond;
Z₁ and Z₂ are independently selected from CHR₆, NR₇, oxygen or carbonyl, and Z₁ and Z₂ are not both NR₇ or oxygen;
Z₃ is CHR₆ or carbonyl;
Z₄, Z₅ and Z₆ are independently selected from CR₆ or N;
n is an integer from 0 to 4;
R₁ is selected from hydrogen, R₆-(CO)-, R₆-(SO)-, R₆-(SO₂)-, R₆-O(CO)-, R₆R₇N(CO)-, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₆ groups;
R₂ is selected from -(CO)O-(CH₂)ₘR₆, wherein m is an integer from 0 to 4, or R₂ is selected from C₆-C₁₅ aryl or 5- to 15-membered heteroaryl, wherein C₆-C₁₅ aryl or 5- to 15-membered heteroaryl is optionally substituted with 0 to 8 R₆ groups;
R₃ and R₄ are respectively independently selected from hydrogen or C₁-C₁₅ alkyl, wherein C₁-C₁₅ alkyl is optionally substituted with 0 to 8 R₆ groups; and R₃ and R₄ are not both hydrogen;
R₆ is selected from hydrogen, halogen, -CN, -NO₂, -OR₇, -NR₇R₈, -SR₇, -(CO)-R₇, -(SO)-R₇, -(SO₂)-R₇, -NR₇-(CO)-R₈, -(CO)-NR₇R₈, -O(CO)-R₇, -(CO)OR₇, -O(CO)OR₇, -O(CO)-NR₇R₈, -NR₇-(CO)-NR₈R₉, -NR₇-(CO)OR₈, -NR₇-(SO₂)-R₈, -(SO₂)-NR₇R₃, -O(SO₂)-R₇, -(SO₂)OR₇, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₁₀ groups;
R₇, R₈ and R₉ are respectively independently selected from hydrogen, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups; and
R₁₀ is selected from hydrogen, halogen, cyano, hydroxyl, mercapto, ether, nitro, amino, carboxyl, sulfo, ester, amide, sulfonate, sulfonamide, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3-to 15-membered heterocycloalkyl, C₆-C₁₅ aryl or 5- to 15-membered heteroaryl is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups.

4. The compound represented by formula (Ia) or (Ib), or the pharmaceutically acceptable salt, prodrug, deuterated compound, hydrate, solvate, enantiomer, diastereoisomer or racemate thereof according to claim 3,
wherein, the compound represented by formula (Ia) is a compound represented by the following formula (Ia-1), and the compound represented by formula (Ib) is a compound represented by the following formula (Ib-1): 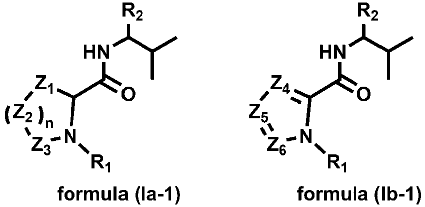
wherein R₁, R₂, Z₁, Z₂, Z₃, Z₄, Z₅, Z₆ and n are as defined in claim 3.

5. The compound represented by general formula (Ia) or (Ib), or the pharmaceutically acceptable salt, prodrug, deuterated compound, hydrate, solvate, enantiomer, diastereoisomer or racemate thereof according to claim 3,
wherein, the compound represented by formula (Ia) is a compound represented by the following formula (Ia-1'), 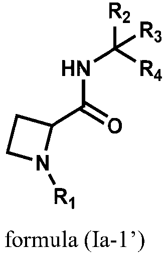
wherein R₁, R₂, R₃ and R₄ are as defined in claim 3.

6. The compound represented by formula (Ia) or (Ib), or the pharmaceutically acceptable salt, prodrug, deuterated compound, hydrate, solvate, enantiomer, diastereoisomer or racemate thereof according to claim 5,
wherein, the compound represented by formula (Ia) is a compound represented by the following formula (Ia-2), 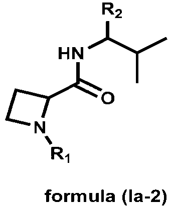
wherein R₁ and R₂ are as defined in claim 5.

7. The compound represented by formula (Ia) or (Ib), or the pharmaceutically acceptable salt, prodrug, deuterated compound, hydrate, solvate, enantiomer, diastereoisomer or racemate thereof according to claim 6,
wherein, the compound represented by formula (Ia) is a compound represented by the following formula (Ia-3), 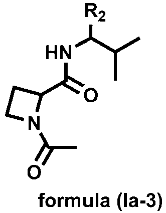
wherein R₂ is as defined in claim 6.

8. The compound represented by formula (Ia) or (Ib), or the pharmaceutically acceptable salt, prodrug, deuterated compound, hydrate, solvate, enantiomer, diastereoisomer or racemate thereof according to any one of claims 3 to 7,
wherein:
R₂ is selected from -(CO)O-(CH₂)ₘR₆, wherein m is an integer from 0 to 4.

9. The compound represented by formula (Ia) or (Ib), or the pharmaceutically acceptable salt, prodrug, deuterated compound, hydrate, solvate, enantiomer, diastereoisomer or racemate thereof according to any one of claims 3 to 7,
wherein, R₂ is selected from C₆-C₁₅ aryl or 5- to 15-membered heteroaryl, wherein C₆-C₁₅ aryl or 5- to 15-membered heteroaryl is optionally substituted with 0 to 8 R₆ groups.

10. The compound represented by formula (Ia) or (Ib), or the pharmaceutically acceptable salt, prodrug, deuterated compound, hydrate, solvate, enantiomer, diastereoisomer or racemate thereof according to claim 9,
wherein, R₂ is selected from:

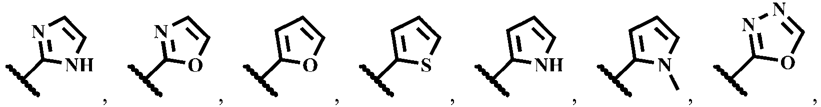

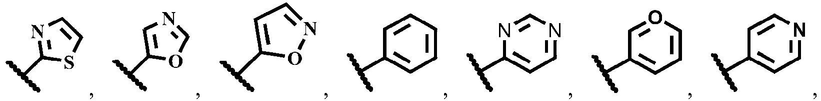

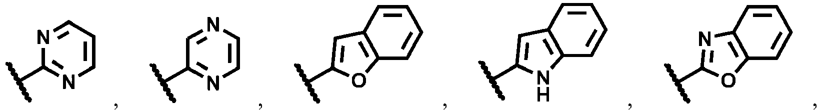

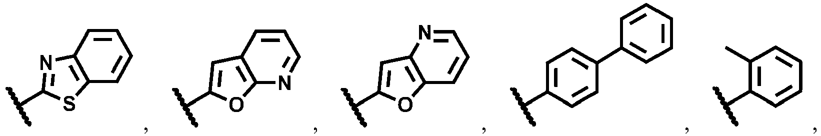

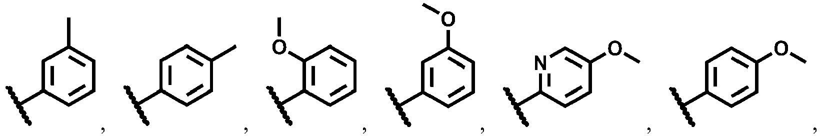

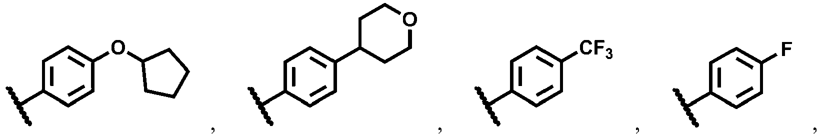

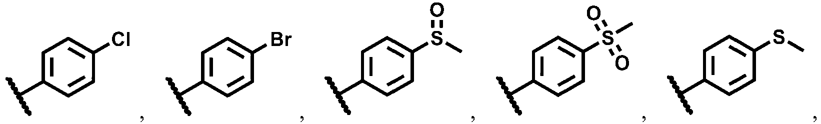

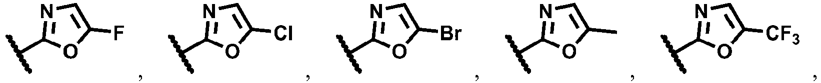

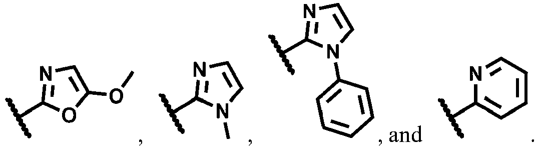

11. The compound represented by general formula (Ia) or (Ib), or the pharmaceutically acceptable salt, prodrug, deuterated compound, hydrate, solvate, enantiomer, diastereoisomer or racemate thereof according to claim 2:
wherein:
X is a bond;
Z₁ and Z₂ are independently selected from CHR₆, NR₇, oxygen or carbonyl, and Z₁ and Z₂ are not both NR₇ or oxygen;
Z₃ is CHR₆ or carbonyl;
Z₄, Z₅ and Z₆ are independently selected from CR₆ or N;
n is an integer from 0 to 4;
R₁ is selected from hydrogen, R₆-(CO)-, R₆-(SO)-, R₆-(SO₂)-, R₆-O(CO)-, R₆R₇N(CO)-, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₆ groups;
R₂ is selected from C₆-C₁₅ aryl or 5- to 15-membered heteroaryl, wherein C₆-C₁₅ aryl or 5- to 15-membered heteroaryl is optionally substituted with 0 to 8 R₆ groups;
R₃ and R₄ are respectively independently selected from hydrogen, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₆ groups; and R₃ and R₄ are not both hydrogen;
R₆ is selected from hydrogen, halogen, -CN, -NO₂, -OR₇, -NR₇R₈, -SR₇, -(CO)-R₇, -(SO)-R₇, -(SO₂)-R₇, -NR₇-(CO)-R₈, -(CO)-NR₇R₈, -O(CO)-R₇, -(CO)OR₇, -O(CO)OR₇, -O(CO)-NR₇R₈, -NR₇-(CO)-NR₈R₉, -NR₇-(CO)OR₈, -NR7-(SO₂)-R₈, -(SO₂)-NR₇R₃, -O(SO₂)-R₇, -(SO₂)OR₇, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 R₁₀ groups;
R₇, R₈ and R₉ are respectively independently selected from hydrogen, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups; and
R₁₀ is selected from hydrogen, halogen, cyano, hydroxyl, mercapto, ether, nitro, amino, carboxyl, sulfo, ester, amide, sulfonate, sulfonamide, C₁-C₁₅ alkyl, C₁-C₁₅ alkoxy, C₃-C₁₅ cycloalkyl, 3- to 15-membered heterocycloalkyl, C₆-C₁₅ aryl or 5- to 15-membered heteroaryl, wherein C₁-C₁₅ alkyl, C₁-C₁₅ alkoxy, C₃-C₁₅ cycloalkyl, 3-to 15-membered heterocycloalkyl, C₆-C₁₅ aryl or 5- to 15-membered heteroaryl is optionally substituted with 0 to 8 halogen, cyano, hydroxyl, mercapto, ether, nitro, alkoxy, amino, carboxyl, sulfo, ester, amide, sulfonate, sulfonamide, alkyl or haloalkyl groups.

12. The compound represented by formula (Ia) or (Ib), or the pharmaceutically acceptable salt, prodrug, deuterated compound, hydrate, solvate, enantiomer, diastereoisomer or racemate thereof according to claim 11,
wherein, the compound represented by formula (Ia) is a compound represented by the following formula (Ia-4), and the compound represented by formula (Ib) is a compound represented by the following formula (Ib-2): 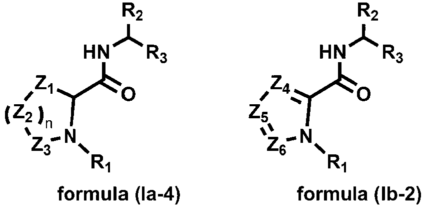
wherein R₁, R₂, R₃, Z₁, Z₂, Z₃, Z₄, Z₅, Z₆ and n are as defined in claim 11.

13. The compound represented by formula (Ia) or (Ib), or the pharmaceutically acceptable salt, prodrug, deuterated compound, hydrate, solvate, enantiomer, diastereoisomer or racemate thereof according to claim 12,
wherein, the compound represented by formula (Ia) is a compound represented by the following formula (Ia-5), 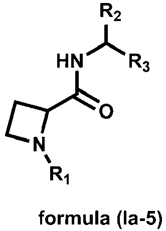
wherein R₁, R₂ and R₃ are as defined in claim 12.

14. The compound represented by general formula (Ia) or (Ib), or the pharmaceutically acceptable salt, prodrug, deuterated compound, hydrate, solvate, enantiomer, diastereoisomer or racemate thereof according to claim 13,
wherein, the compound represented by formula (Ia) is a compound represented by the following formula (Ia-6), 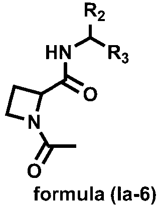
wherein R₂ and R₃ are as defined in claim 13.

15. A compound, or a pharmaceutically acceptable salt, prodrug, deuterated compound, hydrate, solvate, enantiomer, diastereoisomer or racemate thereof, selected from any one of the following compounds:
| | | | |
|---|---|---|---|
| A-1-1 | 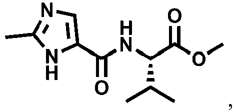 | A-1-2 | 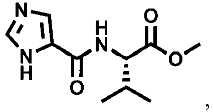 |
| A-1-3 | 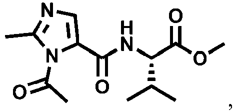 | A-1-4 | 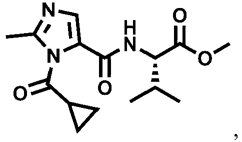 |
| A-1-5 | 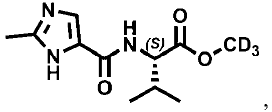 | A-1-6 | 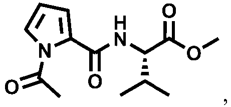 |
| A-2-1 | 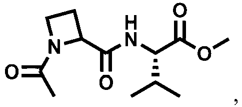 | A-2-2 | 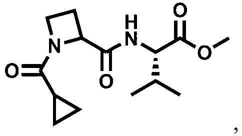 |
| A-2-3 | 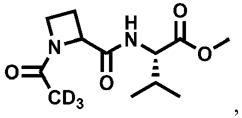 | A-2-4 | 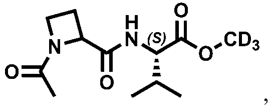 |
| A-2-5 | 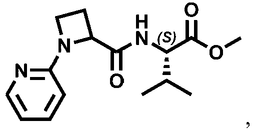 | A-2-6 | 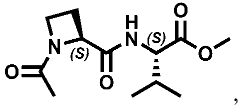 |
| A-2-7 | 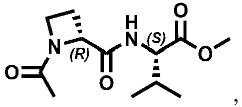 | A-2-8 | 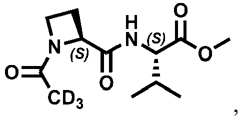 |
| A-2-9 | 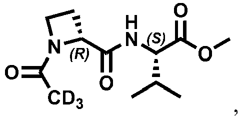 | A-2-10 | 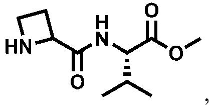 |
| A-2-11 | 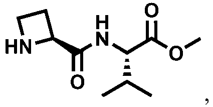 | A-2-12 | 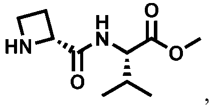 |
| A-2-13 | 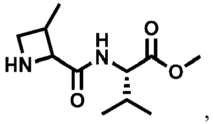 | A-2-14 | 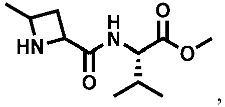 |
| A-2-15 | 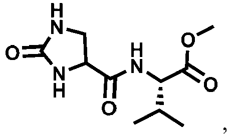 | A-2-16 | 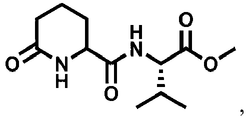 |
| A-2-17 | 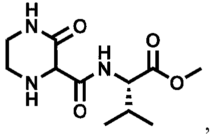 | A-2-18 | 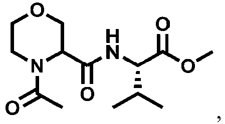 |
| A-2-20 | 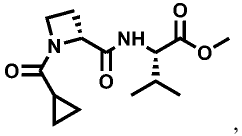 | A-2-21 | 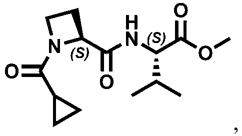 |
| A-2-22 | 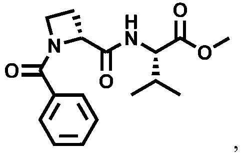 | A-2-23 | 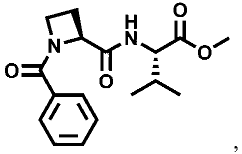 |
| A-2-24 | 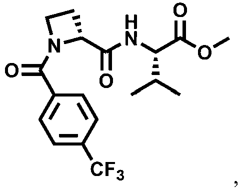 | A-2-25 | 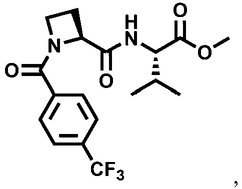 |
| A-2-26 | 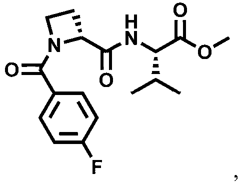 | A-2-27 | 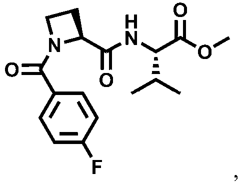 |
| A-2-28 | 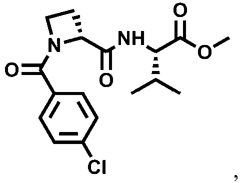 | A-2-29 | 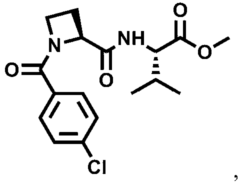 |
| A-2-30 | 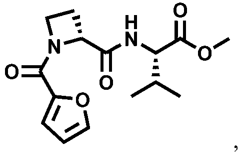 | A-2-31 | 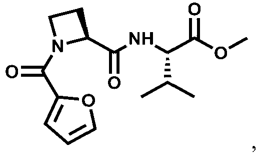 |
| A-2-32 | 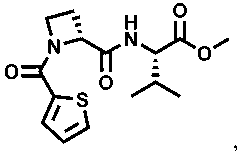 | A-2-33 | 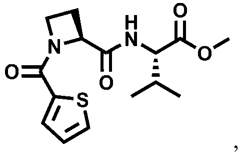 |
| A-2-34 | 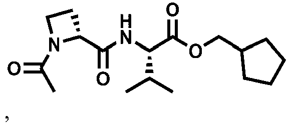 | A-2-35 | 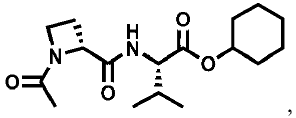 |
| A-2-36 | 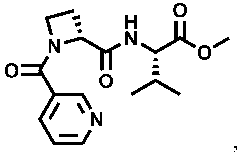 | A-2-37 | 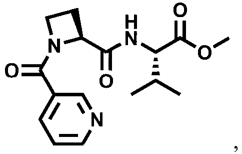 |
| A-2-38 | 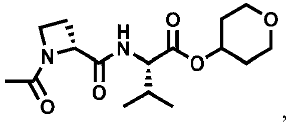 | A-2-39 | 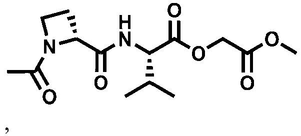 |
| A-2-40 | 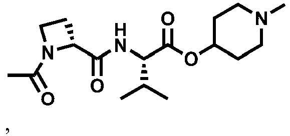 | A-2-41 | 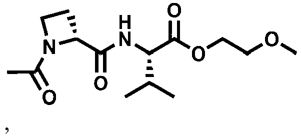 |
| A-2-42 | 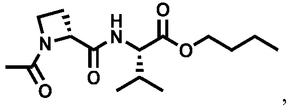 | A-2-43 | 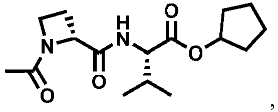 |
| A-2-44 | 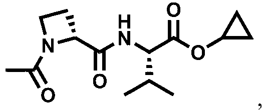 | | |
| B-1-1 | 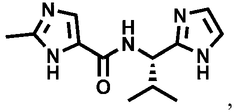 | B-2-1 | 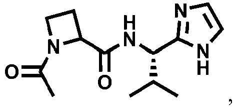 |
| B-2-2 | 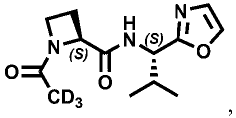 | B-2-3 | 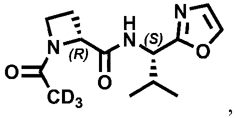 |
| B-2-4 | 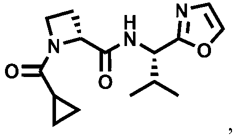 | B-2-5 | 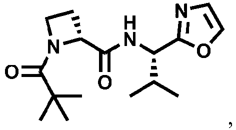 |
| B-2-8 | 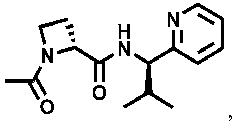 | B-2-9 | 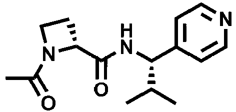 or |
| | | | 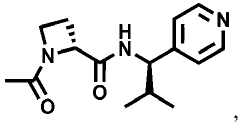 |
| B-2-10 | 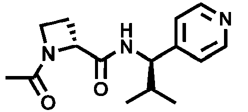 or | B-2-11 | 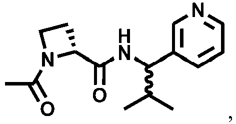 |
| | 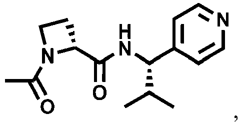 | | |
| B-2-12 | 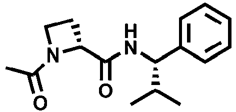 or | B-2-13 | 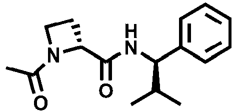 or |
| | 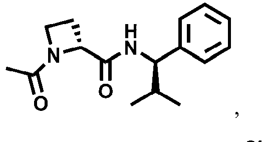 | | 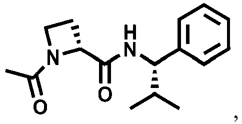 |
| | 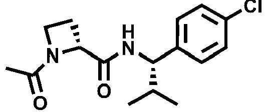 | | 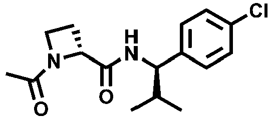 |
| B-2-14 | or | B-2-15 | or |
| | 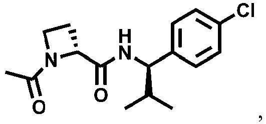 | | 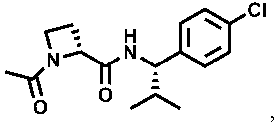 |
| B-2-16 | 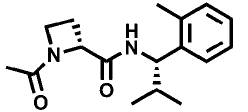 or | B-2-17 | 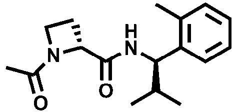 or |
| | 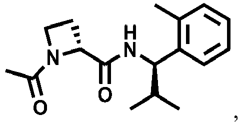 | | 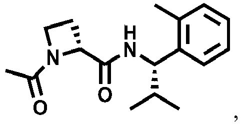 |
| B-2-18 | 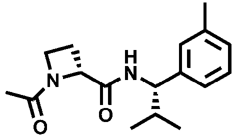 or | B-2-19 | 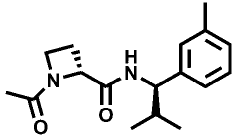 or |
| | 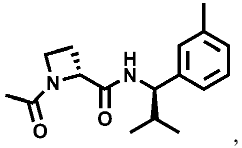 | | 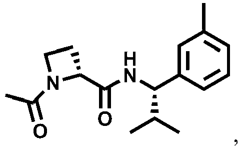 |
| B-2-20 | 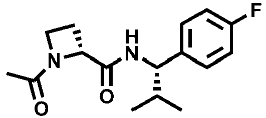 | B-2-21 | 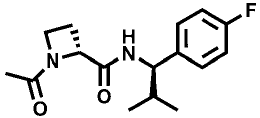 or |
| | or | | |
| | 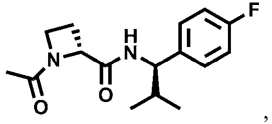 | | 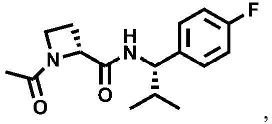 |
| B-2-22 | 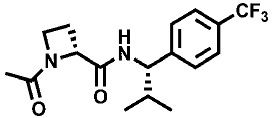 | B-2-23 | 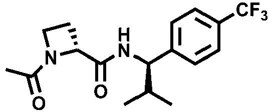 |
| | or | | or |
| | 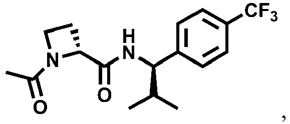 | | 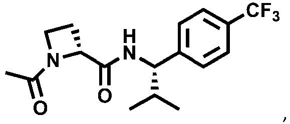 |
| B-2-24 | 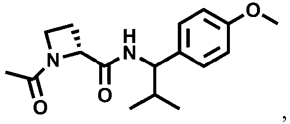 | B-2-25 | 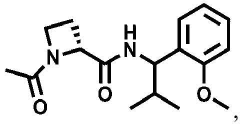 |
| B-2-26 | 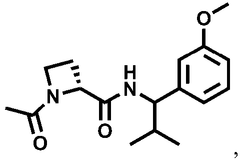 | B-2-27 | 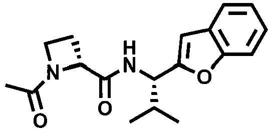 |
| | | | or |
| | | | 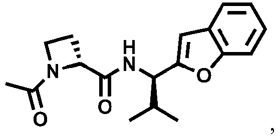 |
| B-2-28 | 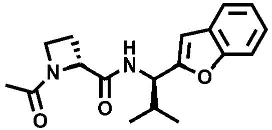 | B-2-29 | 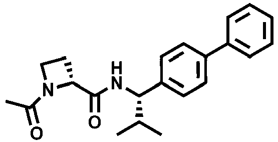 |
| | or | | or |
| | 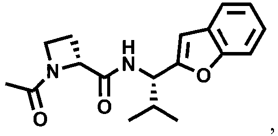 | | 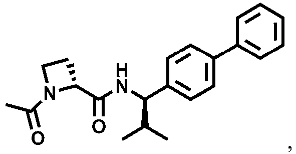 |
| B-2-30 | 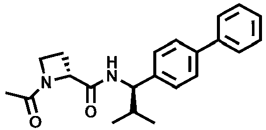 | B-2-31 | 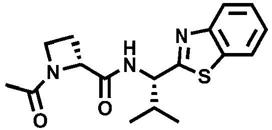 |
| | or | | or |
| | 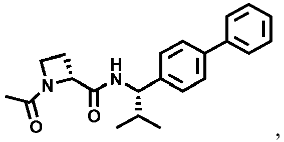 | | 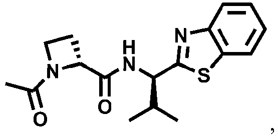 |
| B-2-32 | 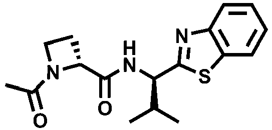 or | B-2-33 | 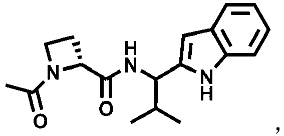 |
| | 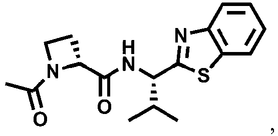 | | |
| B-2-34 | 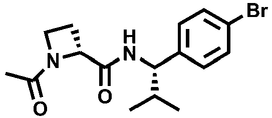 | B-2-35 | 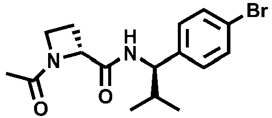 |
| | or | | or |
| | 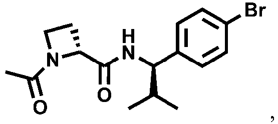 | | 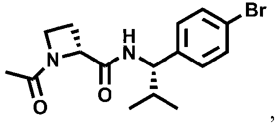 |
| B-2-36 | 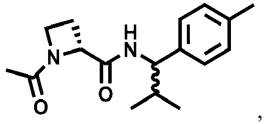 | B-2-37 | 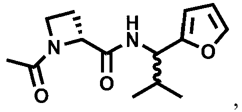 |
| B-2-38 | 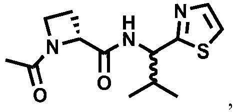 | B-2-39 | 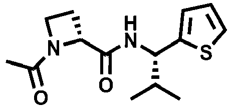 or |
| | | | 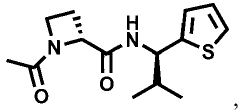 |
| B-2-40 | 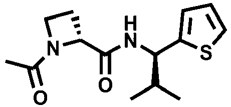 or | B-2-41 | 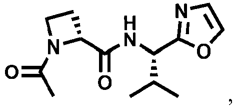 |
| | 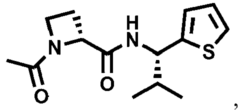 | | |
| B-2-42 | 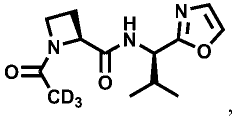 | B-2-43 | 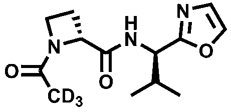 and |
| B-2-44 | 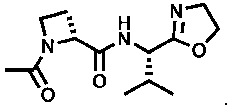 | | |

16. A compound, or a pharmaceutically acceptable salt, prodrug, deuterated compound, hydrate, solvate, enantiomer, diastereoisomer or racemate thereof, selected from any one of the following compounds: 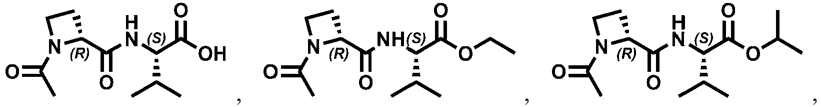 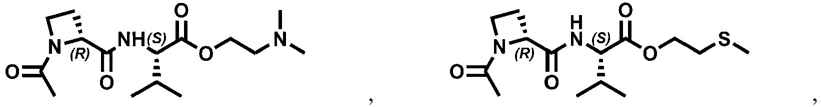 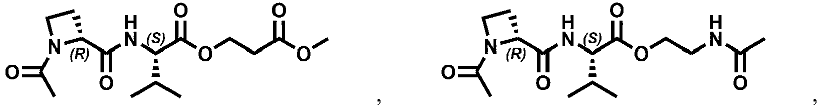 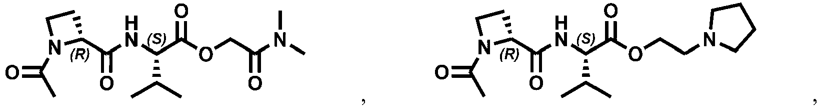 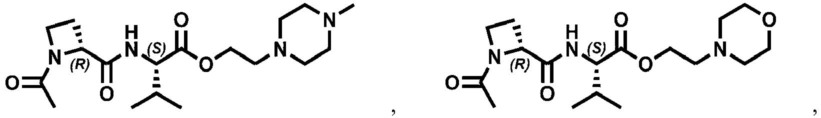 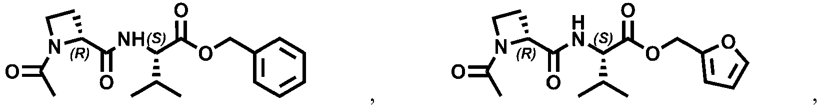 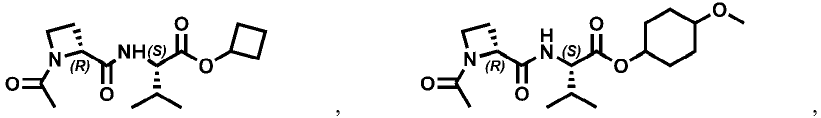 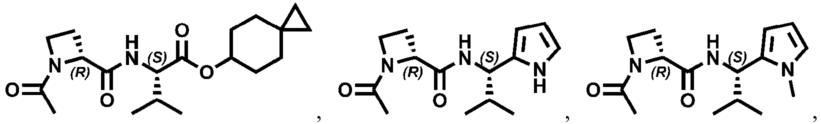 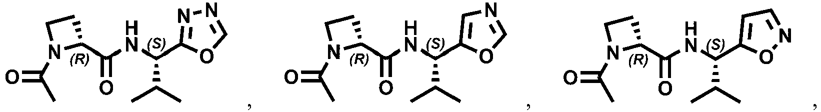 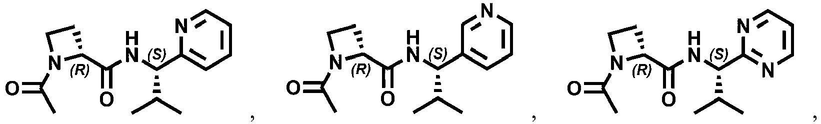 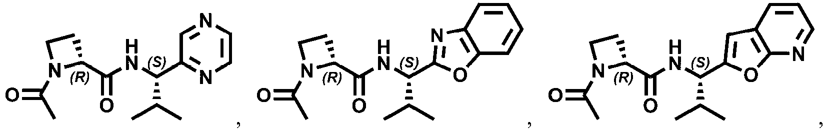 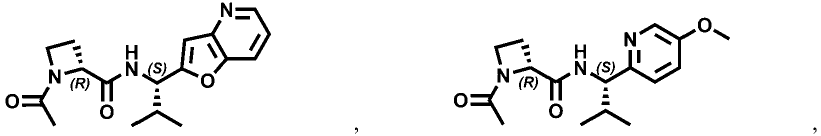 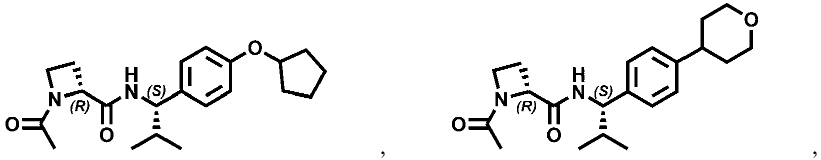 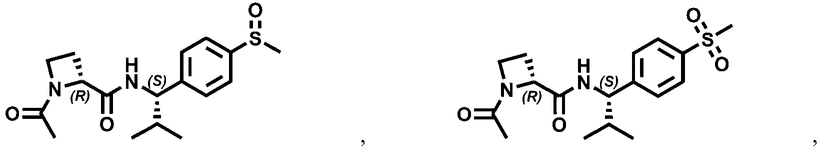 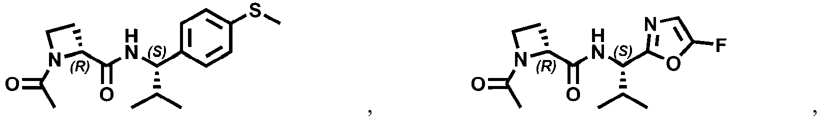 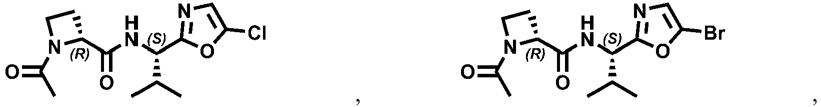 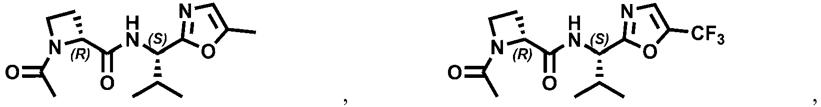 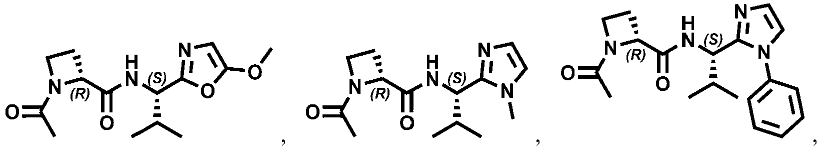 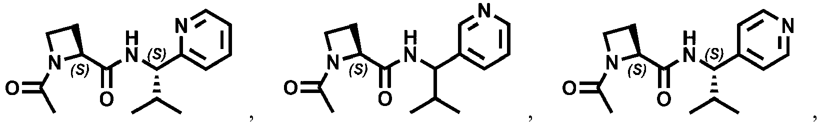 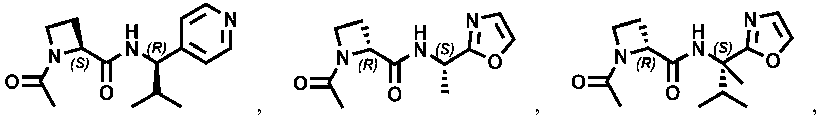 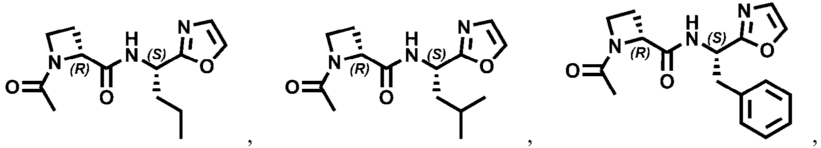 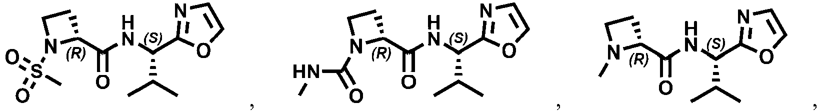 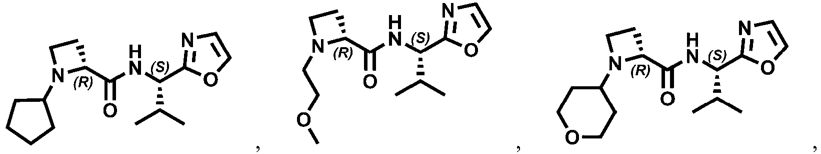 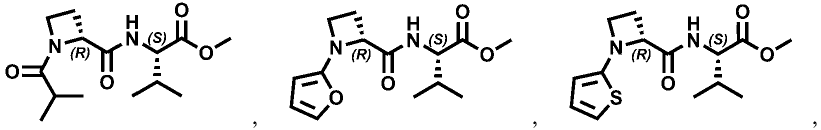 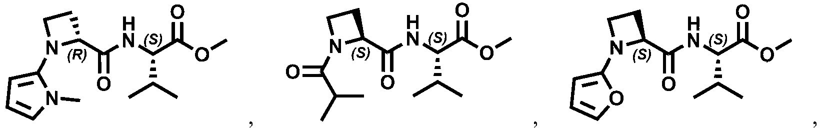 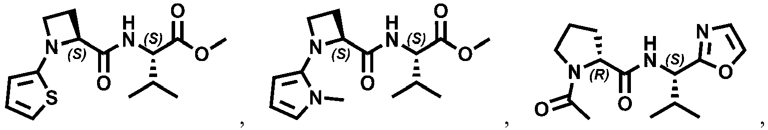 and

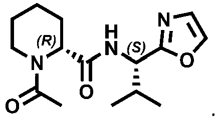

17. A pharmaceutical composition comprising a therapeutically effective amount of the compound, or the pharmaceutically acceptable salt, prodrug, deuterated compound, hydrate, solvate, enantiomer, diastereoisomer or racemate thereof according to any one of claims 1 to 16, and a pharmaceutically acceptable excipient or an auxiliary ingredient.

18. A use of the compound, or the pharmaceutically acceptable salt, prodrug, deuterated compound, hydrate, solvate, enantiomer, diastereoisomer or racemate thereof according to any one of claims 1 to 16, or the pharmaceutical composition according to claim 17 in preparation of a drug for preventing and/or treating depression.

19. The use according to claim 18, wherein the drug is a quick-acting drug.
